# EUROPEAN PATENT APPLICATION

(11) **EP 1 854 487 A2**
(43) Date of publication of application: **14.11.2007**
(21) Application number: 07114939.7
(22) Date of filing: 06.04.2004
(51) Int. Cl.: A61K 45/06, A61P 35/00

(54) **Combinations of an 11-beta-hydroxysteroid dehaydrogenase type 1 inhibitor and a glucocorticoid receptor agonist**

(30) Priority: 11.04.2003 DK 200300569; 11.04.2003 DK 200300566; 11.04.2003 DK 200300565; 11.04.2003 DK 200300571; 11.04.2003 DK 200300570; 11.04.2003 DK 200300568; 02.05.2003 US 467284 P; 02.05.2003 US 467453 P; 02.05.2003 US 467363 P; 02.05.2003 US 467362 P; 02.05.2003 US 467443 P; 02.05.2003 US 467800 P; 22.05.2003 DK 200300778; 22.05.2003 DK 200300776; 02.06.2003 US 475157 P; 02.06.2003 US 475195 P; 27.06.2003 DK 200300972; 30.06.2003 DK 200300990; 30.06.2003 DK 200300989; 30.06.2003 DK 200300988; 02.07.2003 DK 200300998; 10.07.2003 US 486078 P; 10.07.2003 US 486098 P; 10.07.2003 US 486094 P; 10.07.2003 US 486095 P; 10.07.2003 US 486097 P; 22.12.2003 DK 200301910; 06.01.2004 DK 200400009; 16.01.2004 US 537099 P
(62) Divisional of application: 04725890.0
(71) Applicant: NOVO NORDISK A/S, 2880 Bagsvaerd (DK)
(72) Inventor: Kampen, Gita, DK-2850 Nærum (DK); Andersen, Henrik, Sune, 2800 Lyngby (DK)

(57) **Abstract**

Combination therapy comprising the administration of an 11β-hydroxysteroid dehydrogenase type 1 inhibitor and a glucocorticoid receptor agonist for treating some forms of cancer, diseases and disorders having inflammation as a component, and to minimize the side effects associated with glucorticoid receptor agonist therapy.

## Description

### FIELD OF THE INVENTION

The instant invention involves a combination therapy comprising the administration of an 11β-hydroxysteroid dehydrogenase type 1 inhibitor and a glucocorticoid receptor agonist for treating some forms of cancer, diseases and disorders having inflammation as a component, and to minimize the side effects associated with glucorticoid receptor agonist therapy.

### BACKGROUND OF THE INVENTION

Glucocorticoid receptor agonists are widely used as anti-inflammatory and disease-modifying treatment in a broad spectrum of diseases with an inflammatory component. Glucocorticoid receptor agonists are also used as a component of some forms of cancer chemotherapy. However, the use of glucocorticoid receptor agonists is often limited by severe side effects caused by glucocorticoid receptor agonism in organs and tissues that are not targets for treatment. These side effects include osteoporosis, decreased linear growth (children), aseptic bone necrosis, cushingoid fat distribution, mental changes, insulin resistance, hypertension, myopathy, cataract and glaucoma (Harrison's Principles of Internal Medicine, 14th edition, Eds. Fauci et al., McGraw-Hill, New York, USA).

Like the endogenous active glucocorticoids (e.g. cortisol in humans, corticosterone in rodents), glucocorticoid receptor agonists bind to and stimulate ubiquitously expressed intracellular glucocorticoid receptors. Hence, the degree of receptor agonism depends on the intracellular concentration of ligand (reviewed e.g. in Yudt & Cidlowski, Mol Endocrinol; 16, 1719 (2002)).

11 β-hydroxysteroid dehydrogenase type 1 (11β-HSD1) is an intracellular enzyme that physiologically catalyses the conversion of biologically inactive endogenous glucocorticoid (e.g. cortisone in man, 11-dehydrocorticosterone in rodents) to active glucocorticoid (e.g. cortisol in man, corticosterone in rodents). 11β-HSD1 is expressed in several tissues and organs including the liver, adipose tissue, skeletal muscle, bone, pancreas, endothelium, ocular tissue and certain parts of the central nervous system. Thus, 11β-HSD1 serves to increase the local levels of active endogenous glucocorticoid in many of the tissues and organs that are the origin of the side effects (but not the beneficial effects) of treatment with glucocorticoid receptor agonists (Tannin et al., J. Biol. Chem., 266, 16653 (1991); Bujalska et al., Endocrinology, 140, 3188 (1999); Whorwood et al., J. Clin. Endocrinol. Metab., 86, 2296 (2001); Cooper et al., Bone, 27, 375 (2000); Davani et al., J. Biol. Chem., 275, 34841 (2000); Brem et al., Hypertension, 31, 459 (1998); Rauz et al., Invest. Ophthalmol. Vis. Sci., 42, 2037 (2001); Moisan et al., Endocrinology, 127, 1450 (1990)).

During the course of a treatment with a glucocorticoid receptor agonist, the degree of stimulation of the glucocorticoid receptor will reflect the sum of contributions from the active endogenous glucocorticoid generated locally by 11β-HSD1, the active endogenous glucocorticoid derived from plasma and the exogenous glucocoid receptor agonist.

Consequently, inhibition of 11β-HSD1 decreases the total stimulation of the glucocorticoid receptor. Due to the expression pattern of 11β-HSD1, levels of active glucocorticoid are decreased in the tissues and organs that are negatively affected by therapy with glucocorticoid receptor agonists while the desired therapeutic effects remain intact.

The impact of the effect of decreased 11β-HSD1 activity is highlighted by the 11β-HSD1 knockout mouse that has increased plasma levels of endogenous active glucocorticoid, but inspite of this remains protected from insulin resistance, presents with an anti-atherogenic plasma lipid profile and benefits from decreased age-related cognitive impairment (Morton et al., J. Biol. Chem. 276, 41293; Kotelevtsev et al., Proc. Natl. Acad. Sci. USA, 94, 14924 (1997); Yau et al., Proc. Natl. Acad. Sci. USA, 98, 4716 (2001). In addition, a clinical case report demonstrates that a partial defect in/reduced activity of 11β-HSD1 abolishes the obesity and hypertension normally associated with Cushing's disease, i.e. increased pituitary ACTH secretion resulting in increased synthesis of cortisol in the adrenal glands (Tomlinson et al., J. Clin. Endocrinol. Metab., 87, 57 (2002).

The instant invention addresses the clinical problems related to side effects of treatment with glucocorticoid receptor agonists by providing a combination therapy comprised of an 11β-HSD1 inhibitor and a glucocorticoid receptor agonist. When administered as a part of a combination therapy, the 11β-HSD1 inhibitor together with the glucocorticoid receptor agonist allow for control of the disease while minimizing the side effects. Due to the drug combination, and hence the decreased severity of side effects, dosage of the glucocorticoid receptor agonist can be optimized to meet the required therapeutic effect, providing improved means of achieving the desired clinical result.

Examples of inhibitors and/or modulators of the human 11β-hydroxysteroid dehydrogenase type 1 enzyme can be found in WO 01/90090, WO 01/90091, WO 01/90092, WO 01/90093, WO 01/90094, WO 02/72084 and WO 02/076435, as well as the following patent applications under common ownership of the present application: PA 2003 00569 filed 11 April 2003 DK, PA 2003 00565 filed 11 April 2003 DK, PA 2003 00571 filed 11 April 2003 DK, PA 2003 00570 filed 11 April 2003 DK, PA 2003 00566 filed 11 April 2003 DK, PA 2003 00972 filed 27 June 2003 DK, PA 2003 00998 filed 02 July 2003 DK, PA 2003 00988 filed 30 June 2003 DK, PA 2003 00989 filed 30 June 2003 DK, PA 2003 00990 filed 30 June 2003 DK, and PA 2003 01910 filed 22 December 2003 DK, the contents of which are hereby incorporated by reference in their entirety.

### SUMMARY OF THE INVENTION

An object of the present invention is to provide a novel combination therapy comprised of a therapeutically effective amount of a glucocorticoid receptor agonist in combination with an 11β-hydroxysteroid dehydrogenase type 1 (11β-HSD1) inhibitor for the reduction of undesirable side effects occurring during glucocorticoid receptor agonist therapy, and for treating some forms of cancer, diseases and disorders having inflammation as a component.

### DEFINITIONS

In the following structural formulas and throughout the present specification, the following terms have the indicated meaning:

The term "halo" includes fluorine, chlorine, bromine, and iodine.

The term "trihalomethyl" includes trifluoromethyl, trichloromethyl, tribromomethyl, and triiodomethyl.

The term "trihalomethoxy" includes trifluorometoxy, trichlorometoxy, tribromometoxy, and triiodometoxy.

The term "alkyl" includes C₁-C₆ straight chain saturated and methylene aliphatic hydrocarbon groups, C₃-C₆ branched saturated hydrocarbon groups having the specified number of carbon atoms. For example, this definition shall include but is not limited to methyl (Me), ethyl (Et), propyl (Pr), butyl (Bu), pentyl, hexyl, isopropyl (i-Pr), isobutyl (i-Bu), *tert*-butyl (t-Bu), sec-butyl (s-Bu), isopentyl, neopentyl, and the like.

The term "alkenyl" includes C₂-C₆ straight chain unsaturated aliphatic hydrocarbon groups and branched C₃-C₆ unsaturated aliphatic hydrocarbon groups having the specified number of carbon atoms. For example, this definition shall include but is not limited to ethenyl, propenyl, butenyl, pentenyl, hexenyl, methylpropenyl, methylbutenyl and the like.

The term "alkynyl" includes C₂-C₆ straight chain unsaturated aliphatic hydrocarbon groups and C₄-C₆ branched unsaturated aliphatic hydrocarbon groups having the specified number of carbon atoms. For example, this definition shall include but is not limited to ethynyl, propynyl, butynyl, pentynyl, hexynyl, methylbutynyl, and the like.

The term "saturated or partially saturated cyclic, bicyclic or tricyclic ring system" represents but are not limit to aziridinyl, pyrrolinyl, pyrrolidinyl, 2-imidazolinyl, imidazolidinyl, 2-pyrazolinyl, morpholinyl, piperidinyl, thiomorpholinyl, piperazinyl, phthalimide, 1,2,3,4-tetrahydro-quinolinyl, 1,2,3,4-tetrahydro-isoquinolinyl, 1,2,3,4-tetrahydro-quinoxalinyl, and indolinyl.

The term "saturated or partially saturated cyclic ring system" represents but are not limited to cyclopropyl, cyclobutyl, cyclopentyl, cyclohexyl, cyclobutenyl, cyclopentenyl, cyclohexenyl, cycloheptenyl, cyclooctenyl, cyclononenyl, cyclodecenyl, tetrahydrofuranyl or tetrahydropyranyl.

The term "saturated or partially saturated aromatic ring system" represents but are not limited to cyclopentyl, cyclohexyl, cyclobutenyl, cyclopentenyl, cyclohexenyl, cycloheptenyl, cyclooctenyl, cyclononenyl, cyclodecenyl, tetrahydrofuranyl, tetrahydropyranyl, phenyl, pyridyl or pyrimidinyl.

The term "cycloalkyl" (e.g. cyclopropyl, cyclobutyl, cyclopentyl, cyclohexyl, cycloheptyl, cyclooctyl, cyclononyl, cyclodecyl, bicyclo[3.2.1]octyl, spiro[4.5]decyl, norpinyl, norbonyl, norcaryl, adamantyl and the like) represents a saturated, mono-, bi-, tri- or spirocarbocyclic group having the specified number of carbon atoms.

The term "cycloalkylalkyl" (e.g. cyclopropylmethyl, cyclobutylethyl, adamantylmethyl and the like) represents a cycloalkyl group as defined above attached through an alkyl group having the indicated number of carbon atoms or substituted alkyl group as defined above.

The term "cycloalkenyl" (e.g. cyclobutenyl, cyclopentenyl, cyclohexenyl, cycloheptenyl, cyclooctenyl, cyclononenyl, cyclodecenyl and the like) represents a partially saturated, mono-, bi-, tri- or spirocarbocyclic group having the specified number of carbon atoms.

The term "hetcycloalkyl" (tetrahydrofuranyl, tetrahydropyranyl, tertahydrothiopyranyl, and the like) represents a saturated mono-, bi-, tri- or spirocarbocyclic group having the specified number of carbon atoms and one or two additional heteroatoms or groups selected from nitrogen, oxygen, sulphur, SO or SO₂.

The term "alkyloxy" (e.g. methoxy, ethoxy, propyloxy, allyloxy, cyclohexyloxy) represents an alkyl group as defined above having the indicated number of carbon atoms attached through an oxygen bridge.

The term "alkyloxyalkyl" (e.g. methyloxymethyl and the like) represents an alkyloxy group as defined above attached through an "alkyl" group.

The term "aryloxyhetaryl" (e.g. 2-phenoxy-pyridyl and the like) represents an aryloxy group as defined below attached through a "hetaryl" group.

The term "aryloxy" (e.g. phenoxy, naphthyloxy and the like) represents an aryl group as defined below attached through an oxygen bridge.

The term "hetaryloxy" (e.g. 2-pyridyloxy and the like) represents a hetaryl group as defined below attached through an oxygen bridge.

The term "arylalkyloxy" (e.g. phenethyloxy, naphthylmethyloxy and the like) represents an arylalkyl group as defined below attached through an oxygen bridge.

The term "hetarylalkyloxy" (e.g. 2-pyridylmethyloxy and the like) represents a hetarylalkyl group as defined below attached through an oxygen bridge.

The term "alkyloxycarbonyl" (e.g. methylformiat, ethylformiat and the like) represents an alkyloxy group as defined above attached through a carbonyl group.

The term "aryloxycarbonyl" (e.g. phenylformiat, 2-thiazolylformiat and the like) represents an aryloxy group as defined above attached through a carbonyl group.

The term "arylalkyloxycarbonyl" (e.g. benzylformiat, phenyletylformiat and the like) represents an "arylalkyloxy" group as defined above attached through a carbonyl group.

The term "arylalkyl" (e.g. benzyl, phenylethyl, 3-phenylpropyl, 1-naphtylmethyl, 2-(1-naphtyl)ethyl and the like) represents an aryl group as defined below attached through an alkyl having the indicated number of carbon atoms or substituted alkyl group as defined above.

The term "hetarylalkyl" (e.g. (2-furyl)methyl, (3-furyl)methyl, (2-thienyl)methyl, (3-thienyl)methyl, (2-pyridyl)methyl, 1-methyl-1-(2-pyrimidyl)ethyl and the like) represents a hetaryl group as defined below attached through an alkyl having the indicated number of carbon atoms or substituted alkyl group as defined above.

The term "alkylcarbonyl" (e.g. octylcarbonyl, pentylcarbonyl, 3-hexenylcarbonyl) represents an alkyl group as defined above having the indicated number of carbon atoms attached through a carbonyl group.

The term "arylcarbonyl" (e.g. benzoyl) represents an aryl group as defined below attached through a carbonyl group.

The term "hetarylcarbonyl" (e.g. 2-thiophenylcarbonyl, 3-methoxy-anthrylcarbonyl, oxazolylcarbonyl and the like) represents a hetaryl group as defined below attached through a carbonyl group.

The term "carbonylalkyl" (e.g. acetyl and the like) represents a carbonyl group attached through alkyl group as defined above having the indicated number of carbon atoms.

The term "alkylcarbonylalkyl" (e.g. propan-2-one, 4,4-dimethyl-pentan-2-one and the like) represents an alkylcarbonyl group as defined above attached through an alkyl group as defined above having the indicated number of carbon atoms.

The term "arylcarbonylalkyl" (e.g. 1-phenyl-propan-1-one, 1-(3-chloro-phenyl)-2-methyl-butan-1-one and the like) represents a arylcarbonyl group as defined above attached through an alkyl group as defined above having the indicated number of carbon atoms.

The term "hetarylcarbonylalkyl" (e.g. 1-pyridin-2-yl-propan-1-one, 1-(1-*H*-imidazol-2-yl)-propan-1-one and the like) represents a hetarylcarbonyl group as defined above attached through an alkyl group as defined above having the indicated number of carbon atoms.

The term "arylalkylcarbonyl" (e.g. phenylpropylcarbonyl, phenylethylcarbonyl and the like) represents an arylalkyl group as defined above having the indicated number of carbon atoms attached through a carbonyl group.

The term "hetarylalkylcarbonyl" (e.g. imidazolylpentylcarbonyl and the like) represents an hetarylalkyl group as defined above wherein the alkyl group is in turn attached through a carbonyl.

The term "alkylcarboxy" (e.g. heptylcarboxy, cyclopropylcarboxy, 3-pentenylcarboxy) represents an alkylcarbonyl group as defined above wherein the carbonyl is in turn attached through an oxygen bridge.

The term "arylcarboxy" (e.g. benzoic acid and the like) represents an arylcarbonyl group as defined above wherein the carbonyl is in turn attached through an oxygen bridge.

The term "alkylcarboxyalkyl" (e.g. heptylcarboxymethyl, propylcarboxy *tert*-butyl, 3-pentylcarboxyethyl) represents an

The term "arylalkylcarboxy" (e.g. benzylcarboxy, phenylpropylcarboxy and the like) represents an arylalkylcarbonyl group as defined above wherein the carbonyl is in turn attached through an oxygen bridge.

The term "arylalkylcarboxyalkyl" (e.g. benzylcarboxymethyl, phenylpropylcarboxypropyl and the like) represents an arylalkylcarboxy group as defined above wherein the carboxy group is in turn attached through an alkyl group as defined above having the indicated number of carbon atoms.

The term "hetarylcarboxy" (e.g. pyridine-2-carboxylic acid and the like) represents a hetarylcarbonyl group as defined above wherein the carbonyl is in turn attached through an oxygen bridge.

The term "hetarylalkylcarboxy" (e.g. (1-*H*-imidazol-2-yl)-acetic acid, 3-pyrimidin-2-yl-propionic acid and the like) represents a hetarylalkyl group as defined above wherein the carbonyl is in turn attached through an oxygen bridge.

The term "alkylthio" (e.g. methylthio, ethylthio and the like) represents an alkyl group as defined above attached through a sulphur bridge.

The term "arylthio" (e.g. benzenthiol, naphthylthiol and the like) represents an aryl group as defined below attached through a sulphur bridge.

The term "hetarylthio" (e.g. pyridine-2-thiol, thiazole-2-thiol and the like) represents an hetaryl group as defined below attached through a sulphur bridge.

The term "arylthioalkyl" (e.g. methylsulfanyl benzene, ethylsulfanyl naphthalene and the like) represents an arylthio group as defined below attached through an alkyl group having the indicated number of carbon atoms.

The term "hetarylthioalkyl" (e.g. 2-methylsulfanyl-pyridine, 1-ethylsulfanyl-isoquinoline and the like) represents a hetarylthio group as defined below attached through an alkyl group having the indicated number of carbon atoms.

The term "hetaryloxyaryl" (e.g. 1-phenoxy-isoquinolyl, 2-phenoxypyridyl and the like) represents a hetaryloxy group as defined above attached through an "aryl" group as defined below.

The term "hetaryloxyhetaryl" (e.g. 1-(2-pyridyloxy-isoquinoline), 2-(imidazol-2-yloxypyridine) and the like) represents a hetaryloxy group as defined above attached through a "hetaryl" group as defined below.

The term "aryloxyalkyl" (e.g. phenoxymethyl, naphthyloxyethyl and the like) represents an aryloxy group as defined above attached through an "alkyl" group having the indicated number of carbon atoms.

The term "aryloxyaryl" (e.g. 1-phenoxy-naphthalene, phenyloxyphenyl and the like) represents an aryloxy group as defined above attached through an "aryl" group as defined below.

The term "arylalkyloxyalkyl" (e.g. ethoxymethyl-benzene, 2-methoxymethyl-naphthalene and the like) represents an arylalkyloxy group as defined above attached through an "alkyl" group having the indicated number of carbon atoms.

The term "hetaryloxyalkyl" (e.g. 2-pyridyloxymethyl, 2-quinolyloxyethyl and the like) represents a hetaryloxy group as defined above attached through an "alkyl" group having the indicated number of carbon atoms.

The term "hetarylalkyloxyalkyl" (e.g. 4-methoxymethyl-pyrimidine, 2-methoxymethyl-quinoline and the like) represents a hetarylalkyloxy group as defined above attached through an "alkyl" group having the indicated number of carbon atoms.

The term "alkylcarbonylamino" (e.g. methylcarbonylamino, cyclopentylcarbonylaminomethyl, methylcarbonylaminophenyl) represents an "alkylcarbonyl" group as defined above wherein the carbonyl is in turn attached through the nitrogen atom of an amino group. The nitrogen atom may itself be substituted with an alkyl or aryl group.

The term "alkylcarbonylaminoalkyl" (e.g.N-propyl-acetamide, N-butyl-propionamide and the like) represents an "alkylcarbonylamino" group attached through an alkyl group as defined above having the indicated number of carbon atoms.

The term "arylalkylcarbonylamino" (e.g. phenylacetamide, 3phenyl-propionamide and the like) represents an "arylalkylcarbonyl" group as defined above attached through an amino group.

The term "arylalkylcarbonylaminoalkyl" (e.g. N-ethyl-phenylacetamide, N-butyl-3-phenyl-propionamide and the like) represents an "arylalkylcarbonylamino" group attached through an alkyl group as defined above having the indicated number of carbon atoms.

The term "arylcarbonylamino" (e.g. benzamide, naphthalene-1-carboxylic acid amide and the like) represents an "arylcarbonyl" group as defined above attached through an amino group.

The term "arylcarbonylaminoalkyl" (e.g. N-propyl-benzamide, N-Butyl-naphthalene-1-carboxylic acid amide and the like) represents an "arylcarbonylamino" group attached through an alkyl group as defined above having the indicated number of carbon atoms.

The term "aryl" includes but is not limited to a carbocyclic aromatic ring system being either monocyclic, bicyclic, or polycyclic, such as phenyl, biphenyl, naphthyl, anthracenyl, phenanthrenyl, fluorenyl, indenyl, pentalenyl, azulenyl, biphenylenyl and the like. Aryl is also intended to include the partially hydrogenated derivatives of the carbocyclic aromatic systems enumerated above. Non-limiting examples of such partially hydrogenated derivatives are 1,2,3,4-tetrahydronaphthyl, 1,4-dihydronaphthyl and the like.

The term "hetaryl" includes but is not limited to pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), oxazolyl (2-oxazolyl, 4-oxazolyl, 5-oxazolyl), isoxazolyl (3-isoxazolyl, 4-isoxazolyl, 5-isoxazolyl), thiazolyl (2-thiazolyl, 4-thiazolyl, 5-thiazolyl), thiophenyl (2-thiophenyl, 3-thiophenyl, 4-thiophenyl, 5-thiophenyl), furanyl (2-furanyl, 3-furanyl, 4-furanyl, 5-furanyl), pyridyl (2-pyridyl, 3-pyridyl, 4-pyridyl, 5-pyridyl), 5-tetrazolyl, pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydro-benzo-[b]furanyl), 6-(2,3-dihydro-benzo-[b]furanyl), 7-(2,3-dihydro-benzo[b]furanyl)), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]thiophenyl (2-(2,3-dihydro-benzo[b]thiophenyl), 3-(2,3-dihydro-benzo[b]thiophenyl), 4-(2,3-dihydro-benzo[b]thiophenyl), 5-(2,3-dihydro-benzo[b]thiophenyl), 6-(2,3-dihydro-benzo[b]thiophenyl), 7-(2,3-dihydro-benzo[b]thiophenyl)), 4,5,6,7-tetrahydro-benzo[b]thiophenyl (2-(4,5,6,7-tetrahydro-benzo[b]thiophenyl), 3-(4,5,6,7-tetrahydro-benzo[b]thiophenyl), 4-(4,5,6,7-tetrahydro-benzo[b]thiophenyl), 5-(4,5,6,7-tetrahydro-benzo[b]thiophenyl), 6-(4,5,6,7-tetrahydro-benzo[b]thiophenyl), 7-(4,5,6,7-tetrahydro-benzo[b]thiophenyl)), 4,5,6,7-tetrahydro-thieno[2,3-c]pyridyl (4-(4,5,6,7-tetrahydro-thieno[2,3-c]pyridyl), 5-4,5,6,7-tetrahydro-thieno[2,3-c]pyridyl), 6-(4,5,6,7-tetrahydro-thieno[2,3-c]pyridyl), 7-(4,5,6,7-tetrahydro-thieno[2,3-c]pyridyl)), indolyl (1-indolyl, 2-indolyl, 3-indolyl, 4-indolyl, 5-indolyl, 6-indolyl, 7-indolyl), isoindolyl (1-isoindolyl, 2-isoindolyl, 3-isoindolyl, 4-isoindolyl, 5-isoindolyl, 6-isoindolyl, 7-isoindolyl), 1,3-dihydro-isoindolyl (1-(1,3-dihydro-isoindolyl), 2-(1,3-dihydro-isoindolyl), 3-(1,3-dihydro-isoindolyl), 4-(1,3-dihydro-isoindolyl), 5-(1,3-dihydro-isoindolyl), 6-(1,3-dihydro-isoindolyl), 7-(1,3-dihydro-isoindolyl)), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benz-oxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl), piperidinyl (2-piperidinyl, 3-piperidinyl, 4-piperidinyl), pyrrolidinyl (1-pyrrolidinyl, 2-pyrrolidinyl, 3-pyrrolidinyl).

With respect to Formula II, the term "NR⁴R⁵carbonylalkyl" (e.g. *N,N*-dimethyl-propionamide, N-isopropyl-N-methyl-propionamide and the like) represents NR⁴R⁵ substituted by a carbonylalkyl group as defined above, wherein R⁴ and R⁵ are as defined for Formula II herein.

With respect to Formula II, the term "alkylR⁶alkyl" (e.g. 2-ethoxymethyl, N-ethyl-N-methy amine, methyl-propyl-amide, ethanesulfonic acid methylamide and the like) represents an alkyl group as defined above, substituted by R⁶, which is substituted by an alkyl group as defined above, wherein R⁶ is as defined for Formula II herein.

With respect to Formula II, the term "arylR⁶alkyl" (e.g. ethoxy-benzene, ethyl-methyl-phenyl-amine, N-ethyl-benzamide, N-isobutyl-benzenesulfonamide and the like) represents an aryl group as defined above, substituted by R⁶, which is substituted by an alkyl group as defined above, wherein R⁶ is as defined for Formula II herein.

With respect to Formula II, the term "arylalkylR⁶alkyl" (e.g. benzyloxymethyl, ethyl-methyl-benzyl-amine, N-ethyl-benzylamide and the like) represents an arylalkyl group as defined above, substituted by R⁶, which is substituted by an alkyl group as defined above, wherein R⁶ is as defined for Formula II herein.

With respect to Formula II, the term "hetarylR⁶alkyl" (e.g. 2-ethoxy-1*H*-imidazol, ethyl-quinolin-2-yl-amine, thiazole-2-carboxylic acid, methyl-propyl-amide, pyridine-3-sulfonic acid isobutyl-amide and the like) represents a hetaryl group as defined above, substituted by R⁶, which is substituted by an alkyl group as defined above, wherein R⁶ is as defined for Formula II herein.

With respect to Formula II, the term "arylcarbonylNR¹⁵" (e.g. *N*-benzyl-*N*-methyl-benzamide and the like) represents an arylcarbonyl group as defined above, substituted by NR¹⁵, wherein R¹⁵ is as defined for Formula II herein.

With respect to Formula II, the term "alkylSOₙ" (e.g. ethylsulfonyl, ethylsulfinyl and the like) represents an alkyl group as defined above, wherein the alkyl group is in turn attached through a sulphur bridge wherein the sulphur is substituted with n oxygen atoms, wherein n is as defined for Formula II herein.

With respect to Formula II, the term "arylSOₘ" (e.g. phenylsulfinyl, naphthyl-2-sulfonyl and the like) represents an aryl group as defined above, wherein the aryl group is in turn attached through a sulphur bridge wherein the sulphur is substituted with m oxygen atoms, wherein m is as defined for Formula II herein.

With respect to Formula II, the term "hetarylSOₘ" (e.g. thiazol-2-sulfinyl, pyridine-2-sulfonyl and the like) represents a hetaryl group as defined above, wherein the hetaryl group is in turn attached through a sulphur bridge wherein the sulphur is substituted with m oxygen atoms, wherein is as defined for Formula II herein.

With respect to Formula II, the term "arylSOₘNR⁸" (e.g. N-methyl-benzenesulfonamide and the like) represents an aryl group as defined above, wherein the aryl group is in turn attached through a SOₘNR⁸ group wherein the sulphur is substituted with m oxygen atoms and the nitrogen atom substituted by R⁸, wherein R⁸ and m are as defined for Formula II herein.

With respect to Formula V, the term "NR⁴R⁵carbonylalkyl" (e.g. *N,N*-dimethyl-propionamide, *N*-isopropyl-*N*-methyl-propionamide and the like) represents NR⁴R⁵ substituted by a carbonylalkyl group as defined above, wherein R⁴ and R⁵ are as defined for Formula V herein.

With respect to Formula V, the term "arylR⁸alkyl" (e.g. ethoxy-benzene, N-ethyl-N-methyl-phenyl-amine, *N*-ethyl-benzamide, *N*-isobutyl-benzenesulfonamide and the like) represents an aryl group as defined above, substituted by R⁸, which is substituted by an alkyl group as defined above, wherein R⁸ is as defined for Formula V herein.

With respect to Formula V, the term "hetarylR⁸alkyl" (e.g. 2-ethoxy-1*H*-imidazol, ethyl-quinolin-2-yl-amine, thiazole-2-carboxylic acid, methyl-propyl-amide, pyridine-3-sulfonic acid isobutyl-amide and the like) represents a hetaryl group as defined above, substituted by R⁸, which is substituted by an alkyl group as defined above, wherein R⁸ is as defined for Formula V herein.

With respect to Formula V, the term "arylcarbonylNR¹⁵" (e.g. *N*-benzyl-*N*-methyl-benzamide and the like) represents an arylcarbonyl group as defined above, substituted by NR¹⁵, wherein R¹⁵ is as defined for Formula V herein.

Certain of the above defined terms may occur more than once in the structural formulae, and upon such occurrence each term shall be defined independently of the other.

The term "optionally substituted" as used herein means that the groups in question are either unsubstituted or substituted with one or more of the substituents specified. When the groups in question are substituted with more than one substituent, the substituents may be the same or different.

The term "treatment" is defined as the management and care of a patient for the purpose of combating or alleviating the disease, condition or disorder, and the term includes the administration of the active compound to prevent the onset of the symptoms or complications, or alleviating the symptoms or complications, or eliminating the disease, condition, or disorder.

The term "pharmaceutically acceptable" is defined as being suitable for administration to humans without adverse events.

The term "prodrug" is defined as a chemically modified form of the active drug, said prodrug being administered to the patient and subsequently being converted to the active drug. Techniques for development of prodrugs are well known in the art.

The term "combination therapy" is defined as the administration of a single pharmaceutical dosage formulation which comprises the 11 β-HSD1 inhibitor and the glucocorticoid receptor agonist, as well as administration of each active agent in its own separate pharmaceutical dosage formulation. Where separate dosage formulations are used, the 11β-HSD1 inhibitor and the glucocorticoid receptor agonist can be administered to the patient at essentially the same time, i.e. concurrently, or at separate staggered times, i.e. sequentially. When given by different dosage formulations, the route of administration may be the same or different for each agent. Any route of administration known or contemplated for the individual agents is acceptable for the practice of the present invention.

The term "therapeutically effective amount" is defined as the amount of drug or pharmaceutical agent that will elicit the biological or medical response of a tissue, a system or a mammal that is sought by the treating individual, i.e. medical doctor or other clinician.

As used herein, the various forms of the term "modulation" are intended to include stimulation (e.g., increasing or upregulating a particular response or activity) and inhibition (e.g., decreasing or downregulating a particular response or activity).

As used herein, the term "agonist" is intended to indicate a substance that activates the receptor(s).

As used herein, the term "glucocorticoid receptor agonist" is intended to indicate a substance that activates glucocorticoid receptor(s) without dependence on prior modification of the substance by 11 β HSD1.

### DETAILED DECSRIPTION OF THE INVENTION

Synthetic schemes for general formula (I) through general formula (V) compounds described herein, as well as examples, are found in the following patent applications under common ownership of the present application: PA 2003 00569 filed 11 April 2003 DK, PA 2003 00565 filed 11 April 2003 DK, PA 2003 00571 filed 11 April 2003 DK, PA 2003 00570 filed 11 April 2003 DK, PA 2003 00566 filed 11 April 2003 DK, PA 2003 00972 filed 27 June 2003 DK, PA 2003 00998 filed 02 July 2003 DK, PA 2003 00988 filed 30 June 2003 DK, PA 2003 00989 filed 30 June 2003 DK, PA 2003 00990 filed 30 June 2003 DK, and PA 2003 01910 filed 22 December 2003 DK, the contents of which are hereby incorporated by reference in their entirety.

In one embodiment of the present invention said substituted pyrazolo[1,5-a]pyrimidines, or a prodrug thereof, as a component of the combination therapy is of the general formula (I) 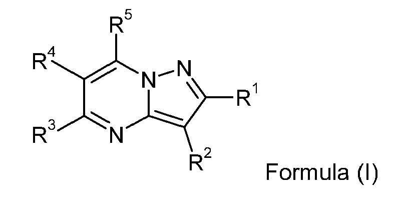 wherein
R¹ and R² independently are hydrogen, halo, C(=O)NR⁶R⁷, CO₂R¹⁵, NR⁶C(=O)R¹¹, OR¹² or SR¹²;
R³ and R⁵ independently are hydrogen, NR¹³R¹⁴, trihalomethyl, trihalomethoxy, C₁-C₆alkyl, C₂-C₆alkenyl, C₂-C₆alkynyl, C₁-C₆alkyloxy, C₁-C₆alkylthio, aryl, arylC₁-C₆alkyl, hetaryl or hetarylC₁-C₆alkyl, wherein alkyl, alkynyl, alkenyl, aryl, hetaryl, arylalkyl or hetarylalkyl groups independently are optionally substituted with one or more of R⁸;
R⁴ is hydrogen, cyano, halo, CO₂R¹⁵, C(=O)NR⁶R⁷, C₁-C₆alkyl, C₂-C₆alkenyl, C₂-C₆alkynyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, or C₁-C₆alkyloxyC₁-C₆alkyl, wherein the alkyl, alkynyl, alkenyl, cycloalkyl, aryl, hetaryl, arylalkyl or hetarylalkyl groups independently are optionally substituted with one or more of R⁹;
R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰; or
R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R⁸ and R⁹ independently are hydrogen, halo, hydroxy, oxo, cyano, nitro, C₃-C₁₀cycloalkyl, C₃-C₁₀hetocycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆-alkylcarboxy, hetarylC₁-C₆alkylcarboxy, C₁-C₆alkylcarbonylamino or arylC₁-C₆alkylcarbonylamino;
R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, trihalomethyl, trihalometh dialkylamino oxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R¹¹ is C₁-C₆alkyl, C₂-C₆alkenyl, C₂-C₆alkynyl, arylC₁-C₆alkyl, hetarylC₁-C₆alkyl, C₁-C₆alkylcarbonylC₁-C₆alkyl, C₁-C₆alkyloxy, aryloxy, C₁-C₆alkyloxy, arylcarbonyl, arylC₁-C₆alkylcarbonyl, hetarylcarbonylC₁-C₆alkyl, wherein the alkyl, alkenyl, alkynyl, aryl and hetaryl groups independently are optionally substituted with one or more of R⁹;
R¹² is C₁-C₆alkylcarbonylaminoC₁-C₆alkyl, arylcarbonylaminoC₁-C₆alkyl or arylC₁-C₆alkylcarbonylaminoC₁-C₆alkyl;
R¹³and R¹⁴ independently are hydrogen, oxo, C₃-C₁₀cycloalkyl, C₁-C₆alkyl, C₁-C₆alkylcarbonyl, arylcarbonyl, hetarylcarbonyl, arylC₁-C₆alkylcarbonyl, hetarylC₁-C₆alkylcarbonyl;
R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl; or
a salt thereof with a pharmaceutically acceptable acid or base, or any optical isomer or mixture of optical isomers, including a racemic mixture, or any tautomeric forms.

In another embodiment of the present invention said substituted pyrazolo[1,5-a]pyrimidines, or a prodrug thereof, as a component of the combination therapy is of the general formula (I) wherein
R¹ and R² independently are hydrogen, halo, C(=O)NR⁶R⁷, NC(=O)R¹¹, OR¹² or SR¹²;
R³ and R⁵ independently are hydrogen, NR¹³R¹⁴, trihalomethyl, trihalomethoxy, C₁-C₆alkyl, C₂-C₆alkenyl, C₂-C₆alkynyl, C₁-C₆alkyloxy, C₁-C₆alkylthio, aryl, arylC₁-C₆alkyl, hetaryl or hetaryl-C₁-C₆alkyl, wherein alkyl, alkynyl, alkenyl, aryl, hetaryl, arylalkyl or hetarylalkyl groups independently are optionally substituted with one or more of R⁸;
R⁴ is hydrogen, cyano, halo, CO₂R¹⁵, C(=O)NR⁶R⁷, C₁-C₆alkyl, C₂-C₆alkenyl, C₂-C₆alkynyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, or C₁-C₆alkyloxyC₁-C₆alkyl, wherein the alkyl, alkynyl, alkenyl, cycloalkyl, aryl, hetaryl, arylalkyl or hetarylalkyl groups independently are optionally substituted with one or more of R⁹;
R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰; or
R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆-alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R⁸ and R⁹ independently are hydrogen, halo, hydroxy, oxo, cyano, nitro, C₃-C₁₀cycloalkyl, C₃-C₁₀hetocycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy, hetarylC₁-C₆alkylcarboxy, C₁-C₆alkylcarbonylamino or arylC₁-C₆alkylcarbonylamino;
R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, trihalomethyl, trihalometh dialkylamino oxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R¹¹ is C₁-C₆alkyl, C₂-C₆alkenyl, C₂-C₆alkynyl, arylC₁-C₆alkyl, hetarylC₁-C₆alkyl, C₁-C₆alkylcarbonylC₁-C₆alkyl, C₁-C₆alkyl, arylcarbonyl, arylC₁-C₆alkylcarbonyl, hetarylcarbonylC₁-C₆alkyl, wherein the alkyl, alkenyl, alkynyl, aryl and hetaryl groups independently are optionally substituted with one or more of R⁹;
R¹² is C₁-C₆alkylcarbonylaminoC₁-C₆alkyl, arylcarbonylaminoC₁-C₆alkyl or arylC₁-C₆alkylcarbonylaminoC₁-C₆alkyl;
R¹³ and R¹⁴ independently are hydrogen, oxo, C₃-C₁₀cycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy;
R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl; or
a salt thereof with a pharmaceutically acceptable acid or base, or any optical isomer or mixture of optical isomers, including a racemic mixture, or any tautomeric forms.

In another embodiment of the present invention said substituted pyrazolo[1,5-a]pyrimidines, or a prodrug thereof, as a component of the combination therapy is of the general formula (I) wherein R¹ and R² independently are hydrogen and/or C(=O)NR⁶R⁷.

In another embodiment of the present invention said substituted pyrazolo[1,5-a]pyrimidines, or a prodrug thereof, as a component of the combination therapy is of the general formula (I) wherein R³ and R⁵ independently are hydrogen, trihalomethyl, C₁-C₆alkyl, C₁-C₆alkyloxy, aryl, arylC₁-C₆alkyl, hetaryl or hetarylC₁-C₆alkyl, wherein alkyl, aryl, hetaryl, arylalkyl or hetarylalkyl groups independently are optionally substituted with one or more of R⁸.

In another embodiment of the present invention said substituted pyrazolo[1,5-a]pyrimidines, or a prodrug thereof, as a component of the combination therapy is of the general formula (I) wherein R⁴ is hydrogen, cyano, halo, C(=O)NR⁶R⁷, C₁-C₆alkyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, wherein the alkyl, aryl, hetaryl, arylalkyl or hetarylalkyl groups independently are optionally substituted with one or more of R⁹.

In another embodiment of the present invention said substituted pyrazolo[1,5-a]pyrimidines, or a prodrug thereof, as a component of the combination therapy is of the general formula (I) wherein R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰.

In another embodiment of the present invention said substituted pyrazolo[1,5-a]pyrimidines, or a prodrug thereof, as a component of the combination therapy is of the general formula (I) R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen or oxygen, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁₀C₆alkylcarboxy or hetarylC₁-C₆alkylcarboxy.

In another embodiment of the present invention said substituted pyrazolo[1,5-a]pyrimidines, or a prodrug thereof, as a component of the combination therapy is of the general formula (I) wherein R⁶ and R⁷ together with the nitrogen to which they are attached, are forming a saturated or partially saturated cyclic, bicyclic or tricyclic ring system containing from 4 to 10 carbon atoms and from 0 to 2 additional nitrogen atoms, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆alkyloxyC₁-C₆alkyl.

In another embodiment of the present invention said substituted pyrazolo[1,5-a]pyrimidines, or a prodrug thereof, as a component of the combination therapy is of the general formula (I) wherein R⁸ and R⁹ independently are hydrogen, halo, hydroxy, oxo, cyanoC₃-C₁₀cycloalkyl, C₃-C₁₀hetocycloalkyl, C₁-C₆alkyl, C₁-C₆alkyloxy, trihalomethyl, arylC₁C₆alkyloxy, hetarylC₁-C₆alkyloxy.

In another embodiment of the present invention said substituted pyrazolo[1,5-a]pyrimidines, or a prodrug thereof, as a component of the combination therapy is of the general formula (I) wherein R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy.

In another embodiment of the present invention said substituted pyrazolo[1,5-a]pyrimidines, or a prodrug thereof, as a component of the combination therapy is of the general formula (I) wherein R¹¹ is C₁-C₆alkyl, arylC₁-C₆alkyl, hetarylC₁-C₆alkyl, C₁-C₆alkyloxy, aryloxy, C₁-C₆alkyloxy, wherein the alkyl, aryl and hetaryl groups independently are optionally substituted with one or more of R⁹.

In another embodiment of the present invention said substituted pyrazolo[1,5-a]pyrimidines, or a prodrug thereof, as a component of the combination therapy is of the general formula (I) wherein R¹² is C₁-C₆alkylcarbonylaminoC₁-C₆alkyl, arylcarbonylaminoC₁-C₆alkyl or arylC₁-C₆alkylcarbonylaminoC₁-C₆alkyl.

In another embodiment of the present invention said substituted pyrazolo[1,5-a]pyrimidines, or a prodrug thereof, as a component of the combination therapy is of the general formula (I) wherein R¹³and R¹⁴ independently are hydrogen, C₃-C₁₀cycloalkyl, C₁-C₆alkyl, C₁-C₆alkylcarbonyl, arylcarbonyl, hetarylcarbonyl, arylC₁-C₆alkylcarbonyl.

In another embodiment of the present invention said substituted pyrazolo[1,5-a]pyrimidines, or a prodrug thereof, as a component of the combination therapy is of the general formula (I) wherein R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl.

In another embodiment of the present invention said substituted pyrazolo[1,5-a]pyrimidines, or a prodrug thereof, as a component of the combination therapy is selected from the group consisting of:
(3-Bromo-5-thiophen-2-yl-7-trifluoromethyl-pyrazolo[1,5-a]pyrimidin-2-yl)-(2,6-dimethyl-piperidin-1-yl-)methanone;
(3-Bromo-5-thiophen-2-yl-7-trifluoromethyl-pyrazolo[1,5-a]pyrimidin-2-yl)-(2-ethyl-piperidin-1-yl-)methanone;
(5-Thiophen-2-yl-7-trifluoromethyl-pyrazolo[1,5-*a*]pyrimidin-2-yl)-(1,3,3-trimethyl-6-aza-bicyclo[3.2.1]oct-6-yl)methanone;
(3-Chloro-5-thiophen-2-yl-7-trifluoromethyl-pyrazolo[1,5-*a*]pyrimidin-2-yl)-(2-methyl-piperidin-1-yl-)methanone;
5-Methyl-7-phenyl-pyrazolo[1,5-*a*]pyrimidine-2-carboxylic acid cyclohexylamide;
Azepan-1-yl-(3-chloro-5-thiophen-2-yl-7-trifluoromethyl-pyrazolo[1,5-*a*]pyrimidin-2-yl)-methanone;
Azepan-1-yl-(3-chloro-5-thiophen-2-yl-7-trifluoromethyl-pyrazolo[1,5-*a*]pyrimidin-3-yl)-methanone;
(2,6-Dimethyl-piperidin-1-yl-)-(3-chloro-5-thiophen-2-yl-7-trifluoromethyl-pyrazolo[1,5-a]pyrimidin-3-yl)methanone;
3-Bromo-5-furan-2-yl-7-trifluoromethyl-pyrazolo[1,5-a]pyrimidin-2-carboxylic acid cyclohexylamide;
(7-Methyl-5-phenyl-pyrazolo[1,5-a]pyrimidin-3-yl)-(4-methyl-piperidin-1-yl)methanone;
5-(-4-Methoxy-phenyl-7-trifluoromethyl-pyrazolo[1,5-a]pyrimidine-3-carboxylic acid benzyl-methyl amide;
[5-(-4-Ethoxy-phenyl-7-methyl-pyrazolo[1,5-a]pyrimidine-2-yl])-piperidin-1-yl-methanone;
Azepan-1-yl-(3-bromo-5-furan-2-yl-7-trifluoromethyl-pyrazolo[1,5-a]pyrimidin-2-yl)methanone;
5-Benzo[1,3]dioxol-5-yl-7-trifluoromethyl-pyrazolo[1,5-a]pyrimidine-3-carboxylic acid cyclohexyl-methyl amide;
(3,4-Dihydro-1*H*-isoquinolin-2-yl)-[5-(-4-Methoxy-phenyl-7-trifluoromethyl-pyrazolo[1,5-*a*]pyrimidin-3-yl]methanone;
Azepan-1-yl-[5-(4-bromo-phenyl)-7-trifluoromethyl-pyrazolo[1,5-a]pyrimidin-3-yl)methanone
Azepan-1-yl-(5,7-diphenyl)-pyrazolo[1,5-a]pyrimidin-3-yl)methanone;
[5-(4-bromo-phenyl)-7-trifluoromethyl-pyrazolo[1,5-a]pyrimidin-3-yl]-(2-methyl-piperidin-1-yl)-methanone;
(5-Furan-2-yl-7-trifluoromethyl-pyrazolo[1,5-a]pyrimidin-3-yl)-(2-methyl-piperidin-1-yl)-methanone;
(2,6-Dimethyl-piperidin-1-yl-)-[7-(4-ethoxy-phenyl)-5-methyl-pyrazolo[1,5-a]pyrimidin-2-yl] methanone;
5-(4-Ethoxy-phenyl)-7-methyl-pyrazolo[1,5-a]pyrimidine-2-carboxylic acid cyclohexyl amide;
Piperidin-1-yl-(5-thiophen-2-yl-7-trifluoromethyl-pyrazolo[1,5-*a*]pyrimidin-3-yl)methanone;
(5-Furan-2-yl-7-trifluoromethyl-pyrazolo[1,5-*a*]pyrimidin-3-yl)-(2-methyl-piperidin-1-yl)-methanone;
(2-Methyl-piperidin-1-yl)-(5-thiophen-2-yl-7-trifluoromethyl-pyrazolo[1,5-*a*]pyrimidin-3-yl)-methanone;
Azepan-1-yl-[5-(4-methoxy-phenyl)-7-trifluoromethyl-pyrazolo[1,5-a]pyrimidin-3-yl]-methanone;
5-Thiophen-2-yl -7-trifluoromethyl-pyrazolo[1,5-a]pyrimidine-3-carboxylic acid benzyl-methyl-amide;
5-p-Tolyl-7-trifluoromethyl-pyrazolo[1,5-a]pyrimidine-3-carboxylic acid (1,3-dimethyl-1*H-*pyrazol-4-ylmethyl)-methyl amide;
5-Methyl-7-phenyl-pyrazolo[1 ,5-a]pyrimidine-2-carboxylic acid cyclohexyl-methyl amide; 7-Methyl-5-phenyl-pyrazolo[1,5-a]pyrimidine-2-carboxylic acid cyclohexyl-methyl amide;
(5-Phenyl-7-trifluoromethyl-pyrazolo[1,5-a]pyrimidin-2-yl)-piperidin-1-yl-methanone;
5-Naphthalen-1-yl-7-trifluoromethyl-pyrazolo[1,5-a]pyrimidine-2-carboxylic acid cyclohexyl-methyl amide;
5-Naphthalen-1-yl-7-trifluoromethyl-pyrazolo[1,5-a]pyrimidine-2-carboxylic acid cyclohexyl amide;
(5-Naphthalen-1-yl-7-trifluoromethyl-pyrazolo[1,5-a]pyrimidin-2-yl)-piperidin-1-yl-methanone
(2-Methyl-piperidin-1-yl)-(5-naphthalen-1-yl-7-trifluoromethyl-pyrazolo[1,5-a]pyrimidin-2-yl)-methanone;
5,7-Diphenyl-pyrazolo[1,5-a]pyrimidine-2-carboxylic acid cyclohexyl amide; 5,7-Diphenyl-pyrazolo[1,5-a]pyrimidine-2-carboxylic acid cyclohexyl-methyl amide;
(5,7-Diphenyl-pyrazolo[1,5-a]pyrimidin-2-yl)-(2-methyl-piperidin-1-yl)methanone;
(5,7-Diphenyl-pyrazolo[1,5-a]pyrimidin-2-yl)-piperidin-1-yl-methanone;
5-Methyl-7-trifluoromethyl-pyrazolo[1,5-a]pyrimidine-2-carboxylic acid cyclohexyl amide;
(5-Methyl-7-trifluoromethyl-pyrazolo[1,5-a]pyrimidin-2-yl)-piperidin-1-yl-methanone;
5-Methyl-7-trifluoromethyl-pyrazolo[1,5-a]pyrimidine-2-carboxylic acid cyclohexyl-methyl-amide;
(2-Methyl-piperidin-1-yl)-(5-methyl-7-trifluoromethyl-pyrazolo[1,5-*a*]pyrimidin-2-yl)-methanone;
5-Phenyl-7-trifluoromethyl-pyrazolo[1,5-*a*]pyrimidine-2-carboxylic acid cyclohexyl amide;
(5-Phenyl-7-trifluoromethyl-pyrazolo[1,5-*a*]pyrimidin-2-yl)-piperidin-1-yl-methanone;
7-Phenyl-5-trifluoromethyl-pyrazolo[1,5-*a*]pyrimidine-2-carboxylic acid cyclohexyl-methyl-amide;
5-Phenyl-7-trifluoromethyl-pyrazolo[1,5-*a*]pyrimidine-3-carboxylic acid cyclohexyl amide; (5-Phenyl-7-trifluoromethyl-pyrazolo[1,5-*a*]pyrimidin-3-yl)-piperidin-1-yl-methanone; or
a salt thereof with a pharmaceutically acceptable acid or base, or any optical isomer or mixture of optical isomers, including a racemic mixture, or any tautomeric forms.

In another embodiment of the present invention said substituted pyrazolo[1,5-a]pyrimidines, or a prodrug thereof, as a component of the combination therapy is selected from the group of
5-Methyl-7-trifluoromethyl-pyrazolo[1,5-a]pyrimidine-2-carboxylic acid methyl ester;
5-Methyl-7-phenyl-pyrazolo[1,5-a]pyrimidine-2-carboxylic acid methyl ester;
7-Methyl-5-phenyl-pyrazolo[1,5-a]pyrimidine-2-carboxylic acid methyl ester;
(5-methyl-7-trifluoromethyl-pyrazolo[1,5-a]pyrimidin-2-yl)- (piperidin-1-yl)methanone;
(5-methyl-7-phenyl-pyrazolo[1,5-a]pyrimidine-2-carboxylic acid cyclohexyl-methyl amide;
7-Methyl-5-phenyl-pyrazolo[1,5-a]pyrimidine-2-carboxylic acid cyclohexyl-methyl-amide;
7-Phenyl-5-trifluoromethyl-pyrazolo[1,5-a]pyrimidine-2-carboxylic acid cyclohexyl-methyl-amide;
(5-Phenyl-7-trifluoromethyl-pyrazolo[1,5-a]pyrimidin-2-yl)-piperidin-1-yl-methanone;
(2-Methyl-piperidin-1-yl)-(5-methyl-7-trifluoromethyl-pyrazolo[1,5-a]pyrimidin-2-yl)-methanone;
5-Methyl-7-trifluoromethyl-pyrazolo[1,5-a]pyrimidine-2-carboxylic acid cyclohexyl-methyl-amide;
(5-Methyl-7-trifluoromethyl-pyrazolo[1,5-a]pyrimidin-2-yl)-piperidin-1-yl-methanone;
(5,7-Diphenyl-pyrazolo[1,5-a]pyrimidin-2-yl)-piperidin-1-yl-methanone;
(5,7-Diphenyl-pyrazolo[1,5-a]pyrimidin-2-yl)-(2-methyl-piperidin-1-yl)-methanone;
5,7-Diphenyl-pyrazolo[1,5-a]pyrimidine-2-carboxylic acid cyclohexyl-methyl-amide;
(2-Methyl-piperidin-1-yl)-(5-naphthalen-1-yl-7-trifluoromethyl-pyrazolo[1,5-a]pyrimidin-2-yl)-methanone;
(5-Naphthalen-1-yl-7-trifluoromethyl-pyrazolo[1,5-a]pyrimidin-2-yl)-piperidin-1-yl-methanone;
5-Naphthalen-1-yl-7-trifluoromethyl-pyrazolo[1,5-a]pyrimidine-2-carboxylic acid cyclohexyl-methyl-amide;
[7-(4-Ethoxy-phenyl)-5-methyl-pyrazolo[1,5-a]pyrimidin-2-yl]-(1,3,3-trimethyl-6-aza-bicyclo[3.2.1]oct-6-yl)-methanone;
(2-Methyl-piperidin-1-yl)-(7-phenyl-5-trifluoromethyl-pyrazolo[1,5-a]pyrimidin-2-yl)-methanone;
(5-Methyl-7-trifluoromethyl-pyrazolo[1,5-a]pyrimidin-2-yl)-(1,3,3-trimethyl-6-aza-bicyclo[3.2.1]oct-6-yl)-methanone;
(5-Phenyl-7-trifluoromethyl-pyrazolo[1,5-a]pyrimidin-2-yl)-(1,3,3-trimethyl-6-aza-bicyclo[3.2.1]oct-6-yl)-methanone;
(6-Bromo-3-chloro-pyrazolo[1,5-a]pyrimidin-2-yl)-morpholin-4-yl-methanone;
(5-Phenyl-7-trifluoromethyl-pyrazolo[1,5-a]pyrimidin-2-yl)-piperidin-1-yl-methanone;
Morpholin-4-yl-(5-phenyl-7-trifluoromethyl-pyrazolo[1,5-a]pyrimidin-2-yl)-methanone; (6-Bromo-pyrazolo[1,5-a]pyrimidin-2-yl)-piperidin-1-yl-methanone;
(6-Bromo-pyrazolo[1,5-a]pyrimidin-2-yl)-morpholin-4-yl-methanone;
(3,6-Dibromo-pyrazolo[1,5-a]pyrimidin-2-yl)-morpholin-4-yl-methanone;
(3-Bromo-5-phenyl-7-trifluoromethyl-pyrazolo[1,5-a]pyrimidin-2-yl)-piperidin-1-yl-methanone;
(3-Bromo-5-phenyl-7-trifluoromethyl-pyrazolo[1,5-a]pyrimidin-2-yl)-morpholin-4-yl-methanone;
(3-Chloro-5-phenyl-7-trifluoromethyl-pyrazolo[1,5-a]pyrimidin-2-yl)-piperidin-1-yl-methanone;
(3-Chloro-5-phenyl-7-trifluoromethyl-pyrazolo[1,5-a]pyrimidin-2-yl)-morpholin-4-yl-methanone;
(3,6-Dibromo-pyrazolo[1,5-a]pyrimidin-2-yl)-piperidin-1-yl-methanone;
[4-(4-Fluoro-phenyl)-piperazin-1-yl]-(5-thiophen-2-yl-7-trifluoromethyl-pyrazolo[1,5-a]pyrimidin-2-yl)-methanone;
5-Thiophen-2-yl-7-trifluoromethyl-pyrazolo[1,5-a]pyrimidine-2-carboxylic acid cyclohexyl-methyl-amide;
(5-Benzo[1,3]dioxol-5-yl-7-trifluoromethyl-pyrazolo[1,5-a]pyrimidin-2-yl)-(4-methyl-piperazin-1-yl)-methanone;
[5-(3,4-Dichloro-phenyl)-7-trifluoromethyl-pyrazolo[1,5-a]pyrimidin-2-yl]-(2-methyl-piperidin-1-yl)-methanone;
3-Chloro-5-(4-methoxy-phenyl)-7-trifluoromethyl-pyrazolo[1,5-a]pyrimidine-2-carboxylic acid methyl-(2-pyridin-2-yl-ethyl)-amide;
[3-Chloro-5-(4-chloro-phenyl)-7-trifluoromethyl-pyrazolo[1,5-a]pyrimidin-2-yl]-morpholin-4-yl-methanone;
(3-Chloro-5-thiophen-2-yl-7-trifluoromethyl-pyrazolo[1,5-a]pyrimidin-2-yl)-(3,4-dihydro-2H-quinolin-1-yl)-methanone;
[5-(3,4-Dichloro-phenyl)-7-trifluoromethyl-pyrazolo[1,5-a]pyrimidin-2-yl]-[4-(2-methoxyphenyl)-piperazin-1-yl]-methanone;
(4-Benzoyl-piperazin-1-yl)-[3-chloro-5-(4-chloro-phenyl)-7-trifluoromethyl-pyrazolo[1,5-a]pyrimidin-2-yl]-methanone;
4-(5-Benzo[1,3]dioxol-5-yl-7-trifluoromethyl-pyrazolo[1,5-a]pyrimidine-2-carbonyl)-piperazine-1-carboxylic acid ethyl ester;
(5-Benzo[1,3]dioxol-5-yl-7-trifluoromethyl-pyrazolo[1,5-a]pyrimidin-2-yl)-(6,7-dimethoxy-1-methyl-3,4-dihydro-1H-isoquinolin-2-yl)-methanone;
[4-(Furan-2-carbonyl)-piperazin-1-yl]-[5-(4-methoxy-phenyl)-7-trifluoromethyl-pyrazolo[1,5-a]pyrimidin-2-yl]-methanone;
(3-Chloro-5-thiophen-2-yl-7-trifluoromethyl-pyrazolo[1,5-a]pyrimidin-2-yl)-[4-(2-methoxyphenyl)-piperazin-1-yl]-methanone;
[5-(4-Methoxy-phenyl)-7-trifluoromethyl-pyrazolo[1,5-a]pyrimidin-2-yl]-piperidin-1-yl-methanone;
(3,4-Dihydro-2*H*-quinolin-1-yl)-(5-phenyl-7-trifluoromethyl-pyrazolo[1,5-a]pyrimidin-2-yl)-methanone;
(3-Bromo-5-thiophen-2-yl-7-trifluoromethyl-pyrazolo[1,5-a]pyrimidin-2-yl)-(2,6-dimethyl-piperidin-1-yl)-methanone;
(3-Bromo-5-thiophen-2-yl-7-trifluoromethyl-pyrazolo[1,5-a]pyrimidin-2-yl)-(2-ethyl-piperidin-1-yl)-methanone;
3-Bromo-5-thiophen-2-yl-7-trifluoromethyl-pyrazolo[1,5-a]pyrimidine-2-carboxylic acid cyclohexyl-methyl-amide;
[5-(4-Bromo-phenyl)-3-chloro-7-trifluoromethyl-pyrazolo[1,5-a]pyrimidin-2-yl]-pyrrolidin-1-yl-methanone;
(3-Chloro-5-furan-2-yl-7-trifluoromethyl-pyrazolo[1,5-a]pyrimidin-2-yl)-[4-(4-fluoro-phenyl)-piperazin-1-yl]-methanone;
(3-Chloro-5-furan-2-yl-7-trifluoromethyl-pyrazolo[1,5-a]pyrimidin-2-yl)-(4-phenyl-piperazin-1-yl)-methanone;
[3-Chloro-5-(4-chloro-phenyl)-7-trifluoromethyl-pyrazolo[1,5-a]pyrimidin-2-yl]-(4-methyl-piperazin-1-yl)-methanone;
Azepan-1-yl-[3-chloro-5-(4-chloro-phenyl)-7-trifluoromethyl-pyrazolo[1,5-a]pyrimidin-2-yl]-methanone;
[3-Chloro-5-(3,4-dimethoxy-phenyl)-7-trifluoromethyl-pyrazolo[1,5-a]pyrimidin-2-yl]-pyrrolidin-1-yl-methanone;
[5-(4-Chloro-phenyl)-7-trifluoromethyl-pyrazolo[1,5-a]pyrimidin-2-yl]-(2-methyl-piperidin-1-yl)-methanone;
(5-Thiophen-2-yl-7-trifluoromethyl-pyrazolo[1,5-a]pyrimidin-2-yl)-(1,3,3-trimethyl-6-aza-bicyclo[3.2.1]oct-6-yl)-methanone;
[4-(2-Chloro-phenyl)-piperazin-1-yl]-(5-thiophen-2-yl-7-trifluoromethyl-pyrazolo[1,5-a]pyrimidin-2-yl)-methanone;
(3,4-Dihydro-1H-isoquinolin-2-yl)-(5-thiophen-2-yl-7-trifluoromethyl-pyrazolo[1,5-a]pyrimidin-2-yl)-methanone;
(4-Methyl-piperazin-1-yl)-[5-(4-nitro-phenyl)-7-trifluoromethyl-pyrazolo[1,5-a]pyrimidin-2-yl]-methanone;
[5-(3,4-Dichloro-phenyl)-7-trifluoromethyl-pyrazolo[1,5-a]pyrimidin-2-yl]-thiomorpholin-4-yl-methanone;
(3-Bromo-5-furan-2-yl-7-trifluoromethyl-pyrazolo[1,5-a]pyrimidin-2-yl)-(3,4-dihydro-2H-quinolin-1-yl)-methanone;
(5-Furan-2-yl-7-trifluoromethyl-pyrazolo[1,5-a]pyrimidin-2-yl)-pyrrolidin-1-yl-methanone;
[4-(Furan-2-carbonyl)-piperazin-1-yl]-[5-(4-methoxy-phenyl)-7-trifluoromethyl-pyrazolo[1,5-a]pyrimidin-2-yl]-methanone;
[5-(4-Nitro-phenyl)-7-trifluoromethyl-pyrazolo[1,5-a]pyrimidin-2-yl]-piperidin-1-yl-methanone;
(5-Furan-2-yl-7-trifluoromethyl-pyrazolo[1,5-a]pyrimidin-2-yl)-pyrrolidin-1-yl-methanone;
(4-Methyl-piperidin-1-yl)-(5-phenyl-7-trifluoromethyl-pyrazolo[1,5-a]pyrimidin-2-yl)-methanone;
(3-Bromo-5-thiophen-2-yl-7-trifluoromethyl-pyrazolo[1,5-a]pyrimidin-2-yl)-[4-(4-fluoro-phenyl)-piperazin-1-yl]-methanone;
[4-(4-Fluoro-phenyl)-piperazin-1-yl]-(5-phenyl-7-trifluoromethyl-pyrazolo[1,5-a]pyrimidin-2-yl)-methanone;
[4-(4-Fluoro-phenyl)-piperazin-1-yl]-(5-thiophen-2-yl-7-trifluoromethyl-pyrazolo[1,5-a]pyrimidin-2-yl)-methanone;
[3-Bromo-5-(4-methoxy-phenyl)-7-trifluoromethyl-pyrazolo[1,5-a]pyrimidin-2-yl]-morpholin-4-yl-methanone;
[4-(4-Fluoro-phenyl)-piperazin-1-yl]-(7-methyl-5-phenyl-pyrazolo[1,5-a]pyrimidin-2-yl)-methanone;
(4-Benzoyl-piperazin-1-yl)-(5-phenyl-7-trifluoromethyl-pyrazolo[1,5-a]pyrimidin-2-yl)-methanone;
(3-Chloro-5-thiophen-2-yl-7-trifluoromethyl-pyrazolo[1,5-a]pyrimidin-2-yl)-(4-phenyl-piperazin-1-yl)-methanone;
(2-Ethyl-piperidin-1-yl)-(5-phenyl-7-trifluoromethyl-pyrazolo[1,5-a]pyrimidin-2-yl)-methanone;
(5,7-Diphenyl-pyrazolo[1,5-a]pyrimidin-2-yl)-(4-methyl-piperazin-1-yl)-methanone;
(4-Benzyl-piperazin-1-yl)-(3-chloro-5-phenyl-7-trifluoromethyl-pyrazolo[1,5-a]pyrimidin-2-yl)-methanone;
(3-Chloro-5-thiophen-2-yl-7-trifluoromethyl-pyrazolo[1,5-a]pyrimidin-2-yl)-(4-methyl-piperazin-1-yl)-methanone;
(4-Phenyl-piperazin-1-yl)-(5-thiophen-2-yl-7-trifluoromethyl-pyrazolo[1,5-a]pyrimidin-2-yl)-methanone;
[5-(4-Bromo-phenyl)-7-trifluoromethyl-pyrazolo[1,5-a]pyrimidin-2-yl]-pyrrolidin-1-yl-methanone;
(5,7-Diphenyl-pyrazolo[1,5-a]pyrimidin-2-yl)-pyrrolidin-1-yl-methanone;
(3-Chloro-5-thiophen-2-yl-7-trifluoromethyl-pyrazolo[1,5-a]pyrimidin-2-yl)-(2-ethyl-piperidin-1-yl)-methanone;
(3-Chloro-5-thiophen-2-yl-7-trifluoromethyl-pyrazolo[1,5-a]pyrimidin-2-yl)-(2-methyl-piperidin-1-yl)-methanone;
5-Methyl-7-phenyl-pyrazolo[1,5-a]pyrimidine-2-carboxylic acid cyclohexyl-methyl-amide;
(3-Chloro-5-thiophen-2-yl-7-trifluoromethyl-pyrazolo[1,5-a]pyrimidin-2-yl)-morpholin-4-yl-methanone;
(3-Chloro-5-thiophen-2-yl-7-trifluoromethyl-pyrazolo[1,5-a]pyrimidin-2-yl)-[4-(4-fluoro-phenyl)-piperazin-1-yl]-methanone;
(3-Bromo-5-thiophen-2-yl-7-trifluoromethyl-pyrazolo[1,5-a]pyrimidin-2-yl)-(4-methyl-piperazin-1-yl)-methanone;
(7-Methyl-5-phenyl-pyrazolo[1,5-a]pyrimidin-2-yl)-piperidin-1-yl-methanone;
Azepan-1-yl-(3-chloro-5-thiophen-2-yl-7-trifluoromethyl-pyrazolo[1,5-a]pyrimidin-2-yl)-methanone;
Azepan-1-yl-[3-bromo-5-(4-bromo-phenyl)-7-trifluoromethyl-pyrazolo[1,5-a]pyrimidin-2-yl]-methanone;
3-Bromo-5-(4-bromo-phenyl)-7-trifluoromethyl-pyrazolo[1,5-a]pyrimidine-2-carboxylic acid cyclohexyl-methyl-amide;
(2,6-Dimethyl-piperidin-1-yl)-[5-(4-methoxy-phenyl)-7-trifluoromethyl-pyrazolo[1,5-a]pyrimidin-2-yl]-methanone;
[5-(4-Bromo-phenyl)-7-trifluoromethyl-pyrazolo[1,5-a]pyrimidin-2-yl]-(4-methyl-piperazin-1-yl)-methanone;
5-Phenyl-7-trifluoromethyl-pyrazolo[1,5-a]pyrimidine-2-carboxylic acid benzyl-methyl-amide;
[5-(4-Ethoxy-phenyl)-7-methyl-pyrazolo[1,5-a]pyrimidin-2-yl]-(4-phenyl-piperazin-1-yl)-methanone;
(3,4-Dihydro-1*H*-isoquinolin-2-yl)-[5-(4-methoxy-phenyl)-7-trifluoromethyl-pyrazolo[1,5-a]pyrimidin-2-yl]-methanone;
Morpholin-4-yl-[5-(4-nitro-phenyl)-7-trifluoromethyl-pyrazolo[1,5-a]pyrimidin-2-yl]-methanone;
(Hexahydro-pyrrolo[1,2-a]pyrazin-2-yl)-(5-phenyl-7-trifluoromethyl-pyrazolo[1,5-a]pyrimidin-2-yl)-methanone;
(Hexahydro-pyrrolo[1,2-a]pyrazin-2-yl)-(5-p-tolyl-7-trifluoromethyl-pyrazolo[1,5-a]pyrimidin-2-yl)-methanone;
(Hexahydro-pyrrolo[1,2-a]pyrazin-2-yl)-(5-thiophen-2-yl-7-trifluoromethyl-pyrazolo[1,5-a]pyrimidin-2-yl)-methanone;
(3-Chloro-5-phenyl-7-trifluoromethyl-pyrazolo[1,5-a]pyrimidin-2-yl)-(hexahydro-pyrrolo[1,2-a]pyrazin-2-yl)-methanone;
[5-(4-Fluoro-phenyl)-7-methyl-pyrazolo[1,5-a]pyrimidin-2-yl]-morpholin-4-yl-methanone;
(5,7-Diphenyl-pyrazolo[1,5-a]pyrimidin-2-yl)-(2-methyl-piperidin-1-yl)-methanone;
5-Phenyl-7-trifluoromethyl-pyrazolo[1,5-a]pyrimidine-2-carboxylic acid (1,3-dimethyl-1*H-*pyrazol-4-ylmethyl)-methyl-amide;
5-Thiophen-2-yl-7-trifluoromethyl-pyrazolo[1,5-a]pyrimidine-2-carboxylic acid (1,5-dimethyl-1*H*-pyrazol-4-ylmethyl)-methyl-amide;
5-Thiophen-2-yl-7-trifluoromethyl-pyrazolo[1,5-a]pyrimidine-2-carboxylic acid methyl-(1,3,5-trimethyl-1*H*-pyrazol-4-ylmethyl)-amide;
3-Chloro-5-(4-methoxy-phenyl)-7-trifluoromethyl-pyrazolo[1,5-a]pyrimidine-2-carboxylic acid (1,3-dimethyl-1H-pyrazol-4-ylmethyl)-methyl-amide;
5-(4-Chloro-phenyl)-7-trifluoromethyl-pyrazolo[1,5-a]pyrimidine-2-carboxylic acid (1,3-dimethyl-1H-pyrazol-4-ylmethyl)-methyl-amide;
5-(3,4-Dimethoxy-phenyl)-7-trifluoromethyl-pyrazolo[1,5-a]pyrimidine-2-carboxylic acid (1,3-dimethyl-1*H*-pyrazol-4-ylmethyl)-methyl-amide;
(2,5-Dimethyl-piperidin-1-yl)-(5-phenyl-7-trifluoromethyl-pyrazolo[1,5-a]pyrimidin-2-yl)-methanone;
5-Furan-2-yl-7-trifluoromethyl-pyrazolo[1,5-a]pyrimidine-2-carboxylic acid methyl-(1,3,5-trimethyl-1*H*-pyrazol-4-ylmethyl)-amide;
5-(4-Chloro-phenyl)-7-trifluoromethyl-pyrazolo[1,5-a]pyrimidine-2-carboxylic acid (1,5-dimethyl-1*H*-pyrazol-4-ylmethyl)-methyl-amide;
(2,4-Dimethyl-piperidin-1-yl)-(5-phenyl-7-trifluoromethyl-pyrazolo[1,5-a]pyrimidin-2-yl)-methanone;
(3-Bromo-5-phenyl-7-trifluoromethyl-pyrazolo[1,5-a]pyrimidin-2-yl)-(hexahydro-pyrrolo[1,2-a]pyrazin-2-yl)-methanone;
[5-(3,4-Dimethoxy-phenyl)-7-trifluoromethyl-pyrazolo[1,5-a]pyrimidin-2-yl]-(hexahydro-pyrrolo[1,2-a]pyrazin-2-yl)-methanone;
(3-Chloro-5-p-tolyl-7-trifluoromethyl-pyrazolo[1,5-a]pyrimidin-2-yl)-(hexahydro-pyrrolo[1,2-a]pyrazin-2-yl)-methanone;
(3-Bromo-5-furan-2-yl-7-trifluoromethyl-pyrazolo[1,5-a]pyrimidin-2-yl)-piperidin-1-yl-methanone;
(3-Chloro-5-phenyl-7-trifluoromethyl-pyrazolo[1,5-a]pyrimidin-2-yl)-pyrrolidin-1-yl-methanone;
(7-Methyl-5-phenyl-pyrazolo[1,5-a]pyrimidin-2-yl)-(4-methyl-piperidin-1-yl)-methanone;
(4-Methyl-piperazin-1-yl)-(5-thiophen-2-yl-7-trifluoromethyl-pyrazolo[1,5-a]pyrimidin-2-yl)-methanone;
[3-Bromo-5-(4-methoxy-phenyl)-7-trifluoromethyl-pyrazolo[1,5-a]pyrimidin-2-yl]-piperidin-1-yl-methanone;
(4-Methyl-piperazin-1-yl)-(5-phenyl-7-trifluoromethyl-pyrazolo[1,5-a]pyrimidin-2-yl)-methanone;
[4-(4-Fluoro-phenyl)-piperazin-1-yl]-(5-furan-2-yl-7-trifluoromethyl-pyrazolo[1,5-a]pyrimidin-2-yl)-methanone;
[5-(4-Methoxy-phenyl)-7-trifluoromethyl-pyrazolo[1,5-a]pyrimidin-2-yl]-morpholin-4-yl-methanone;
(3,4-Dihydro-2*H*-quinolin-1-yl)-(5-thiophen-2-yl-7-trifluoromethyl-pyrazolo[1,5-a]pyrimidin-2-yl)-methanone;
(3-Bromo-5-thiophen-2-yl-7-trifluoromethyl-pyrazolo[1,5-a]pyrimidin-2-yl)-morpholin-4-yl-methanone;
Azepan-1-yl-(3-chloro-5-phenyl-7-trifluoromethyl-pyrazolo[1,5-a]pyrimidin-2-yl)-methanone;
(4-Benzoyl-piperazin-1-yl)-[5-(4-bromo-phenyl)-7-trifluoromethyl-pyrazolo[1,5-a]pyrimidin-2-yl]-methanone;
(4-Benzyl-piperazin-1-yl)-[5-(4-bromo-phenyl)-3-chloro-7-trifluoromethyl-pyrazolo[1,5-a]pyrimidin-2-yl]-methanone;
[5-(4-Bromo-phenyl)-3-chloro-7-trifluoromethyl-pyrazolo[1,5-a]pyrimidin-2-yl]-(4-methyl-piperazin-1-yl)-methanone;
Azepan-1-yl-[5-(4-bromo-phenyl)-7-trifluoromethyl-pyrazolo[1,5-a]pyrimidin-2-yl]-methanone;
[3-Bromo-5-(4-bromo-phenyl)-7-trifluoromethyl-pyrazolo[1,5-a]pyrimidin-2-yl]-(3,4-dihydro-2H-quinolin-1-yl)-methanone;
[5-(3,4-Dimethoxy-phenyl)-7-trifluoromethyl-pyrazolo[1,5-a]pyrimidin-2-yl]-morpholin-4-yl-methanone;
(3-Bromo-5-furan-2-yl-7-trifluoromethyl-pyrazolo[1,5-a]pyrimidin-2-yl)-(4-methyl-piperazin-1-yl)-methanone;
(4-Benzoyl-piperazin-1-yl)-(3-bromo-5-furan-2-yl-7-trifluoromethyl-pyrazolo[1,5-a]pyrimidin-2-yl)-methanone;
(3-Bromo-5-furan-2-yl-7-trifluoromethyl-pyrazolo[1,5-a]pyrimidin-2-yl)-(3,4-dihydro-2H-quinolin-1-yl)-methanone;
[5-(4-Bromo-phenyl)-7-trifluoromethyl-pyrazolo[1,5-a]pyrimidin-2-yl]-piperidin-1-yl-methanone;
[5-(4-Ethoxy-phenyl)-7-methyl-pyrazolo[1,5-a]pyrimidin-2-yl]-piperidin-1-yl-methanone;
[5-(4-Ethoxy-phenyl)-7-methyl-pyrazolo[1,5-a]pyrimidin-2-yl]-morpholin-4-yl-methanone;
Azepan-1-yl-(3-bromo-5-furan-2-yl-7-trifluoromethyl-pyrazolo[1,5-a]pyrimidin-2-yl)-methanone;
(3,4-Dihydro-1 H-isoquinolin-2-yl)-(5-thiophen-2-yl-7-trifluoromethyl-pyrazolo[1,5-a]pyrimidin-2-yl)-methanone;
(4-Benzyl-piperazin-1-yl)-(3-bromo-5-furan-2-yl-7-trifluoromethyl-pyrazolo[1,5-a]pyrimidin-2-yl)-methanone;
Azepan-1-yl-[5-(4-bromo-phenyl)-3-chloro-7-trifluoromethyl-pyrazolo[1,5-a]pyrimidin-2-yl]-methanone;
(3-Chloro-5-thiophen-2-yl-7-trifluoromethyl-pyrazolo[1,5-a]pyrimidin-2-yl)-(3,4-dihydro-1*H-*isoquinolin-2-yl)-methanone;
(5-Benzo[1,3]dioxol-5-yl-7-trifluoromethyl-pyrazolo[1,5-a]pyrimidin-2-yl)-piperidin-1-yl-methanone;
Azepan-1-yl-(5,7-diphenyl-pyrazolo[1,5-a]pyrimidin-2-yl)-methanone;
Azepan-1-yl-(5-benzo[1,3]dioxol-5-yl-7-trifluoromethyl-pyrazolo[1,5-a]pyrimidin-2-yl)-methanone;
[5-(4-Bromo-phenyl)-7-trifluoromethyl-pyrazolo[1,5-a]pyrimidin-2-yl]-morpholin-4-yl-methanone;
[3-Bromo-5-(4-bromo-phenyl)-7-trifluoromethyl-pyrazolo[1,5-a]pyrimidin-2-yl]-morpholin-4-yl-methanone;
[5-(4-Methoxy-phenyl)-7-trifluoromethyl-pyrazolo[1,5-a]pyrimidin-2-yl]-thiomorpholin-4-yl-methanone;
[5-(4-Bromo-phenyl)-3-chloro-7-trifluoromethyl-pyrazolo[1,5-a]pyrimidin-2-yl]-piperidin-1-yl-methanone;
(5-Benzo[1,3]dioxol-5-yl-7-trifluoromethyl-pyrazolo[1,5-a]pyrimidin-2-yl)-(3,4-dihydro-2H-quinolin-1-yl)-methanone;
(5-Benzo[1,3]dioxol-5-yl-7-trifluoromethyl-pyrazolo[1,5-a]pyrimidin-2-yl)-(4-benzyl-piperazin-1-yl)-methanone;
(5-Benzo[1,3]dioxol-5-yl-7-trifluoromethyl-pyrazolo[1,5-a]pyrimidin-2-yl)-morpholin-4-yl-methanone;
[5-(4-Bromo-phenyl)-7-trifluoromethyl-pyrazolo[1,5-a]pyrimidin-2-yl]-[4-(2-methoxy-phenyl)-piperazin-1-yl]-methanone;
[3-Bromo-5-(4-bromo-phenyl)-7-trifluoromethyl-pyrazolo[1,5-a]pyrimidin-2-yl]-(4-methyl-piperazin-1-yl)-methanone;
Azepan-1-yl-[5-(4-methoxy-phenyl)-7-trifluoromethyl-pyrazolo[1,5-a]pyrimidin-2-yl]-methanone;
(2,6-Dimethyl-piperidin-1-yl)-[7-(4-ethoxy-phenyl)-5-methyl-pyrazolo[1,5-a]pyrimidin-2-yl]-methanone;
(3,4-Dihydro-2H-quinolin-1-yl)-(5,7-diphenyl-pyrazolo[1,5-a]pyrimidin-2-yl)-methanone;
(3,6-Dibromo-pyrazolo[1,5-a]pyrimidin-2-yl)-piperidin-1-yl-methanone;
(3-Bromo-5-furan-2-yl-7-trifluoromethyl-pyrazolo[1,5-a]pyrimidin-2-yl)-piperidin-1-yl-methanone;
[5-(4-Methoxy-phenyl)-7-trifluoromethyl-pyrazolo[1,5-a]pyrimidin-2-yl]-thiomorpholin-4-yl-methanone;
[5-(3,4-Dichloro-phenyl)-7-trifluoromethyl-pyrazolo[1,5-a]pyrimidin-2-yl]-thiomorpholin-4-yl-methanone;
(3,4-Dihydro-1 H-isoquinolin-2-yl)-[5-(4-nitro-phenyl)-7-trifluoromethyl-pyrazolo[1,5-a]pyrimidin-2-yl]-methanone;
Azepan-1-yl-[5-(4-methoxy-phenyl)-7-trifluoromethyl-pyrazolo[1,5-a]pyrimidin-2-yl]-methanone;
Azepan-1-yl-[5-(4-nitro-phenyl)-7-trifluoromethyl-pyrazolo[1,5-a]pyrimidin-2-yl]-methanone;
[5-(3,4-Dichloro-phenyl)-7-trifluoromethyl-pyrazolo[1,5-a]pyrimidin-2-yl]-morpholin-4-yl-methanone;
Azepan-1-yl-[5-(3,4-dichloro-phenyl)-7-trifluoromethyl-pyrazolo[1,5-a]pyrimidin-2-yl]-methanone;
(3,4-Dihydro-1 H-isoquinolin-2-yl)-(5-phenyl-7-trifluoromethyl-pyrazolo[1,5-a]pyrimidin-2-yl)-methanone;
[5-(4-Bromo-phenyl)-3-chloro-7-trifluoromethyl-pyrazolo[1,5-a]pyrimidin-2-yl]-pyrrolidin-1-yl-methanone;
5,7-Diphenyl-pyrazolo[1,5-a]pyrimidine-2-carboxylic acid cyclohexyl-methyl-amide;
(3,4-Dihydro-1*H-*isoquinolin-2-yl)-(5-furan-2-yl-7-trifluoromethyl-pyrazolo[1,5-a]pyrimidin-2-yl)-methanone;
(4-Benzyl-piperazin-1-yl)-(3-chloro-5-furan-2-yl-7-trifluoromethyl-pyrazolo[1,5-a]pyrimidin-2-yl)-methanone;
[3-Chloro-5-(4-methoxy-phenyl)-7-trifluoromethyl-pyrazolo[1,5-a]pyrimidin-2-yl]-piperidin-1-yl-methanone;
(3-Chloro-5-*p*-tolyl-7-trifluoromethyl-pyrazolo[1,5-a]pyrimidin-2-yl)-(4-methyl-piperazin-1-yl)-methanone;
(3-Chloro-5-*p*-tolyl-7-trifluoromethyl-pyrazolo[1,5-a]pyrimidin-2-yl)-(4-phenyl-piperazin-1-yl)-methanone;
[3-Chloro-5-(3,4-dimethoxy-phenyl)-7-trifluoromethyl-pyrazolo[1,5-a]pyrimidin-2-yl]-piperidin-1-yl-methanone;
[4-(4-Fluoro-phenyl)-piperazin-1-yl]-(5-p-tolyl-7-trifluoromethyl-pyrazolo[1,5-a]pyrimidin-2-yl)-methanone;
(4-Phenyl-piperazin-1-yl)-(5-*p*-tolyl-7-trifluoromethyl-pyrazolo[1,5-a]pyrimidin-2-yl)-methanone;
Morpholin-4-yl-(5-*p*-tolyl-7-trifluoromethyl-pyrazolo[1,5-a]pyrimidin-2-yl)-methanone;
(4-Benzyl-piperazin-1-yl)-(5-*p*-tolyl-7-trifluoromethyl-pyrazolo[1,5-a]pyrimidin-2-yl)-methanone;
[5-(4-Chloro-phenyl)-7-trifluoromethyl-pyrazolo[1,5-a]pyrimidin-2-yl]-(4-methyl-piperazin-1-yl)-methanone;
[5-(4-Chloro-phenyl)-7-trifluoromethyl-pyrazolo[1,5-a]pyrimidin-2-yl]-morpholin-4-yl-methanone;
(4-Benzyl-piperazin-1-yl)-[5-(4-chloro-phenyl)-7-trifluoromethyl-pyrazolo[1,5-a]pyrimidin-2-yl]-methanone;
[5-(4-Fluoro-phenyl)-7-trifluoromethyl-pyrazolo[1,5-a]pyrimidin-2-yl]-morpholin-4-yl-methanone;
[5-(4-Fluoro-phenyl)-7-trifluoromethyl-pyrazolo[1,5-a]pyrimidin-2-yl]-piperidin-1-yl-methanone;
(3,4-Dihydro-2H-quinolin-1-yl)-[5-(4-fluoro-phenyl)-7-trifluoromethyl-pyrazolo[1,5-a]pyrimidin-2-yl]-methanone;
Azepan-1-yl-[5-(3,4-dimethoxy-phenyl)-7-trifluoromethyl-pyrazolo[1,5-a]pyrimidin-2-yl]-methanone;
(4-Benzyl-piperazin-1-yl)-[5-(4-methoxy-phenyl)-7-trifluoromethyl-pyrazolo[1,5-a]pyrimidin-2-yl]-methanone;
[5-(4-Methoxy-phenyl)-7-trifluoromethyl-pyrazolo[1,5-a]pyrimidin-2-yl]-pyrrolidin-1-yl-methanone;
(5-Benzo[1,3]dioxol-5-yl-7-trifluoromethyl-pyrazolo[1,5-a]pyrimidin-2-yl)-pyrrolidin-1-yl-methanone;
(3,4-Dihydro-2H-quinolin-1-yl)-[5-(4-nitro-phenyl)-7-trifluoromethyl-pyrazolo[1,5-a]pyrimidin-2-yl]-methanone;
[3-Chloro-5-(4-methoxy-phenyl)-7-trifluoromethyl-pyrazolo[1,5-a]pyrimidin-2-yl]-(4-methyl-piperidin-1-yl)-methanone;
[3-Chloro-5-(4-methoxy-phenyl)-7-trifluoromethyl-pyrazolo[1,5-a]pyrimidin-2-yl]-(3,4-dihydro-2H-quinolin-1-yl)-methanone;
3-Chloro-5-(4-methoxy-phenyl)-7-trifluoromethyl-pyrazolo[1,5-a]pyrimidine-2-carboxylic acid cyclohexyl-methyl-amide;
[3-Chloro-5-(4-fluoro-phenyl)-7-trifluoromethyl-pyrazolo[1,5-a]pyrimidin-2-yl]-(4-methyl-piperazin-1-yl)-methanone;
(3-Chloro-5-p-tolyl-7-trifluoromethyl-pyrazolo[1,5-a]pyrimidin-2-yl)-morpholin-4-yl-methanone;
Azepan-1-yl-(3-chloro-5-p-tolyl-7-trifluoromethyl-pyrazolo[1,5-a]pyrimidin-2-yl)-methanone;
Azepan-1-yl-[3-chloro-5-(3,4-dimethoxy-phenyl)-7-trifluoromethyl-pyrazolo[1,5-a]pyrimidin-2-yl]-methanone;
[5-(3,4-Dichloro-phenyl)-7-trifluoromethyl-pyrazolo[1,5-a]pyrimidin-2-yl]-(4-methyl-piperazin-1-yl)-methanone;
5,7-Diphenyl-pyrazolo[1,5-a]pyrimidine-2-carboxylic acid benzyl-methyl-amide; (5,7-Diphenyl-pyrazolo[1,5-a]pyrimidin-2-yl)-(4-phenyl-piperazin-1-yl)-methanone;
Morpholin-4-yl-(5-phenyl-7-trifluoromethyl-pyrazolo[1,5-a]pyrimidin-2-yl)-methanone;
Morpholin-4-yl-(5-thiophen-2-yl-7-trifluoromethyl-pyrazolo[1,5-a]pyrimidin-2-yl)-methanone;
Piperidin-1-yl-(5-thiophen-2-yl-7-trifluoromethyl-pyrazolo[1,5-a]pyrimidin-2-yl)-methanone;
[3-Bromo-5-(4-bromo-thiophen-2-yl)-7-trifluoromethyl-pyrazolo[1,5-a]pyrimidin-2-yl]-piperidin-1-yl-methanone;
(3-Bromo-5-thiophen-2-yl-7-trifluoromethyl-pyrazolo[1,5-a]pyrimidin-2-yl)-(3,4-dihydro-2*H-*quinolin-1-yl)-methanone;
(3-Chloro-5-thiophen-2-yl-7-trifluoromethyl-pyrazolo[1,5-a]pyrimidin-2-yl)-piperidin-1-yl-methanone;
(7-Methyl-5-phenyl-pyrazolo[1,5-a]pyrimidin-2-yl)-morpholin-4-yl-methanone;
(7-Methyl-5-phenyl-pyrazolo[1,5-a]pyrimidin-2-yl)-pyrrolidin-1-yl-methanone;
7-Methyl-5-phenyl-pyrazolo[1,5-a]pyrimidine-2-carboxylic acid benzyl-methyl-amide;
(2,6-Dimethyl-piperidin-1-yl)-[5-(4-fluoro-phenyl)-7-trifluoromethyl-pyrazolo[1,5-a]pyrimidin-2-yl]-methanone;
Azepan-1-yl-[5-(4-fluoro-phenyl)-7-trifluoromethyl-pyrazolo[1,5-a]pyrimidin-2-yl]-methanone;
[5-(4-Fluoro-phenyl)-7-trifluoromethyl-pyrazolo[1,5-a]pyrimidin-2-yl]-(4-methyl-piperazin-1-yl)-methanone;
[5-(4-Fluoro-phenyl)-7-trifluoromethyl-pyrazolo[1,5-a]pyrimidin-2-yl]-(4-phenyl-piperazin-1-yl)-methanone;
(2-Ethyl-piperidin-1-yl)-[5-(4-fluoro-phenyl)-7-trifluoromethyl-pyrazolo[1,5-a]pyrimidin-2-yl]-methanone;
(5-Phenyl-7-trifluoromethyl-pyrazolo[1,5-a]pyrimidin-3-yl)-piperidin-1-yl-methanone;
(5-Methyl-7-phenyl-pyrazolo[1,5-a]pyrimidin-3-yl)-(1,3,3-trimethyl-6-aza-bicyclo[3.2.1]oct-6-yl)-methanone;
(5-Thiophen-2-yl-7-trifluoromethyl-pyrazolo[1,5-a]pyrimidin-3-yl)-(1,3,3-trimethyl-6-aza-bicyclo[3.2.1]oct-6-yl)-methanone;
(4-Phenyl-piperazin-1-yl)-pyrazolo[1,5-a]pyrimidin-3-yl-methanone;
Pyrazolo[1,5-a]pyrimidine-3-carboxylic acid benzyl-(2-hydroxy-ethyl)-amide; [5-(4-Bromo-phenyl)-7-trifluoromethyl-pyrazolo[1,5-a]pyrimidin-3-yl]-(3,4-dihydro-1 H-isoquinolin-2-yl)-methanone;
(5,7-Dimethyl-pyrazolo[1,5-a]pyrimidin-3-yl)-[4-(4-methoxy-phenyl)-piperazin-1-yl]-methanone;
(3,4-Dihydro-2H-quinolin-1-yl)-[5-(4-fluoro-phenyl)-7-trifluoromethyl-pyrazolo[1,5-a]pyrimidin-3-yl]-methanone;
(5-Furan-2-yl-7-trifluoromethyl-pyrazolo[1,5-a]pyrimidin-3-yl)-piperidin-1-yl-methanone;
Azepan-1-yl-[7-trifluoromethyl-5-(3,4,5-trimethoxy-phenyl)-pyrazolo[1,5-a]pyrimidin-3-yl]-methanone;
(5-Benzo[1,3]dioxol-5-yl-7-trifluoromethyl-pyrazolo[1,5-a]pyrimidin-3-yl)-pyrrolidin-1-yl-methanone;
Azepan-1-yl-(5,7-diphenyl-pyrazolo[1,5-a]pyrimidin-3-yl)-methanone;
4-(5,7-Dimethyl-pyrazolo[1,5-a]pyrimidine-3-carbonyl)-piperazine-1-carboxylic acid ethyl ester;
(3,4-Dihydro-1*H*-isoquinolin-2-yl)-(5,7-dimethyl-pyrazolo[1,5-a]pyrimidin-3-yl)-methanone;
[4-(3-Chloro-phenyl)-piperazin-1-yl]-(5-p-tolyl-7-trifluoromethyl-pyrazolo[1,5-a]pyrimidin-3-yl)-methanone;
(4-Methyl-piperidin-1-yl)-pyrazolo[1,5-a]pyrimidin-3-yl-methanone;
[4-(2-Methoxy-phenyl)-piperazin-1-yl]-pyrazolo[1,5-a]pyrimidin-3-yl-methanone;
(3,4-Dihydro-2H-quinolin-1-yl)-[5-(4-methoxy-phenyl)-7-trifluoromethyl-pyrazolo[1,5-a]pyrimidin-3-yl]-methanone;
[5-(4-Methoxy-phenyl)-7-trifluoromethyl-pyrazolo[1,5-a]pyrimidin-3-yl]-thiomorpholin-4-yl-methanone;
(5-Furan-2-yl-7-trifluoromethyl-pyrazolo[1,5-a]pyrimidin-3-yl)-(2-methyl-piperidin-1-yl)-methanone;
(4-Benzyl-piperazin-1-yl)-(5,7-dimethyl-pyrazolo[1,5-a]pyrimidin-3-yl)-methanone;
[5-(4-Bromo-phenyl)-7-trifluoromethyl-pyrazolo[1,5-a]pyrimidin-3-yl]-morpholin-4-yl-methanone;
(5,7-Dimethyl-pyrazolo[1,5-a]pyrimidin-3-yl)-piperidin-1-yl-methanone;
(5-Furan-2-yl-7-trifluoromethyl-pyrazolo[1,5-a]pyrimidin-3-yl)-[4-(2-methoxy-phenyl)-piperazin-1-yl]-methanone;
(5-Phenyl-7-trifluoromethyl-pyrazolo[1,5-a]pyrimidin-3-yl)-piperidin-1-yl-methanone;
Azepan-1-yl-(5-thiophen-2-yl-7-trifluoromethyl-pyrazolo[1,5-a]pyrimidin-3-yl)-methanone;
(2,6-Dimethyl-piperidin-1-yl)-(5-thiophen-2-yl-7-trifluoromethyl-pyrazolo[1,5-a]pyrimidin-3-yl)-methanone;
[5-(4-Methoxy-phenyl)-7-trifluoromethyl-pyrazolo[1,5-a]pyrimidin-3-yl]-(4-methyl-piperazin-1-yl)-methanone;
(4-Methyl-piperazin-1-yl)-(5-thiophen-2-yl-7-trifluoromethyl-pyrazolo[1,5-a]pyrimidin-3-yl)-methanone;
[5-(4-Bromo-phenyl)-7-trifluoromethyl-pyrazolo[1,5-a]pyrimidin-3-yl]-(4-methyl-piperazin-1-yl)-methanone;
(Hexahydro-pyrrolo[1,2-a]pyrazin-2-yl)-(5-*p*-tolyl-7-trifluoromethyl-pyrazolo[1,5-a]pyrimidin-3-yl)-methanone;
[5-(4-Bromo-phenyl)-7-trifluoromethyl-pyrazolo[1,5-a]pyrimidin-3-yl]-(hexahydro-pyrrolo[1,2-a]pyrazin-2-yl)-methanone;
[5-(3,4-Dimethoxy-phenyl)-7-trifluoromethyl-pyrazolo[1,5-a]pyrimidin-3-yl]-(hexahydro-pyrrolo[1,2-a]pyrazin-2-yl)-methanone;
1-[5-(3-Methoxy-phenyl)-7-trifluoromethyl-pyrazolo[1,5-a]pyrimidine-3-carbonyl]-piperidine-4-carboxylic acid ethyl ester;
4-[5-(3-Methoxy-phenyl)-7-trifluoromethyl-pyrazolo[1,5-a]pyrimidine-3-carbonyl]-piperazine-1-carboxylic acid ethyl ester;
(3,4-Dihydro-2*H*-quinolin-1-yl)-[5-(3-methoxy-phenyl)-7-trifluoromethyl-pyrazolo[1,5-a]pyrimidin-3-yl]-methanone;
[4-(4-Fluoro-phenyl)-piperazin-1-yl]-[5-(3-methoxy-phenyl)-7-trifluoromethyl-pyrazolo[1,5-a]pyrimidin-3-yl]-methanone;
5-*p*-Tolyl-7-trifluoromethyl-pyrazolo[1,5-a]pyrimidine-3-carboxylic acid (1,3-dimethyl-1*H-*pyrazol-4-ylmethyl)-methyl-amide;
5-*p*-Tolyl-7-trifluoromethyl-pyrazolo[1,5-a]pyrimidine-3-carboxylic acid methyl-(1,3,5-trimethyl-1 H-pyrazol-4-ylmethyl)-amide;
[7-Difluoromethyl-5-(4-methoxy-phenyl)-pyrazolo[1,5-a]pyrimidin-3-yl]-(2-ethyl-imidazol-1-yl)-methanone;
[7-Difluoromethyl-5-(4-methoxy-phenyl)-pyrazolo[1,5-a]pyrimidin-3-yl]-morpholin-4-yl-methanone;
1-[7-Difluoromethyl-5-(4-methoxy-phenyl)-pyrazolo[1,5-a]pyrimidine-3-carbonyl]-1*H*-indole-3-carboxylic acid methyl ester;
[4-(4-Chloro-phenyl)-piperazin-1-yl]-[7-difluoromethyl-5-(4-methoxy-phenyl)-pyrazolo[1,5-a]pyrimidin-3-yl]-methanone;
[7-Difluoromethyl-5-(4-methoxy-phenyl)-pyrazolo[1,5-a]pyrimidin-3-yl]-(2-ethyl-piperidin-1-yl)-methanone;
7-Difluoromethyl-5-p-tolyl-pyrazolo[1,5-a]pyrimidine-3-carboxylic acid (1-ethyl-5-methyl-1*H-*pyrazol-4-ylmethyl)-methyl-amide;
1-(7-Difluoromethyl-5-methyl-pyrazolo[1,5-a]pyrimidine-3-carbonyl)-piperidine-4-carboxylic acid amide;
(7-Difluoromethyl-5-phenyl-pyrazolo[1,5-a]pyrimidin-3-yl)-(2-ethyl-imidazol-1-yl)-methanone;
(7-Difluoromethyl-5-phenyl-pyrazolo[1,5-a]pyrimidin-3-yl)-thiomorpholin-4-yl-methanone;
(7-Difluoromethyl-5-*p*-tolyl-pyrazolo[1,5-a]pyrimidin-3-yl)-(4-pyridin-2-yl-piperazin-1-yl)-methanone;
[7-Difluoromethyl-5-(4-methoxy-phenyl)-pyrazolo[1,5-a]pyrimidin-3-yl]-[4-(furan-2-carbonyl)-piperazin-1-yl]-methanone;
7-Difluoromethyl-5-phenyl-pyrazolo[1,5-a]pyrimidine-3-carboxylic acid (1-ethyl-3-methyl-1*H-*pyrazol-4-ylmethyl)-methyl-amide;
[7-Difluoromethyl-5-(4-methoxy-phenyl)-pyrazolo[1,5-a]pyrimidin-3-yl]-thiomorpholin-4-yl-methanone;
(7-Difluoromethyl-5-phenyl-pyrazolo[1,5-a]pyrimidin-3-yl)-(3,4-dihydro-1H-isoquinolin-2-yl)-methanone;
(7-Difluoromethyl-5-methyl-pyrazolo[1,5-a]pyrimidin-3-yl)-piperidin-1-yl-methanone;
Azepan-1-yl-(7-difluoromethyl-5-phenyl-pyrazolo[1,5-a]pyrimidin-3-yl)-methanone;
7-Difluoromethyl-5-*p*-tolyl-pyrazolo[1,5-a]pyrimidine-3-carboxylic acid methyl-(1-methyl-1*H-*pyrazol-4-ylmethyl)-amide;
(7-Difluoromethyl-5-*p*-tolyl-pyrazolo[1,5-a]pyrimidin-3-yl)-(3-methyl-piperidin-1-yl)-methanone;
(7-Difluoromethyl-5-*p*-tolyl-pyrazolo[1,5-a]pyrimidin-3-yl)-(4-ethyl-piperazin-1-yl)-methanone;
(7-Difluoromethyl-5-*p*-tolyl-pyrazolo[1,5-a]pyrimidin-3-yl)-morpholin-4-yl-methanone;
7-Difluoromethyl-5-phenyl-pyrazolo[1,5-a]pyrimidine-3-carboxylic acid methyl-(1,3,5-trimethyl-1*H*-pyrazol-4-ylmethyl)-amide;
(7-Difluoromethyl-5-phenyl-pyrazolo[1,5-a]pyrimidin-3-yl)-[4-(2-methyl-2H-pyrazole-3-carbonyl)-piperazin-1-yl]-methanone;
7-Difluoromethyl-5-*p*-tolyl-pyrazolo[1,5-a]pyrimidine-3-carboxylic acid benzyl-methyl-amide; 7-Difluoromethyl-5-(4-methoxy-phenyl)-pyrazolo[1,5-a]pyrimidine-3-carboxylic acid (1-ethyl-3-methyl-1*H*-pyrazol-4-ylmethyl)-methyl-amide;
7-Difluoromethyl-5-phenyl-pyrazolo[1,5-a]pyrimidine-3-carboxylic acid methyl-(4-pyrazol-1-yl-benzyl)-amide;
Azepan-1-yl-(5-cyclopropyl-7-difluoromethyl-pyrazolo[1,5-a]pyrimidin-3-yl)-methanone;
(4-Benzyl-piperazin-1-yl)-[5-(4-methoxy-phenyl)-7-trifluoromethyl-pyrazolo[1,5-a]pyrimidin-3-yl]-methanone;
(3,4-Dihydro-2*H*-quinolin-1-yl)-[5-(4-methoxy-phenyl)-7-trifluoromethyl-pyrazolo[1,5-a]pyrimidin-3-yl]-methanone;
5-(4-Methoxy-phenyl)-7-trifluoromethyl-pyrazolo[1,5-a]pyrimidine-3-carboxylic acid benzyl-methyl-amide;
(5-Furan-2-yl-7-trifluoromethyl-pyrazolo[1,5-a]pyrimidin-3-yl)-(4-methyl-piperazin-1-yl)-methanone;
(5-Furan-2-yl-7-trifluoromethyl-pyrazolo[1,5-a]pyrimidin-3-yl)-thiomorpholin-4-yl-methanone;
[5-(4-Methoxy-phenyl)-7-trifluoromethyl-pyrazolo[1,5-a]pyrimidin-3-yl]-morpholin-4-yl-methanone;
(4-Benzyl-piperazin-1-yl)-(5-thiophen-2-yl-7-trifluoromethyl-pyrazolo[1,5-a]pyrimidin-3-yl)-methanone;
[5-(4-Bromo-phenyl)-7-trifluoromethyl-pyrazolo[1,5-a]pyrimidin-3-yl]-morpholin-4-yl-methanone;
Azepan-1-yl-(5-benzo[1,3]dioxol-5-yl-7-trifluoromethyl-pyrazolo[1,5-a]pyrimidin-3-yl)-methanone;
(5-Benzo[1,3]dioxol-5-yl-7-trifluoromethyl-pyrazolo[1,5-a]pyrimidin-3-yl)-(3,4-dihydro-2H-quinolin-1-yl)-methanone;
5-Benzo[1,3]dioxol-5-yl-7-trifluoromethyl-pyrazolo[1,5-a]pyrimidine-3-carboxylic acid cyclohexyl-methyl-amide;
[5-(4-Bromo-phenyl)-7-trifluoromethyl-pyrazolo[1,5-a]pyrimidin-3-yl]-(3,4-dihydro-2H-quinolin-1-yl)-methanone;
[5-(4-Bromo-phenyl)-7-trifluoromethyl-pyrazolo[1,5-a]pyrimidin-3-yl]-piperidin-1-yl-methanone;
Azepan-1-yl-(5-furan-2-yl-7-trifluoromethyl-pyrazolo[1,5-a]pyrimidin-3-yl)-methanone;
(5-Benzo[1,3]dioxol-5-yl-7-trifluoromethyl-pyrazolo[1,5-a]pyrimidin-3-yl)-pyrrolidin-1-yl-methanone;
(5-Benzo[1,3]dioxol-5-yl-7-trifluoromethyl-pyrazolo[1,5-a]pyrimidin-3-yl)-(4-methyl-piperazin-1-yl)-methanone;
(3,4-Dihydro-1*H*-isoquinolin-2-yl)-[5-(4-methoxy-phenyl)-7-trifluoromethyl-pyrazolo[1,5-a]pyrimidin-3-yl]-methanone;
Azepan-1-yl-[5-(4-bromo-phenyl)-7-trifluoromethyl-pyrazolo[1,5-a]pyrimidin-3-yl]-methanone;
(3,4-Dihydro-2*H*-quinolin-1-yl)-(5,7-diphenyl-pyrazolo[1,5-a]pyrimidin-3-yl)-methanone;
Azepan-1-yl-(5,7-diphenyl-pyrazolo[1,5-a]pyrimidin-3-yl)-methanone;
(5-Benzo[1,3]dioxol-5-yl-7-trifluoromethyl-pyrazolo[1,5-a]pyrimidin-3-yl)-(4-benzyl-piperazin-1-yl)-methanone;
(4-Benzyl-piperazin-1-yl)-(5,7-diphenyl-pyrazolo[1,5-a]pyrimidin-3-yl)-methanone;
(5,7-Diphenyl-pyrazolo[1,5-a]pyrimidin-3-yl)-(4-methyl-piperazin-1-yl)-methanone;
[5-(4-Bromo-phenyl)-7-trifluoromethyl-pyrazolo[1,5-a]pyrimidin-3-yl]-(2-methyl-piperidin-1-yl)-methanone;
(3,4-Dihydro-2*H*-quinolin-1-yl)-(5-furan-2-yl-7-trifluoromethyl-pyrazolo[1,5-a]pyrimidin-3-yl)-methanone;
(5-Furan-2-yl-7-trifluoromethyl-pyrazolo[1,5-a]pyrimidin-3-yl)-(2-methyl-piperidin-1-yl)-methanone;
[5-(4-Methoxy-phenyl)-7-trifluoromethyl-pyrazolo[1,5-a]pyrimidin-3-yl]-piperidin-1-yl-methanone;
(5-Benzo[1,3]dioxol-5-yl-7-trifluoromethyl-pyrazolo[1,5-a]pyrimidin-3-yl)-morpholin-4-yl-methanone;
(5-Benzo[1,3]dioxol-5-yl-7-trifluoromethyl-pyrazolo[1,5-a]pyrimidin-3-yl)-piperidin-1-yl-methanone;
Morpholin-4-yl-[7-trifluoromethyl-5-(3,4,5-trimethoxy-phenyl)-pyrazolo[1,5-a]pyrimidin-3-yl]-methanone;
(4-Benzyl-piperazin-1-yl)-[7-trifluoromethyl-5-(3,4,5-trimethoxy-phenyl)-pyrazolo[1,5-a]pyrimidin-3-yl]-methanone;
(3,4-Dihydro-2*H*-quinolin-1-yl)-[7-trifluoromethyl-5-(3,4,5-trimethoxy-phenyl)-pyrazolo[1,5-a]pyrimidin-3-yl]-methanone;
(4-Methyl-piperazin-1-yl)-[7-trifluoromethyl-5-(3,4,5-trimethoxy-phenyl)-pyrazolo[1,5-a]pyrimidin-3-yl]-methanone;
Azepan-1-yl-[7-trifluoromethyl-5-(3,4,5-trimethoxy-phenyl)-pyrazolo[1,5-a]pyrimidin-3-yl]-methanone;
[5-(4-Methoxy-phenyl)-7-trifluoromethyl-pyrazolo[1,5-a]pyrimidin-3-yl]-piperidin-1-yl-methanone;
[5-(4-Methoxy-phenyl)-7-trifluoromethyl-pyrazolo[1,5-a]pyrimidin-3-yl]-pyrrolidin-1-yl-methanone;
Piperidin-1-yl-(5-thiophen-2-yl-7-trifluoromethyl-pyrazolo[1,5-a]pyrimidin-3-yl)-methanone;
(4-Benzyl-piperazin-1-yl)-[5-(4-methoxy-phenyl)-7-trifluoromethyl-pyrazolo[1,5-a]pyrimidin-3-yl]-methanone;
(3,4-Dihydro-2H-quinolin-1-yl)-[5-(4-methoxy-phenyl)-7-trifluoromethyl-pyrazolo[1,5-a]pyrimidin-3-yl]-methanone;
(5-Furan-2-yl-7-trifluoromethyl-pyrazolo[1,5-a]pyrimidin-3-yl)-(2-methyl-piperidin-1-yl)-methanone;
(5-Furan-2-yl-7-trifluoromethyl-pyrazolo[1,5-a]pyrimidin-3-yl)-morpholin-4-yl-methanone;
(5-Furan-2-yl-7-trifluoromethyl-pyrazolo[1,5-a]pyrimidin-3-yl)-thiomorpholin-4-yl-methanone;
[4-(4-Fluoro-phenyl)-piperazin-1-yl]-(5-thiophen-2-yl-7-trifluoromethyl-pyrazolo[1,5-a]pyrimidin-3-yl)-methanone;
(4-Methyl-piperidin-1-yl)-(5-thiophen-2-yl-7-trifluoromethyl-pyrazolo[1,5-a]pyrimidin-3-yl)-methanone;
(5-Furan-2-yl-7-trifluoromethyl-pyrazolo[1,5-a]pyrimidin-3-yl)-(4-methyl-piperazin-1-yl)-methanone;
(4-Benzyl-piperazin-1-yl)-(5-thiophen-2-yl-7-trifluoromethyl-pyrazolo[1,5-a]pyrimidin-3-yl)-methanone;
[5-(4-Methoxy-phenyl)-7-trifluoromethyl-pyrazolo[1,5-a]pyrimidin-3-yl]-morpholin-4-yl-methanone;
Piperidin-1-yl-[7-trifluoromethyl-5-(3,4,5-trimethoxy-phenyl)-pyrazolo[1,5-a]pyrimidin-3-yl]-methanone;
(2-Methyl-piperidin-1-yl)-(5-thiophen-2-yl-7-trifluoromethyl-pyrazolo[1,5-a]pyrimidin-3-yl)-methanone;
Azepan-1-yl-[5-(4-methoxy-phenyl)-7-trifluoromethyl-pyrazolo[1,5-a]pyrimidin-3-yl]-methanone;
5-Thiophen-2-yl-7-trifluoromethyl-pyrazolo[1,5-a]pyrimidine-3-carboxylic acid benzyl-methyl-amide;
[5-(4-Methoxy-phenyl)-7-trifluoromethyl-pyrazolo[1,5-a]pyrimidin-3-yl]-pyrrolidin-1-yl-methanone;
[5-(4-Chloro-phenyl)-2-methyl-7-trifluoromethyl-pyrazolo[1,5-a]pyrimidin-3-yl]-piperidin-1-yl-methanone;
Azepan-1-yl-[5-(4-chloro-phenyl)-2-methyl-7-trifluoromethyl-pyrazolo[1,5-a]pyrimidin-3-yl]-methanone;
[5-(4-Chloro-phenyl)-2-methyl-7-trifluoromethyl-pyrazolo[1,5-a]pyrimidin-3-yl]-(4-methyl-piperazin-1-yl)-methanone;
Morpholin-4-yl-(5-p-tolyl-7-trifluoromethyl-pyrazolo[1,5-a]pyrimidin-3-yl)-methanone;
(4-Benzyl-piperazin-1-yl)-(5-p-tolyl-7-trifluoromethyl-pyrazolo[1,5-a]pyrimidin-3-yl)-methanone;
5-(4-Methoxy-phenyl)-7-trifluoromethyl-pyrazolo[1,5-a]pyrimidine-3-carboxylic acid benzyl-methyl-amide;
(4-Benzyl-piperazin-1-yl)-(5-phenyl-7-trifluoromethyl-pyrazolo[1,5-a]pyrimidin-3-yl)-methanone;
Morpholin-4-yl-(5-phenyl-7-trifluoromethyl-pyrazolo[1,5-a]pyrimidin-3-yl)-methanone; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (II) 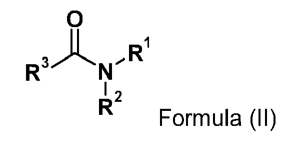 wherein
R¹ is C₃-C₁₀cycloalkyl, C₃-C₁₀hetcycloalkyl, C₁-C₈alkyl, aryl, hetaryl, arylC₁-C₆alkyl or hetarylC₁-C₆alkyl, wherein the cycloalkyl, hetcycloalkyl, alkyl, arylalkyl and hetarylalkyl groups independently are optionally substituted with one or more of R⁴.
R² is hydrogen, C₁-C₈alkyl, aryl, hetaryl, arylC₁-C₆alkyl, C₃-C₁₀cycloalkylC₁-C₆alkyl, C₁-C₆alkyl-carboxyC₁-C₆alkyl wherein the alkyl, aryl and cycloalkyl groups independently are optionally substituted with one or more of R⁵; or
R¹ and R² together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁴;
R³ is C₁-C₈alkyl, C₁-C₆alkenyl, C₁-C₆alkynyl, C₃-C₁₀cycloalkyl, C₃-C₁₀hetcycloalkyl, aryl, hetaryl, arylC₁-C₆alkyl, C₁-C₆alkyloxyC₁-C₆alkyl, hetarylC₁-C₆alkyl, aryl-R⁶-C₁-C₆alkyl, hetaryl-R⁶-C₁-C₆alkyl or arylC₁-C₆alkyl-R⁶-C₁-C₆alkyl wherein the alkyl, cycloalkyl, hetcycloalkyl, alkenyl, alkynyl, aryl and hetaryl groups independently are optionally substituted with one or more of R⁷;
R⁴ and R⁵ independently are hydrogen, hydroxy, oxo, cyano, halo, methylendioxo, NR⁸R⁹, C₁-C₈alkyl, C₁-C₆alkyloxy, trihalomethyl, trihalomethyloxy, C₃-C₁₀cycloalkyl, C₃-C₁₀hetcycloalkyl, C₃-C₁₀cycloalkenyl, aryl, hetaryl, hetarylSOₙ, arylC₁-C₆alkyloxy, hetarylC₁-C₆alkyloxy, C₁-C₆alkyl-R⁶-C₁-C₆alkyl, arylC₁-C₆alkyl-R⁶-C₁-C₆alkyl, C₁-C₆alkylcarbonyl, arylcarbonyl, arylC₁-C₆alkylcarbonyl, hetarylcarbonyl, hetarylC₁-C₆alkyl-carbonyl, C₁-C₆alkylSOₙ, C₁-C₆-alkyl-carboxy, arylcarboxy, hetarylcarboxy, arylC₁-C₆alkylcarboxy or hetarylC₁-C₆alkylcarboxy wherein the alkyl, cycloalkyl, hetcycloalkyl, aryl and hetaryl groups independently are optionally substituted with one ore more of R¹⁵;
R⁶ is oxygen, sulphur, SOₙ or NR¹⁶;
R⁷ is hydrogen, halo, hydroxy, cyano, nitro, COOR¹⁷, C₁-C₈alkyl, C₃-C₁₀cycloalkyl, C₃-C₁₀hetcycloalkyl, methylendioxo, trihalomethyl, trihalomethyloxy, aryl, arylC₁-C₆alkyl, C₁-C₆alkyloxy, C₁-C₆alkyloxyC₁-C₆alkyl, aryloxy, arylC₁-C₆alkyloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyl-oxyC₁-C₆alkyl, NR⁸R⁹, SO₂NR⁸R⁹, NR⁴R⁵carbonylC₁-C₆alkyl, arylthio, hetarylthio, R¹⁸carbonylNR⁸, arylSOₙ, hetarylSOₙ, R¹⁹SOₘNR⁸, arylthioC₁-C₆alkyl, hetarylthioC₁-C₆alkyl or arylC₁-C₆alkylR⁶C₁-C₆alkyl; wherein the aryl and hetaryl groups independently are optionally substituted with one or more R¹⁰;
R⁸ and R⁹ independently are hydrogen, C₁-C₈alkyl, aryl, hetaryl, arylC₁-C₆alkyl or hetarylC₁-C₆alkyl wherein the alkyl, aryl and hetaryl groups independently are optionally substituted with one or more of R¹¹; or
R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one halo, cyano, C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R¹⁰ and R¹¹ independently are hydrogen, hydroxy, oxo, halo, cyano, nitro, C₁-C₈alkyl, C₁-C₆alkyloxy, NR¹²R¹³, methylendioxo, trihalomethyl or trihalomethyloxy;
R¹² and R¹³ independently are hydrogen, C₁-C₈alkyl or arylC₁-C₆alkyl;
R¹⁴ is hydrogen, halo, hydroxy, oxo, nitro, cyano, C₁-C₈alkyl, C₁-C₆alkyloxy or aryloxy;
R¹⁵ is hydrogen, halo, hydroxy, oxo, nitro, cyano, CONR⁸R⁹ or COOR¹⁷;
R¹⁶ is hydrogen, C₁-C₈alkyl, C₃-C₁₀cycloalkyl, C₃-C₁₀hetcycloalkyl, aryl, arylC₁-C₆alkyl, hetaryl, hetarylC₁-C₆alkyl, alkylcarbonyl, arylcarbonyl, arylC₁-C₆alkylcarbonyl, aryloxyC₁-C₆alkyl, hetaryloxyC₁-C₆alkyl, arylthioC₁-C₆alkyl or hetarylthioC₁-C₆alkyl; wherein the alkyl, cycloalkyl, hetcycloalkyl, aryl and hetaryl groups independently are optionally substituted with one or more of R¹⁰;
R¹⁷ is hydrogen, C₁-C₈alkyl, aryl or arylC₁-C₆alkyl;
R¹⁸ is C₁-C₆alkyl, C₂-C₆alkenyl, aryl, arylC₁-C₆alkyl, hetaryl, hetarylC₁-C₆alkyl, C₃-C₁₀cycloalkyl, C₃-C₁₀hetcycloalkyl, C₁-C₆alkyloxy, aryloxy, arylC₁-C₆alkyloxy, arylC₁-C₆alkyloxyC₁-C₆alkyl, hetaryloxy, hetarylC₁-C₆alkyloxy, hetarylC₁-C₆alkyloxyC₁-C₆alkyl or R⁸R⁹NC₁-C₆alkyl wherein the alkyl, alkenyl, cycloalkyl, hetcycloalkyl, aryl and hetaryl groups are optionally substituted with R¹⁵;
R¹⁹ is C₁-C₆alkyl, C₃-C₁₀cydoalkyl, C₃-C₁₀hetcydoalkyl, aryl, arylC₁-C₆alkyl, hetaryl, hetaryl-C₁-C₆alkyl;
m is 1 or 2;
n is 0, 1 or 2; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (II) wherein:
R¹ is C₃-C₁₀cycloalkyl, C₃-C₁₀hetcycloalkyl, C₁-C₈alkyl, aryl, hetaryl, arylC₁-C₆alkyl or hetaryl-C₁-C₆alkyl, wherein the cycloalkyl, hetcycloalkyl, alkyl, arylalkyl and hetarylalkyl groups independently are optionally substituted with one or more of R⁴;
R² is hydrogen, C₁-C₈alkyl, aryl, hetaryl, arylC₁-C₆alkyl, C₃-C₁₀cycloalkylC₁-C₆alkyl, C₁-C₆-alkylcarboxyC₁-C₆alkyl wherein the alkyl, aryl and cycloalkyl groups independently are optionally substituted with one or more of R⁵; or
R¹ and R² are together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-₆alkylcarboxy wherein the alkyl and aryl groups independently are optionally substituted with one ore more of R¹⁴;
R³ is C₁-C₈alkyl, C₁-C₆alkenyl, C₁-C₆alkynyl, C₃-C₁₀cycloalkyl, C₃-C₁₀hetcycloalkyl, aryl, hetaryl, arylC₁-C₆alkyl, C₁-C₆alkyloxyC₁-C₆alkyl, hetarylC₁-C₆alkyl, aryl-R⁶-C₁-C₆alkyl, hetaryl-R⁶-C₁-C₆alkyl or arylC₁-C₆alkyl-R⁶-C₁-C₆alkyl wherein the alkyl, cycloalkyl, hetcycloalkyl, alkenyl, alkynyl, aryl and hetaryl groups independently are optionally substituted with one or more of R⁷;
R⁴ and R⁵ independently are hydrogen, hydroxy, oxo, cyano, halo, methylendioxo, NR⁸R⁹, C₁-C₈alkyl, C₁-C₆alkyloxy, trihalomethyl, trihalomethyloxy, C₃-C₁₀cycloalkyl, C₃-C₁₀hetcycloalkyl, C₃-C₁₀cycloalkenyl, aryl, hetaryl, hetarylSOₙ, arylC₁-C₆alkyloxy, hetarylC₁-C₆alkyloxy, C₁-C₆alkyl-R⁶-C₁-C₆alkyl, arylC₁-C₆alkyl-R⁶-C₁-C₆alkyl, C₁-C₆alkylcarbonyl, arylcarbonyl, arylC₁-C₆alkylcarbonyl, hetarylcarbonyl, hetarylC₁-C₆alkyl-carbonyl, C₁-C₆alkylSOₙ, C₁-C₆alkyl-carboxy, arylcarboxy, hetarylcarboxy, arylC₁-C₆alkylcarboxy or hetarylC₁-C₆alkylcarboxy wherein the alkyl, cycloalkyl, hetcycloalkyl, aryl and hetaryl groups independently are optionally substituted with one ore more of R¹⁵;
R⁶ is oxygen, sulphur, SOₙ, NR¹⁶;
R⁷ is hydrogen, halo, hydroxyl, cyano, nitro, COOR¹⁷, C₁-C₈alkyl, C₃-C₁₀cycloalkyl, C₃-C₁₀hetcycloalkyl, methylendioxo, trihalomethyl, trihalomethyloxy, aryl, arylC₁-C₆alkyl, C₁-C₆alkyloxy, C₁-C₆alkyloxyC₁-C₆alkyl, aryloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyloxyC₁-C₆alkyl, NR⁸R⁹, SO₂NR⁸R⁹, NR⁴R⁵carbonylalkyl, arylcarbonylNR⁸, arylthio, hetarylthio, arylSOₙ, hetarylSOₙ, arylSOₘNR⁸, arylthioC₁-C₆alkyl, hetarylthioC₁-C₆alkyl or arylC₁-C6alkylR⁶C₁-C₆alkyl; wherein the aryl and hetaryl groups independently are optionally substituted with one or more R¹⁰;
R⁸ and R⁹ independently are hydrogen, C₁-C₈alkyl, aryl, hetaryl, arylC₁-C₆alkyl or hetarylC₁-C₆alkyl wherein the alkyl, aryl and hetaryl groups independently are optionally substituted with one or more of R¹¹; or
R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkyl-carboxy or hetarylC₁-C₆alkylcarboxy;
R¹⁰ and R¹¹ independently are hydrogen, hydroxy, oxo, halo, cyano, nitro, C₁-C₆alkyl, C₁-C₆alkyloxy, NR¹²R¹³, methylendioxo, trihalomethyl or trihalomethyloxy;
R¹² and R¹³ independently are hydrogen, C₁-C₈alkyl or arylC₁-C₆alkyl;
R¹⁴ is hydrogen, halo, hydroxy, oxo, nitro, cyano, C₁-C₈alkyl, C₁-C₆alkyloxy or aryloxy;
R¹⁵ is hydrogen, halo, hydroxy, oxo, nitro, cyano or COOR¹⁷;
R¹⁶ is hydrogen, C₁-C₈alkyl, C₃-C₁₀cycloalkyl, C₃-C₁₀hetcycloalkyl, aryl, arylC₁-C₆alkyl, hetaryl, hetarylC₁-C₆alkyl, alkylcarbonyl, arylcarbonyl, arylC₁-C₆alkylcarbonyl, aryloxyC₁-C₆alkyl, hetaryloxyC₁-C₆alkyl, arylthioC₁-C₆alkyl or hetarylthioC₁-C₆alkyl; wherein the alkyl, cycloalkyl, hetcycloalkyl, aryl and hetaryl groups independently are optionally substituted with one or more of R¹⁰;
R¹⁷ is hydrogen, C₁-C₈alkyl, aryl or arylC₁-C₆alkyl;
m is 1 or 2;
n is 0, 1 or 2; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (II) wherein R¹ is C₃-C₁₀cycloalkyl or C₃-C₁₀hetcycloalkyl wherein the cycloalkyl and hetcycloalkyl groups independently are optionally substituted with one or more of R⁴ as defined above.

In another embodiment of the present invention said substituted amides, or a prodrug thereof, as a component of the combination therapy is of the general formula (II) wherein R¹ is C₃-C₁₀cydoalkyl optionally substituted with one or more of R⁴ as defined above.

In another embodiment of the present invention said substituted amides, or a prodrug thereof, as a component of the combination therapy is of the general formula (II) wherein R² is hydrogen or C₁-C₈alkyl, wherein the the alkyl group is optionally substituted with one or more of R⁵ as defined above.

In another embodiment of the present invention said substituted amides, or a prodrug thereof, as a component of the combination therapy is of the general formula (II) wherein R² is C₁-C₈alkyl optionally substituted with one or more of R⁵ as defined above.

In another embodiment of the present invention said substituted amides, or a prodrug thereof, as a component of the combination therapy is of the general formula (II) wherein R³ is C₃-C₁₀cycloalkyl, C₃-C₁₀hetcycloalkyl, aryl, hetaryl, arylC₁-C₆alkyl, hetarylC₁-C₆alkyl, aryl-R⁶-C₁-C₆alkyl, hetaryl-R⁶-C₁-C₆alkyl or arylC₁-C₆alkyl-R⁶-C₁-C₆alkyl wherein the alkyl, cycloalkyl, hetcycloalkyl, aryl and hetaryl groups independently are optionally substituted with one or more of R⁷.

In another embodiment of the present invention said substituted amides, or a prodrug thereof, as a component of the combination therapy is of the general formula (II) wherein R³ is aryl or hetaryl, wherein the aryl and hetaryl groups are optionally substituted with one or more of R⁷ as defined above.

In another embodiment of the present invention said substituted amides, or a prodrug thereof, as a component of the combination therapy is of the general formula (II) wherein R³ is is phenyl optionally substituted with one or more of R⁷ as defined above.

In another embodiment of the present invention said substituted amides, or a prodrug thereof, as a component of the combination therapy is of the general formula (II) wherein R³ is phenyl optionally substituted independently in position 2(ortho) or 4(para) with one or more of R⁷ as defined above.

In another embodiment of the present invention said substituted amides, or a prodrug thereof, as a component of the combination therapy is of the general formula (II) wherein R⁴ and R⁵ independently are hydrogen, hydroxy, oxo, halo, C₁-C₈alkyl, wherein the alkyl group is optionally substituted with one ore more of R¹⁵.

In another embodiment of the present invention said substituted amides, or a prodrug thereof, as a component of the combination therapy is of the general formula (II) wherein R⁶ is oxygen.

In another embodiment of the present invention said substituted amides, or a prodrug thereof, as a component of the combination therapy is of the general formula (II) wherein R⁷is hydrogen, halo, hydroxy, cyano, C₁-C₈alkyl, C₃-C₁₀cycloalkyl, C₃-C₁₀hetcycloalkyl, trihalomethyl, aryl, arylC₁-C₆alkyl, C₁-C₆alkyloxy, C₁-C₆alkyloxyC₁-C₆alkyl, aryloxy, arylC₁-C₆alkyloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyl-oxyC₁-C₆alkyl, NR⁸R⁹, NR⁴R⁵carbonylC₁-C₆alkyl, R¹⁸carbonylNR⁸, R¹⁹SOₘNR⁸, wherein the aryl and hetaryl groups independently are optionally substituted with one or more R¹⁰.

In another embodiment of the present invention said substituted amides, or a prodrug thereof, as a component of the combination therapy is of the general formula (II) wherein R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one halo, cyano, C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy.

In another embodiment of the present invention said substituted amides, or a prodrug thereof, as a component of the combination therapy is of the general formula (II) wherein R¹⁵ is CONR⁸R⁹.

In another embodiment of the present invention said substituted amides, or a prodrug thereof, as a component of the combination therapy is of the general formula (II) wherein R¹⁸ is C₁-C₆alkyl optionally substituted with R¹⁵.

In another embodiment of the present invention said substituted amide, or a prodrug thereof, as a component of the combination therapy is selected from the group consisting of:
3-(10,11-Dihydro-dibenzo[b,f]azepin-5-yl)-1-[4-(1H-imidazol-4-yl)-piperidin-1-yl]-propan-1-one;
4-(10,11-Dihydro-dibenzo[b,f]azepin-5-yl)-1-[4-(3H-imidazol-4-yl)-piperidin-1-yl]-butan-1-one;
2,4-Bis-benzyloxy-benzamide;
(1H-Indol-4-yl)-piperidin-1-yl-methanone;
N-(1,4-Dioxo-1,4-dihydro-naphthalen-2-yl)-benzamide;
N-(2,3-Dihydroxy-propyl)-2-(2-phenyl-adamantan-2-yl)-acetamide;
(6-Fluoro-2-methyl-3,4-dihydro-2H-quinolin-1-yl)-phenyl-methanone;
(2-Chloro-phenyl)-(6-fluoro-2-methyl-3,4-dihydro-2H-quinolin-1-yl)-methanone;
3-Cyclopentyl-1-(6-fluoro-2-methyl-3,4-dihydro-2H-quinolin-1-yl)-propan-1-one;
(3-Chloro-thieno[2,3-b]thiophen-2-yl)-thiomorpholin-4-yl-methanone;
2-[2-(4-Chloro-phenyl)-adamantan-2-yl]-1-[4-(4-methoxy-phenyl)-piperazin-1-yl]-ethanone;
1-(4-Benzyl-piperazin-1-yl)-2-[2-(4-chloro-phenyl)-adamantan-2-yl]-ethanone;
2-[2-(4-Chloro-phenyl)-adamantan-2-yl]-1-(4-methyl-piperazin-1-yl)-ethanone;
1-[4-(6-Chloro-pyridin-2-yl)-piperazin-1-yl]-2-(2-phenyl-adamantan-2-yl)-ethanone;
4-Chloro-N-(1,7,7-trimethyl-bicyclo[2.2.1]hept-2-yl)-benzamide;
3-Chloro-benzo[b]thiophene-2-carboxylic acid (2-cyano-ethyl)-cyclohexyl-amide;
2-[2-(Bicyclo[2.2.1]hept-5-en-2-ylamino)-4-oxo-4,5-dihydro-thiazol-5-yl]-N-(2-chloro-phenyl)-acetamide;
[3-(4-sec-Butyl-phenoxy)-phenyl]-piperidin-1-yl-methanone;
3-(6-Chloro-pyridin-2-yloxy)-N-ethyl-benzamide;
N-Benzyl-2,4-dichloro-N-pyridin-2-yl-benzamide;
2-[Benzoyl-(3-chloro-4-fluoro-phenyl)-amino]-propionic acid butyl ester; 2-[Benzoyl-(3-chloro-4-fluoro-phenyl)-amino]-propionic acid pentyl ester;
3-(4-Fluoro-phenyl)-1-(4-phenyl-piperidin-1-yl)-but-2-en-1-one;
N-(1,7,7-Trimethyl-bicyclo[2.2.1]hept-2-yl)-benzamide;
1-(3-Cyclopentyl-propionyl)-piperidine-3-carboxylic acid ethyl ester;
4-Phenyl-1-phenylacetyl-piperidine-4-carbonitrile;
1-Octanoyl-4-phenyl-piperidine-4-carbonitrile;
2,2-Dimethyl-1-(1,3,3-trimethyl-6-aza-bicydo[3.2.1]oct-6-yl)-propan-1-one;
(4-Chloro-phenyl)-(1,3,3-trimethyl-6-aza-bicyclo[3.2.1]oct-6-yl)-methanone;
N-[1-(4-Methanesulfonyl-phenyl)-ethyl]-N-(tetrahydro-furan-2-ylmethyl)-benzamide;
2-(2-Amino-phenylsulfanyl)-1-(5-fluoro-2,3-dihydro-indol-1-yl)-ethanone;
N-(1-Methyl-2,3-dihydro-1 H-indol-5-ylmethyl)-N-(tetrahydro-furan-2-ylmethyl)-benzamide;
1-Benzoyl-piperidine-2-carboxylic acid ethyl ester;
N-(2-Chloro-phenyl)-2-(1,2,3,4-tetrahydro-isoquinolin-1-yl)-acetamide;
(Decahydro-naphthalen-1-yl)-(4-methyl-piperazin-1-yl)-methanone;
(4-Methyl-piperazin-1-yl)-(2-p-tolylsulfanyl-phenyl)-methanone;
Adamantane-1-carboxylic acid (3-benzyloxy-2-ethyl-6-methyl-pyridin-4-yl)-amide;
(6-Fluoro-2-methyl-3,4-dihydro-2H-quinolin-1-yl)-(3,4,5-trimethoxy-phenyl)-methanone;
N-Bicyclo[2.2.1]hept-2-yl-3-cyclopentyl-propionamide;
(2-Benzo[1,2,5]oxadiazol-5-yl-thiazol-4-yl)-piperidin-1-yl-methanone;
Thiophene-2-carboxylic acid [4-(4-fluoro-phenyl)-4-hydroxy-butyl]-isopropyl-amide;
N-Cyclohexyl-3-(1,3-dioxo-1,3-dihydro-isoindol-2-yl)-propionamide;
2-[(Adamantane-1-carbonyl)-amino]-3-(1H-indol-3-yl)-propionic acid methyl ester; Adamantane-1-carboxylic acid [3-(1 H-benzoimidazol-2-ylsulfanyl)-5-nitro-phenyl]-amide;
N-Benzyl-N-(1-cyclopropyl-ethyl)-4-fluoro-benzamide;
Thiophene-2-carboxylic acid 2-[2-(2-phenyl-adamantan-2-yl)-acetylamino]-ethyl ester;
N-(4-Acetyl-phenyl)-2-(2-phenyl-adamantan-2-yl)-acetamide;
2-[2-(4-Chloro-phenyl)-adamantan-2-yl]-N-(2-hydroxy-ethyl)-acetamide;
(4-Benzoyl-piperidin-1-yl)-thiophen-2-yl-methanone;
N-(2-Benzoyl-4-methyl-phenyl)-3-phenyl-acrylamide;
N-(2-Benzoyl-4-methyl-phenyl)-2-fluoro-benzamide;
Adamantane-1-carboxylic acid (4-ethoxy-benzothiazol-2-yl)-amide;
Adamantane-1-carboxylic acid (5-benzoyl-4-phenyl-thiazol-2-yl)-amide;
3-(2-Hydroxy-phenyl)-1,3-di-piperidin-1-yl-propan-1-one;
N-(1-Methyl-2-phenyl-ethyl)-3-phenyl-propionamide;
4-Oxo-4-piperidin-1-yl-butyric acid 4-tert-butyl-cyclohexyl ester;
N-tert-Butyl-N-(4-methoxy-benzyl)-4-nitro-benzamide;
{4-[(Adamantane-1-carbonyl)-amino]-phenoxy}-acetic acid;
2-(4-Isobutyl-phenyl)-N-(1-phenyl-ethyl)-propionamide;
Adamantane-1-carboxylic acid 4-[(adamantane-1-carbonyl)-amino]-2,6-dimethyl-pyridin-3-yl ester;
2-Phenyl-1-(3-phenyl-pyrrolidin-1-yl)-ethanone;
Adamantane-1-carboxylic acid 4-[(adamantane-1-carbonyl)-amino]-2-ethyl-6-methyl-pyridin-3-yl ester;
N-(1,3-Dioxo-1,3-dihydro-isoindol-2-ylmethyl)-N-(4-hydroxy-phenyl)-benzamide;
Biphenyl-4-yl-piperidin-1-yl-methanone;
Azepan-1-yl-(3,5-dichloro-phenyl)-methanone;
Azepan-1-yl-biphenyl-4-yl-methanone;
Azepan-1-yl-(4-chloro-phenyl)-methanone;
3-Heptylcarbamoyl-bicyclo[2.2.1]hept-5-ene-2-carboxylic acid;
Adamantan-1-yl-azepan-1-yl-methanone;
N,N-Dibenzyl-3,4-dimethoxy-benzamide;
N-Benzyl-N-isopropyl-4-nitro-benzamide;
N-[2-(1H-Indol-3-yl)-1-methyl-ethyl]-2-(4-isobutyl-phenyl)-propionamide;
N-tert-Butyl-2-(4-isobutyl-phenyl)-propionamide;
Adamantane-1-carboxylic acid (2-acetyl-phenyl)-amide;
N-(1,3-Dioxo-1,3-dihydro-isoindol-2-ylmethyl)-N-(4-fluoro-phenyl)-benzamide;
(Octahydro-quinolin-1-yl)-phenyl-methanone;
(7-Hydroxy-octahydro-quinolin-1-yl)-phenyl-methanone;
N-(1,3-Dioxo-1,3-dihydro-isoindol-2-ylmethyl)-N-p-tolyl-benzamide;
N, N-Dibenzyl-4-methyl-benzamide;
(2-Chloro-phenyl)-(2-methyl-piperidin-1-yl)-methanone;
[4-Bromo-3-(piperidine-1-sulfonyl)-phenyl]-piperidin-1-yl-methanone;
2-Chloro-N-(3,4-dimethyl-phenyl)-benzamide;
1-Azepan-1-yl-2-(3,3-dimethyl-3,4-dihydro-2H-isoquinolin-1-ylidene)-ethanone;
N-Cyclohexyl-4-(2,4-dichloro-phenoxy)-butyramide;
N-Benzo[1,3]dioxol-5-yl-2-chloro-benzamide;
(4-Benzyl-piperidin-1-yl)-(2-chloro-phenyl)-methanone;
2-(Benzothiazol-2-ylsulfanyl)-N-cyclohexyl-acetamide;
Cyclohexanecarboxylic acid (7,7-dimethyl-5-oxo-5,6,7,8-tetrahydro-quinazolin-2-yl)-amide; 2,4-Dichloro-N-ethyl-N-o-tolyl-benzamide;
(4-Benzyl-piperidin-1-yl)-(4-fluoro-phenyl)-methanone;
N-Cyclohexyl-4-(2,4-dichloro-phenoxy)-N-methyl-butyramide;
3-[2-(1,3-Dioxo-1,3-dihydro-isoindol-2-yl)-ethyl]-adamantane-1-carboxylic acid;
Morpholin-4-yl-(3-p-tolyl-adamantan-1-yl)-methanone;
N-Benzyl-2,4-dichloro-N-methyl-benzamide;
Thiophene-2-carboxylic acid dibenzylamide;
Azepan-1-yl-(2-bromo-phenyl)-methanone;
(3,4-Dichloro-phenyl)-(4-methyl-piperidin-1-yl)-methanone;
N,N-Dibenzyl-3,4-dichloro-benzamide;
4-(2,4-Dichloro-phenoxy)-1-piperidin-1-yl-butan-1-one;
N, N-Dibenzyl-2-fluoro-benzamide;
(2-Chloro-phenyl)-piperidin-1-yl-methanone;
2-Chloro-N-(3-trifluoromethyl-phenyl)-benzamide;
N-Benzyl-N-ethyl-2-phenyl-acetamide;
(3,4-Dihydro-2H-quinolin-1-yl)-p-tolyl-methanone;
Thiophene-2-carboxylic acid benzyl-ethyl-amide;
N-Adamantan-1-yl-2-dibenzylamino-acetamide;
N-Cyclohexyl-2-(1,3-dioxo-1,3-dihydro-isoindol-2-yl)-3-phenyl-propionamide;
Thiophene-2-carboxylic acid cycloheptylamide;
N-Cyclohexyl-3-diethylsulfamoyl-4-methyl-benzamide;
4-Benzoyl-N-methyl-N-phenyl-benzamide;
N-Benzyl-2-bromo-N-methyl-benzamide;
2-Chloro-N-methyl-N-phenyl-benzamide;
4-Chloro-N-ethyl-N-o-tolyl-benzamide;
N-Benzyl-4, N-dimethyl-benzamide;
2-(4-Chloro-3,5-dimethyl-phenoxy)-N-cyclohexyl-N-methyl-acetamide;
N-Benzyl-2-bromo-N-isopropyl-benzamide;
3-(2-Chloro-phenyl)-N-cyclohexyl-N-methyl-acrylamide;
N-Phenyl-N-(2,2,5-trimethyl-hex-4-enyl)-acetamide;
N-m-Tolyl-N-(2,2, 5-tri methyl-hex-4-enyl)-acetamide;
(3-Chloro-benzo[b]thiophen-2-yl)-(4-methyl-piperazin-1-yl)-methanone;
Adamantane-1-carboxylic acid (5-methyl-pyridin-2-yl)-amide;
3-Bromo-N-[2-methyl-1-(1,3,3-trimethyl-6-aza-bicyclo[3.2.1]octane-6-carbonyl)-butyl]-benzamide;
4-Chloro-N-[2-methyl-1-(1,3,3-trimethyl-6-aza-bicyclo[3.2.1]octane-6-carbonyl)-butyl]-benzamide;
4-Methyl-N-[2-methyl-1-(1,3,3-trimethyl-6-aza-bicyclo[3.2.1]octane-6-carbonyl)-butyl]-benzamide;
Cyclohexanecarboxylic acid [2-methyl-1-(1,3,3-trimethyl-6-aza-bicyclo[3.2.1]octane-6-carbonyl)-butyl]-amide;
3-Cyclopentyl-N-[2-methyl-1-(1,3,3-trimethyl-6-aza-bicyclo[3.2.1]octane-6-carbonyl)-butyl]-propionamide;
2-Chloro-N-[2-(4-ethyl-benzoylamino)-ethyl]-N-(4-fluoro-phenyl)-benzamide;
N-{1-Benzyl-2-[4-(3-cyclopentyl-propionyl)-piperazin-1-yl]-2-oxo-ethyl}-3-cyclopentyl-propionamide;
N-Bicyclo[2.2.1]hept-5-en-2-ylmethyl-3-cyclopentyl-N-[2-(1H-indol-3-yl)-ethyl]-propionamide;
N-Bicyclo[2.2.1]hept-5-en-2-ylmethyl-2,4-dichloro-N-[2-(1H-indol-3-yl)-ethyl]-benzamide;
Naphthalene-2-carboxylic acid (2-oxo-azepan-3-yl)-thiophen-3-ylmethyl-amide;
3,4,5-Trimethoxy-N-(4-methyl-benzyl)-N-[6-(pyridin-2-ylamino)-hexyl]-benzamide;
3-Cyclopentyl-N-(4-methyl-benzyl)-N-[6-(pyridin-2-ylamino)-hexyl]-propionamide;
N-(3,4-Dimethoxy-benzyl)-3-methoxy-N-[6-(pyridin-2-ylamino)-hexyl]-benzamide;
N, N-Dimethyl-2-[3-(4-n itro-phenyl)-adamantan-1-yl]-acetamide;
Adamantane-1-carboxylic acid [4-(1,3-dioxo-1,3-dihydro-isoindol-2-yl)-butyl]-p-tolyl-amide;
2-(1,3-Dioxo-1,3-dihydro-isoindol-2-yl)-3-methyl-N-(2-trifluoromethyl-phenyl)-butyramide;
2-(4-Chloro-2-methyl-phenoxy)-N-(2-trifluoromethyl-phenyl)-propionamide;
4-(2,4-Dichloro-phenoxy)-1-[4-(4-fluoro-phenyl)-piperazin-1-yl]-butan-1-one;
(3,4-Dihydro-2H-quinolin-1-yl)-[3-(piperidine-1-sulfonyl)-phenyl]-methanone;
Acetic acid 4-(azepane-1-carbonyl)-phenyl ester;
N-Adamantan-1-ylmethyl-benzamide;
[3-(4-Nitro-phenyl)-adamantan-1-yl]-piperidin-1-yl-methanone;
N-(1,1-Dimethyl-hexyl)-2-morpholin-4-yl-acetamide;
Adamantyl-1-carboxylic acid (2-methoxy-ethyl)-amide;
N-(4-Adamantan-1-yl-2-methyl-phenyl)-acetamide;
3-p-Tolyl-adamantane-1-carboxylic acid (2,5-dichloro-phenyl)-amide;
(3-Chloro-adamantan-1-yl)-pyrrolidin-1-yl-methanone;
2-Amino-5-cyclohexylcarbamoyl-4-methyl-thiophene-3-carboxylic acid ethyl ester;
N-(2-Chloro-phenyl)-2-{3-[(2-chloro-phenylcarbamoyl)-methyl]-adamantan-1-yl}-acetamide;
3-p-Tolyl-adamantane-1-carboxylic acid (2,4-difluoro-phenyl)-amide;
Adamantyl-1-carboxylic acid tert-butylamide;
2-Adamantan-1-yl-N-tert-butyl-acetamide;
N-Methyl-N-phenyl-4-(pyrrolidine-1-sulfonyl)-benzamide;
N-(1-Adamantan-1-yl-ethyl)-2-fluoro-benzamide;
Adamantane-1-carboxylic acid [2-(3,4-dimethoxy-phenyl)-ethyl]-amide;
Adamantane-1-carboxylic acid dimethylamide;
N-Benzyl-4-chloro-N-(1-cyclopropyl-vinyl)-benzamide;
3,5-Dimethyl-adamantane-1-carboxylic acid benzylamide;
2,4-Dichloro-N-cyclohexyl-N-(2-hydroxy-ethyl)-benzamide;
N-Adamantan-1-yl-2,4-dichloro-N-ethyl-benzamide;
2-[(3-p-Tolyl-adamantane-1-carbonyl)-amino]-propionic acid methyl ester; N-Adamantan-1-yl-3-morpholin-4-yl-propionamide;
3-p-Tolyl-adamantane-1-carboxylic acid isopropylamide;
N-Adamantan-1-yl-2-benzylamino-acetamide;
N-Benzyl-2,4-dichloro-N-(1-cyclopropyl-ethyl)-5-methyl-benzamide;
2-[(Adamantane-1-carbonyl)-amino]-benzoic acid ethyl ester;
N-Benzyl-N-isopropyl-4-methyl-3-nitro-benzamide;
(3,4-Dihydro-2H-quinolin-1-yl)-(2-fluoro-phenyl)-methanone;
N-Ethyl-2-fluoro-N-phenyl-benzamide;
2-Phenethyl-N-(2-trifluoromethyl-phenyl)-benzamide;
1-(3,4-Dihydro-2H-quinolin-1-yl)-2-o-tolyloxy-ethanone;
2-(1-Benzyl-1H-imidazol-2-ylsulfanyl)-N-cyclohexyl-acetamide;
Cyclohexanecarboxylic acid (2-propoxy-phenyl)-amide;
2-{3-[4-(2-Chloro-phenyl)-piperazin-1-yl]-3-oxo-propyl}-isoindole-1,3-dione;
N-Cyclopentyl-2-(2,4-dichloro-phenoxy)-propionamide;
Adamantane-1-carboxylic acid (2-trifluoromethyl-phenyl)-amide;
(4-Chloro-3-nitro-phenyl)-(2,6-dimethyl-piperidin-1-yl)-methanone;
4-(2-Ethyl-phenyl)-4-aza-tricyclo[5.2.2.0^{2,6}]undec-8-ene-3,5-dione;
2-Phenyl-N-(2-trifluoromethyl-phenyl)-butyramide;
N-Adamantan-1-yl-4-chloro-2-nitro-benzamide;
3-p-Tolyl-adamantane-1-carboxylic acid (2,3-dimethyl-phenyl)-amide;
N-Benzyl-3-methyl-4-p-tolyl-butyramide;
N-(2-Cyclohex-1-enyl-ethyl)-2-(1,3-dioxo-1,3-dihydro-isoindol-2-yl)-propionamide;
(4-tert-Butyl-phenyl)-(3,4-dihydro-1H-isoquinolin-2-yl)-methanone;
2-[1-(4-Chloro-benzenesulfonyl)-1H-benzoimidazol-2-ylsulfanyl]-N-thiophen-2-ylmethyl-acetamide;
2-Phenoxy-1-[4-(2-trifluoromethyl-benzyl)-piperazin-1-yl]-ethanone;
Cyclohexanecarboxylic acid [5-(2-fluoro-benzylsulfanylmethyl)-[1,3,4]thiadiazol-2-yl]-amide;
4-Methyl-2-phenyl-thiazole-5-carboxylic acid naphthalen-1-ylamide;
4-Fluoro-N-[4-(1,3,3-trimethyl-6-aza-bicyclo[3.2.1]octane-6-carbonyl)-phenyl]-benzenesulfonamide;
(3-Methoxy-phenyl)-(4-o-tolyl-piperazin-1-yl)-methanone;
N-Adamantan-1-yl-3-(1,3-dioxo-1,3-dihydro-isoindol-2-yl)-propionamide;
N-Cyclooctyl-2-methoxy-3-methyl-benzamide;
2-[4-(2,3,3-Trimethyl-6-aza-bicyclo[3.2.1]octane-6-carbonyl)-phenyl]-isoindole-1,3-dione;
(2,3-Diphenyl-quinoxalin-6-yl)-(2,3,3-trimethyl-6-aza-bicyclo[3.2.1]oct-6-yl)-methanone;
Adamantan-1-yl-(1,3,4,5-tetrahydro-pyrido[4,3-b]indol-2-yl)-methanone;
N-{4-[1-(Naphthalene-2-sulfonyl)-piperidin-3-yl]-butyl}-N'-p-tolyl-oxalamide;
N-Benzyl-N-(2-oxo-2-pyrrolidin-1-yl-ethyl)-benzenesulfonamide;
(4-Amino-phenyl)-(1,3,3-trimethyl-6-aza-bicyclo[3.2.1]oct-6-yl)-methanone;
1-[4-(4-Fluoro-phenyl)-piperazin-1-yl]-2-(2-isopropyl-5-methyl-phenoxy)-ethanone;
Adamantane-1-carboxylic acid benzyl-pyridin-2-yl-amide;
Adamantan-1-yl-piperidin-1-yl-methanone;
Adamantan-1-yl-pyrrolidin-1-yl-methanone;
(3,4-Dihydro-2H-quinolin-1-yl)-o-tolyl-methanone;
Adamantyl-1-carboxylic acid benzylamide;
Pyridine-2-carboxylic acid adamantan-2-ylamide;
(3-Chloro-adamantan-1-yl)-piperidin-1-yl-methanone;
Adamantan-1-yl-(4-methyl-piperidin-1-yl)-methanone;
2-[3-(Azepane-1-carbonyl)-phenyl]-isoindole-1,3-dione;
2-[3-(Piperidine-1-carbonyl)-phenyl]-isoindole-1,3-dione;
4-(Benzyl-methanesulfonyl-amino)-N-furan-2-ylmethyl-benzamide;
(4-Nitro-phenyl)-(1,3,3-trimethyl-6-aza-bicyclo[3.2.1]oct-6-yl)-methanone;
1-Cyclohexyl-5-oxo-pyrrolidine-3-carboxylic acid (4-chloro-3-nitro-phenyl)-amide;
N-(2-Chloro-phenyl)-2-(2-oxo-2-phenyl-ethylsulfanyl)-acetamide;
3-(4-Hydroxy-phenyl)-N-isochroman-1-ylmethyl-3-phenyl-propionamide;
(4-Ethoxy-phenyl)-(2-methyl-piperidin-1-yl)-methanone;
1-Cyclohexyl-5-oxo-pyrrolidine-3-carboxylic acid (3-chloro-phenyl)-amide;
N-[4-(Benzyl-isopropyl-sulfamoyl)-phenyl]-acetamide;
N-(3,4-Dimethyl-phenyl)-N-[4-(piperidine-1-carbonyl)-benzyl]-methanesulfonamide;
2-(5-Phenyl-1H-imidazol-2-ylsulfanyl)-N-(1,1,3,3-tetramethyl-butyl)-acetamide;
2-(Benzothiazol-2-ylsulfanyl)-N-(1,1,3,3-tetramethyl-butyl)-acetamide;
2-(Benzooxazol-2-ylsulfanyl)-N-(1,1,3,3-tetramethyl-butyl)-acetamide;
2-(Naphthalen-2-ylcarbamoylmethylsulfanyl)-N-(1,1,3,3-tetramethyl-butyl)-acetamide;
Acetic acid 4-(3,5-dimethyl-piperidine-1-carbonyl)-phenyl ester;
(4-Chloro-3-nitro-phenyl)-(2,6-dimethyl-piperidin-1-yl)-methanone;
[1-(4-Chloro-benzenesulfonyl)-piperidin-3-yl]-(octahydro-quinolin-1-yl)-methanone;
2,4-Dichloro-N-cyclohexyl-N-(2-hydroxy-ethyl)-benzamide;
(4-Fluoro-phenyl)-(3,4,4a,8a-tetrahydro-2H-quinolin-1-yl)-methanone;
N-Adamantan-1-yl-2-ethoxy-acetamide;
2-(2-Oxo-2-phenothiazin-10-yl-ethyl)-hexahydro-isoindole-1,3-dione;
Adamantane-1-carboxylic acid (tetrahydro-furan-2-ylmethyl)-amide;
2-Bromo-N-cycloheptyl-benzamide;
Bicyclo[2.2.1]hept-2-yl-[4-(2-ethoxy-phenyl)-piperazin-1-yl]-methanone;
N-Furan-2-ylmethyl-2-phenyl-2-phenylsulfanyl-acetamide;
Adamantane-1-carboxylic acid benzyl-methyl-amide;
1-(3,4-Dihydro-2H-quinolin-1-yl)-3-(4-fluoro-phenyl)-propenone;
Adamantan-1-yl-(2,6-dimethyl-piperidin-1-yl)-methanone;
4-Methyl-N-homoadamantyl-3-yl-benzamide;
(3,5-Dimethyl-piperidin-1-yl)-(3-methyl-4-nitro-phenyl)-methanone;
Quinoline-2-carboxylic acid cyclooctylamide;
Adamantane-1-carboxylic acid [2-(2,4-dimethoxy-phenyl)-ethyl]-amide;
(3,4-Dihydro-1 H-isoquinolin-2-yl)-o-tolyl-methanone;
(3,6-Dichloro-benzo[b]thiophen-2-yl)-(4-methyl-piperazin-1-yl)-methanone;
3-(1-Benzyl-1 H-imidazol-2-ylsulfanyl)-N-cyclohexyl-propionamide;
Propionic acid 2-amino-4-methyl-5-p-tolylcarbamoyl-thiophen-3-yl ester;
2-Cyclohexyl-N-(2,6-dimethyl-phenyl)-N-furan-2-ylmethyl-acetamide;
(3-Methoxy-phenyl)-(2,2,4-trimethyl-4-phenyl-3,4-dihydro-2H-quinolin-1-yl)-methanone;
1-[4-(2,3,3-Trimethyl-6-aza-bicyclo[3.2.1]octane-6-carbonyl)-phenyl]-pyrrolidine-2,5-dione;
1-(3,4-Dihydro-2H-quinolin-1-yl)-2-(1-naphthalen-1-yl-1H-tetrazol-5-ylsulfanyl)-ethanone;
[4-(2,3-Dimethyl-phenyl)-piperazin-1-yl]-o-tolyl-methanone;
(4-Benzyl-piperidin-1-yl)-(4-methyl-3-nitro-phenyl)-methanone;
N-(2-Cyano-phenyl)-2-(9-ethyl-9H-1,3,4,9-tetraaza-fluoren-2-ylsulfanyl)-acetamide;
N-(2-Cyano-phenyl)-2-(9-methyl-9H-1,3,4,9-tetraaza-fluoren-2-ylsulfanyl)-acetamide;
1-(Thiophene-2-carbonyl)-2,3-dihydro-1H-quinolin-4-one;
(3-Chloro-6-nitro-benzo[b]thiophen-2-yl)-piperidin-1-yl-methanone;
(4-Bromo-phenyl)-(3,5-dimethyl-piperidin-1-yl)-methanone;
2-Morpholin-4-yl-N-(1-phenyl-cyclopentylmethyl)-acetamide;
9-Oxo-9H-fluorene-1-carboxylic acid (3-methyl-butyl)-amide;
[4-(2,5-Dimethyl-pyrrol-1-yl)-phenyl]-(4-methyl-piperidin-1-yl)-methanone;
N-Cycloheptyl-3-diethylsulfamoyl-benzamide;
(4-Methoxy-phenyl)-(3-phenyl-piperidin-1-yl)-methanone;
3-Amino-N-cyclohexyl-N-methyl-benzamide;
N-Ethyl-3,4-dimethyl-N-phenyl-benzamide;
N-Benzyl-3,4, N-trimethyl-benzamide;
(4-Fluoro-phenyl)-(3-phenyl-piperidin-1-yl)-methanone;
[4-(2,3-Dimethyl-phenyl)-piperazin-1-yl]-(3-methoxy-phenyl)-methanone;
Furan-2-carboxylic acid [4-(4-methyl-piperidine-1-sulfonyl)-phenyl]-amide;
N-(2-Cyclohex-1-enyl-ethyl)-2-o-tolyloxy-acetamide;
5-(2-Chloro-phenoxymethyl)-furan-2-carboxylic acid (1-bicyclo[2.2.1]hept-2-yl-ethyl)-amide;
3-(2-Chloro-phenyl)-1-[4-(2,3-dimethyl-phenyl)-piperazin-1-yl]-propenone;
N-[3-(Azepane-1-carbonyl)-phenyl]-benzamide;
[3-(Piperidine-1-carbonyl)-pyrazol-1-yl]-o-tolyl-methanone;
N-(1-Phenyl-cyclopentylmethyl)-2-piperidin-1-yl-propionamide;
2-Morpholin-4-yl-N-(1-phenyl-cyclopentylmethyl)-propionamide;
N-[4-(Azepane-1-sulfonyl)-phenyl]-2,2,2-trifluoro-acetamide;
2,3-Dihydro-benzo[1,4]dioxine-6-carboxylic acid (1-adamantan-1-yl-ethyl)-amide;
N-Adamantan-1-yl-2-(3-methoxy-phenoxy)-acetamide;
3-Chloro-benzo[b]thiophene-2-carboxylic acid (2-cyano-ethyl)-phenyl-amide;
[4-(4-Nitro-benzyl)-piperidin-1-yl]-phenyl-methanone;
[4-(2-Nitro-benzyl)-piperidin-1-yl]-phenyl-methanone;
3-[5-(4-Fluoro-phenyl)-furan-2-yl]-1-(1,3,3-trimethyl-6-aza-bicyclo[3.2.1]oct-6-yl)-propenone;
2-(3-Fluoro-benzoylamino)-4-methyl-5-(piperidine-1-carbonyl)-thiophene-3-carboxylic acid methyl ester;
N-(2-Ethyl-phenyl)-2-(3-methyl-piperidin-1-yl)-acetamide;
2-(2-Methoxy-benzoylamino)-4-methyl-5-(piperidine-1-carbonyl)-thiophene-3-carboxylic acid methyl ester;
1-Phenyl-cyclopentanecarboxylic acid (4-phenyl-tetrahydro-pyran-4-ylmethyl)-amide;
4-(2,4-Dichloro-phenoxy)-1-(4-phenyl-piperazin-1-yl)-butan-1-one;
Naphthalene-1-carboxylic acid cycloheptylamide;
N-Indan-5-yl-2-methyl-3-nitro-benzamide;
N-Cyclohexyl-3-(2,2,2-trifluoro-ethoxymethyl)-benzamide;
2-Methoxy-N-(1-phenyl-cyclopentylmethyl)-benzamide;
[5-(2,5-Dichloro-phenoxymethyl)-furan-2-yl]-(2,6-dimethyl-morpholin-4-yl)-methanone;
[5-(2-Bromo-phenoxymethyl)-furan-2-yl]-(2-methyl-piperidin-1-yl)-methanone;
5-(2-Methoxy-phenoxymethyl)-furan-2-carboxylic acid cycloheptylamide;
(3,4-Dihydro-1H-isoquinolin-2-yl)-[1-(2-nitro-benzenesulfonyl)-piperidin-3-yl]-methanone;
N-Cyclooctyl-2-(4-methoxy-phenoxy)-acetamide;
N-(2,3-Dimethyl-phenyl)-4-[methyl-(toluene-4-sulfonyl)-amino]-butyramide;
N-Phenyl-N-[4-(piperidine-1-carbonyl)-benzyl]-benzenesulfonamide;
N-[4-(3,4-Dihydro-1 H-isoquinoline-2-carbonyl)-benzyl]-N-(3,4-dimethyl-phenyl)-methanesulfonamide;
2,3-Dihydro-benzo[1,4]dioxine-2-carboxylic acid bicyclo[2.2.1]hept-2-ylamide; 4,5,6,7-Tetrahydro-benzo[b]thiophene-3-carboxylic acid cycloheptylamide;
N-(2-Azepan-1-yl-2-oxo-ethyl)-N-benzyl-4-fluoro-benzenesulfonamide;
1-(2,6-Dimethyl-morpholin-4-yl)-3,3-diphenyl-propan-1-one;
N-Bicyclo[2.2.1]hept-2-yl-4-morpholin-4-ylmethyl-benzamide;
[3-(2-Chloro-6-nitro-phenoxy)-phenyl]-piperidin-1-yl-methanone;
N-Adamantan-1-yl-2-(4-methyl-quinolin-2-ylsulfanyl)-acetamide;
Cyclohexanecarboxylic acid (2-phenylsulfanyl-phenyl)-amide;
(4-Hydroxy-4-phenyl-octahydro-quinolin-1-yl)-phenyl-methanone;
3-Cyclohexyl-N-(3-phenyl-propyl)-propionamide;
2-[1-(2,5-Dimethyl-phenyl)-1H-tetrazol-5-ylsulfanyl]-N-isopropyl-N-phenyl-acetamide;
N-{2-[4-(3,4-Dihydro-1H-isoquinoline-2-sulfonyl)-phenyl]-ethyl}-acetamide;
N-Benzyl-N-[2-oxo-2-(4-phenyl-piperazin-1-yl)-ethyl]-benzenesulfonamide;
[4-(2-Chloro-6-nitro-phenoxy)-phenyl]-piperidin-1-yl-methanone;
N-Cycloheptyl-3-phenyl-propionamide;
(3-Chloro-6-methyl-benzo[b]thiophen-2-yl)-piperidin-1-yl-methanone;
N-Cycloheptyl-2,4-dimethoxy-benzamide;
N-(3-Chloro-phenyl)-2-(8,11,11-trimethyl-3,4,6-triaza-tricyclo[6.2.1.0^{2,7}]undeca-2(7),3,5-trien-5-ylsulfanyl)-acetamide;
N-(2-Nitro-phenyl)-2-(8,11,11-trimethyl-3,4,6-triaza-tricyclo[6.2.1.0^{2,7}]undeca-2(7),3,5-trien-5-ylsulfanyl)-acetamide;
N-Phenyl-2-(8,11,11-trimethyl-3,4,6-triaza-tricyclo[6.2.1.0^{2,7}]undeca-2(7),3,5-trien-5-ylsulfanyl)-acetamide;
N-Ethyl-3-methyl-N-o-tolyl-benzamide;
N-[5-(2,4-Dichloro-benzylsulfanyl)-[1,3,4]thiadiazol-2-yl]-2,2-dimethyl-propionamide;
4-Fluoro-N-[4-(1,3,3-trimethyl-6-aza-bicyclo[3.2.1]octane-6-carbonyl)-phenyl]-benzenesulfonamide;
4-Bromo-1-ethyl-1H-pyrazole-3-carboxylic acid (2-methylsulfanyl-phenyl)-amide; 5-Benzoyl-furan-2-carboxylic acid diisopropylamide;
2-[3-(Piperidine-1-carbonyl)-phenyl]-isoindole-1,3-dione;
N-{2-[2-(4-Bromo-phenyl)-2-oxo-ethylsulfanyl]-benzothiazol-6-yl}-acetamide;
2-(6-Amino-benzothiazol-2-ylsulfanyl)-N-cyclohexyl-acetamide;
N-(2-Cyclohexylcarbamoylmethylsulfanyl-benzothiazol-6-yl)-2-methoxy-benzamide;
Benzofuran-2-yl-(4-phenyl-piperidin-1-yl)-methanone;
1-(2-Nitro-phenyl)-piperidine-3-carboxylic acid diethylamide;
1-(4-Nitro-phenyl)-piperidine-3-carboxylic acid diethylamide;
5-Bromo-furan-2-carboxylic acid adamantan-2-ylamide;
3,3-Dimethyl-pentanedioic acid bis-[(2,4-difluoro-phenyl)-amide];
2-(3-Bromo-benzylsulfanyl)-1-(4-phenyl-piperazin-1-yl)-ethanone;
N-(2-Azepan-1-yl-2-oxo-ethyl)-N-benzyl-4-bromo-benzenesulfonamide;
1-(2,3-Dihydro-indol-1-yl)-2-p-tolylsulfanyl-ethanone;
[4-(4-Bromo-benzenesulfonyl)-piperazin-1-yl]-furan-2-yl-methanone;
[5-(2-Bromo-phenoxymethyl)-furan-2-yl]-(2,6-dimethyl-morpholin-4-yl)-methanone;
5-(2-Chloro-phenoxymethyl)-furan-2-carboxylic acid diethylamide; 5-(2-Bromo-phenoxymethyl)-furan-2-carboxylic acid diethylamide; 5-(2-Chloro-phenoxymethyl)-furan-2-carboxylic acid methyl-phenyl-amide;
[5-(2-Chloro-phenoxymethyl)-furan-2-yl]-(4-methyl-piperidin-1-yl)-methanone;
[3-(2,5-Dichloro-phenoxymethyl)-phenyl]-pyrrolidin-1-yl-methanone;
[5-(4-Ethoxy-phenoxymethyl)-furan-2-yl]-(4-methyl-piperidin-1-yl)-methanone;
3-(1,3-Dioxo-1,3-dihydro-isoindol-2-yl)-N-(2-methyl-cyclohexyl)-propionamide;
3-(3,4-Dihydro-2H-quinoline-1-carbonyl)-N-phenyl-benzenesulfonamide;
[3-(2,3-Dihydro-indole-1-sulfonyl)-phenyl]-(3,4-dihydro-2H-quinolin-1-yl)-methanone;
[3-(2,5-Dimethyl-pyrrol-1-yl)-phenyl]-(4-methyl-piperidin-1-yl)-methanone;
N-Cyclohexyl-3-(2-hydroxy-4-methyl-phenyl)-3-phenyl-propionamide;
2-Diethylamino-N-(1-phenyl-cyclopentylmethyl)-propionamide;
(6-Fluoro-2-methyl-3,4-dihydro-2H-quinolin-1-yl)-(3-trifluoromethyl-phenyl)-methanone;
(2,6-Dimethyl-morpholin-4-yl)-[4-(naphthalen-1-yloxymethyl)-phenyl]-methanone;
N-Benzyl-4-bromo-N-ethyl-benzamide;
(3-Methyl-piperidin-1-yl)-[4-(naphthalen-1-yloxymethyl)-phenyl]-methanone;
Azepan-1-yl-[5-(2-chloro-phenoxymethyl)-furan-2-yl]-methanone;
(4-Methyl-piperidin-1-yl)-[4-(naphthalen-1-yloxymethyl)-phenyl]-methanone;
Azepan-1-yl-[5-(2,4-dichloro-phenoxymethyl)-furan-2-yl]-methanone;
N-Cycloheptyl-4-(4-methoxy-2-methyl-phenyl)-butyramide;
2-(4-Benzothiazol-2-yl-piperazin-1-yl)-N-cyclohexyl-acetamide;
N-Cycloheptyl-2-(2,6-dimethyl-phenoxy)-acetamide;
(3,4-Dihydro-2H-quinolin-1-yl)-(3-iodo-phenyl)-methanone;
N-Cycloheptyl-3-(2,2,2-trifluoro-ethoxymethyl)-benzamide;
Azepan-1-yl-[4-(2-chloro-phenoxymethyl)-phenyl]-methanone;
(2,6-Dimethyl-morpholin-4-yl)-[4-(naphthalen-2-yloxymethyl)-phenyl]-methanone;
Azepan-1-yl-[3-(4-ethoxy-phenoxymethyl)-phenyl]-methanone;
Benzo[b]thiophene-3-carboxylic acid (1,2,3,4-tetrahydro-naphthalen-1-yl)-amide;
2-(4-Chloro-2-methyl-phenoxy)-N-cycloheptyl-acetamide;
2,4-Dichloro-N-cyclohexyl-N-methyl-benzamide;
N-Cyclooctyl-2-p-tolyloxy-acetamide;
(3,5-Dimethyl-piperidin-1-yl)-(4-methyl-3-nitro-phenyl)-methanone;
Biphenyl-4-yl-(2,6-dimethyl-piperidin-1-yl)-methanone;
N-Cyclohexyl-4-fluoro-N-methyl-benzamide;
N-[4-(Azepane-1-carbonyl)-phenyl]-N-methyl-benzenesulfonamide;
N-Cycloheptyl-2-fluoro-benzamide;
N-Cycloheptyl-4-methyl-benzamide;
(3-Methyl-piperidin-1-yl)-p-tolyl-methanone;
[2-(3,4-Dimethoxy-phenylcarbamoyl)-piperidin-1-yl]-acetic acid benzyl ester;
N-[4-(2-Methyl-piperidine-1-sulfonyl)-phenyl]-acetamide;
2-(2,4-Dichloro-phenoxy)-N-(2-methyl-butyl)-propionamide;
[4-(2-Chloro-6-nitro-phenyl)-piperazin-1-yl]-(4-methoxy-phenyl)-methanone;
N-Cyclohexyl-4-(4-methoxy-3-methyl-phenyl)-butyramide;
(3-Chloro-6-methoxy-benzo[b]thiophen-2-yl)-(3,4-dihydro-1 H-isoquinolin-2-yl)-methanone;
2-(4-Methyl-benzylsulfanyl)-1-piperidin-1-yl-ethanone;
N-Cyclohexyl-N-[(4-phenyl-thiazol-2-ylcarbamoyl)-methyl]-benzamide;
N-(2-Azepan-1-yl-2-oxo-ethyl)-N-(4-isopropyl-phenyl)-methanesulfonamide;
N-Adamantan-1-yl-3-p-tolylsulfanyl-propionamide;
6-(2,4-Dichloro-phenylcarbamoyl)-3,4-dimethyl-cyclohex-3-enecarboxylic acid;
(4-Butyl-cyclohexyl)-morpholin-4-yl-methanone;
(3,4-Dichloro-phenyl)-(3,4-dihydro-2H-quinolin-1-yl)-methanone;
N-(2-Cyclohex-1-enyl-ethyl)-3-methoxy-benzamide;
N-Adamantan-2-yl-3-(1,5-dimethyl-1H-pyrazol-4-yl)-acrylamide;
N-Adamantan-1-yl-N-methyl-4-(4-nitro-pyrazol-1-ylmethyl)-benzamide;
5-(4-Chloro-3,5-dimethyl-pyrazol-1-ylmethyl)-furan-2-carboxylic acid adamantan-2-ylamide;
2-(4-Chloro-phenoxy)-N-(2-fluoro-5-methyl-phenyl)-2-methyl-propionamide;
N-Adamantan-1-yl-2-(4-chloro-3,5-dimethyl-phenoxy)-acetamide;
2-[(3-Carboxy-bicyclo[2.2.1]heptane-2-carbonyl)-amino]-5,6-dihydro-4H-cyclopenta[b]thiophene-3-carboxylic acid propyl ester;
2-Adamantan-1-yl-N-[1-(2,5-dimethyl-phenyl)-ethyl]-acetamide;
3-Methyl-thiophene-2-carboxylic acid cyclooctylamide;
N-p-Tolyl-2-(8,11,11-trimethyl-3,4,6-triaza-tricyclo[6.2.1.0^{2,7}]undeca-2,4,6-trien-5-ylsulfanyl)-propionamide;
Azepan-1-yl-[5-(4-chloro-5-methyl-3-nitro-pyrazol-1-ylmethyl)-furan-2-yl]-methanone;
N-Ad a mantan-2-yl-3-(4-bromo-3-n itro-pyrazol-1-yl methyl)-benzam ide;
N-Bicyclo[2.2.1]hept-2-yl-2-chloro-benzamide;
[5-(3-Chloro-phenoxymethyl)-furan-2-yl]-piperidin-1-yl-methanone;
1-(4-Ethyl-benzoyl)-6-methoxy-2-methyl-2,3-dihydro-1H-quinolin-4-one;
6-Fluoro-2-methyl-1-{3-[4-(propane-1-sulfonyl)-phenoxymethyl]-benzoyl}-2,3-dihydro-1H-quinolin-4-one;
N-Cycloheptyl-2-(naphthalen-1-yloxy)-acetamide;
N-Cyclohexyl-4-o-tolyloxy-butyramide;
(2-Benzylsulfanyl-phenyl)-morpholin-4-yl-methanone;
(2-Chloro-5,6-difluoro-3-methyl-phenyl)-(4-methyl-piperidin-1-yl)-methanone;
(3-Bromo-phenyl)-[4-(4-chloro-2-nitro-phenyl)-piperazin-1-yl]-methanone;
2-Bromo-N-(1,1,3,3-tetramethyl-butyl)-benzamide;
N-Adamantan-1-yl-2-(2-benzoyl-5-methoxy-phenoxy)-acetamide;
N-Cyclohexyl-3-methyl-4-p-tolyl-butyramide;
[5-(4-Methyl-2-nitro-phenoxymethyl)-furan-2-yl]-thiomorpholin-4-yl-methanone;
[5-(2,5-Dichloro-phenoxymethyl)-furan-2-yl]-thiomorpholin-4-yl-methanone;
5-(4-Chloro-2-nitro-phenoxymethyl)-furan-2-carboxylic acid adamantan-1-ylamide; 4,5,6,7-Tetrahydro-benzo[b]thiophene-3-carboxylic acid cyclohexylamide;
4-Chloro-1,5-dimethyl-1H-pyrazole-3-carboxylic acid adamantan-1-yl-methyl-amide;
4-(4-Methoxy-3-methyl-phenyl)-N-(2-methyl-cyclohexyl)-butyramide;
3-Benzo[1,3]dioxol-5-yl-1-(3,4-dihydro-1 H-isoquinolin-2-yl)-propenone;
N-Bicyclo[2.2.1]hept-2-yl-3-phenylsulfanyl-propionamide;
Azepan-1-yl-[5-(2-nitro-phenoxymethyl)-furan-2-yl]-methanone;
N-Benzyl-2-(4-chloro-phenylsulfanyl)-N-methyl-acetamide;
1-(4-Benzyl-piperidin-1-yl)-2-benzylsulfanyl-ethanone;
2-(4-tert-Butyl-phenoxy)-1-(4-ethyl-piperazin-1-yl)-ethanone;
[4-(4-Ethoxy-phenoxymethyl)-phenyl]-(4-methyl-piperidin-1-yl)-methanone;
5-(4-Bromo-3,5-dimethyl-pyrazol-1-ylmethyl)-furan-2-carboxylic acid adamantan-2-ylamide;
1-Azepan-1-yl-3-(4-chloro-phenylsulfanyl)-propan-1-one;
N-Bicyclo[2.2.1]hept-2-yl-2-(2-chloro-benzylsulfanyl)-acetamide;
2-(2-Methyl-benzylsulfanyl)-1-(4-phenyl-piperazin-1-yl)-ethanone;
N-[2-(1-Benzo[1,3]dioxol-5-yl-3-furan-2-yl-3-oxo-propylsulfanyl)-phenyl]-acetamide;
(3,5-Dimethyl-piperidin-1-yl)-(3-iodo-phenyl)-methanone;
[5-(2-Bromo-phenoxymethyl)-furan-2-yl]-(6-fluoro-2-methyl-3,4-dihydro-2H-quinolin-1-yl)-methanone;
N-Benzyl-N-cyclohex-1-enyl-isonicotinamide;
1-[4-(4-Fluoro-phenyl)-piperazin-1-yl]-2-(2-methyl-benzylsulfanyl)-ethanone;
2-(2-Bromo-4-methyl-phenoxy)-N-(2-cyclohex-1-enyl-ethyl)-acetamide;
2-[5-(2-Hydroxy-phenyl)-[1,3,4]oxadiazol-2-ylsulfanyl]-1-piperidin-1-yl-ethanone;
5-(4-Nitro-pyrazol-1-ylmethyl)-furan-2-carboxylic acid adamantan-2-ylamide;
3-Benzo[1,3]dioxol-5-yl-3-(2-methoxy-phenyl)-1-pyrrolidin-1-yl-propan-1-one;
N-Adamantan-2-yl-3,4-dichloro-benzamide;
Benzo[b]thiophen-3-yl-(6-fluoro-2-methyl-3,4-dihydro-2H-quinolin-1-yl)-methanone;
2-Adamantan-1-yl-1-(3,4-dihydro-1 H-isoquinolin-2-yl)-ethanone;
4,5,6,7-Tetrahydro-benzo[b]thiophene-3-carboxylic acid (2-cyclohex-1-enyl-ethyl)-amide; Benzo[b]thiophene-3-carboxylic acid (3,3,5-trimethyl-cyclohexyl)-amide;
2-(2,6-Dimethyl-phenoxy)-N-(2-isopropyl-phenyl)-acetamide;
4-Bromo-N-[3-(piperidine-1-carbonyl)-phenyl]-benzamide;
N-Benzo[1,3]dioxol-5-ylmethyl-2-(2-cyano-phenylsulfanyl)-benzamide;
N-Adamantan-1-yl-2-(naphthalen-2-yloxy)-acetamide;
[4-(4-Chloro-phenylsulfanylmethyl)-phenyl]-morpholin-4-yl-methanone;
Thiophene-2-carboxylic acid (3,3,5-trimethyl-cyclohexyl)-amide;
Benzo[1,3]dioxol-5-yl-(3,4-dihydro-2H-quinolin-1-yl)-methanone;
3-Chloro-benzo[b]thiophene-2-carboxylic acid cyclooctylamide;
2-[2-Morpholin-4-yl-1-(4-nitro-benzyl)-2-oxo-ethyl]-isoindole-1,3-dione;
2-Hydroxy-4,4-dimethyl-6-oxo-cyclohex-1-enecarboxylic acid phenylamide;
(2,4-Dichloro-phenyl)-(2,6-dimethyl-piperidin-1-yl)-methanone;
Adamantane-1-carboxylic acid furan-2-ylmethyl-p-tolyl-amide;
Azocan-1-yl-(4-tert-butyl-phenyl)-methanone;
3-Chloro-benzo[b]thiophene-2-carboxylic acid benzyl-methyl-amide;
Adamantane-1-carboxylic acid (2-fluoro-phenyl)-amide;
2-(Piperidine-1-carbonyl)-5-piperidin-1-yl-oxazole-4-carbonitrile;
N-(4,6-Dimethyl-5-nitro-pyridin-3-yl)-benzamide;
Adamantan-1-yl-[4-(2-methoxy-phenyl)-piperazin-1-yl]-methanone;
(2-Methyl-piperidin-1-yl)-o-tolyl-methanone;
N-Benzyl-4-chloro-N-isopropyl-3-nitro-benzamide;
N-(3-Hexylsulfanyl-[1,2,4]thiadiazol-5-yl)-3-methyl-butyramide;
4,N-Dimethyl-N-[4-(piperidine-1-carbonyl)-phenyl]-benzenesulfonamide;
Azepan-1-yl-(5-tert-butyl-2H-pyrazol-3-yl)-methanone;
2-Amino-4-methyl-5-(piperidine-1-carbonyl)-thiophene-3-carboxylic acid ethyl ester; 5-Methyl-furan-2-carboxylic acid (1-adamantan-1-yl-ethyl)-amide;
(3-Chloro-6-methyl-benzo[b]thiophen-2-yl)-(3,4-dihydro-1H-isoquinolin-2-yl)-methanone;
N-Adamantan-1-yl-2-trifluoromethyl-benzamide;
(3-Bromo-phenyl)-(2,2,4-trimethyl-4-phenyl-3,4-dihydro-2H-quinolin-1-yl)-methanone;
Benzo[1,3]dioxole-5-carboxylic acid dipropylamide;
N-(3,3-Diphenyl-propyl)-4-methoxy-benzamide;
[4-(2-Chloro-6-nitro-phenyl)-piperazin-1-yl]-p-tolyl-methanone;
Furan-2-yl-[4-(toluene-4-sulfonyl)-piperazin-1-yl]-methanone;
3-(2-Chloro-6-fluoro-phenyl)-1-(3,4-dihydro-2H-quinolin-1-yl)-propenone;
2-Chloro-N-cycloheptyl-benzamide;
1-[4-(4-Nitro-phenyl)-piperazin-1-yl]-3-phenyl-propan-1-one;
(3,4-Dihydro-1 H-isoquinolin-2-yl)-(3,4-dimethyl-phenyl)-methanone;
(1-Adamantan-1-yl-4-bromo-1 H-pyrazol-3-yl)-morpholin-4-yl-methanone;
2-Phenyl-cyclopropanecarboxylic acid cyclooctylamide;
3-[4-(2-Ethoxy-phenyl)-piperazine-1-carbonyl]-isochromen-1-one;
[3-(4-Bromo-pyrazol-1-ylmethyl)-phenyl]-(4-methyl-piperidin-1-yl)-methanone;
2-Azepan-1-yl-N-biphenyl-2-yl-acetamide;
N-[5-(3,4-Dimethoxy-benzyl)-[1,3,4]thiadiazol-2-yl]-3-methyl-butyramide;
Adamantan-1-yl-(4-phenyl-piperidin-1-yl)-methanone;
N-(2-Azepan-1-yl-2-oxo-ethyl)-N-(4-ethoxy-phenyl)-4-methylsulfanyl-benzenesulfonamide;
1-Adamantan-1-yl-4-bromo-1H-pyrazole-3-carboxylic acid diethylamide;
(6-Fluoro-2-methyl-3,4-dihydro-2H-quinolin-1-yl)-(2-fluoro-phenyl)-methanone;
3-[4-(2,3-Dimethyl-phenyl)-piperazine-1-carbonyl]-isochromen-1-one;
N-Cyclooctyl-2-(2-methoxy-phenoxy)-acetamide;
N-Cyclohexyl-N-methyl-2-nitro-benzamide;
Adamantane-1-carboxylic acid (1,1-dioxo-tetrahydro-thiophen-3-yl)-amide;
N-Adamantan-2-yl-2-(4-chloro-phenyl)-acetamide;
(2,4-Dichloro-phenyl)-(3-methyl-piperidin-1-yl)-methanone;
2-(4-tert-Butyl-phenoxy)-N-cyclooctyl-acetamide;
(4-Hydroxy-4-phenyl-octahydro-quinolin-1-yl)-phenyl-methanone;
(2-Chloro-phenyl)-(2-methyl-piperidin-1-yl)-methanone;
(10,11-Dihydro-dibenzo[b,f]azepin-5-yl)-(2-methoxy-phenyl)-methanone;
(3-Chloro-phenyl)-(2-methyl-piperidin-1-yl)-methanone;
(3-Chloro-6-nitro-benzo[b]thiophen-2-yl)-(3-methyl-piperidin-1-yl)-methanone;
(2,5-Dichloro-phenyl)-(4-methyl-piperidin-1-yl)-methanone;
N-[5-(3,4-Dichloro-benzyl)-[1,3,4]thiadiazol-2-yl]-2,2-dimethyl-propionamide;
4-(4-Chloro-2-methyl-phenoxy)-1-(3,4-dihydro-2H-quinolin-1-yl)-butan-1-one;
(3,4-Dichloro-phenyl)-[4-(2,3-dimethyl-phenyl)-piperazin-1-yl]-methanone;
Cyclooctanecarboxylic acid [1-(naphthalene-2-sulfonyl)-pyrrolidin-2-yl]-amide;
1-Butyl-pyrrolidine-2-carboxylic acid benzo[1,3]dioxol-4-ylamide;
5-Methyl-furan-2-carboxylic acid dibenzylamide;
(3,4-Dihydro-2H-quinolin-1-yl)-[3-(4-phenyl-piperazine-1-sulfonyl)-phenyl]-methanone;
Bicyclo[2.2.1]hept-2-yl-[4-(2,3-dimethyl-phenyl)-piperazin-1-yl]-methanone;
N-Adamantan-1-yl-2-benzoyl-benzamide;
[5-(2-Chloro-phenoxymethyl)-furan-2-yl]-(3-methyl-piperidin-1-yl)-methanone;
(3,5-Dimethyl-piperidin-1-yl)-(2-iodo-phenyl)-methanone;
1-Benzenesulfonyl-pyrrolidine-2-carboxylic acid cyclooctylamide;
(3,4-Dimethoxy-phenyl)-(6-fluoro-2-methyl-3,4-dihydro-2H-quinolin-1-yl)-methanone;
3-(2,6-Dichloro-phenyl)-1-(2-ethyl-piperidin-1-yl)-propenone;
N-(3,4-Difluoro-phenyl)-2,6-difluoro-benzamide;
2,6-Difluoro-N-naphthalen-1-yl-benzamide;
(4-Chloro-phenyl)-(3,5-dimethyl-piperidin-1-yl)-methanone;
N-[4-(2,6-Dimethyl-piperidine-1-carbonyl)-phenyl]-2-(naphthalen-2-yloxy)-acetamide;
(2-Chloro-phenyl)-(3-methyl-piperidin-1-yl)-methanone;
N-{2-[4-(Piperidine-1-sulfonyl)-phenyl]-ethyl}-acetamide;
N-Biphenyl-2-yl-2-(pyridin-2-ylsulfanyl)-acetamide;
Azepan-1-yl-[5-(4-chloro-3,5-dimethyl-pyrazol-1-ylmethyl)-furan-2-yl]-methanone;
Acetic acid 4-(4-methyl-piperidine-1-carbonyl)-phenyl ester;
Acetic acid 4-(4-benzyl-piperidine-1-carbonyl)-phenyl ester;
Benzo[1,3]dioxole-5-carboxylic acid cycloheptylamide;
2-(2,4-Dimethyl-phenoxy)-1-(6-fluoro-2-methyl-3,4-dihydro-2H-quinolin-1-yl)-ethanone;
Acetic acid 4-(3,4-dihydro-2H-quinoline-1-carbonyl)-phenyl ester;
Azepan-1-yl-(3,5-dibromo-phenyl)-methanone;
(3,5-Dibromo-phenyl)-[4-(2-methoxy-phenyl)-piperazin-1-yl]-methanone;
N-Cyclooctyl-4-isopropyl-benzamide;
N-Cyclooctyl-2-(4-methoxy-phenyl)-acetamide;
(4-tert-Butyl-piperidin-1-yl)-phenyl-methanone;
N-(4-tert-Butyl-3-nitro-phenyl)-acetamide;
(2,6-Dimethyl-piperidin-1-yl)-[5-(2,3,5,6-tetrafluoro-phenoxymethyl)-furan-2-yl]-methanone;
2-(4-Chloro-3,5-dimethyl-phenoxy)-N-cyclohexyl-N-methyl-acetamide;
N-Cyclohexyl-3-(1,3-dioxo-1,3-dihydro-isoindol-2-yl)-N-methyl-propionamide;
2-(4-Chloro-3-methyl-phenoxy)-N-cyclohexyl-N-methyl-acetamide;
N-Cyclopentyl-3-(3,4-dihydro-2H-quinoline-1-carbonyl)-benzenesulfonamide;
(3,4-Dihydro-1 H-isoquinolin-2-yl)-(3-dimethylamino-phenyl)-methanone;
3-Cyclohexylcarbamoyl-bicyclo[2.2.1]hept-5-ene-2-carboxylic acid isopropyl ester;
1-(3,4-Dihydro-1 H-isoquinolin-2-yl)-2-(2-methoxy-phenyl)-ethanone;
N-Benzyl-N-cyclohex-1-enyl-benzamide;
[1-(Thiophene-2-sulfonyl)-piperidin-4-yl]-(1,3,3-trimethyl-6-aza-bicyclo[3.2.1]oct-6-yl)-methanone;
N-Adamantan-1-yl-2-(1-phenyl-1 H-tetrazol-5-ylsulfanyl)-acetamide;
(3,4-Dihydro-2H-quinolin-1-yl)-[4-(morpholine-4-sulfonyl)-phenyl]-methanone;
(3,4-Dihydro-2H-quinolin-1-yl)-[4-(pyrrolidine-1-sulfonyl)-phenyl]-methanone;
(3,4-Dihydro-2H-quinolin-1-yl)-[1-(thiophene-2-sulfonyl)-piperidin-4-yl]-methanone;
(1-Benzenesulfonyl-piperidin-3-yl)-(3,4-dihydro-1H-isoq u inolin-2-yl)-methanone;
6,7-Dimethyl-4-oxa-tricyclo[4.3.0.0^{3,7}]nonane-3-carboxylic acid cyclohexylamide;
6,7-Dimethyl-4-oxa-tricyclo[4.3.0.0^{3,7}]nonane-3-carboxylic acid (2-chloro-phenyl)-amide;
(6,7-Dimethyl-4-oxa-tricyclo[4.3.0.0^{3,7}]non-3-yl)-piperidin-1-yl-methanone;
2-[(Adamantane-1-carbonyl)-amino]-3-(1H-indol-3-yl)-propionic acid methyl ester;
2-(5,6-Dimethyl-4-oxo-3,4-dihydro-thieno[2,3-d]pyrimidin-2-ylsulfanyl)-N-furan-2-ylmethyl-acetamide;
N-Allyl-2-(5,6-dimethyl-4-oxo-3,4-dihydro-thieno[2,3-d]pyrimidin-2-ylsulfanyl)-acetamide;
N-Adamantan-1-yl-2-(5,6,7,8-tetrahydro-benzo[4,5]thieno[2,3-d]pyrimidin-4-ylsulfanyl)-acetamide;
1-(3,4-Dihydro-2H-quinoline-1-carbonyl)-4,7,7-trimethyl-2-oxa-bicyclo[2.2.1]heptan-3-one;
1-(3,4-Dihydro-1H-isoquinoline-2-carbonyl)-4,7,7-trimethyl-2-oxa-bicyclo[2.2.1]heptan-3-one;
Azepan-1-yl-(6,7-dimethyl-4-oxa-tricyclo[4.3.0.0^{3,7}]non-3-yl)-methanone;
2,5-Dimethyl-furan-3-carboxylic acid (1-adamantan-1-yl-ethyl)-amide;
1-Cyclohexyl-5-oxo-pyrrolidine-3-carboxylic acid (3-cyano-4,5,6,7-tetrahydro-benzo[b]thiophen-2-yl)-amide;
6-(2,4-Dichloro-phenylcarbamoyl)-3,4-dimethyl-cyclohex-3-enecarboxylic acid;
2-(2-Cyano-phenylsulfanyl)-N-cyclopentyl-benzamide;
[5-(2-Methoxy-4-propyl-phenoxymethyl)-furan-2-yl]-(3-methyl-piperidin-1-yl)-methanone;
(4-tert-Butyl-phenyl)-(3,5-dimethyl-piperidin-1-yl)-methanone;
[4-(2-Methoxy-naphthalen-1-ylmethyl)-piperazin-1-yl]-(4-methoxy-phenyl)-methanone;
(3,4-Dichloro-phenyl)-(3,5-dimethyl-piperidin-1-yl)-methanone;
(4-Ethoxy-phenyl)-(4-methyl-piperidin-1-yl)-methanone;
2-Phenethyl-N-(tetrahydro-furan-2-ylmethyl)-benzamide;
N-Cycloheptyl-2-phenoxy-benzamide;
Adamantane-1-carboxylic acid (2-ethoxy-phenyl)-amide;
N-Adamantan-2-yl-2-o-tolyloxy-acetamide;
(2-Chloro-phenyl)-(3,5-dimethyl-piperidin-1-yl)-methanone;
1-Morpholin-4-yl-2-[3-(4-nitro-phenyl)-adamantan-1-yl]-ethanone;
2-Dimethylamino-N-(2-nitro-phenyl)-benzamide;
N-Benzyl-2-(4,4,6-trimethyl-1-p-tolyl-1,4-dihydro-pyrimidin-2-ylsulfanyl)-acetamide;
[4-(3,5-Dinitro-phenoxy)-phenyl]-(2-ethyl-piperidin-1-yl)-methanone;
1-(4-Chloro-benzoyl)-2,3-dihydro-1H-benzo[g]quinolin-4-one;
2-[(Adamantane-1-carbonyl)-amino]-3-phenyl-propionic acid methyl ester;
[Benzyl-(4-nitro-benzoyl)-amino]-acetic acid ethyl ester;
9-Oxo-9H-fluorene-3-carboxylic acid methyl-(4-nitro-phenyl)-amide;
Adamantane-1-carboxylic acid [2-(4-methoxy-phenyl)-ethyl]-amide;
(10,11-Dihydro-dibenzo[b,f]azepin-5-yl)-(4-fluoro-phenyl)-methanone;
2-Benzylsulfanyl-N-[2-(2-methoxy-phenoxy)-ethyl]-acetamide;
N-Adamantan-1-yl-2-(2-oxo-4-phenyl-pyrrolidin-1-yl)-acetamide;
2-Bromo-N-tricyclo[3.2.1.0^{2,4}]oct-6-ylmethyl-benzamide;
Adamantane-1-carboxylic acid (2,6-dimethoxy-pyrimidin-4-yl)-amide;
Hexanedioic acid (2,7,7-trimethyl-bicyclo[2.2.1]hept-1-yl)-amide (1,7,7-trimethyl-bicyclo[2.2.1]hept-2-yl)-amide;
2-Chloro-N-(2-cyclohexyl-ethyl)-benzamide;
2-[3-(2-Ethyl-piperidin-1-yl)-3-oxo-propyl]-isoindole-1,3-dione;
N-Adamantan-1-yl-2-(2-oxo-4-phenyl-pyrrolidin-1-yl)-acetamide;
N-Adamantan-1-yl-2-hydroxy-2,2-diphenyl-acetamide;
Adamantane-1-carboxylic acid (naphthalen-1-ylmethyl)-amide;
Adamantane-1-carboxylic acid (benzo[1,3]dioxol-5-ylmethyl)-amide;
1-(Azepane-1-carbonyl)-fluoren-9-one;
2-(Quinolin-2-ylsulfanyl)-N-p-tolyl-acetamide;
2,4-Dichloro-N-[3-(piperidine-1-carbonyl)-phenyl]-benzamide;
2-Chloro-4,5-difluoro-N-(3,3,5-trimethyl-cyclohexyl)-benzamide;
2-(2-Chloro-benzylsulfanyl)-N-p-tolyl-acetamide;
[4-(4-Chloro-phenylsulfanylmethyl)-phenyl]-pyrrolidin-1-yl-methanone;
N-Adamantan-1-yl-N-methyl-isonicotinamide;
Azepan-1-yl-[4-(4-chloro-phenylsulfanylmethyl)-phenyl]-methanone;
(2-Chloro-phenyl)-(1,5,7-trimethyl-3,7-diaza-bicyclo[3.3.1]non-3-yl)-methanone;
(3-Chloro-benzo[b]thiophen-2-yl)-(4-methyl-piperidin-1-yl)-methanone;
Benzoic acid 1-benzoyl-decahydro-quinolin-4-yl ester;
2-(3-Bromo-benzylsulfanyl)-1-[4-(2-methoxy-phenyl)-piperazin-1-yl]-ethanone;
4-Methyl-N-[2-(phenoxazine-10-carbonyl)-phenyl]-benzenesulfonamide;
2-[1-(Azepane-1-carbonyl)-2-methyl-propyl]-isoindole-1,3-dione;
2-(3-Bromo-benzylsulfanyl)-1-piperidin-1-yl-ethanone;
1-[3-(4-Bromo-phenyl)-1-furan-2-yl-3-oxo-propyl]-pyrrolidin-2-one;
2-Chloro-N-cyclooctyl-4,5-difluoro-benzamide;
2,4-Dichloro-N-(2-furan-2-ylmethyl-cyclohexyl)-benzamide;
N-(4-Benzoyl-furazan-3-yl)-2-fluoro-benzamide;
N-Adamantan-1-yl-2-(3-cyano-4-methoxymethyl-6-methyl-pyridin-2-ylsulfanyl)-acetamide;
4-tert-Butyl-N-cyclooctyl-benzamide;
N-Adamantan-1-yl-2-phenyl-butyramide;
(3-Chloro-6-methoxy-benzo[b]thiophen-2-yl)-piperidin-1-yl-methanone;
(3,7-Dichloro-6-methoxy-benzo[b]thiophen-2-yl)-piperidin-1-yl-methanone;
Acetic acid 1-benzoyl-decahydro-quinolin-4-yl ester;
2-Bromo-N-methyl-N-phenyl-benzamide;
N-Benzo[1,3]dioxol-5-yl-2,4-dichloro-benzamide;
(3-Chloro-6-fluoro-benzo[b]thiophen-2-yl)-piperidin-1-yl-methanone;
N-(1,2,3,5,6,7-Hexahydro-s-indacen-1 -yl)-2-piperidin-1 -yl-acetamide;
2-[(Adamantane-1-carbonyl)-amino]-3-(1H-indol-3-yl)-propionic acid methyl ester;
2-[2-(1,3-Dioxo-1,3-dihydro-isoindol-2-yl)-3-phenyl-propionylamino]-3-methyl-butyric acid methyl ester;
2-(6-Oxo-6-piperidin-1-yl-hexyl)-isoindole-1,3-dione;
2-[2-(1,3-Dioxo-1,3-dihydro-isoindol-2-yl)-3-phenyl-propionylamino]-3-methyl-butyric acid methyl ester;
Adamantane-1-carboxylic acid (2,6-dihydroxy-pyrimidin-4-yl)-amide;
Adamantane-1-carboxylic acid [3-(1 H-benzoimidazol-2-ylsulfanyl)-5-nitro-phenyl]-amide;
Adamantane-1-carboxylic acid methyl-phenyl-amide;
3-Chloro-benzo[b]thiophene-2-carboxylic acid dibenzylamide;
N-Adamantan-1-yl-2-(3-cyano-6-methyl-4-trifluoromethyl-pyridin-2-ylsulfanyl)-acetamide;
2-(3-Oxo-3-phenyl-propenyl)-isoindole-1,3-dione;
Adamantane-1-carboxylic acid (4-ethoxy-benzothiazol-2-yl)-amide;
N-[5-(5-Chloro-benzooxazol-2-yl)-2-methyl-phenyl]-2-methoxy-benzamide;
N-[2-(2-Bromo-phenyl)-benzooxazol-5-yl]-2-methoxy-benzamide;
2-(4-Chloro-phenoxy)-N-(4-chloro-3-trifluoromethyl-phenyl)-acetamide;
2,2-Dimethyl-N-(5-propyl-[1,3,4]thiadiazol-2-yl)-propionamide;
2-[2-(2,6-Dimethyl-morpholin-4-yl)-1-methyl-2-oxo-ethyl]-isoindole-1,3-dione;
2-(2-Cyano-phenylsulfanyl)-N-(2-trifluoromethyl-phenyl)-benzamide;
Azepan-1-yl-(3,6-dichloro-benzo[b]thiophen-2-yl)-methanone;
Benzo[1,3]dioxol-5-yl-(4-benzyl-piperidin-1-yl)-methanone;
Azepan-1-yl-(3-chloro-6-methyl-benzo[b]thiophen-2-yl)-methanone;
N-(5-Hexyl-[1,3,4]thiadiazol-2-yl)-isobutyramide;
(3-Chloro-phenyl)-(10,11-dihydro-dibenzo[b,f]azepin-5-yl)-methanone;
(2-Chloro-phenyl)-(10,11-dihydro-dibenzo[b,f]azepin-5-yl)-methanone;
2-Amino-5-(azepane-1-carbonyl)-4-methyl-thiophene-3-carboxylic acid ethyl ester;
Adamantan-1-yl-(4-cyclopropyl-1,4,6,7-tetrahydro-imidazo[4,5-c]pyridin-5-yl)-methanone;
Adamantan-1-yl-(4-trifluoromethyl-1,4,6,7-tetrahydro-imidazo[4,5-c]pyridin-5-yl)-methanone;
Adamantan-1-yl-[4-(1H-benzoimidazol-2-ylsulfanyl)-piperidin-1-yl]-methanone;
Adamantan-1-yl-(1,4,6,7-tetrahydro-imidazo[4,5-c]pyridin-5-yl)-methanone;
[4-(1H-Imidazol-4-yl)-piperidin-1-yl]-(4-pentyl-phenyl)-methanone;
3-Cyclohexyl-1-[4-(1H-imidazol-4-yl)-piperidin-1-yl]-propan-1-one;
1-(4-Propyl-piperazin-1-yl)-3-(4-trifluoromethyl-phenyl)-propan-1-one;
N-(2-Hydroxy-benzyl)-3-thiophen-3-yl-N-(2-thiophen-2-yl-ethyl)-acrylamide;
N-(1,3-Dimethyl-pentyl)-2-(3-fluoro-phenyl)-N-(4-hydroxy-benzyl)-acetamide;
N-Cyclobutyl-2-(3-fluoro-phenyl)-N-(4-hydroxy-benzyl)-acetamide;
N-Cyclobutyl-N-(4-hydroxy-benzyl)-4-trifluoromethyl-benzamide;
N-(3-Hydroxy-benzyl)-2-methyl-3-nitro-N-(4-sulfamoyl-benzyl)-benzamide;
N--(4-Bromo-benzyl)-N-(4-hydroxy-benzyl)-2-naphthalen-1-yl-acetamide;
6-(2-Bromo-phenylsulfanyl)-hexanoic acid (3-amino-2,2-dimethyl-propyl)-amide;
N-(3-Amino-2,2-dimethyl-propyl)-4-[2-(2-isopropyl-phenylsulfanyl)-ethyl]-benzamide;
N-(3-Amino-2,2-dimethyl-propyl)-4-[4-(4-chloro-phenyl)-pyrimidin-2-ylsulfanylmethyl]-3-nitro-benzamide;
4-(4-Bromo-phenyl)-N-(2-hydroxy-benzyl)-4-oxo-N-thiophen-2-ylmethyl-butyramide;
N-[2-(2,4-Dichloro-phenyl)-ethyl]-N-(4-hydroxy-benzyl)-2-thiophen-3-yl-acetamide;
N-(2-Chloro-benzyl)-N-(4-hydroxy-benzyl)-2-thiophen-2-yl-acetamide;
Heptanoic acid benzyl-(4-hydroxy-benzyl)-amide;
N-(4-Fluoro-benzyl)-N-(4-hydroxy-benzyl)-2-thiophen-3-yl-acetamide;
4-Methyl-pentanoic acid (4-fluoro-benzyl)-(4-hydroxy-benzyl)-amide;
N-Allyl-2-(4-chloro-phenyl)-N-(4-hydroxy-benzyl)-acetamide;
N-Allyl-2-benzo[b]thiophen-3-yl-N-(4-hydroxy-benzyl)-acetamide;
Heptanoic acid (3-ethoxy-propyl)-(4-hydroxy-benzyl)-amide;
Dec-3-enoic acid (4-hydroxy-benzyl)-(4-trifluoromethyl-benzyl)-amide;
6-Oxo-6-phenyl-hexanoic acid (4-hydroxy-benzyl)-(4-trifluoromethyl-benzyl)-amide;
2-(3,4-Difluoro-phenyl)-N-(4-hydroxy-benzyl)-N-thiophen-2-ylmethyl-acetamide;
2-Methyl-pent-4-enoic acid (3-hydroxy-benzyl)-[2-(2-methoxy-phenyl)-ethyl]-amide;
Heptanoic acid (3-hydroxy-benzyl)-(4-isopropyl-benzyl)-amide;
5-(2,6-Dichloro-phenylsulfanyl)-pentanoic acid (naphthalen-1-ylmethyl)-amide;
N-(6,6-Dimethyl-bicyclo[3.1.1]hept-2-ylmethyl)-4-[2-(5-methyl-1H-benzoimidazol-2-ylsulfanyl)-ethyl]-benzamide;
N-(6,6-Dimethyl-bicyclo[3.1.1]hept-2-ylmethyl)-4-[2-(4-phenoxy-pyrimidin-2-ylsulfanyl)-ethyl]-benzamide;
N-(6,6-Dimethyl-bicyclo[3.1.1]hept-2-ylmethyl)-4-[2-(4-fluoro-phenylsulfanyl)-ethyl]-benzamide;
4-(2,6-Dichloro-phenylsulfanyl)-N-(6,6-dimethyl-bicyclo[3.1.1]hept-2-ylmethyl)-butyramide;
N-(6,6-Dimethyl-bicyclo[3.1.1]hept-2-ylmethyl)-4-[2-(5-methyl-1H-benzoimidazol-2-ylsulfanyl)-ethyl]-benzamide;
N-(6,6-Dimethyl-bicyclo[3.1.1]hept-2-ylmethyl)-4-[2-(4-fluoro-phenylsulfanyl)-ethyl]-benzamide;
5-(3-Methylsulfanyl-[1,2,4]thiadiazol-5-ylsulfanyl)-pentanoic acid (6,6-dimethyl-bicyclo[3.1.1]hept-2-ylmethyl)-amide;
5-(2,6-Dichloro-phenylsulfanyl)-pentanoic acid [2-(3-trifluoromethyl-phenyl)-ethyl]-amide;
4-[2-(2,6-Dichloro-phenylsulfanyl)-ethyl]-N-[2-(2-fluoro-phenyl)-ethyl]-benzamide;
2-Cyclohexylamino-thiazole-4-carboxylic acid (1,2,3,4-tetrahydro-naphthalen-1-yl)-amide;
2-Cyclohexylamino-thiazole-4-carboxylic acid (3-chloro-4-hydroxy-phenyl)-amide;
2-Cyclohexylamino-thiazole-4-carboxylic acid (1,2-dimethyl-propyl)-amide;
2-Cyclohexylamino-thiazole-4-carboxylic acid (1-ethyl-propyl)-amide;
2-Cyclohexylamino-thiazole-4-carboxylic acid [3-(1-hydroxy-ethyl)-phenyl]-amide;
2-Cyclohexylamino-thiazole-4-carboxylic acid (1-ethynyl-cyclohexyl)-amide; 2-Cyclohexylamino-thiazole-4-carboxylic acid (2-methoxy-dibenzofuran-3-yl)-amide; 2-Cyclohexylamino-thiazole-4-carboxylic acid [2-(4-hydroxy-phenyl)-ethyl]-amide; 2-Cyclohexylamino-thiazole-4-carboxylic acid (4-hydroxy-cyclohexyl)-amide;
2-(2,6-Difluoro-benzylamino)-N-[2-(3-trifluoromethyl-phenyl)-ethyl]-acetamide;
4-{4-[2-(4-Dimethylamino-phenyl)-acetyl]-piperazin-1-yl}-3-(2-phenyl-propylamino)-benzamide;
2-(2-Ethyl-phenylsulfanyl)-3-[methyl-(2-pyridin-4-yl-ethyl)-amino]-N-prop-2-ynyl-propionamide;
4-Methyl-cyclohexanecarboxylic acid {[2-(2-chloro-6-fluoro-benzylsulfanyl)-ethylcarbamoyl]-methyl}-prop-2-ynyl-amide;
2-Benzylsulfanyl-N-{[2-(2-chloro-6-fluoro-benzylsulfanyl)-ethylcarbamoyl]-methyl}-N-(2-methoxy-ethyl)-acetamide;
4-[2-(5-Cyclopropylmethylsulfanyl-[1,3,4]thiadiazol-2-ylsulfanyl)-ethyl]-N-(6,6-dimethyl-bicyclo[3.1.1]hept-2-ylmethyl)-benzamide;
N-(6,6-Dimethyl-bicyclo[3.1.1]hept-2-ylmethyl)-4-[2-(4-phenoxy-pyrimidin-2-ylsulfanyl)-ethyl]-benzamide;
N-(6,6-Dimethyl-bicyclo[3.1.1]hept-2-ylmethyl)-2-p-tolyloxy-acetamide;
Bicyclo[2.2.1]hept-5-ene-2-carboxylic acid [4-(2,5-difluoro-phenoxy)-butyl]-amide;
4-Trifluoromethyl-cyclohexanecarboxylic acid [6-(2,6-difluoro-phenoxy)-hexyl]-amide;
N-Cyclopropyl-3-methoxy-N-(2-piperidin-4-yl-ethyl)-5-(pyridine-2-carbonyl)-benzamide;
3-Methoxy-N-(2-methoxy-ethyl)-N-(2-piperidin-4-yl-ethyl)-5-(pyridine-2-carbonyl)-benzamide;
3-Methoxy-N-(2-piperidin-4-yl-ethyl)-5-(pyridine-2-carbonyl)-N-(tetrahydro-furan-2-ylmethyl)-benzamide;
3-Methoxy-N-(2-oxo-azepan-3-yl)-N-(2-piperidin-4-yl-ethyl)-5-(pyridine-2-carbonyl)-benzamide;
3-Methoxy-N-[3-(2-oxo-pyrrolidin-1-yl)-propyl]-N-(2-piperidin-4-yl-ethyl)-5-(pyridine-2-carbonyl)-benzamide;
3-Methoxy-N-methyl-N-(2-piperidin-4-yl-ethyl)-5-(pyridine-2-carbonyl)-benzamide;
[2-({Cyclopropyl-[3-methoxy-5-(pyridine-2-carbonyl)-benzoyl]-amino}-methyl)-phenoxy]-acetic acid;
(2-{[[3-Methoxy-5-(pyridine-2-carbonyl)-benzoyl]-(3-methyl-butyl)-amino]-methyl}-phenoxy)-acetic acid;
[2-({Cyclopentyl-[3-methoxy-5-(pyridine-2-carbonyl)-benzoyl]-amino}-methyl)-phenoxy]-acetic acid;
[2-({(2-Methoxy-ethyl)-[3-methoxy-5-(pyridine-2-carbonyl)-benzoyl]-amino}-methyl)-phenoxy]-acetic acid;
[2-({Carbamoylmethyl-[3-methoxy-5-(pyridine-2-carbonyl)-benzoyl]-amino}-methyl)-phenoxy]-acetic acid;
[2-({[3-Methoxy-5-(pyridine-2-carbonyl)-benzoyl]-pyridin-4-yl-amino}-methyl)-phenoxy]-acetic acid;
[2-({Cyclopropylmethyl-[3-methoxy-5-(pyridine-2-carbonyl)-benzoyl]-amino}-methyl)-phenoxy]-acetic acid;
[2-({[3-Methoxy-5-(pyridine-2-carbonyl)-benzoyl]-methyl-amino}-methyl)-phenoxy]-acetic acid;
[4-(4-Hydroxy-benzyl)-piperazin-1-yl]-[3-methoxy-5-(pyridine-2-carbonyl)-phenyl]-methanone;
{Carbamoylmethyl-[3-methoxy-5-(pyridine-2-carbonyl)-benzoyl]-amino}-acetic acid;
{(3-Imidazol-1-yl-propyl)-[3-methoxy-5-(pyridine-2-carbonyl)-benzoyl]-amino}-acetic acid;
{[3-Methoxy-5-(pyridine-2-carbonyl)-benzoyl]-pyridin-4-yl-amino}-acetic acid;
[[3-Methoxy-5-(pyridine-2-carbonyl)-benzoyl]-(2-oxo-azepan-3-yl)-amino]-acetic acid;
3-Methoxy-N-(2-methoxy-ethyl)-N-piperidin-3-ylmethyl-5-(pyridine-2-carbonyl)-benzamide;
4-[3-Methoxy-5-(pyridine-2-carbonyl)-benzoylamino]-piperidine-1-carboxylic acid ethyl ester;
3-Methoxy-N-[3-(2-oxo-pyrrolidin-1-yl)-propyl]-5-(pyridine-2-carbonyl)-benzamide;
3-({Carbamoylmethyl-[3-methoxy-5-(pyridine-2-carbonyl)-benzoyl]-amino}-methyl)-benzoic acid;
3-({(3-Imidazol-1-yl-propyl)-[3-methoxy-5-(pyridine-2-carbonyl)-benzoyl]-amino}-methyl)-benzoic acid;
4-Amino-N-(3-hydroxy-benzyl)-N-indan-2-yl-2-propionylamino-butyramide;
5-Amino-2-propionylamino-pentanoic acid (3-hydroxy-benzyl)-indan-2-yl-amide;
N-Ethyl-2-hexylamino-N-(4-hydroxy-benzyl)-acetamide;
2-Hexylamino-N-(4-hydroxy-benzyl)-N-methyl-acetamide;
1-[1-(6-Phenyl-hexanoyl)-piperidin-4-yl]-1,3-dihydro-benzoimidazol-2-one;
1-[1-(3-Cyclohexyl-propionyl)-piperidin-4-yl]-1,3-dihydro-benzoimidazol-2-one;
N-(2-Hydroxy-benzyl)-N-isobutyl-benzamide;
N-(2-Hydroxy-benzyl)-2-(4-hydroxy-phenyl)-N-isobutyl-acetamide;
N-(2-Hydroxy-benzyl)-N-(3-methyl-butyl)-benzamide;
N-(4-Hydroxy-benzyl)-N-isobutyl-benzamide;
4-Hydroxy-N-(4-hydroxy-benzyl)-N-isobutyl-benzamide;
N-(4-Hydroxy-benzyl)-2-(4-hydroxy-phenyl)-N-isobutyl-acetamide;
N-(4-Hydroxy-benzyl)-N-(3-methyl-butyl)-benzamide;
N-(2-Ethoxy-ethyl)-4-hydroxy-N-(4-hydroxy-benzyl)-benzamide;
N-(4-Hydroxy-benzyl)-N-(3-isopropoxy-propyl)-benzamide;
N-(3-Hydroxy-benzyl)-N-(4-methyl-pentyl)-benzamide;
N-(3-Hydroxy-benzyl)-2-(4-hydroxy-phenyl)-N-(4-methyl-pentyl)-acetamide;
N-(3-Hydroxy-benzyl)-N-(3-isopropoxy-propyl)-benzamide;
N-(2-Hydroxy-benzyl)-N-(3-methyl-butyl)-4-propyl-benzamide;
N-(4-Hydroxy-benzyl)-N-(6-hydroxy-hexyl)-4-propyl-benzamide;
N-(4-Hydroxy-benzyl)-N-(3-methyl-butyl)-4-propyl-benzamide;
N-[2-(4-Fluoro-benzylamino)-thiazol-4-ylmethyl]-N-phenethyl-butyramide; or
a salt thereof with a pharmaceutically acceptable acid or base, or any optical isomer or mixture of optical isomers, including a racemic mixture, or any tautomeric forms.

In another embodiment of the present invention said substituted amide, or a prodrug thereof, as a component of the combination therapy is selected from the group consisting of::
(4-Tetrazol-1-yl-phenyl)-(1,3,3-trimethyl-6-aza-bicyclo[3.2.1]oct-6-yl)-methanone;
N-Cyclohexyl-N-methyl-2-phenoxymethyl-benzamide;
4-Amino-N-cyclohexyl-N-methyl-benzamide;
N-Cycloheptyl-N-methyl-2-phenoxymethyl-benzamide;
N-Cyclohexyl-N-methyl-benzamide;
2-Chloro-N-cyclohexyl-6-fluoro-N-methyl-benzamide;
N-Cyclohexyl-N-methyl-4-trifluoromethoxy-benzamide;
N-Cyclohexyl-2,3, N-trimethyl-benzamide;
3,5-Dichloro-N-cyclohexyl-N-methyl-benzamide;
N-Cyclohexyl-N-methyl-2-phenoxy-benzamide;
2,4-Bis-benzyloxy-N-cyclohexyl-N-methyl-benzamide;
2-Benzyloxy-N-cyclohexyl-N-methyl-benzamide;
N-Cyclohexyl-N-methyl-4-phenoxy-benzamide;
4-Benzyloxy-N-cyclohexyl-N-methyl-benzamide;
N-Cyclohexyl-N-methyl-4-phenoxymethyl-benzamide;
2-Chloro-N-cyclohexyl-N-ethyl-4-nitro-benzamide;
4-Chloro-N-cyclohexyl-N-ethyl-3-nitro-benzamide;
6-Fluoro-4H-benzo[1,3]dioxine-8-carboxylic acid cyclohexyl-methyl-amide;
Azepan-1-yl-(2-chloro-phenyl)-methanone;
Azepan-1-yl-(3-chloro-phenyl)-methanone;
Azepan-1-yl-phenyl-methanone;
2-(Biphenyl-4-yloxy)-N-cyclohexyl-N-methyl-benzamide;
N-Cyclohexyl-2-(3,5-dimethoxy-phenoxy)-N-methyl-benzamide;
N-Cyclohexyl-2-(2,3-dimethoxy-phenoxy)-N-methyl-benzamide;
2,4-Dichloro-N-(3,3-dimethyl-1,5-dioxa-spiro[5.5]undec-9-yl)-N-methyl-benzamide;
2,4-Dichloro-N-methyl-N-(4-oxo-cyclohexyl)-benzamide;
N-Cyclohexyl-2-hydroxy-N-methyl-benzamide;
N-Cyclohexyl-3-methoxy-N-methyl-benzamide;
Benzo[1,3]dioxole-5-carboxylic acid cyclohexyl-methyl-amide;
3-Benzyloxy-N-cyclohexyl-N-methyl-benzamide;
N-Cyclohexyl-3-hydroxy-N-methyl-benzamide;
[4-(Morpholine-4-sulfonyl)-phenyl]-(1,3,3-trimethyl-6-aza-bicyclo[3.2.1]oct-6-yl)-methanone;
N-Benzyl-3-(1,3,3-trimethyl-6-aza-bicyclo[3.2.1]octane-6-carbonyl)-benzenesulfonamide;
[4-Fluoro-3-(morpholine-4-sulfonyl)-phenyl]-(1,3,3-trimethyl-6-aza-bicyclo[3.2.1]oct-6-yl)-methanone;
Thiophene-2-sulfonic acid [4-(1,3,3-trimethyl-6-aza-bicyclo[3.2.1]octane-6-carbonyl)-phenyl]-amide;
N-Phenyl-4-(1,3,3-trimethyl-6-aza-bicyclo[3.2.1]octane-6-carbonyl)-benzenesulfonamide;
(4-Phenoxy-phenyl)-(1,3,3-trimethyl-6-aza-bicyclo[3.2.1]oct-6-yl)-methanone;
N-(2,4-Dimethyl-phenyl)-3-(1,3,3-trimethyl-6-aza-bicyclo[3.2.1]octane-6-carbonyl)-benzenesulfonamide;
(2-Phenoxymethyl-phenyl)-(1,3,3-trimethyl-6-aza-bicyclo[3.2.1]oct-6-yl)-methanone;
4-(3-Aza-bicyclo[3.2.2]nonane-3-carbonyl)-N, N-dipropyl-benzenesulfonamide;
2-Bromo-N-cyclohexyl-N-methyl-benzamide;
N-[4-(1,3,3-Trimethyl-6-aza-bicyclo[3.2.1]octane-6-carbonyl)-phenyl]-acetamide;
(4-Dimethylamino-phenyl)-(1,3,3-trimethyl-6-aza-bicyclo[3.2.1]oct-6-yl)-methanone;
(4-Pyrrol-1-yl-phenyl)-(1,3,3-trimethyl-6-aza-bicyclo[3.2.1]oct-6-yl)-methanone;
(4-Imidazol-1-yl-phenyl)-(1,3,3-trimethyl-6-aza-bicyclo[3.2.1]oct-6-yl)-methanone;
(4-Amino-2-methoxy-phenyl)-(trimethyl-6-aza-bicyclo[3.2.1]oct-6-yl)-methanone;
(4-Methanesulfonyl-phenyl)-(1,3,3-trimethyl-6-aza-bicyclo[3.2.1]oct-6-yl)-methanone;
(3-Methanesulfonyl-phenyl)-(1,3,3-trimethyl-6-aza-bicyclo[3.2.1]oct-6-yl)-methanone;
(4-Benzenesulfonyl-phenyl)-(1,3,3-trimethyl-6-aza-bicyclo[3.2.1]oct-6-yl)-methanone;
Azepan-1-yl-[4-(3,4-dihydro-1 H-isoquinolin-2-ylmethyl)-phenyl]-methanone;
Azepan-1-yl-(4-morpholin-4-ylmethyl-phenyl)-methanone;
[4-(3-Trifluoromethyl-pyrazol-1-yl)-phenyl]-(1,3,3-trimethyl-6-aza-bicyclo[3.2.1]oct-6-yl)-methanone;
(4-[1,2,4]Triazol-1-yl-phenyl)-(1,3,3-trimethyl-6-aza-bicyclo[3.2.1]oct-6-yl)-methanone;
(4-Pyrazol-1-yl-phenyl)-(1,3,3-trimethyl-6-aza-bicyclo[3.2.1]oct-6-yl)-methanone;
2-Benzyloxymethyl-N-cyclohexyl-N-methyl-benzamide;
N-Cyclohexyl-N-methyl-4-(3-methyl-5-oxo-4,5-dihydro-pyrazol-1-yl)-benzamide;
5-Methyl-2-[4-(1,3,3-trimethyl-6-aza-bicyclo[3.2.1]octane-6-carbonyl)-phenyl]-2,4-dihydro-pyrazol-3-one;
(9H-Carbazol-3-yl)-(1,3,3-trimethyl-6-aza-bicyclo[3.2.1]oct-6-yl)-methanone;
[4-(3,5-Dimethyl-pyrazol-1-yl)-phenyl]-(1,3,3-trimethyl-6-aza-bicyclo[3.2.1]oct-6-yl)-methanone;
Phenyl-(1,3,3-trimethyl-6-aza-bicyclo[3.2.1]oct-6-yl)-methanone;
Azepan-1-yl-(2-bromo-phenyl)-methanone;
(3-Aza-bicyclo[3.2.2]non-3-yl)-(2-bromo-phenyl)-methanone;
(4-Benzyl-piperidin-1-yl)-quinolin-2-yl-methanone;
(2-Methyl-piperidin-1-yl)-quinolin-2-yl-methanone;
(3-Aza-bicyclo[3.2.2]non-3-yl)-quinolin-2-yl-methanone;
Quinoline-2-carboxylic acid cyclohexyl-methyl-amide;
Quinolin-2-yl-(1,3,3-trimethyl-6-aza-bicyclo[3.2.1]oct-6-yl)-methanone;
1-[4-(1,3,3-Trimethyl-6-aza-bicydo[3.2.1]octane-6-carbonyl)-phenyl]-pyrrolidine-2,5-dione;
Pyridin-3-yl-(1,3,3-trimethyl-6-aza-bicyclo[3.2.1]oct-6-yl)-methanone;
Pyridin-4-yl-(1,3,3-trimethyl-6-aza-bicyclo[3.2.1]oct-6-yl)-methanone;
Pyridin-2-yl-(1,3,3-trimethyl-6-aza-bicyclo[3.2.1]oct-6-yl)-methanone;
(6-Pyrazol-1-yl-pyridin-3-yl)-(1,3,3-trimethyl-6-aza-bicyclo[3.2.1]oct-6-yl)-methanone;
4-(1,3,3-Trimethyl-6-aza-bicyclo[3.2.1]octane-6-carbonyl)-benzoic acid;
Imidazo[2,1-b]thiazol-6-yl-(1,3,3-trimethyl-6-aza-bicyclo[3.2.1]oct-6-yl)-methanone;
8-(4-Dimethylamino-benzoyl)-8-aza-bicyclo[3.2.1]octan-3-one;
(4-Dimethylamino-phenyl)-(3-hydroxy-8-aza-bicyclo[3.2.1]oct-8-yl)-methanone;
(4-Dimethylamino-phenyl)-(3-hydroxy-3-methyl-8-aza-bicyclo[3.2.1]oct-8-yl)-methanone;
Trifluoro-acetic acid 8-(4-dimethylamino-benzoyl)-8-aza-bicyclo[3.2.1]oct-3-yl ester; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIa) 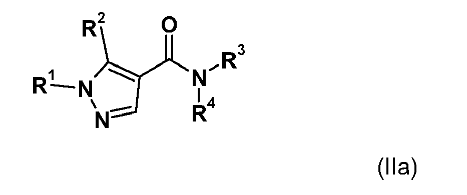 wherein
R¹ is aryl, arylC₁-C₆alkyl, hetaryl or hetarylC₁-C₆alkyl optionally substituted with one or more of R⁶ independently;
R² is halo, C₁-C₆alkyl, C₂-C₆alkenyl, C₂-C₆alkynyl, C₃-C₁₀cycloalkyl, C₃-C₁₀cycloalkylC₁C₆alkyl, trihalomethyl, aryl, arylC₁-C₆alkyl, C₁-C₆alkyloxyC₁-C₆alkyl, arylC₁-C₆alkyloxyC₁-C₆alkyl, C₁-C₆alkylNR⁵C₁-C₆alkyl, arylC₁-C₆alkylNR⁵C₁-C₆alkyl, hetaryl or hetarylC₁-C₆alkyl wherein the alkyl, alkenyl, alkynyl, cycloalkyl and aryl groups independently are optionally substituted with one or more R⁷;
R³ is C₁-C₆alkyl optionally substituted with one or more of R⁸;
R⁴ is C₆-C₁₀cycloalkyl, C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸; or
R³ and R⁴ together with the nitrogen to which they are attached, are forming a saturated or partially saturated bicyclic/bridge ring system containing from 7 to 12 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetaryl-C₁-C₆alkyl, hydroxy, oxo, C₁-C₆alkyloxy, arylC₁-C₆alkyloxy or hetarylC₁-C₆alkyloxy, wherein the alkyl and aryl groups independently are optionally substituted with one ore more of R⁹;
R⁵ is C₁-C₆alkyl, C₂-C₆alkenyl, C₂-C₆alkynyl, C₃-C₁₀cycloalkyl, C₃-C₁₀hetcycloalkyl, C₃-C₁₀cycloalkylC₁-C₆alkyl, C₃-C₁₀hetcycloalkylC₁-C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;
R⁶ and R⁷ independently are hydrogen, hydroxy, oxo, halo, nitro, cyano, C₁-C₆alkyl, C₁-C₆alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl, arylC₁-C₆alkyloxycarbonyl, C₁-C₆alkylcarboxy, arylcarboxy or arylC₁-C₆alkylcarboxy;
R⁸ and R⁹ independently are hydrogen, C₁-C₆alkyl, aryl, hetaryl, arylC₁-C₆alkyl, hetarylC₁-C₆alkyl, hydroxy, oxo, cyano, NR¹⁰R¹¹, C₁-C₆alkyloxy, aryloxy, arylC₁-C₆alkyloxy, hetaryloxy, hetarylC₁-C₆alkyloxy, C₁-C₆alkyloxyCₗ-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R¹⁰ and R¹¹ independently are hydrogen, C₁-C₈alkyl, aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarbonyl, C₁-C₆alkylcarboxyC₁-C₆alkyl; or
R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-₆alkylcarboxy; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIa) wherein:
R¹ is aryl or hetaryl optionally substituted with one or more R⁶ independently;
R² is halo, C₁-C₆alkyl, C₂-C₆alkenyl, C₂-C₆alkynyl, C₃-C₁₀cycloalkyl, C₃-C₁₀cycloalkylC₁-C₆alkyl, aryl, arylC₁-C₆alkyl, C₁-C₆alkyloxyC₁-C₆alkyl, arylC₁-C₆alkyloxylC₁-C₆alkyl, C₁-C₆alkylNR⁵C₁-C₆alkyl, arylC₁-C₆alkylNR⁵C₁-C₆alkyl, hetaryl or hetarylC₁-C₆alkyl wherein the alkyl, alkenyl, alkynyl, cycloalkyl and aryl groups independently are optionally substituted with one or more R⁷;
R³ is C₁-C₆alkyl optionally substituted with one or more of R⁸;
R⁴ is C₆-C₁₀cycloalkyl, C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸;
R⁵ is C₁-C₆alkyl, C₂-C₆alkenyl, C₂-C₆alkynyl, C₃-C₁₀cycloalkyl, C₃-C₁₀hetcycloalkyl, C₃-C₁₀cycloalkylC₁-C₆alkyl, C₃-C₁₀hetcycloalkylC₁-C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;
R⁶ and R⁷ independently are hydrogen, hydroxy, oxo, halo, nitro, cyano, C₁-C₆alkyl, C₁-C₆alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl, arylC₁-C₆alkyloxycarbonyl, C₁-C₆alkylcarboxy, arylcarboxy or arylC₁-C₆alkylcarboxy;
R⁸ and R⁹ independently are hydrogen, C₁-C₆alkyl, aryl, hetaryl, arylC₁-C₆alkyl, hetarylC₁-C₆alkyl, hydroxy, oxo, cyano, NR¹⁰R¹¹, C₁-C₆alkyloxy, aryloxy, arylC₁-C₆alkyloxy, hetaryloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylcarbonyl, hetarylC₁-C₆alkyl-carbonyl, C₁-C₆alkylcarboxy, arylcarboxy or arylC₁-C₆alkylcarboxy;
R¹⁰ and R¹¹ independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkylcarboxyC₁-C₆alkyl; or
R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetaryl C₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁₋₆alkylcarboxy; or
a salt thereof with a pharmaceutically acceptable acid or base, or any optical isomer or mixture of optical isomers, including a racemic mixture, a prodrug thereof, or any tautomeric forms.

In another embodiment of the present invention said substituted amides, or a prodrug thereof, as a component of the combination therapy is of the general formula (IIa) wherein R¹ is aryl, arylC₁-C₆alkyl or hetaryl optionally substituted with one or more of R⁶.

In another embodiment of the present invention said substituted amides, or a prodrug thereof, as a component of the combination therapy is of the general formula (IIa) wherein R¹ is aryl optionally substituted with one or more of R⁶.

In another embodiment of the present invention said substituted amides, or a prodrug thereof, as a component of the combination therapy is of the general formula (IIa) wherein R¹ is arylC₁-C₆alkyl optionally substituted with one or more of R⁶.

In another embodiment of the present invention said substituted amides, or a prodrug thereof, as a component of the combination therapy is of the general formula (IIa) wherein R¹ is hetaryl optionally substituted with one or more of R⁶.

In another embodiment of the present invention said substituted amides, or a prodrug thereof, as a component of the combination therapy is of the general formula (lla) wherein R² is C₁-C₆alkyl, C₃-C₁₀cycloalkyl, C₃-C₁₀cycloalkylC₁-C₆alkyl, trihalomethyl, arylC₁-C₆alkyl, or hetarylC₁-C₆alkyl wherein the alkyl, cycloalkyl and aryl groups independently are optionally substituted with one or more R⁷.

In another embodiment of the present invention said substituted amides, or a prodrug thereof, as a component of the combination therapy is of the general formula (IIa) wherein R² is C₁-C₆alkyl optionally substituted with one or more R⁷.

In another embodiment of the present invention said substituted amides, or a prodrug thereof, as a component of the combination therapy is of the general formula (IIa) wherein R² is trihalomethyl.

In another embodiment of the present invention said substituted amides, or a prodrug thereof, as a component of the combination therapy is of the general formula (IIa) wherein R³ is C₁-C₆alkyl optionally substituted with one or more of R⁸.

In another embodiment of the present invention said substituted amides, or a prodrug thereof, as a component of the combination therapy is of the general formula (IIa) wherein R⁴ is C₆-C₁₀cycloalkyl, or C₆-C₁₀hetcycloalkyl, wherein the cycloalkyl and hetcycloalkyl groups independently are optionally substituted with one or more of R⁸.

In another embodiment of the present invention said substituted amides, or a prodrug thereof, as a component of the combination therapy is of the general formula (IIa) wherein R⁴ is C₆-C₁₀cycloalkyl optionally substituted with one or more of R⁸.

In another embodiment of the present invention said substituted amides, or a prodrug thereof, as a component of the combination therapy is of the general formula (IIa) wherein R⁴ is C₆ C₆-C₁₀hetcycloalkyl optionally substituted with one or more of R⁸.

In another embodiment of the present invention said substituted amides, or a prodrug thereof, as a component of the combination therapy is of the general formula (IIa) wherein R³ and R⁴ together with the nitrogen to which they are attached, are forming a saturated or partially saturated bicyclic/bridge ring system containing from 7 to 12 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, arylC₁-C₆alkyloxy or hetarylC₁-C₆alkyloxy, wherein the alkyl and aryl groups independently are optionally substituted with one ore more of R⁹.

In another embodiment of the present invention said substituted amides, or a prodrug thereof, as a component of the combination therapy is of the general formula (IIa) wherein the saturated or partially saturated bicyclic/bridge ring system is 6-aza-bicyclo[3.2.1]octane.

In another embodiment of the present invention said substituted amides, or a prodrug thereof, as a component of the combination therapy is of the general formula (IIa) wherein R⁶ and R⁷ independently are hydrogen, hydroxy, oxo, halo, cyano, C₁-C₆alkyl, C₁-C₆alkyloxy, trihalomethyl, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl or arylC₁-C₆alkyloxycarbonyl.

In another embodiment of the present invention said substituted amides, or a prodrug thereof, as a component of the combination therapy is of the general formula (IIa) wherein R⁸ and R⁹ independently are hydrogen, C₁-C₆alkyl, hydroxy, oxo, C₁-C₆alkyloxy or arylC₁-C₆alkyloxy.

In another embodiment of the present invention said substituted amides, or a prodrug thereof, as a component of the combination therapy is of the general formula (IIa) wherein R¹⁰ and R¹¹ independently are hydrogen or C₁-C₈alkyl.

In another embodiment of the present invention said substituted amides, or a prodrug thereof, as a component of the combination therapy is selected from the group consisting of:
1-(4-Chloro-phenyl)-5-propyl-1*H*-pyrazole-4-carboxylic acid cyclohexyl-methyl-amide; 1-(4-Chloro-phenyl)-5-trifluoromethyl-1H-pyrazole-4-carboxylic acid cyclohexyl-methyl-amide;
[1-(4-Methoxy-phenyl)-5-methyl-1*H*-pyrazol-4-yl]-(1,3,3-trimethyl-6-aza-bicyclo[3.2.1]oct-6-yl)-methanone; 1 [1-(4-Chloro-phenyl)-5-propyl-1*H*-pyrazol-4-yl]-(1,3,3-trimethyl-6-aza-bicyclo[3.2.1]oct-6-yl)-methanone;
[1-(3,5-Dichloro-phenyl)-5-propyl-1*H*-pyrazol-4-yl]-(1,3,3-trimethyl-6-aza-bicyclo[3.2.1]oct-6-yl)-methanone; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is selected from the group consisting of:
1-(Phenyl)-5-methyl-1*H*-pyrazole-4-carboxylic acid cyclohexyl-methyl-amide;
1-(4-Fluoro-phenyl)-5-methyl-1*H*-pyrazole-4-carboxylic acid cyclohexyl-methyl-amide;
1-(4-Methoxy-phenyl)-5-methyl-1*H*-pyrazole-4-carboxylic acid cyclohexyl-methyl-amide;
1-(4-Chloro-phenyl)-5-methyl-1*H*-pyrazole-4-carboxylic acid cyclohexyl-methyl-amide;
1-(2-Methyl-phenyl)-5-methyl-1*H*-pyrazole-4-carboxylic acid cyclohexyl-methyl-amide;
1-(4-Amino-phenyl)-5-methyl-1*H*-pyrazole-4-carboxylic acid cyclohexyl-methyl-amide;
1-(2-Pyridyl)-5-methyl-1*H*-pyrazole-4-carboxylic acid cyclohexyl-methyl-amide;
1-(2-Pyridyl)-5-propyl-1*H*-pyrazole-4-carboxylic acid cyclohexyl-methyl-amide; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIb) 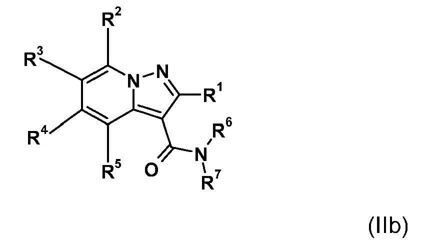 wherein
R¹ is hydrogen, trihalomethyl, C₁-C₆alkyl, C₁-C₆alkyloxy, C₁-C₆alkylthio, aryl, arylC₁-C₆alkyl, hetaryl or hetaralkyl, wherein the alkyl, aryl and hetaryl groups independently are optionally substituted with one or more of R⁸;
R² , R³, R⁴ and R⁵ independently are hydrogen, halo, nitro, cyano, hydroxy, NR⁹R¹⁰, trihalomethyl, C₁-C₆alkyl, C₁-C₆alkyloxy, C₁-C₆alkylthio, aryl, arylC₁-C₆alkyl, hetaryl or hetaralkyl, wherein the alkyl, aryl and hetaryl groups independently are optionally substituted with one or more of R⁸; or
R² together with R³ are forming a saturated or partially saturated cyclic ring system containing from 3 to 6 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy; or
R³ together with R⁴ are forming a saturated or partially saturated cyclic ring system containing from 3 to 6 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy; or
R⁴ together with R⁵ are forming a saturated or partially saturated cyclic ring system containing from 3 to 6 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy;
R⁶ is aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl, wherein the alkyl, aryl and cycloalkyl groups independently are optionally substituted with one or more of R¹¹;
R⁷ is C₁-C₈alkyl, aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl, wherein the alkyl, aryl and cycloalkyl groups independently are optionally substituted with one or more of R¹¹; or
R⁶ and R⁷, together with the nitrogen to which they are attached, are forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁸;
R⁹ and R¹⁰ independently are hydrogen, C₁-C₈alkyl, aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl, wherein the alkyl, aryl and cycloalkyl groups independently are optionally substituted with one or more of R¹¹; or
R⁹ and R¹⁰, together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁸;
R⁸ and R¹¹ independently are hydrogen, halo, hydroxy, oxo, nitro, cyano, C₁-C₈alkyl, C₁-C₆alkyloxy or aryloxy; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIb) wherein R¹ is hydrogen or C₁-C₆alkyl, wherein the alkyl group is optionally substituted with one or more of R⁸.

In another embodiment of the present invention said substituted amides, or a prodrug thereof, as a component of the combination therapy is of the general formula (IIb) wherein R¹ is hydrogen.

In another embodiment of the present invention said substituted amides, or a prodrug thereof, as a component of the combination therapy is of the general formula (IIb) wherein R², R³, R⁴ and R⁵ are hydrogen.

In another embodiment of the present invention said substituted amides, or a prodrug thereof, as a component of the combination therapy is of the general formula (llb) wherein R³ together with R⁴ are forming a saturated or partially saturated cyclic ring system containing from 3 to 6 carbon atoms and from 0 to 2 additional heteroatoms selected from nitrogen or oxygen, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy.

In another embodiment of the present invention said substituted amides, or a prodrug thereof, as a component of the combination therapy is of the general formula (IIb) wherein R⁴ together with R⁵ are forming a saturated or partially saturated cyclic ring system containing from 3 to 6 carbon atoms and from 0 to 2 additional heteroatoms selected from nitrogen or oxygen, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy.

In another embodiment of the present invention said substituted amides, or a prodrug thereof, as a component of the combination therapy is of the general formula (IIb) wherein R⁶ and R⁷, together with the nitrogen to which they are attached, are forming a saturated or partially saturated cyclic, bicyclic or tricyclic ring system containing from 6 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁸;

In another embodiment of the present invention said substituted amides, or a prodrug thereof, as a component of the combination therapy is of the general formula (IIb) wherein R⁶ and R⁷; together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁸.

In another embodiment of the present invention said substituted amides, or a prodrug thereof, as a component of the combination therapy is of the general formula (IIb) wherein R⁹ and R¹⁰, together with the nitrogen to which they are attached, are forming a saturated or partially saturated cyclic, bicyclic or tricyclic ring system containing from 4 to 10 carbon atoms and from 0 to 2 additional heteroatoms selected from nitrogen or oxygen, the ring system optionally being substituted with at least one of C₁C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁸.

In another embodiment of the present invention said substituted amides, or a prodrug thereof, as a component of the combination therapy is selected from the group consisting of:
pyrazolo[1,5-a]pyridin-3-yl-(1,3,3-trimethyl-6-aza-bicyclo[3.2.1]oct-6-yl)-methanone;
(2-Methyl-pyrazolo[1,5-a]pyridin-3-yl)-(1,3,3-trimethyl-6-aza-bicyclo[3.2.1]oct-6-yl)-methanone;
Pyrazolo[1,5-a]pyridine-3-carboxylic acid cyclohexyl-methyl-amide; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIc): 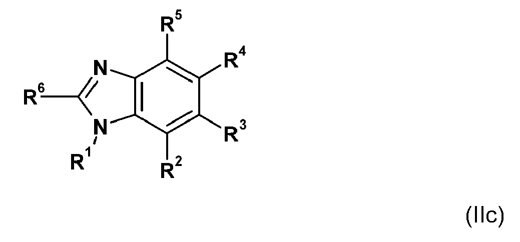 wherein
R¹ is hydrogen, C₁-C₈alkyl, C₁-C₆alkyloxyC₁-C₆alkyl, aryl, hetaryl, arylC₁-C₆alkyl, hetarylC₁-C₆alkyl, C₁-C₆SO₂, arylSO₂, hetarylSO₂, arylC₁-C₆alkylSO₂ or hetarylC₁-C₆alkylSO₂ all of which is optionally substituted with one or more R⁸;
R² and R⁵ independently are hydrogen, halo, nitro, cyano, trihalomethyl, C₁-C₆alkyl, aryl, arylC₁-C₆alkyl, hetaryl or hetarylC₁-C₆alkyl wherein the alkyl, aryl, arylalkyl, hetaryl and hetarylalkyl groups independently are substituted with one or more R⁹; and
either R³ is hydrogen; and R⁴ is C(O)NR⁷R⁸; or R³ is C(O)NR⁷R⁸; and R⁴ is hydrogen; and
R⁶ is hydrogen, halo, cyano, trihalomethyl, NR¹²R¹³, C₁-C₆alkyl, aryl, arylC₁-C₆alkyl, hetaryl or hetarylC₁-C₆alkyl wherein the alkyl, aryl, arylalkyl, hetaryl and hetarylalkyl groups independently are substituted with one or more R⁹; and
R⁷ and R⁸ independently are C₁-C₈alkyl, C₃-C₁₀cycloalkyl, C₃-C₁₀hetcycloalkyl, C₃-C₁₀cycloalkylC₁-C₆alkyl, wherein the alkyl, cycloalkyl and hetcycloalkyl groups independently are optionally substituted with one or more of R¹⁰; or
R⁷ and R⁸ together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₈alkyl, arylC₁-C₆alkyl, hetarylC₁-C₆alkyl, hydroxy, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹;
R⁹ is hydrogen, hydroxy, oxo, halo, nitro, cyano, C₁-C₆alkyl, C₁-C₆alkyloxy, trihalomethyl, trihalomethoxy, NR¹²R¹³, C(O)NR¹²R¹³, arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R¹⁰ and R¹¹ independently are hydrogen, halo, oxo, hydroxy, C₁-C₆alkyl, aryl, arylC₁-C₆alkyl, hetaryl or hetarylalkyl;
R¹² and R¹³ independently are hydrogen, C₁-C₈alkyl, aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarbonyl, arylcarbonyl, arylC₁-C₈alkylcarbonyl, hetarylcarbonyl, hetarylC₁-C₆alkylcarbonyl, C₁-C₆alkylcarboxyC₁-C₆alkyl; or
R¹² and R¹³ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-C₆alkyl-carbonyl, hetarylC₁-C₆alkylcarbonyl, C₁-C₆alkylcarboxy, arylcarboxy or arylC₁-C₆alkylcarboxy; or
a salt thereof with a pharmaceutically acceptable acid or base, or any optical isomer or mixture of optical isomers, including a racemic mixture, a prodrug thereof, or any tautomeric forms.

In another embodiment of the present invention said substituted amides, or a prodrug thereof, as a component of the combination therapy is of the general formula (IId) 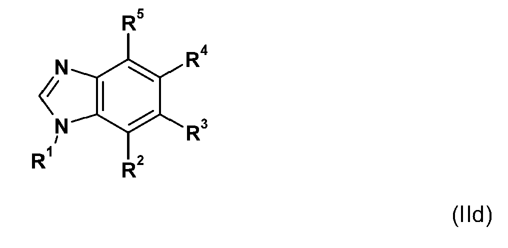 wherein
R¹ is hydrogen, C₁-C₈alkyl, aryl, hetaryl, arylC₁-C₆alkyl or hetarylC₁-C₆alkyl optionally substituted with one or more R⁸;
R² and R⁵ independently are hydrogen, halo, nitro, cyano, trihalomethyl, C₁-C₆alkyl, aryl, arylC₁-C₆alkyl, hetaryl or hetarylC₁-C₆alkyl wherein the alkyl, aryl, arylalkyl, hetaryl and hetarylalkyl groups independently are substituted with one or more R⁸; and
either R³ is hydrogen; and R⁴ is C(O)NR⁶R⁷; or R³ is C(O)NR⁶R⁷; and R⁴ is hydrogen;
R⁶ and R⁷ independently are C₁-C₈alkyl, C₃-C₁₀cycloalkyl, C₃-C₁₀hetcycloalkyl, C₃-C₁₀cycloalkylC₁-C₆alkyl, wherein the alkyl, cycloalkyl and hetcycloalkyl groups independently are optionally substituted with one or more of R⁹; or
R⁶ and R⁷ together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₈alkyl, arylC₁-C₆alkyl, hetarylC₁-C₆alkyl, hydroxy, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁰;
R⁸is hydrogen, hydroxy, oxo, halo, nitro, cyano, C₁-C₆alkyl, C₁-C₆alkyloxy, trihalomethyl, trihalomethoxy, NR¹¹R¹², arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R⁹ and R¹⁰ independently are hydrogen, halo, oxo, hydroxy, C₁-C₆alkyl, aryl, arylC₁-C₆alkyl, hetaryl or hetarylalkyl;
R¹¹ and R¹² independently are hydrogen, C₁-C₈alkyl, aryl, hetaryl, arylC₁-C₆alkyl, C₃-C₁₀cycloalkyl, C₃-C₁₀hetcydoalkyl, C₃-C₁₀cycloalkylC₁-C₆alkyl, C₁-C₆alkyl-carboxyC₁-C₆alkyl; or
R¹¹ and R¹² together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-C₆alkyl-carbonyl, hetarylC₁-C₆alkylcarbonyl, C₁-C₆alkylcarboxy, arylcarboxy or arylC₁-C₆alkylcarboxy; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIc) and (IId) wherein R¹ is hydrogen, C₁-C₈alkyl, arylC₁-C₆alkyl, hetarylC₁-C₆alkyl, arylSO₂, hetarylSO₂, arylC₁-C₆alkylSO₂ or hetarylC₁-C₆alkylSO₂ all of which is optionally substituted with one or more R⁸.

In another embodiment of the present invention said substituted amides, or a prodrug thereof, as a component of the combination therapy is of the general formula (IIc) and (IId) wherein R¹ is hydrogen, C₁-C₈alkyl, arylC₁-C₆alkyl, hetarylC₁-C₆alkyl all of which is optionally substituted with one or more R⁸.

In another embodiment of the present invention said substituted amides, or a prodrug thereof, as a component of the combination therapy is of the general formula (IIc) and (IId) wherein R¹ is arylSO₂, hetarylSO₂, arylC₁-C₆alkylSO₂ or hetarylC₁-C₆alkylSO₂ all of which is optionally substituted with one or more R⁸.

In another embodiment of the present invention said substituted amides, or a prodrug thereof, as a component of the combination therapy is of the general formula (IIc) and (IId) wherein R² is hydrogen.

In another embodiment of the present invention said substituted amides, or a prodrug thereof, as a component of the combination therapy is of the general formula (IIc) and (IId) wherein R³ is hydrogen and R⁴ is C(O)NR⁷R⁸.

In another embodiment of the present invention said substituted amides, or a prodrug thereof, as a component of the combination therapy is of the general formula (IIc) and (IId) wherein R³ is C(O)NR⁷R⁸ and R⁴ is hydrogen.

In another embodiment of the present invention said substituted amides, or a prodrug thereof, as a component of the combination therapy is of the general formula (IIc) and (IId) wherein R⁵ is hydrogen.

In another embodiment of the present invention said substituted amides, or a prodrug thereof, as a component of the combination therapy is of the general formula (IIc) wherein R⁶ is hydrogen, NR¹²R¹³, C₁-C₆alkyl, aryl or hetaryl wherein the alkyl, aryl and hetaryl independently are substituted with one or more R⁹.

In another embodiment of the present invention said substituted amides, or a prodrug thereof, as a component of the combination therapy is of the general formula (IIc) and (IId) wherein R⁷ and R⁸ independently are C₁-C₈alkyl or C₃-C₁₀cydoalkyl, wherein the alkyl and cycloalkyl groups independently are optionally substituted with one or more of R¹⁰.

In another embodiment of the present invention said substituted amides, or a prodrug thereof, as a component of the combination therapy is of the general formula (IIc) and (IId) wherein R⁷ and R⁸ together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₈alkyl, arylC₁-C₆alkyl, hetarylC₁-C₆alkyl, hydroxy, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹.

In another embodiment of the present invention said substituted amides, or a prodrug thereof, as a component of the combination therapy is of the general formula (IIc) and (lld) wherein R⁹ is hydrogen, hydroxy, oxo, halo, nitro, cyano, C₁-C₆alkyl, C₁-C₆alkyloxy, trihalomethyl, trihalomethoxy, NR¹²R¹³, C(O)NR¹²R¹³, arylC₁-C₆alkyloxy, C₁-C₆alkylcarbonyl, arylcarbonyl, arylC₁-C₆alkylcarbonyl.

In another embodiment of the present invention said substituted amides, or a prodrug thereof, as a component of the combination therapy is of the general formula (IIc) and (IId) wherein R¹⁰ and R¹¹ independently are hydrogen, halo, oxo, hydroxy, C₁-C₆alkyl, aryl, arylC₁-C₆alkyl, hetaryl or hetarylalkyl.

In another embodiment of the present invention said substituted amides, or a prodrug thereof, as a component of the combination therapy is of the general formula (IIc) and (IId) wherein R¹¹ and R¹² together with the nitrogen to which they are attached, are forming a saturated or partially saturated cyclic, bicyclic or tricyclic ring system containing from 4 to 10 carbon atoms and from 0 to 2 additional heteroatoms selected from nitrogen or oxygen, the ring system optionally being substituted with at least one of C₁C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-C₆alkyl-carbonyl, hetarylC₁-C₆alkylarbonyl, C₁-C₆alkylcarboxy, arylcarboxy or arylC₁-C₆alkylcarboxy.

In another embodiment of the present invention said substituted amides, or a prodrug thereof, as a component of the combination therapy is selected from the gropu consisting of:
1*H*-Benzoimidazole-5-carboxylic acid cyclohexyl-methyl-amide;
1-Benzyl-1*H*-benzoimidazole-5-carboxylic acid cyclohexyl-methyl-amide;
(1*H*-Benzoimidazol-5-yl)-(1,3,3-trimethyl-6-aza-bicyclo[3.2.1]oct-6-yl)-methanone; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is selected from the group consisting of:
Isopropyl-2-trifluoromethyl-1*H*-benzoimidazole-5-carboxylic acid cyclohexyl-methyl-amide;
1-Benzyl-1*H*-benzoimidazole-5-carboxylic acid cyclohexyl-methyl-amide;
2-Methyl-1*H*-benzoimidazole-5-carboxylic acid cyclohexyl-methyl-amide;
2-Hydroxymethyl-1*H*-benzoimidazole-5-carboxylic acid cyclohexyl-methyl-amide;
2-(4-Amino-phenyl)-1*H*-benzoimidazole-5-carboxylic acid cyclohexyl-methyl-amide;
(1*H*-Benzoimidazol-5-yl)-(1,3,3-trimethyl-6-aza-bicyclo[3.2.1]oct-6-yl)-methanone;
(2-Methyl-1*H*-benzoimidazol-5-yl)-(1,3,3-trimethyl-6-aza-bicyclo[3.2.1]oct-6-yl)-methanone;
(2-Amino-1*H*-benzoimidazol-5-yl)-(1,3,3-trimethyl-6-aza-bicyclo[3.2.1]oct-6-yl)-methanone;
(2-Benzo[1,3]dioxol-5-yl-1*H*-benzoimidazol-5-yl)-(1,3,3-trimethyl-6-aza-bicyclo[3.2.1]oct-6-yl)-methanone;
3-[5-(1,3,3-Trimethyl-6-aza-bicyclo[3.2.1]octane-6-carbonyl)-1*H*-benzoimidazol-2-yl]-benzoic acid methyl ester;
(2-Thiophen-2-yl-1*H*-benzoimidazol-5-yl)-(1,3,3-trimethyl-6-aza-bicyclo[3.2.1]oct-6-yl)-methanone;
[2-(2-Nitro-phenyl)-*1H*-benzoimidazol-5-yl]-(1,3,3-trimethyl-6-aza-bicyclo[3.2.1]oct-6-yl)-methanon; or
a salt thereof with a pharmaceutically acceptable acid or base, or any optical isomer or mixture of optical isomers, including a racemic mixture, a prodrug thereof, or any tautomeric forms.

In another embodiment of the present invention said substituted amides, or a prodrug thereof, as a component of the combination therapy is of the general formula (IIe) 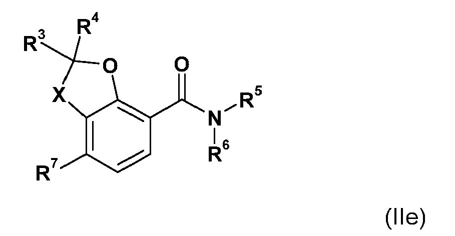 wherein
X is oxygen or (CR¹R²)ₙ;
R¹, R², R³, and R⁴ independently are hydrogen, C₁-C₆alkyl, aryl, arylC₁-C₆alkyl, hetaryl or hetarylC₁-C₆alkyl optionally substituted with one or more R⁸ independently; or
R¹ and either R³ or R⁴ together are forming a saturated or partially saturated ring system containing from 4 to 8 carbon atoms, the ring system optionally being substituted with at least one of C₁-C₆alkyl, hydroxy, oxo, aryl, hetaryl, arylC₁-C₆alkyl or hetarylC₁-C₆alkyl; or
R¹ and either R³ or R⁴ together with the single bond are forming a carbon-carbon double bond;
R⁵ is C₁-C₈alkyl optionally substituted with one or more of R⁹;
R⁶ is C₃-C₁₀cycloalkyl, C₃-C₁₀hetcycloalkyl, C₃-C₁₀cycloalkylC₁-C₆alkyl, wherein the alkyl, cycloalkyl and hetcycloalkyl groups independently are optionally substituted with one or more of R⁹; or
R⁵ and R⁶ together with the nitrogen to which they are attached, are forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁰;
R⁷ is hydrogen, halo, nitro, NR¹²R¹³, cyano, trihalomethyl, C₁-C₆alkyl, aryl, arylC₁-C₆alkyl, C₁-C₆alkyloxy, aryloxy, arylC₁-C₆alkyloxy, hetaryl, hetarylC₁-C₆alkyl, hetaryloxy or hetarylC₁-C₆alkyloxy optionally substituted with one or more R¹¹ independently;
R⁸ and R⁹ independently are hydrogen, hydroxy, oxo, halo, nitro, cyano, C₁-C₆alkyl, C₁-C₆alkyloxy, trihalomethyl, trihalomethoxy, NR¹²R¹³, arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R¹⁰ is hydrogen, C₁-C₈alkyl, arylC₁-C₆alkyl, hetarylC₁-C₆alkyl, C₁-C₆alkyloxy, arylC₁-C₆alkyloxy, hetarylC₁-C₆alkyloxy;
R¹¹ is hydrogen, halo, hydroxy, oxo, nitro, cyano, C₁-C₈alkyl, C₁-C₆alkyloxy, aryloxy or hetaryloxy;
R¹² and R¹³ independently are hydrogen, C₁-C₈alkyl, aryl, hetaryl, arylC₁-C₆alkyl, C₃-C₁₀cycloalkyl, C₃-C₁₀hetcycloalkyl, C₁-C₆alkylcarbonyl, arylC₁-C₆alkylcarbonyl, C₃-C₁₀cycloalkylC₁-C₆alkyl, C₁-C₆alkyloxycarbonyl; or
R¹² and R¹³ are together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-₆alkylcarboxy;
n is 1 or 2; or
a salt thereof with a pharmaceutically acceptable acid or base, or any optical isomer or mixture of optical isomers, including a racemic mixture, a prodrug thereof, or any tautomeric forms.

In another embodiment of the present invention said substituted amides, or a prodrug thereof, as a component of the combination therapy is of the general formula (IIe) wherein X is (CR¹R²)ₙ, wherein R¹, R² and n are as defined above.

In another embodiment of the present invention said substituted amides, or a prodrug thereof, as a component of the combination therapy is of the general formula (IIe) wherein n is 1.

In another embodiment of the present invention said substituted amides, or a prodrug thereof, as a component of the combination therapy is of the general formula (IIe) wherein X is oxygen.

In another embodiment of the present invention said substituted amides, or a prodrug thereof, as a component of the combination therapy is of the general formula (IIe) wherein R¹, R², R³, and R⁴ independently are hydrogen, C₁-C₆alkyl or arylC₁-C₆alkyl, optionally substituted with one or more R⁸.

In another embodiment of the present invention said substituted amides, or a prodrug thereof, as a component of the combination therapy is of the general formula (IIe) wherein R¹ and either R³ or R⁴ together with the single bond are forming a carbon-carbon double bond.

In another embodiment of the present invention said substituted amides, or a prodrug thereof, as a component of the combination therapy is of the general formula (IIe) wherein R⁵ is C₁-C₈alkyl optionally substituted with one or more of R⁹.

In another embodiment of the present invention said substituted amides, or a prodrug thereof, as a component of the combination therapy is of the general formula (IIe) wherein R⁶ is C₃-C₁₀cycloalkyl or C₃-C₁₀hetcycloalkyl each of which is optionally substituted with one or more of R⁹.

In another embodiment of the present invention said substituted amides, or a prodrug thereof, as a component of the combination therapy is of the general formula (IIe) wherein R⁶ is C₃-C₁₀cycloalkyl optionally substituted with one or more of R⁹.

In another embodiment of the present invention said substituted amides, or a prodrug thereof, as a component of the combination therapy is of the general formula (IIe) wherein R⁵ and R⁶ together with the nitrogen to which they are attached, are forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁰.

In another embodiment of the present invention said substituted amides, or a prodrug thereof, as a component of the combination therapy is of the general formula (lle) wherein R⁷ is hydrogen, halo, NR¹²R¹³, trihalomethyl, C₁-C₆alkyloxy, aryloxy, arylC₁-C₆alkyloxy or hetaryloxy optionally substituted with one or more R¹¹ independently.

In another embodiment of the present invention said substituted amides, or a prodrug thereof, as a component of the combination therapy is of the general formula (Ile) wherein R⁸ and R⁹ independently are hydrogen, hydroxy, oxo, halo, nitro, cyano, C₁-C₆alkyl, C₁-C₆alkyloxy, trihalomethyl, or NR¹²R¹³.

In another embodiment of the present invention said substituted amides, or a prodrug thereof, as a component of the combination therapy is of the general formula (IIe) wherein R¹⁰ is hydrogen or C₁-C₈alkyl.

In another embodiment of the present invention said substituted amides, or a prodrug thereof, as a component of the combination therapy is of the general formula (IIe) wherein the bicyclic ring system is 6-aza-bicyclo[3.2.1]octane optionally substituted with one or more of C₁-C₆alkyl.

In another embodiment of the present invention said substituted amides, or a prodrug thereof, as a component of the combination therapy is of the general formula (IIe) wherein the bicyclic ring system is 1,3,3-trimethyl-6-aza-bicyclo[3.2.1]octane.

In another embodiment of the present invention said substituted amides, or a prodrug thereof, as a component of the combination therapy is selected from the group consisting of:
2,3-Dimethyl-2,3-dihydro-benzofuran-7-carboxylic acid cyclohexyl-methyl-amide; 2,5-Dimethyl-3-phenyl-benzofuran-7-carboxylic acid cyclohexyl-methyl-amide; 2,2-Dimethyl-2,3-dihydro-benzofuran-7-carboxylic acid cyclohexyl-methyl-amide; 2-Methyl-2,3-dihydro-benzofuran-7-carboxylic acid cyclohexyl-methyl-amide;
(2,3-Dimethyl-2,3-dihydro-benzofuran-7-yl)-(2,4,4-trimethyl-6-aza-bicyclo[3.2.1]oct-6-yl)-methanone;
4-Methoxy-2-methyl-2,3-dihydro-benzofuran-7-carboxylic acid cyclohexyl-methyl-amide;
2-Methyl-benzofuran-7-carboxylic acid cyclohexyl-methyl-amide;
(2-Methyl-2,3-dihydro-benzofuran-7-yl)-(1,3,3-trimethyl-6-aza-bicyclo[3.2.1]oct-6-yl)-methanone;
Benzofuran-7-carboxylic acid cyclohexyl-methyl-amide;
2,3-Dihydro-benzofuran-7-carboxylic acid cyclohexyl-methyl-amide;
3,3-Dimethyl-2,3-dihydro-benzofuran-7-carboxylic acid cyclohexyl-methyl-amide;
Chroman-8-carboxylic acid cyclohexyl-methyl-amide; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is selected from the group consisting of:
2,3-Dihydro-benzofuran-7-carboxylic acid cyclohexyl-methyl-amide;
Benzofuran-7-carboxylic acid cyclohexyl-methyl-amide;
2-Methyl-2,3-dihydro-benzofuran-7-carboxylic acid cyclohexyl-methyl-amide;
2-Methyl-benzofuran-7-carboxylic acid cyclohexyl-methyl-amide;
3,3-Dimethyl-2,3-dihydro-benzofuran-7-carboxylic acid cyclohexyl-methyl-amide;
(2,3-Dimethyl-2,3-dihydro-benzofuran-7-yl)-(2,4,4-trimethyl-6-aza-bicyclo[3.2.1]oct-6-yl)-methanone;
4-Methoxy-2-methyl-2,3-dihydro-benzofuran-7-carboxylic acid cyclohexyl-methyl-amide;
2,2-Dimethyl-2,3-dihydro-benzofuran-7-carboxylic acid cyclohexyl-methyl-amide;
(2-Methyl-2,3-dihydro-benzofuran-7-yl)-(1,3,3-trimethyl-6-aza-bicyclo[3.2.1]oct-6-yl)-methanone;
Chroman-8-carboxylic acid cyclohexyl-methyl-amide; or
a salt thereof with a pharmaceutically acceptable acid or base, or any optical isomer or mixture of optical isomers, including a racemic mixture, a prodrug thereof, or any tautomeric forms.

In another embodiment of the present invention said substituted amides, or a prodrug thereof, as a component of the combination therapy is of the general formula (IIf) 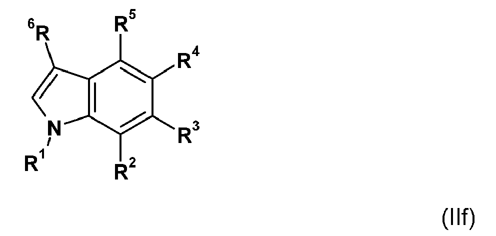 wherein
R¹ is hydrogen, C₁-C₈alkyl, hetaryl, arylC₁-C₆alkyl or hetarylC₁-C₆alkyl optionally substituted with one or more R⁹;
R², R³, R⁴, R⁵ and R⁶ independently are hydrogen, halo, nitro, cyano, trihalomethyl, carboxy, N(R¹²R¹³), C(O)NR⁷R⁸, C₁-C₈alkyl, C₃-C₁₀cycloalkyl, C₃-C₁₀hetcycloalkyl, N(R¹²R¹³)C₁-C₆alkyl, C₁-C₆alkyloxy, C₁-C₆alkyloxyC₁-C₆alkyl, aryl, arylC₁-C₆alkyl, aryloxy, aryloxyC₁-C₆alkyl, arylC₁-C₆alkyloxy, arylC₁-C₆alkyloxyC₁-C₆alkyl, C₁-C₆alkylcarboxy, arylcarboxy, arylC₁-C₆alkylcarboxy, hetaryl, hetarylC₁-C₆alkyl, hetarylC₁-C₆alkyloxy, hetaryloxyC₁-C₆alkyl or hetarylC₁-C₆alkyloxyC₁-C₆alkyl wherein wherein the alkyl, aryl, arylalkyl, hetaryl and hetarylalkyl groups independently are substituted with one or more R⁹;
R⁷ is hydrogen or C₁-C₈alkyl optionally substituted with one or more of R¹⁰;
R⁸is C₃-C₁₀cycloalkyl, C₃-C₁₀hetcydoalkyl , C₃-C₁₀cycloalkylC₁-C₆alkyl, wherein the cycloalkyl and hetcycloalkyl groups independently are optionally substituted with one or more of R¹⁰; or
R⁷ and R⁸ together with the nitrogen to which they are attached, are forming a saturated or partially saturated cyclic, bicyclic or tricyclic ring system containing from 4 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹;
R⁹ is hydrogen, hydroxy, oxo, halo, nitro, cyano, C₁-C₆alkyl, C₁-C₆alkyloxy, trihalomethyl, trihalomethoxy, NR¹²R¹³, arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R¹⁰ and R¹¹ independently are hydrogen, halo, oxo, hydroxy, C₁-C₆alkyl, aryl, arylC₁-C₆alkyl, hetaryl or hetarylalkyl;
R¹² and R¹³ independently are hydrogen, C₁-C₈alkyl, aryl, hetaryl, arylC₁-C₆alkyl, C₃-C₁₀cycloalkyl, C₃-C₁₀hetcydoalkyl, C₃-C₁₀cycloalkylC₁-C₆alkyl, C₁-C₆alkylcarbonyl, arylcarbonyl, arylC₁-C₆alkylcarbonyl, C₃-C₁₀cycloalkylcarbonyl, C₃-C₁₀hetcycloalkylcarbonyl or C₃-C₁₀cycloalkylC₁-C₆alkylcarbonyl wherein the alkyl and aryl groups independently are optionally substituted with one or more of R¹¹, wherein R¹¹ is as defined above; or
R¹² and R¹³ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-C₆alkyl-carbonyl, hetarylC₁-C₆alkylcarbonyl, C₁-C₆alkylcarboxy, arylcarboxy or arylC₁-C₆alkylcarboxy; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIf) wherein
R¹ is hydrogen, C₁-C₈alkyl, hetaryl, arylC₁-C₆alkyl or hetarylC₁-C₆alkyl optionally substituted with one or more R⁹;
R² and R⁵ independently are hydrogen, halo, nitro, cyano, trihalomethyl, C₁-C₆alkyl, C₁-C₆alkyloxy, C₁-C₆alkyloxyC₁-C₆alkyl, aryl, arylC₁-C₆alkyl, aryloxy, aryloxyC₁-C₆alkyl, arylC₁-C₆alkyloxy, arylC₁-C₆alkyloxyC₁-C₆alkyl, hetaryl or hetarylC₁-C₆alkyl wherein the alkyl, aryl, arylalkyl, hetaryl and hetarylalkyl groups independently are substituted with one or more R⁹; and
either R³ is C(O)NR⁷R⁸, and R⁴ is hydrogen; or R³ is hydrogen, and R⁴ is C(O)NR⁷R⁸;
R⁶ is C₁-C₈alkyl, C₃-C₁₀cycloalkyl, C₃-C₁₀hetcycloalkyl, N(R¹²R¹³)C₁-C₆alkyl, C₁-C₆alkyloxyC₁-C₆alkyl, aryloxyC₁-C₆alkyl, arylC₁-C₆alkyloxy or arylC₁-C₆alkyloxyC₁-C₆alkyl;
R⁷ is C₁-C₈alkyl optionally substituted with one or more of R¹⁰;
R⁸ is C₃-C₁₀cycloalkyl, C₃-C₁₀hetcycloalkyl, C₃-C₁₀cycloalkylC₁-C₆alkyl, wherein the cycloalkyl and hetcycloalkyl groups independently are optionally substituted with one or more of R¹⁰; or
R⁷ and R⁸ together with the nitrogen to which they are attached, are forming a saturated or partially saturated cyclic, bicyclic or tricyclic ring system containing from 6 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹;
R⁹ is hydrogen, hydroxy, oxo, halo, nitro, cyano, C₁-C₆alkyl, C₁-C₆alkyloxy, trihalomethyl, trihalomethoxy, NR¹²R¹³, arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R¹⁰ and R¹¹ independently are hydrogen, halo, oxo, hydroxy, C₁-C₆alkyl, aryl, arylC₁-C₆alkyl, hetaryl or hetarylalkyl;
R¹² and R¹³ independently are hydrogen, C₁-C₈alkyl, aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarbonyl, arylcarbonyl, arylC₁-C₆alkylcarbonyl, C₃-C₁₀cycloalkylcarbonyl, C₃-C₁₀hetcycloalkylcarbonyl or C₃-C₁₀cycloalkylC₁-C₆alkylcarbonyl; or
R¹² and R¹³ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyl-oxyC₁-C₆alkyl, C₁-C₆alkylcarbonyl, arylcarbonyl, hetarylcarbonyl, arylC₁-C₆alkyl-carbonyl, hetarylC₁-C₆alkylcarbonyl, C₁-C₆alkylcarboxy, arylcarboxy or arylC₁-C₆alkylcarboxy; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIf) wherein R² is C(O)NR⁷R⁸ and R³ R⁴ and R⁵ are hydrogen, wherein R⁷ and R⁸ are as defined above.

In another embodiment of the present invention said substituted amides, or a prodrug thereof, as a component of the combination therapy is of the general formula (IIf) wherein R³ is C(O)NR⁷R⁸ and R² R⁴ and R⁵ are hydrogen, wherein R⁷ and R⁸ are as defined above.

In another embodiment of the present invention said substituted amides, or a prodrug thereof, as a component of the combination therapy is of the general formula (IIf) wherein R⁴ is C(O)NR⁷R⁸ and R² R³ and R⁵ are hydrogen, wherein R⁷ and R⁸ are as defined above.

In another embodiment of the present invention said substituted amides, or a prodrug thereof, as a component of the combination therapy is of the general formula (IIf) wherein R⁵ is C(O)NR⁷R⁸ and R² R³ and R⁴ are hydrogen, wherein R⁷ and R⁸ are as defined above.

In another embodiment of the present invention said substituted amides, or a prodrug thereof, as a component of the combination therapy is of the general formula (IIf) wherein R⁶ is C(O)NR⁷R⁸, wherein R⁷ and R⁸ are as defined above.

In another embodiment of the present invention said substituted amides, or a prodrug thereof, as a component of the combination therapy is of the general formula (IIf) wherein R³ is C(O)NR⁷R⁸ and R⁴ is hydrogen, wherein R⁷ and R⁸ are as defined above.

In another embodiment of the present invention said substituted amides, or a prodrug thereof, as a component of the combination therapy is of the general formula (IIf) wherein R³ is hydrogen and R⁴ is C(O)NR⁷R⁸, wherein R⁷ and R⁸ are as defined above.

In another embodiment of the present invention said substituted amides, or a prodrug thereof, as a component of the combination therapy is of the general formula (IIf) wherein R⁸ is C₃-C₁₀cycloalkyl or C₃-C₁₀hetcycloalkyl, each of which is optionally substituted with one or more of R¹⁰, wherein R¹⁰ is as defined above.

In another embodiment of the present invention said substituted amides, or a prodrug thereof, as a component of the combination therapy is of the general formula (IIf) wherein R⁷ and R⁸ together with the nitrogen to which they are attached, are forming a saturated or partially saturated bicyclic or tricyclic ring system containing from 6 to 10 carbon atoms and from 0 to 2 additional heteroatoms selected from nitrogen or oxygen, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹, wherein R¹¹ is as defined above.

In another embodiment of the present invention said substituted amides, or a prodrug thereof, as a component of the combination therapy is of the general formula (IIf) wherein the bicyclic ring system is 6-aza-bicyclo[3.2.1]octane optionally substituted with one or more C₁-C₆alkyl.

In another embodiment of the present invention said substituted amides, or a prodrug thereof, as a component of the combination therapy is of the general formula (IIf) wherein the bicyclic ring system is 1,3,3-trimethyl-6-aza-bicyclo[3.2.1]octane.

In another embodiment of the present invention said substituted amides, or a prodrug thereof, as a component of the combination therapy is selected from the group consisting of:
(1*H*-Indol-5-yl)-(1,3,3-trimethyl-6-aza-bicyclo[3.2.1]oct-6-yl)-methanone;
1*H*-Indole-6-carboxylic acid cyclohexyl-methyl-amide;
(1*H*-Indol-7-yl)-(1,3,3-trimethyl-6-aza-bicyclo[3.2.1]oct-6-yl)-methanone;
(1*H*-Indol-6-yl)-(1,3,3-trimethyl-6-aza-bicyclo[3.2.1]oct-6-yl)-methanone;
1*H*-Indole-6-carboxylic acid adamantan-2-ylamide;
(6-Aza-bicyclo[3.2.1]oct-6-yl)-(1*H*-indol-6-yl)-methanone;
1*H*-Indole-6-carboxylic acid (8-methyl-8-aza-bicyclo[3.2.1]oct-3-yl)-amide;
1*H*-Indole-5-carboxylic acid adamantan-2-ylamide;
(6-Aza-bicyclo[3.2.1]oct-6-yl)-(1*H*-indol-5-yl)-methanone;
(1*H*-Indol-4-yl)-(1,3,3-trimethyl-6-aza-bicyclo[3.2.1]oct-6-yl)-methanone;
(1*H*-Indol-3-yl)-(1,3,3-trimethyl-6-aza-bicyclo[3.2.1]oct-6-yl)-methanone;
(1*H*-Indol-2-yl)-(1,3,3-trimethyl-6-aza-bicyclo[3.2.1]oct-6-yl)-methanone;
(1-Methyl-1*H*-indol-3-yl)-(1,3,3-trimethyl-6-aza-bicyclo[3.2.1]oct-6-yl)-methanone;
(3-Aza-bicyclo[3.2.2]non-3-yl)-(1*H*-indol-3-yl)-methanone;
1-Methyl-1*H*-indole-3-carboxylic acid cycloheptylamide;
1-Methyl-1*H*-indole-3-carboxylic acid adamantan-1-ylamide;
(3-Aza-bicyclo[3.2.2]non-3-yl)-(1-methyl-1*H*-indol-3-yl)-methanone;
(1-Methyl-1*H*-indol-3-yl)-(4-methyl-piperazin-1-yl)-methanone;
1-Methyl-1*H*-indole-3-carboxylic acid (3-hydroxy-adamantan-1-yl)-amide;
1-Methyl-1*H*-indole-3-carboxylic acid azepan-1-ylamide;
1-Methyl-1*H*-indole-3-carboxylic acid (2-oxo-azepan-3-yl)-amide;
(4-Benzyl-piperidine-1-yl)-(1-methyl-1*H*-indol-3-yl)-methanone;
1-Methyl-1*H*-indole-3-carboxylic acid (2,6-dimethyl-piperidin-1-yl)-amide;
1-Methyl-1*H*-indole-3-carboxylic acid (2-methyl-piperidin-1-yl)-amide;
(1-Cyclopropylmethyl-6-fluoro-1*H*-indol-3-yl)-(1,3,3-trimethyl-6-aza-bicyclo[3.2.1]oct-6-yl)-methanone;
Azepan-1-yl-(1-methyl-1*H*-indol-3-yl)-methanone;
(5-Benzyloxy-1*H*-indol-3-yl)-(1,3,3-trimethyl-6-aza-bicyclo[3.2.1]oct-6-yl)-methanone;
(5*H*-[1,3]Dioxolo[4,5]indol-7-yl)-(1,3,3-trimethyl-6-aza-bicyclo[3.2.1]oct-6-yl)-methanone;
(5-Chloro-1*H*-indol-3-yl)-(1,3,3-trimethyl-6-aza-bicyclo[3.2.1]oct-6-yl)-methanone;
(6-Trifluoromethyl-1*H*-indol-3-yl)-(1,3,3-trimethyl-6-aza-bicyclo[3.2.1]oct-6-yl)-methanone;
(6-Methyl-1*H*-indol-3-yl)-(1,3,3-trimethyl-6-aza-bicyclo[3.2.1]oct-6-yl)-methanone;
(6-Nitro-1*H*-indol-3-yl)-(1,3,3-trimethyl-6-aza-bicyclo[3.2.1]oct-6-yl)-methanone;
(5-Methoxy-1*H*-indol-3-yl)-(1,3,3-trimethyl-6-aza-bicyclo[3.2.1]oct-6-yl)-methanone;
(6-Fluoro-1*H*-indol-3-yl)-(1,3,3-trimethyl-6-aza-bicyclo[3.2.1]oct-6-yl)-methanone;
(6-Methoxy-1*H-*indol-3-yl)-(1,3,3-trimethyl-6-aza-bicyclo[3.2.1]oct-6-yl)-methanone;
(7-Nitro-1*H*-indol-3-yl)-(1,3,3-trimethyl-6-aza-bicyclo[3.2.1]oct-6-yl)-methanone;
(1*H*-Indol-4-yl)-(1,3,3-trimethyl-6-aza-bicyclo[3.2.1]oct-6-yl)-methanone;
2-(1*H*-Indol-3-yl)-1-(1,3,3-trimethyl-6-aza-bicyclo[3.2.1]oct-6-yl)-ethanone;
1-(3-Aza-bicyclo[3.2.2]non-3-yl)-2-(1*H*-indol-3-yl)-ethanone;
1-(3-Aza-bicyclo[3.2.2]non-3-yl)-2-(1-methyl-1*H*-indol-3-yl)-ethanone;
2-(1-Methyl-1*H*-indol-3-yl)-1-(1,3,3-trimethyl-6-aza-bicyclo[3.2.1]oct-6-yl)-ethanone;
[3-(1,3,3-Trimethyl-6-aza-bicyclo[3.2.1]octane-6-carbonyl)-1*H*-indol-6-yloxy]-acetic acid *tert-*butyl ester;
6-(1,3,3-Trimethyl-6-aza-bicyclo[3.2.1]octane-6-carbonyl)-1*H*-indole-3-carboxylic acid; 6-(1,3,3-Trimethyl-6-aza-bicyclo[3.2.1]octane-6-carbonyl)-1*H*-indole-3-carboxylic acid ethyl ester;
5-(1,3,3-Trimethyl-6-aza-bicyclo[3.2.1]octane-6-carbonyl)-1*H*-indole-3-carboxylic acid; 5-(1,3,3-Trimethyl-6-aza-bicyclo[3.2.1]octane-6-carbonyl)-1*H*-indole-3-carboxylic acid ethyl ester;
4-(1,3,3-Trimethyl-6-aza-bicyclo[3.2.1]octane-6-carbonyl)-1*H*-indole-3-carboxylic acid; 4-(1,3,3-Trimethyl-6-aza-bicyclo[3.2.1]octane-6-carbonyl)-1*H*-indole-3-carboxylic acid ethyl ester;
5-(1,3,3-Trimethyl-6-aza-bicyclo[3.2.1]octane-6-carbonyl)-1*H*-indole-3-carboxylic acid; 5-(1,3,3-Trimethyl-6-aza-bicyclo[3.2.1]octane-6-carbonyl)-1*H*-indole-3-carboxylic acid ethyl ester;
4-(1,3,3-Trimethyl-6-aza-bicyclo[3.2.1]octane-6-carbonyl)-1*H*-indole-3-carboxylic acid; 4-(1,3,3-Trimethyl-6-aza-bicyclo[3.2.1]octane-6-carbonyl)-1*H*-indole-3-carboxylic acid ethyl ester;
[3-(Piperidine-1-carbonyl)-1*H*-indol-5-yl]-(1,3,3-trimethyl-6-aza-bicyclo[3.2.1]oct-6-yl)-methanone;
5-(1,3,3-Trimethyl-6-aza-bicyclo[3.2.1]octane-6-carbonyl)-1*H*-indole-3-carboxylic acid cyanomethyl-amide;
5-(1,3,3-Trimethyl-6-aza-bicyclo[3.2.1]octane-6-carbonyl)-1*H*-indole-3-carboxylic acid benzylamide;
5-(1,3,3-Trimethyl-6-aza-bicyclo[3.2.1]octane-6-carbonyl)-1*H*-indole-3-carboxylic acid dimethylamide;
5-(1,3,3-Trimethyl-6-aza-bicyclo[3.2.1]octane-6-carbonyl)-1*H*-indole-3-carboxylic acid allylamide;
5-(1,3,3-Trimethyl-6-aza-bicyclo[3.2.1]octane-6-carbonyl)-1*H*-indole-3-carboxylic acid (2-dimethylamino-ethyl)-methyl-amide;
5-(1,3,3-Trimethyl-6-aza-bicyclo[3.2.1]octane-6-carbonyl)-1*H*-indole-3-carboxylic acid (2-methoxy-ethyl)-amide;
5-(1,3,3-Trimethyl-6-aza-bicyclo[3.2.1]octane-6-carbonyl)-1*H*-indole-3-carboxylic acid 4-methoxy-benzylamide;
5-(1,3,3-Trimethyl-6-aza-bicyclo[3.2.1]octane-6-carbonyl)-1*H*-indole-3-carboxylic acid (tetrahydro-furan-2-ylmethyl)-amide;
[3-(2-Methoxymethyl-pyrrolidine-1-carbonyl)-1*H*-indol-5-yl]-(1,3,3-trimethyl-6-aza-bicyclo-[3.2.1]oct-6-yl)-methanone;
[3-(2,6-Dimethyl-morpholine-4-carbonyl)-1*H*-indol-5-yl]-(1,3,3-trimethyl-6-aza-bicyclo-[3.2.1]oct-6-yl)-methanone;
5-(1,3,3-Trimethyl-6-aza-bicyclo[3.2.1]octane-6-carbonyl)-1*H*-indole-3-carboxylic acid (1,1-dioxo-tetrahydro-thiophen-3-yl)-amide;
5-(1,3,3-Trimethyl-6-aza-bicyclo[3.2.1]octane-6-carbonyl)-1*H*-indole-3-carboxylic acid [3-(4-methyl-piperazin-1-yl)-propyl]-amide;
5-(1,3,3-Trimethyl-6-aza-bicyclo[3.2.1]octane-6-carbonyl)-1*H*-indole-3-carboxylic acid 4-trifluoromethyl-benzylamide;
5-(1,3,3-Trimethyl-6-aza-bicyclo[3.2.1]octane-6-carbonyl)-1*H*-indole-3-carboxylic acid (furan-2-ylmethyl)-amide;
[3-(2,3,5,6-Tetrahydro-[1,2']bipyrazinyl-4-carbonyl)- 1*H*-indol-5-yl]-(1,3,3-trimethyl-6-aza-bicyclo[3.2.1]oct-6-yl)-methanone;
5-(1,3,3-Trimethyl-6-aza-bicyclo[3.2.1]octane-6-carbonyl)-1*H*-indole-3-carboxylic acid (2*H-*tetrazol-5-ylmethyl)-amide;
[3-(1,3,3-Trimethyl-6-aza-bicyclo[3.2.1]octane-6-carbonyl)-1*H*-indol-5-yl]-(1,3,3-trimethyl-6-aza-bicyclo[3.2.1]oct-6-yl)-methanone;
3-{[5-(1,3,3-Trimethyl-6-aza-bicyclo[3.2.1]octane-6-carbonyl)-1*H*-indole-3-carbonyl]-amino}-propionic acid ethyl ester;
5-(1,3,3-Trimethyl-6-aza-bicyclo[3.2.1]octane-6-carbonyl)-1*H*-indole-3-carboxylic acid (4-methoxy-phenyl)-amide;
3-{[5-(1,3,3-Trimethyl-6-aza-bicyclo[3.2.1]octane-6-carbonyl)-1*H*-indole-3-carbonyl]-amino}-propionic acid;
Azepan-1-yl-(1*H*-indol-5-yl)-methanone;
1*H*-Indole-5-carboxylic acid dibenzylamide;
(3-Aza-bicyclo[3.2.2]non-3-yl)-(1*H*-indol-5-yl)-methanone;
(4-Benzyl-piperidin-1-yl)-(1*H*-indol-5-yl)-methanone;
8-(1*H*-Indole-5-carbonyl)-1-phenyl-1,3,8-triaza-spiro[4.5]decan-4-one;
[4-(4-Chloro-phenyl)-4-hydroxy-piperidin-1-yl]-(1*H*-indol-5-yl)-methanone;
1-[1-(1*H*-Indole-5-carbonyl)-piperidin-4-yl]-1,3-dihydro-benzoimidazol-2-one;
(4-*tert*-Butyl-piperidin-1-yl)-(1*H*-indol-5-yl)-methanone;
1-(1*H*-Indole-5-carbonyl)-4-phenyl-piperidine-4-carbonitrile;
(1*H*-Indol-5-yl)-(4-phenyl-piperidin-1-yl)-methanone;
(5-Benzyl-2,5-diaza-bicyclo[2.2.1]hept-2-yl)-(1*H*-indol-5-yl)-methanone;
(1*H*-Indol-5-yl)-(4-pyrrolidin-1-yl-piperidin-1-yl)-methanone;
1*H*-Indole-5-carboxylic acid (5-hydroxy-1,3,3-trimethyl-cyclohexylmethyl)-amide;
1*H*-Indole-5carboxylic acid (3,4-dihydrospiro(1*H*-indene-1,4-piperidine)-amide;
(3-Methanesulfonylmethyl-1*H*-indol-5-yl)-(1,3,3-trimethyl-6-aza-bicyclo[3.2.1]oct-6-yl)-methanone;
(3-Dimethylaminomethyl-1*H*-indol-6-yl)-(1,3,3-trimethyl-6-aza-bicyclo[3.2.1]oct-6-yl)-methanone;
1-{3-Acetyl-2-[5-(1,3,3-trimethyl-6-aza-bicyclo[3.2.1]octane-6-carbonyl)-1*H*-indol-3-yl]-2,3-dihydro-imidazol-1-yl}-ethanone;
1-Ethyl-3-[5-(1,3,3-trimethyl-6-aza-bicyclo[3.2.1]octane-6-carbonyl)-1*H*-indol-3-yl]-pyrrolidine-2,5-dione;
(3-Thiazol-2-yl-1*H*-indol-5-yl)-(1,3,3-trimethyl-6-aza-bicyclo[3.2.1]oct-6-yl)-methanone;
(3-lodo-1*H*-indol-5-yl)-(1,3,3-trimethyl-6-aza-bicyclo[3.2.1]oct-6-yl)-methanone;
6-(1,3,3-Trimethyl-6-aza-bicyclo[3.2.1]octane-6-carbonyl)-1*H*-indole-3-carbonitrile;
5-(1,3,3-Trimethyl-6-aza-bicyclo[3.2.1]octane-6-carbonyl)-1*H*-indole-3-carbonitrile;
6-(1,3,3-Trimethyl-6-aza-bicyclo[3.2.1]octane-6-carbonyl)-1*H*-indole-3-carboxylic acid amide;
[3-(2H-Tetrazol-5-yl)-1*H*-indol-6-yl]-(1,3,3-trimethyl-6-aza-bicyclo[3.2.1]oct-6-yl)-methanone;
*N*-[3-(1,3,3-Trimethyl-6-aza-bicyclo[3.2.1]octane-6-carbonyl)-1*H*-indol-7-yl]-acetamide;
(1-Benzenesulfonyl-1*H*-indol-5-yl)-(1,3,3-trimethyl-6-aza-bicyclo[3.2.1]oct-6-yl)-methanone;
(1-Benzenesulfonyl-2-methyl-1*H*-indol-5-yl)-(1,3,3-trimethyl-6-aza-bicyclo[3.2.1]oct-6-yl)-methanone;
(1-methyl-1*H*-indol-5-yl)-(1,3,3-trimethyl-6-aza-bicyclo[3.2.1]oct-6-yl)-methanone;
(1-Benzyl-1*H*-indol-5-yl)-(1,3,3-trimethyl-6-aza-bicyclo[3.2.1]oct-6-yl)-methanone;
[6-(1,3,3-Trimethyl-6-aza-bicyclo[3.2.1]octane-6-carbonyl)-indol-1-yl]-acetic acid ethyl ester;
[1-(2-Ethoxy-ethyl)-1*H*-indol-6-yl]-(1,3,3-trimethyl-6-aza-bicyclo[3.2.1]oct-6-yl)-methanone;
{1-[2-(2-Methoxy-ethoxy)-ethyl]-1*H*-indol-6-yl}-(1,3,3-trimethyl-6-aza-bicyclo[3.2.1]oct-6-yl)-methanone;
3-[5-(1,3,3-Trimethyl-6-aza-bicyclo[3.2.1]octane-6-carbonyl)-indol-1-yl]-propionic acid ethyl ester;
(1-Phenethyl-1*H*-indol-5-yl)-(1,3,3-trimethyl-6-aza-bicyclo[3.2.1]oct-6-yl)-methanone;
[1-(Tetrahydro-furan-2-ylmethyl)-1*H*-indol-5-yl]-(1,3,3-trimethyl-6-aza-bicyclo[3.2.1]oct-6-yl)-methanone;
2-[5-(1,3,3-Trimethyl-6-aza-bicyclo[3.2.1]octane-6-carbonyl)-indol-1-yl]-acetamide;
[1-(4-Trifluoromethoxy-benzyl)-1*H*-indol-5-yl]-(1,3,3-trimethyl-6-aza-bicyclo[3.2.1]oct-6-yl)-methanone;
3-[5-(1,3,3-Trimethyl-6-aza-bicyclo[3.2.1]octane-6-carbonyl)-indol-1-ylmethyl]-benzoic acid methyl ester;
4-[5-(1,3,3-Trimethyl-6-aza-bicyclo[3.2.1]octane-6-carbonyl)-indol-1-ylmethyl]-benzonitrile; or a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIg) 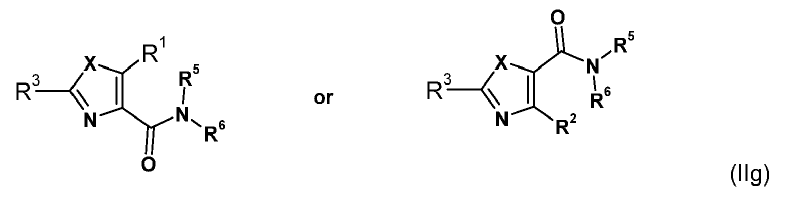 wherein
X is NR⁴, S or O;
R¹ and R² independently are hydrogen, halo, cyano, trihalomethyl, C₁-C₆alkyl or C₁-C₆alkyloxy, wherein the alkyl groups independently are optionally substituted with one or more of R⁷;
R³ is hydrogen, C₁-C₆alkyl, C₃-C₁₀cycloalkyl, C₁-C₆alkyloxy, C₁-C₆alkylthio, aryl, arylC₁-C₆alkyl, hetaryl or hetarylalkyl, wherein the alkyl, cycloalkyl, aryl, hetaryl and hetarylalkyl groups independently are optionally substituted with one or more of R⁷;
R⁴ is hydrogen, C₁-C₈alkyl, C₁-C₆alkyloxyC₁-C₆alkyl, aryl, hetaryl, hetarylC₁-C₆alkyl, arylC₁-C₆alkyl, arylC₁-C₆alkyloxyC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl wherein the alkyl, aryl, hetaryl, cycloalkyl and hetcycloalkyl groups independently are optionally substituted with one or more of R⁷;
R⁵ is hydrogen, and R⁶ is adamantyl optionally substituted with hydroxy, C₁-C₆alkyloxy, aryl, arylC₁-C₆alkyl, aryloxy, arylC₁-C₆alkyloxy, hetaryl, hetaryloxy or hetarylC₁-C₆alkyloxy wherein the alkyl, aryl and hetaryl groups independently are optionally substituted with one or more of R⁷; or
R⁵ and R⁶ are together with the nitrogen to which they are attached, forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylalkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁷;
R⁷ are independently hydrogen, halo, hydroxy, oxo, nitro, NR⁹R¹⁰, cyano, COOR⁸, CONR⁹R¹⁰, C₁-C₈alkyl, C₁-C₆alkyloxy, aryloxy, arylC₁-C₆alkyloxy, hetaryloxy or hetarylC₁-C₆alkyloxy;
R⁸ is hydrogen, C₁-C₆alkyl, aryl, arylC₁-C₆alkyl, hetarylalkyl, wherein the alkyl, aryl and hetarylalkyl groups independently are optionally substituted with one or more of R⁷;
R⁹ and R¹⁰ independently are hydrogen, C₁-C₈alkyl, C₃-C₁₀cycloalkyl, C₃-C₁₀hetcycloalkyl, C₃-C₁₀cycloalkylC₁-C₆alkyl, wherein the alkyl, cycloalkyl and hetcycloalkyl groups independently are optionally substituted with one or more of R⁷; or
R⁹ and R¹⁰ together with the nitrogen to which they are attached, are forming a saturated or partially saturated bicyclic or tricyclic ring system containing from 4 to 10 carbon atoms and from 0 to 2 additional heteroatoms selected from nitrogen or oxygen, the ring system optionally being substituted with at least one of C₁-C₈alkyl, arylC₁-C₆alkyl, hetarylC₁-C₆alkyl, hydroxy, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹;
R¹¹ is hydrogen, halo, oxo, hydroxy, C₁-C₆alkyl, aryl, arylC₁-C₆alkyl, hetaryl or hetarylalkyl;
a salt thereof with a pharmaceutically acceptable acid or base, or any optical isomer or mixture of optical isomers, including a racemic mixture, or any tautomeric forms.
In the definitions of R⁴, in the above formula (IIg), hetcycloalkyl cannot be 7-aza[2,2,1]bicycleheptane.

In another embodiment of the present invention said substituted amides, or a prodrug thereof, as a component of the combination therapy is of the general formula (llg) wherein
X is NR⁴, S or O;
R¹ and R² independently are hydrogen, halo, cyano, trihalomethyl, C₁-C₆alkyl or C₁-C₆alkyloxy, wherein the alkyl groups independently are optionally substituted with one or more of R⁷;
R³ is hydrogen, C₁-C₆alkyl, C₃-C₁₀cycloalkyl, C₁-C₆alkyloxy, C₁-C₆alkylthio, aryl, arylC₁-C₆alkyl, hetaryl or hetarylalkyl, wherein the alkyl, cycloalkyl, aryl, hetaryl and hetarylalkyl groups independently are optionally substituted with one or more of R⁷;
R⁴ is hydrogen, C₁-C₈alkyl, C₁-C₆alkyloxyC₁-C₆alkyl, aryl, hetaryl, arylC₁-C₆alkyl, arylC₁-C₆alkyloxyC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl wherein the alkyl, aryl, hetaryl, cycloalkyl and hetcycloalkyl groups independently are optionally substituted with one or more of R⁷;
R⁵ is hydrogen, and R⁶ is adamantyl optionally substituted with hydroxy, C₁-C₆alkyloxy, aryl, arylC₁-C₆alkyl, aryloxy, arylC₁-C₆alkyloxy, hetaryl, hetaryloxy or hetarylC₁-C₆alkyloxy wherein the alkyl, aryl and hetaryl groups independently are optionally substituted with one or more of R⁷; or
R⁵ and R⁶ are together with the nitrogen to which they are attached, forming a saturated or partially saturated cyclic, bicyclic or tricyclic ring system containing from 5 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylalkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁷;
R⁷ are independently hydrogen, halo, hydroxy, oxo, nitro, NR⁵R⁶, cyano, COOR⁸, CONR⁵R⁶, C₁-C₈alkyl, C₁-C₆alkyloxy, aryloxy or hetaryloxy;
R⁸ is hydrogen, C₁-C₆alkyl, aryl, arylC₁-C₆alkyl, hetarylalkyl, wherein the alkyl, aryl and hetarylalkyl groups independently are optionally substituted with one or more of R⁷; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is of the general formula (IIg) wherein X is NR⁴ or S wherein R⁴ is defined as above.

In another embodiment of the present invention said substituted amides, or a prodrug thereof, as a component of the combination therapy is of the general formula (IIg) wherein X is O.

In another embodiment of the present invention said substituted amides, or a prodrug thereof, as a component of the combination therapy is of the general formula (IIg) wherein X is S.

In another embodiment of the present invention said substituted amides, or a prodrug thereof, as a component of the combination therapy is of the general formula (IIg) wherein is NR⁴ wherein R⁴ is defined as above.

In another embodiment of the present invention said substituted amides, or a prodrug thereof, as a component of the combination therapy is of the general formula (IIg) wherein R¹ and R² independently are hydrogen, halo, trihalomethyl or C₁-C₆alkyl, wherein the alkyl groups independently are optionally substituted with one or more of R⁷.

In another embodiment of the present invention said substituted amides, or a prodrug thereof, as a component of the combination therapy is of the general formula (llg) wherein R³ is hydrogen, C₁-C₆alkyl, aryl, arylC₁-C₆alkyl, hetaryl or hetarylalkyl, wherein the alkyl, cycloalkyl, aryl, hetaryl and hetarylalkyl groups independently are optionally substituted with one or more of R⁷.

In another embodiment of the present invention said substituted amides, or a prodrug thereof, as a component of the combination therapy is of the general formula (IIg) wherein R⁴ is hydrogen, C₁-C₈alkyl, aryl, hetaryl, hetarylC₁-C₆alkyl, arylC₁-C₆alkyl, wherein the alkyl, aryl, hetaryl, groups independently are optionally substituted with one or more of R⁷.

In another embodiment of the present invention said substituted amides, or a prodrug thereof, as a component of the combination therapy is of the general formula (IIg) wherein R⁵ and R⁶ are together with the nitrogen to which they are attached, forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylalkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁷.

In another embodiment of the present invention said substituted amides, or a prodrug thereof, as a component of the combination therapy is of the general formula (IIg) wherein R⁵ and R⁶, together with the nitrogen to which they are attached, are azepane, azo-cane, 6-aza-bicyclo[3.2.1]octane, 8-aza-bicyclo[3.2.1]octane, 3-aza-bicyclo[3.2.1]octane, 2-aza-bicyclo[3.2.1]octane, 3-oxa-6-aza-bicyclo[3.2.1]octane, 6-aza-bicyclo[3.2.2]nonane, 3-aza-bicyclo[3.2.2]nonane, 4-aza-tricydo[4.3.1.1^{3,8}]undecane.

In another embodiment of the present invention said substituted amides, or a prodrug thereof, as a component of the combination therapy is selected from the group consisting of :
(4-Methyl-2-phenyl-thiazol-5-yl)-(1,3,3-trimethyl-6-aza-bicyclo[3.2.1]oct-6-yl)-methanone ;
(2,4-Dimethyl-thiazol-5-yl)-(1,3,3-trimethyl-6-aza-bicyclo[3.2.]oct-6-yl)-methanone;
(4-Methyl-2-pyrazin-2-yl-thiazol-5-yl)-(1,3,3-trimethyl-6-aza-bicyclo[3.2.1]oct-6-yl)-methanone;
[4-Methyl-2-(4-trifluoromethyl-phenyl)-thiazol-5-yl]-(1,3,3-trimethyl-6-aza-bicyclo[3.2.1]oct-6-yl)-methanone;
(3-Aza-bicyclo[3.2.2]non-3-yl)-(2,4-dimethyl-thiazol-5-yl)-methanone;
(1H-lmidazol-4-yl)-(1,3,3-trimethyl-6-aza-bicyclo[3.2.1]oct-6-yl)-methanone;
(3-Aza-bicyclo[3.2.2]non-3-yl)-(4-methyl-2-phenyl-thiazol-5-yl)-methanone;
2,4-Dimethyl-thiazole-5-carboxylic acid cycloheptylamide;
Azepan-1-yl-(2,4-dimethyl-thiazol-5-yl)-methanone;
2,4-Dimethyl-thiazole-5-carboxylic acid adamantan-1-ylamide;
(3-Aza-bicyclo[3.2.2] non-3-yl)-(1H-imidazol-4-yl)-methanone;
2,4-Dimethyl-thiazole-5-carboxylic acid (3-hydroxy-adamantan-1-yl)-amide; or
a salt thereof with a pharmaceutically acceptable acid or base, or any optical isomer or mixture of optical isomers, including a racemic mixture, or any tautomeric forms.

In another embodiment of the present invention said substituted amides, or a prodrug thereof, as a component of the combination therapy is selected from the group consisting of :
(1-Methyl-1H-imidazol-4-yl)-(1,3,3-trimethyl-6-aza-bicyclo[3.2.1]oct-6-yl)-methanone;
[1-(6-Methyl-pyridin-2-yl)-1H-imidazol-4-yl]-(1,3,3-trimethyl-6-aza-bicyclo[3.2.1]oct-6-yl)-methanone;
[1-(4-Chloro-benzyl)-5-methyl-1H-imidazol-4-yl]-(1,3,3-trimethyl-6-aza-bicyclo[3.2.1]oct-6-yl)-methanone; or
a salt thereof with a pharmaceutically acceptable acid or base, or any optical isomer or mixture of optical isomers, including a racemic mixture, or any tautomeric forms.

In another embodiment of the present invention said fused 1,2,4-triazole, or a prodrug thereof, as a component of the combination therapy is of the general formula (III) 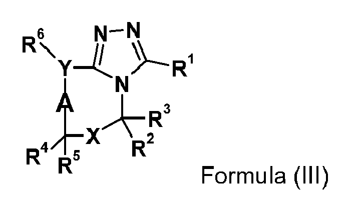 wherein
R¹ is C₅-C₁₀cycloalkyl, C₅-C₁₀hetcycloalkyl, aryl, hetaryl, arylC₁-C₆alkyl or hetarylC₁-C₆alkyl, wherein the cycloalkyl, hetcycloalkyl, aryl, hetaryl and arylalkyl groups independently are optionally substituted with one or more of R⁷;
R² and R³ independently are hydrogen, C₁-C₆alkyl, aryl, hetaryl, arylC₁-C₆alkyl, hetarylC₁-C₆alkyl, aryloxy, hetaryloxy, arylC₁-C₆alkyloxy or hetarylC₁-C₆alkyloxy wherein the alkyl, aryl, hetaryl, arylalkyl and hetarylalkyl groups independently are optionally substituted with one or more of R⁸; or
R² and R³ together with the carbon atom to which they are attached, are forming a saturated or partially saturated cyclic ring system containing from 3 to 6 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy;
R⁴ and R⁵ independently are hydrogen, halo, hydroxy, C₁-C₆alkyl, C₂-C₆alkenyl, C₂-C₆alkynyl, C₁-C₆alkyloxy, aryl, hetaryl, aryloxyC₁-C₆alkyl, aryloxyaryl, hetaryloxyaryl, aryloxyhetaryl, hetaryloxyhetaryl or arylC₁-C₆alkyloxyC₁-C₆alkyl wherein the alkyl, alkenyl, alkynyl, aryl and hetaryl groups independently are optionally substituted with one or more of R⁹; or
R⁴ and R⁵ together with the carbon atoms to which they are attached, are forming a saturated, partially saturated or aromatic ring system containing from 5 to 8 carbon atoms and from 0 to 2 additional heteroatoms selected from nitrogen, oxygen or sulphur, the ring system optionally being substituted with at least one of C₁-C₆alkyl, aryl, arylC₁-C₆alkyl, NR¹⁰R¹¹, halo, trihalomethyl, trihalomethoxy, hydroxy, oxo, C₁-C₆alkyloxy, arylC₁-C₆alkyloxy, C₁-C₆alkyloxy-C₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkylcarboxy; or
R⁴ and either R² or R³ together are forming a saturated or partially saturated bridge containing from 1 to 4 carbon atoms, the bridge can optionally be substituted with at least one of C₁-C₆alkyl, aryl, hetaryl, arylC₁-C₆alkyl or hetarylC₁-C₆alkyl;
R⁶ is hydrogen, C₁-C₆alkyl, aryl, hetaryl, arylC₁-C₆alkyl or hetarylC₁-C₆alkyl; or
R⁶ and either R⁴ or R⁵ together with the carbon atoms to which they are attached, are forming a saturated, partially saturated or aromatic ring system containing from 5 to 8 carbon atoms and from 0 to 2 additional heteroatoms selected from nitrogen, oxygen or sulphur, the ring system optionally being substituted with at least one of C₁-C₆alkyl, aryl, arylC₁-C₆alkyl, NR¹⁰R¹¹, halo, trihalomethyl, trihalomethoxy, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R⁷ and R⁸ independently are hydrogen, halo, hydroxy, cyano, nitro, C₁-C₆alkyl, C₁-C₆alkyloxy, trihalomethyl, trihalomethoxy, aryloxy, hetaryloxy, arylC₁-C₆alkyloxy, hetarylC₁-C₆alkyloxy, C₁-C₆alkyloxyC₁-C₆alkyl, aryloxyC₁-C₆alkyl, arylC₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R⁹ is hydrogen, halo, hydroxy, cyano, C₁-C₆alkyl, methylendioxo, trihalomethyl, trihalomethoxy, aryl, arylC₁-C₆alkyl, aryloxy, NR¹⁰R¹¹ or aryloxyC₁-C₆alkyl, wherein the aryl group is optionally substituted with one or more of R¹²;
R¹⁰ and R¹¹ independently are hydrogen, C₁-C₆alkyl, aryl or arylC₁-C₆alkyl wherein the alkyl and aryl groups independently are optionally substituted with one or more of R¹³; or
R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one of C₁-C₆alkyl, aryl, hetaryl, arylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkyl-carboxy;
R¹² is oxo or halo;
R¹³ is halo, hydroxy, cyano, C₁-C₆alkyl, C₁-C₆alkyloxy, NR¹⁴R¹⁵, methylendioxo, trihalomethyl, or trihalomethoxy;
R¹⁴ and R¹⁵ independently are hydrogen, C₁-C₆alkyl, aryl, hetaryl, arylC₁-C₆alkyl or hetarylC₁-C₆alkyl;
A is a single, double or triple bond;
X is a chemical bond, (CR¹⁶R¹⁷)ₙ or NR¹⁰, wherein R¹⁶ and R¹⁷ independently are hydrogen, oxo or C₁-C₆alkyl, or
X, together with either R² or R³, is a double bond;
Y is CR¹⁸ or nitrogen, wherein R¹⁸ is hydrogen, C₁-C₆alkyl, aryl, hetaryl, arylC₁-C₆alkyl or hetarylC₁-C₆alkyl; or
R¹⁸ and either R² or R³ together are forming a saturated or partially saturated cyclic ring system containg from 1 to 4 carbon atoms, the ring system can optionally be substituted with at least one of C₁-C₆alkyl, aryl, hetaryl, arylC₁-C₆alkyl or hetarylC₁-C₆alkyl; or
R¹⁸ with either R² or R³ and either R⁴ or R⁵ together are forming a saturated or partially saturated cyclic ring system having one common carbon atom containing from 8 to 12 carbon atoms, the ring system can optionally be substituted with at least one of C₁-C₆alkyl, aryl, hetaryl, arylC₁-C₆alkyl or hetarylC₁-C₆alkyl;
n is 0, 1 or 2; or
a salt thereof with a pharmaceutically acceptable acid or base, or any optical isomer or mixture of optical isomers, including a racemic mixture, or any tautomeric forms.

In another embodiment of the present invention said fused 1,2,4-triazole, or a prodrug thereof, as a component of the combination therapy is of the general formula (III) wherein R¹ is C₅-C₁₀cycloalkyl, C₅-C₁₀hetcycloalkyl, aryl, hetaryl, or arylC₁-C₆alkyl, wherein the cycloalkyl, hetcycloalkyl, aryl, hetaryl and arylalkyl groups independently are optionally substituted with one or more of R⁷.

In another embodiment of the present invention said fused 1,2,4-triazole, or a prodrug thereof, as a component of the combination therapy is of the general formula (III) wherein R¹ is C₅-C₁₀cycloalkyl, C₅-C₁₀hetcycloalkyl, aryl, wherein the cycloalkyl, hetcycloalkyl and aryl groups independently are optionally substituted with one or more of R⁷.

In another embodiment of the present invention said fused 1,2,4-triazole, or a prodrug thereof, as a component of the combination therapy is of the general formula (III) wherein R¹ is C₅-C₁₀cycloalkyl optionally substituted with one or more of R⁷.

In another embodiment of the present invention said fused 1,2,4-triazole, or a prodrug thereof, as a component of the combination therapy is of the general formula (III) wherein R¹ is C₅-C₁₀hetcycloalkyl optionally substituted with one or more of R⁷.

In another embodiment of the present invention said fused 1,2,4-triazole, or a prodrug thereof, as a component of the combination therapy is of the general formula (III) wherein R¹ is aryl, optionally substituted with one or more of R⁷.

In another embodiment of the present invention said fused 1,2,4-triazole, or a prodrug thereof, as a component of the combination therapy is of the general formula (III) wherein R¹ is hetaryl optionally substituted with one or more of R⁷.

In another embodiment of the present invention said fused 1,2,4-triazole, or a prodrug thereof, as a component of the combination therapy is of the general formula (III) wherein R¹ is arylC₁-C₆alkyl optionally substituted with one or more of R⁷.

In another embodiment of the present invention said fused 1,2,4-triazole, or a prodrug thereof, as a component of the combination therapy is of the general formula (III) wherein R² and R³ independently are hydrogen or C₁-C₆alkyl.

In another embodiment of the present invention said fused 1,2,4-triazole, or a prodrug thereof, as a component of the combination therapy is of the general formula (III) wherein R² and R³ together with the carbon atom to which they are attached, are forming a saturated or partially saturated cyclic ring system containing from 3 to 6 carbon atoms and from 0 to 2 additional heteroatoms selected from nitrogen or oxygen, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy.

In another embodiment of the present invention said fused 1,2,4-triazole, or a prodrug thereof, as a component of the combination therapy is of the general formula (III) wherein R⁴ and R⁵ independently are hydrogen, C₁-C₆alkyl, C₁-C₆alkyloxy, aryloxyC₁-C₆alkyl, wherein the alkyl and aryl groups independently are optionally substituted with one or more of R⁹.

In another embodiment of the present invention said fused 1,2,4-triazole, or a prodrug thereof, as a component of the combination therapy is of the general formula (III) wherein R⁴ and R⁵ together with the carbon atoms to which they are attached, are forming a saturated or partially saturated ring system containing from 5 to 8 carbon atoms and from 0 to 2 additional heteroatoms selected from nitrogen or oxygen, the ring system optionally being substituted with at least one of C₁-C₆alkyl.

In another embodiment of the present invention said fused 1,2,4-triazole, or a prodrug thereof, as a component of the combination therapy is of the general formula (III) wherein R⁴ and either R² or R³ together are forming a saturated or partially saturated bridge containing from 1 to 4 carbon atoms, the bridge can optionally be substituted with at least one of C₁-C₆alkyl or arylC₁-C₆alkyl.

In another embodiment of the present invention said fused 1,2,4-triazole, or a prodrug thereof, as a component of the combination therapy is of the general formula (III) wherein R⁶ is hydrogen or C₁-C₆alkyl.

In another embodiment of the present invention said fused 1,2,4-triazole, or a prodrug thereof, as a component of the combination therapy is of the general formula (III) wherein R⁶ and either R⁴ or R⁵ together with the carbon atoms to which they are attached, are forming a saturated, partially saturated or aromatic ring system containing from 5 to 8 carbon atoms and from 0 to 2 additional heteroatoms selected from nitrogen or oxygen or sulphur, the ring system optionally being substituted with at least one of C₁-C₆alkyl, halo, trihalomethyl, C₁-C₆alkyloxy or arylC₁-C₆alkyloxy.

In another embodiment of the present invention said fused 1,2,4-triazole, or a prodrug thereof, as a component of the combination therapy is of the general formula (III) wherein R⁷ and R⁸ independently are hydrogen, halo, hydroxy, cyano, C₁-C₆alkyl, C₁-C₆alkyloxy, trihalomethyl, trihalomethoxy, aryloxy, hetaryloxy, arylC₁-C₆alkyloxy, hetarylC₁-C₆alkyloxy, C₁-C₆alkyloxyC₁-C₆alkyl, aryloxyC₁-C₆alkyl or arylC₁-C₆alkyloxyC₁-C₆alkyl.

In another embodiment of the present invention said fused 1,2,4-triazole, or a prodrug thereof, as a component of the combination therapy is of the general formula (III) wherein R⁹ is hydrogen.

In another embodiment of the present invention said fused 1,2,4-triazole, or a prodrug thereof, as a component of the combination therapy is of the general formula (III) wherein A is a double or aromatic bond; X is (CR¹⁶R¹⁷)ₙ, wherein R¹⁶ and R¹⁷ independently are hydrogen or C₁-C₆alkyl and n is 1; Y is CR¹⁸ wherein R¹⁸ is hydrogen, C₁-C₆alkyl, aryl, hetaryl, arylC₁-C₆alkyl or hetarylC₁-C₆alkyl; R⁶ and either R⁴ or R⁵ together with the carbon atoms to which they are attached, are forming an aromatic ring system containing 6 carbon atoms, the ring system optionally being substituted with at least one of C₁-C₆alkyl, aryl, arylC₁-C₆alkyl, NR¹⁰R¹¹, halo, trihalomethyl, trihalomethoxy, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkylcarboxy.

In another embodiment of the present invention said fused 1,2,4-triazole, or a prodrug thereof, as a component of the combination therapy is of the general formula (III) wherein A is a double or aromatic bond.

In another embodiment of the present invention said fused 1,2,4-triazole, or a prodrug thereof, as a component of the combination therapy is of the general formula (III) wherein X is (CR¹⁶R¹⁷)ₙ, wherein R¹⁶ and R¹⁷ independently are hydrogen or C₁-C₆alkyl and n is 1.

In another embodiment of the present invention said fused 1,2,4-triazole, or a prodrug thereof, as a component of the combination therapy is of the general formula (III) wherein Y is CR¹⁸ wherein R¹⁸ is hydrogen, C₁-C₆alkyl, aryl, hetaryl, arylC₁-C₆alkyl or hetarylC₁-C₆alkyl.

In another embodiment of the present invention said fused 1,2,4-triazole, or a prodrug thereof, as a component of the combination therapy is selected from the group consisting:
3-(2-Bromo-phenyl)-6,7,8,9-tetrahydro-5H-[1,2,4]triazolo[4,3-a]azepine;
3-Phenyl-[1,2,4]triazolo[3,4-a]isoquinoline;
(2-Methoxy-benzyl)-(3-phenyl-[1,2,4]triazolo[4,3-b]pyridazin-6-yl)-amine;
3-(2-Fluoro-phenyl)-5-(4-methoxy-phenoxy)-[1,2,4]triazolo[4,3-c]quinazoline;
3-Phenyl-6,7,8,9-tetrahydro-5H-[1,2,4]triazolo[4,3-a]azepine;
3-(4-Chloro-phenyl)-6,7,8,9-tetrahydro-5H-[1,2,4]triazolo[4,3-a]azepine;
3-(3-Chloro-phenyl)-6,7,8,9-tetrahydro-5H-[1,2,4]triazolo[4,3-a]azepine;
3-(3,4-Dichloro-phenyl)-6,7,8,9-tetrahydro-5H-[1,2,4]triazolo[4,3-a]azepine;
5,5-Dimethyl-3-thiophen-2-yl-5,6-dihydro-[1,2,4]triazolo[3,4-a]isoquinoline;
3-(2-Chloro-phenyl)-6,7,8,9-tetrahydro-5H-[1,2,4]triazolo[4,3-a]azepine;
5,5-Dimethyl-3-(3,4,5-trimethoxy-phenyl)-5,6-dihydro-[1,2,4]triazolo[3,4-a]isoquinoline;
3-Furan-2-yl-5,5-dimethyl-5,6-dihydro-[1,2,4]triazolo[3,4-a]isoquinoline;
3-(3-Bromo-phenyl)-6,7,8,9-tetrahydro-5H-[1,2,4]triazolo[4,3-a]azepine;
3-(4-Bromo-phenyl)-5,5-dimethyl-5,6-dihydro-[1,2,4]triazolo[3,4-a]isoquinoline;
4-(5,5-Dimethyl-5,6-dihydro-[1,2,4]triazolo[3,4-a]isoquinolin-3-yl)-phenol;
3-(4-Methoxy-phenyl)-5,5,8,9-tetramethyl-5,6-dihydro-[1,2,4]triazolo[3,4-a]isoquinoline;
5,5-Dimethyl-3-phenyl-5,6-dihydro-[1,2,4]triazolo[3,4-a]isoquinoline;
3-(5,5-Dimethyl-5,6-dihydro-[1,2,4]triazolo[3,4-a]isoquinolin-3-yl)-phenol;
5,5-Dimethyl-3-p-tolyl-5,6-dihydro-[1,2,4]triazolo[3,4-a]isoquinoline;
5,5-Dimethyl-3-thiophen-2-yl-5,6-dihydro-[1,2,4]triazolo[3,4-a]isoquinoline;
7,10-Dimethoxy-5,5-dimethyl-3-phenyl-5,6-dihydro-[1,2,4]triazolo[3,4-a]isoquinoline;
3-(2,4-Dichloro-phenyl)-6,6-dimethyl-5,6,7,8-tetrahydro-[1,2,4]triazolo[4,3-a]pyrimidine;
2-(6,6-Dimethyl-5,6,7,8-tetrahydro-[1,2,4]triazolo[4,3-a]pyrimidin-3-yl)-phenol;
3-(2-Chloro-phenyl)-6,6-dimethyl-5,6,7,8-tetrahydro-[1,2,4]triazolo[4,3-a]pyrimidine;
4-Benzyl-3,5-di-p-tolyl-4H-[1,2,4]triazole;
3-p-Tolyl-6,7,8,9-tetrahydro-5H-[1,2,4]triazolo[4,3-a]azepine;
3-(4-Methoxy-phenyl)-6,7,8,9-tetrahydro-5H-[1,2,4]triazolo[4,3-a]azepine;
3-Pyridin-4-yl-6,7,8,9-tetrahydro-5H-[1,2,4]triazolo[4,3-a]azepine;
3-(4-Bromo-phenyl)-6,7,8,9-tetrahydro-5H-[1,2,4]triazolo[4,3-a]azepine;
3-Furan-2-yl-5,5,8,9-tetramethyl-5,6-dihydro-[1,2,4]triazolo[3,4-a]isoquinoline;
6,6-Dimethyl-3-(2-nitro-phenyl)-5,6,7,8-tetrahydro-[1,2,4]triazolo[4,3-a]pyrimidine;
3-(2,4-Dichloro-phenyl)-5,5-dimethyl-5,6-dihydro-[1,2,4]triazolo[3,4-a]isoquinoline; or
a salt thereof with a pharmaceutically acceptable acid or base, or any optical isomer or mixture of optical isomers, including a racemic mixture, or any tautomeric forms.

In another embodiment of the present invention said fused 1,2,4-triazole, or a prodrug thereof, as a component of the combination therapy is of the general formula (IIIa) 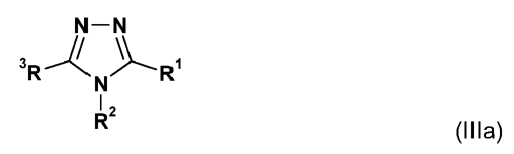 wherein
R¹ is aryl or hetaryl, wherein the aryl and hetaryl groups independently are optionally substituted with one or more of R⁷;
R² and R³ together with the atoms to which they are connected forms a C₅-C₁₀cycloalkyl or C₅-C₁₀hetcycloalkyl, wherein the cycloalkyl and hetcycloalkyl rings independently are optionally substituted with one or more of R⁸; or
R² and R³ are connected to one of the following ring systems at the carbon atoms marked with an asterix (*) 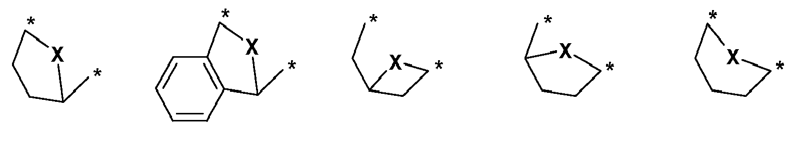 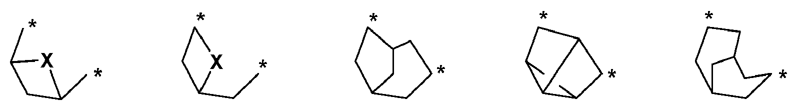 wherein the ring systems independently are optionally substituted with one or more R⁸;
R⁷ is hydrogen, halo, hydroxy, cyano, nitro, C₁-C₆alkyl, C₁-C₆alkyloxy, trihalomethyl, trihalomethoxy, aryloxy, hetaryloxy, NR⁹R¹⁰, arylC₁-C₆alkyloxy, hetarylC₁-C₆alkyloxy, C₁-C₆alkyloxyC₁-C₆alkyl, aryloxyC₁-C₆alkyl, arylC₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R⁸ is hydrogen, C₁-C₆alkyl, halo, aryl, hetaryl, arylC₁-C₆alkyl, NR⁹R¹⁰, trihalomethyl, trihalomethoxy, hydroxy, oxo, C₁-C₆alkyloxy, aryloxy, arylC₁-C₆alkyloxy, hetarylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R⁹ and R¹⁰ independently are hydrogen, C₁-C₆alkyl, aryl or arylC₁-C₆alkyl wherein the alkyl and aryl groups independently are optionally substituted with one or more of R¹¹; or
R⁹ and R¹⁰ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one of C₁-C₆alkyl, aryl, hetaryl, arylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R¹¹ is C₁-C₆alkyl, oxo or halo;
X is (CR¹²R¹³)ₙ, wherein R¹² and R¹³ independently are hydrogen, oxo, hydroxy or C₁-C₆alkyl; and
n is 1 or 2; or
a salt thereof with a pharmaceutically acceptable acid or base, or any optical isomer or mixture of optical isomers, including a racemic mixture, or any tautomeric forms.

In another embodiment of the present invention said fused 1,2,4-triazole, or a prodrug thereof, as a component of the combination therapy is of the general formula (IIIa) wherein R¹ is aryl optionally substituted with R⁷ as defined above.

In another embodiment of the present invention said fused 1,2,4-triazole, or a prodrug thereof, as a component of the combination therapy is of the general formula (IIIa) wherein R¹ is phenyl optionally substituted with R⁷ as defined above.

In another embodiment of the present invention said fused 1,2,4-triazole, or a prodrug thereof, as a component of the combination therapy is of the general formula (IIIa) wherein R² and R³ are connected to one of the following ring systems at the carbon atoms marked with an asterix (*) 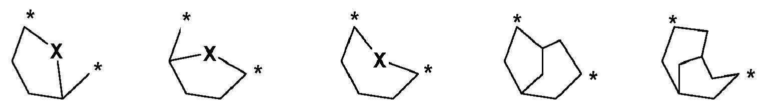 wherein the ring systems independently are optionally substituted with one or more R⁸ as defined above.

In another embodiment of the present invention said fused 1,2,4-triazole, or a prodrug thereof, as a component of the combination therapy is of the general formula (IIIa) wherein R⁷ is hydrogen, halo, hydroxy, cyano, C₁-C₆alkyl, C₁-C₆alkyloxy, trihalomethyl, aryloxy, arylC₁-C₆alkyloxy, hetarylC₁-C₆alkyloxy, C₁-C₆alkyloxyC₁-C₆alkyl, aryloxyC₁-C₆alkyl or arylC₁-C₆alkyloxyC₁-C₆alkyl.

In another embodiment of the present invention said fused 1,2,4-triazole, or a prodrug thereof, as a component of the combination therapy is of the general formula (IIIa) wherein R⁸ is hydrogen, C₁-C₆alkyl or halo.

In another embodiment of the present invention said fused 1,2,4-triazole, or a prodrug thereof, as a component of the combination therapy is of the general formula (IIIb) 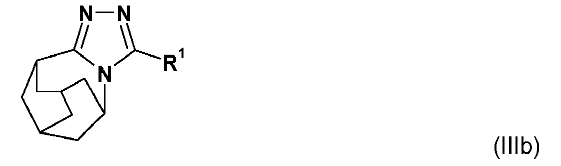 wherein
R¹ is aryl or hetaryl, wherein the aryl and hetaryl groups independently are optionally substituted with one or more of R⁷;
R⁷ is hydrogen, halo, hydroxy, cyano, nitro, C₁-C₆alkyl, C₁-C₆alkyloxy, trihalomethyl, trihalomethoxy, aryloxy, hetaryloxy, NR⁹R¹⁰, arylC₁-C₆alkyloxy, hetarylC₁-C₆alkyloxy, C₁-C₆alkyloxyC₁-C₆alkyl, aryloxyC₁-C₆alkyl, arylC₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R⁹ and R¹⁰ independently are hydrogen, C₁-C₆alkyl, aryl or arylC₁-C₆alkyl wherein the alkyl and aryl groups independently are optionally substituted with one or more of R¹¹; or
R⁹ and R¹⁰ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one of C₁-C₆alkyl, aryl, hetaryl, arylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆a-alkylcarboxy;
R¹¹ is C₁-C₆alkyl, oxo or halo;
a salt thereof with a pharmaceutically acceptable acid or base, or any optical isomer or mixture of optical isomers, including a racemic mixture, or any tautomeric forms.

In another embodiment of the present invention said fused 1,2,4-triazole, or a prodrug thereof, as a component of the combination therapy is of the general formula (IIIb) wherein R¹ is aryl optionally substituted with R⁷ as defined above.

In another embodiment of the present invention said fused 1,2,4-triazole, or a prodrug thereof, as a component of the combination therapy is of the general formula (IIIb) wherein R¹ is phenyl optionally substituted with R⁷ as defined above.

In another embodiment of the present invention said fused 1,2,4-triazole, or a prodrug thereof, as a component of the combination therapy is of the general formula (IIIb) wherein R¹ is phenyl substituted in the ortho position.

In another embodiment of the present invention said fused 1,2,4-triazole, or a prodrug thereof, as a component of the combination therapy is of the general formula (IIIb) wherein R¹ is phenyl substituted in both the ortho and para position.

In another embodiment of the present invention said fused 1,2,4-triazole, or a prodrug thereof, as a component of the combination therapy is of the general formula (IIIb) wherein R⁷ is halo, hydroxy, cyano, C₁-C₆alkyl, C₁-C₆alkyloxy, trihalomethyl, trihalomethoxy, aryloxy, hetaryloxy, NR⁹R¹⁰, arylC₁-C₆alkyloxy, hetarylC₁-C₆alkyloxy, C₁-C₆alkyloxyC₁-C₆alkyl, aryloxyC₁-C₆alkyl, arylC₁-C₆alkyloxyC₁-C₆alkyl, C₁-C₆alkylcarbonyl, arylcarbonyl, hetarylcarbonyl, arylC₁-C₆alkylcarbonyl or hetarylC₁-C₆alkylcarbonyl; wherein R⁹ and R¹⁰ are defined as above.

In another embodiment of the present invention said fused 1,2,4-triazole, or a prodrug thereof, as a component of the combination therapy is of the general formula (IIIb) wherein R⁷ is C₁-C₆alkyloxy, trihalomethoxy, aryloxy, hetaryloxy, arylC₁-C₆alkyloxy, hetarylC₁-C₆alkyloxy, C₁-C₆alkyloxyC₁-C₆alkyl, aryloxyC₁-C₆alkyl or arylC₁-C₆alkyloxyC₁-C₆alkyl.

In another embodiment of the present invention said fused 1,2,4-triazole, or a prodrug thereof, as a component of the combination therapy is selected from the group consisting of:
3-(2-Bromo-phenyl)-6,7,8,9-tetrahydro-5H-[1,2,4]triazolo[4,3-a]azepine;
3-(2-Phenoxymethyl-phenyl)-5,6,7,8-tetrahydro-[1,2,4]triazolo[4,3-a]azepin-9-one;
3-(2-Phenoxymethyl-phenyl)-6,7,8,9,10,11-hexahydro-5*H*-5,9:7,11-dimethano[1,2,4]triazolo[4.3-a]azonine;
3-(5-Bromo-pyridin-3-yl)-6,7,8,9-tetrahydro-5H-[1,2,4]triazolo[4,3-a]azepine;
3-(5-Hex-1-ynyl-pyridin-3-yl)-6,7,8,9-tetrahydro-5H-[1,2,4]triazolo[4,3-a]azepine;
3-(6-Chloro-pyridin-3-yl)-6,7,8,9-tetrahydro-5H-[1,2,4]triazolo[4,3-a]azepine;
3-(6-Morpholin-4-yl-pyridin-3-yl)-6,7,8,9-tetrahydro-5H-[1,2,4]triazolo[4,3-a]azepine;
3-Pyridin-3-yl-6,7,8,9-tetrahydro-5*H*-[1,2,4]triazolo[4,3-a]azepine;
3-(2-Benzyloxymethyl-phenyl)-6,7,8,9-tetrahydro-5*H*-5,9-methano[1,2,4]triazolo[4.3-a]azepine;
3-Phenyl-[1,2,4]triazolo[3,4-a]isoquinoline; or
salt thereof with a pharmaceutically acceptable acid or base, or any optical isomer or mixture of optical isomers, including a racemic mixture, or any tautomeric forms.

In another embodiment of the present invention said alpha-amino ketones, or a prodrug thereof, as a component of the combination therapy is of the general formula (IV) 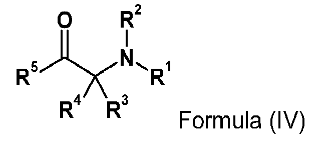 wherein
R¹ and R² independently are hydrogen, C₁-C₆alkyl, C₃-C₁₀cycloalkyl, C₃-C₁₀ het-cycloalkyl, aryl, hetaryl, arylC₁-C₆alkyl or hetarylC₁-C₆alkyl, wherein the alkyl, cycloalkyl, hetcycloalkyl, aryl and hetaryl groups independently are optionally substituted with one or more of R⁶; or
R¹ and R² together with the nitrogen to which they are attached, are forming a saturated or partially saturated cyclic or bicyclic ring system containing from 4 to 8 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetaryl-C₁-C₆alkyl, hydroxy, oxo, C₁-C₆alkyl-oxy, arylC₁-C₆alkyloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylcarbonyl, C₁-C₆alkylcarboxy, arylcarboxy, hetarylcarboxy or arylC₁-C₆alkylcarboxy;
R³ and R⁴ independently are hydrogen, C₁-C₆alkyl, C₁-C₆alkenyl, C₁-C₆alkynyl, aryl, hetaryl, arylC₁-C₆alkyl or hetarylC₁-C₆alkyl, wherein the alkyl, alkenyl, alkynyl, aryl, and hetaryl groups independently are optionally substituted with one or more of R⁷;
R⁵ is C₃-C₁₀cydoalkyl, C₃-C₁₀hetcycloalkyl, aryl or hetaryl, wherein the aryl, hetaryl, cycloalkyl and hetcycloalkyl groups independently are optionally substituted with one or more of R⁸; or R³ and R⁵ together with the carbon atoms to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, C₁-C₆alkylcarboxy, aryl-carboxy, hetarylcarboxy or arylC₁-C₆alkylcarboxy; or
R², R³ and R⁵ together with the atoms to which they are attached, are forming a saturated or partially saturated bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyl-oxy, arylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, C₁-C₆alkylcarboxy, arylcarboxy, hetaryl-carboxy or arylC₁-C₆alkylcarboxy;
R⁶ is hydrogen, hydroxy, oxo, halo, adamantyl, C₁-C₆alkyl, C₁-C₆alkyloxy, trihalomethyl, trihalomethoxy, NR⁹R¹⁰, arylC₁-C₆alkyloxy, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R⁷ and R⁸ independently are hydrogen, hydroxy, oxo, halo, cyano, nitro, NR⁹R¹⁰, C₁-C₆alkyl, C₁-C₆alkyloxy, aryl, hetaryl, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyl-carboxy, arylcarboxy or arylC₁-C₆alkylcarboxy, wherein the aryl and hetaryl groups independently are optionally substituted with C₃-C₁₀cycloalkyl or C₃-C₁₀hetcycloalkyl;
R⁹ and R¹⁰ independently are hydrogen, C₁-C₆alkyl, C₃-C₁₀cycloalkyl, C₃-C₁₀ hetcycloalkyl, aryl, hetaryl, arylC₁-C₆alkyl or hetarylC₁-C₆alkyl; or
R⁹ and R¹⁰ together with the nitrogen to which they are attached, are forming a saturated or partially saturated cyclic, bicyclic or tricyclic ring system containing from 5 to 10 carbon atoms and from 0 to 2 additional heteroatoms selected from nitrogen, oxygen or sulfur, the ring system optionally being substituted with at least one C₁-C₆alkyl, aryl, hetaryl, arylC₁-C₆alkyl, hetarylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, C₁-C₆alkylcarboxy, arylcarboxy, hetarylcarboxy or arylC₁-C₆alkylcarboxy; or
a salt thereof with a pharmaceutically acceptable acid or base, or any optical isomer or mixture of optical isomers, including a racemic mixture, or any tautomeric forms.

In another embodiment of the present invention said alpha-amino ketones, or a prodrug thereof, as a component of the combination therapy is selected from the group consisting of:
1-Adamantan-1-yl-2-morpholin-4-yl-ethanone;
1-Adamantan-1-yl-2-benzylamino-propan-1-one;
1-Adamantan-1-yl-2-benzylamino-ethanone;
2-Pyrrolidin-1-yl-1-(3-p-tolyl-adamantan-1-yl)ethanone;
1-Adamantan-1-yl-2-morpholin-4-yl-propan-1-one;
1-[4-(5-Adamantan-1-yl-[1,2,3]triazol-1-yl)-phenyl]-2-phenylamino-ethanone;
6-Benzo[1,3]dioxol-5-yl-8-benzyl-8-aza-bicyclo[3.2.1]octan-2-one;
2-*tert*-Butylamino-1-(3-p-tolyl-adamantan-1-yl)ethanone;
2-Morpholin-4-yl-1-(3-p-tolyl-adamantan-1-yl)ethanone;
1-Adamantan-1-yl-2-[4-(4-nitro-phenyl)-piperazin-1-yl]ethanone; or
a salt thereof with a pharmaceutically acceptable acid or base, or any optical isomer or mixture of optical isomers, including a racemic mixture, or any tautomeric forms.

In another embodiment of the present invention said substituted 1,2,4-triazole, or a prodrug thereof, as a component of the combination therapy is of the general formula (V) 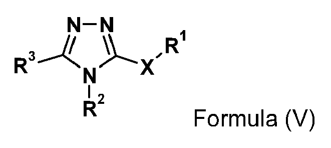 wherein
X is O or S;
R¹ is hydrogen, C₁-C₆alkyl, C₂-C₆alkenyl, C₂-C₆alkynyl, C₃-C₁₀cycloalkyl, arylC₁-C₆alkyl, hetarylC₁-C₆alkyl, R⁴R⁵NcarbonylC₁-C₆alkyl, arylcarbonylC₁-C₆alkyl, hetarylcarbonylC₁-C₆alkyl; wherein the alkyl, alkenyl, alkynyl, aryl and hetaryl groups independently are optionally substituted with one or more of R⁶;
R² is C₁-C₆alkyl, C₁-C₆alkenyl, arylC₁-C₆alkyl, C₃-C₁₀cycloalkyl or hetarylC₁-C₆alkyl; wherein the alkyl, alkenyl, cycloalkyl, aryl and hetaryl groups independently are optionally substituted with one or more of R⁷;
R³ is C₃-C₁₀cycloalkyl, C₃-C₁₀hetcycloalkyl, aryl, hetaryl, arylC₁-C₆alkyl, hetarylC₁-C₆alkyl, arylR⁸C₁-C₆alkyl or hetarylR⁸C₁-C₆alkyl; wherein the alkyl, cycloalkyl, hetcycloalkyl, aryl and hetaryl groups independently are optionally substituted with one or more of R⁹;
R⁴ and R⁵ independently are hydrogen, C₁-C₆alkyl, C₃-C₁₀cycloalkyl, C₅-C₈hetcycloalkyl, aryl, hetaryl, arylC₁-C₆alkyl or hetarylC₁-C₆alkyl; wherein the alkyl, cycloalkyl, hetcycloalkyl, aryl, hetaryl, arylalkyl or hetarylalkyl groups independently are optionally substituted with one or more of R¹¹; or
R⁴ and R⁵ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, arylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkyl-carbonyl, C₁-C₆alkylcarboxy, arylcarboxy or arylC₁-C₆alkylcarboxy;
R⁶, R⁷, R⁹ and R¹¹ independently are hydrogen, halo, NO₂ NH₂, cyano, NR⁴R⁵, CONR⁴R⁵, trihalomethyl, trihalomethoxy, hydroxy, oxo, C₁-C₆alkyl, C₃-C₁₀cycloalkyl, C₃-C₁₀hetcycloalkyl, aryl, hetaryl, arylC₁-C₆alkyl, hetarylC₁-C₆alkyl, C₁-C₆alkylSO₂, R¹⁵R¹⁶NSO₂, C₁-C₆alkyloxy, aryloxy, hetaryloxy, arylC₁-C₆alkyloxy, C₁-C₆alkylcarbonyl, arylcarbonyl, arylC₁-C₆alkylcarbonyl, arylcarbonylNR¹⁵, carboxyC₁-C₆alkyl or carboxyarylC₁-C₆alkyl;
R⁸ is oxygen, NR¹⁰, C(=O)NR¹⁰ or SOₙNR¹⁰; wherein n is 1 or 2;
R¹⁰ is hydrogen, C₁-C₆alkyl, aryl, hetaryl, arylC₁-C₆alkyl or hetarylC₁-C₆alkyl;
R¹⁵ and R¹⁶ independently are hydrogen, C₁-C₆alkyl, C₃-C₁₀cycloalkyl, C₅-C₈hetcycloalkyl, aryl or hetaryl; wherein the alkyl, cycloalkyl, hetcycloalkyl, aryl or hetaryl groups independently are optionally substituted with one or more of R¹¹; or
R¹⁵ and R¹⁶ together with the nitrogen to which they are attached, are forming a saturated or partially saturated cyclic, bicyclic or tricyclic ring system containing from 4 to 8 carbon atoms and from 0 to 2 additional heteroatoms selected from nitrogen, oxygen or sulphur, the ring system optionally being substituted with at least one of C₁-C₆alkyl, aryl, arylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkylcarboxy; or
a salt thereof with a pharmaceutically acceptable acid or base, or any optical isomer or mixture of optical isomers, including a racemic mixture, or any tautomeric forms.

In another embodiment of the present invention said substituted 1,2,4-triazole, or a prodrug thereof, as a component of the combination therapy is of the general formula (V) wherein
X is O or S;
R¹ is hydrogen, C₁-C₆alkyl, C₂-C₆alkenyl, C₂-C₆alkynyl, arylC₁-C₆alkyl, hetarylC₁-C₆alkyl, NR⁴R⁵carbonylC₁-C₆alkyl, arylcarbonylC₁-C₆alkyl, hetarylcarbonylC₁-C₆alkyl; wherein the alkyl, alkenyl, alkynyl, aryl and hetaryl groups independently are optionally substituted with one or more of R⁶;
R² is C₁-C₆alkyl, C₁-C₆alkenyl, arylC₁-C₆alkyl or hetarylC₁-C₆alkyl; wherein the alkyl, alkenyl, aryl and hetaryl groups independently are optionally substituted with one or more of R⁷;
R³ is C₅-C₈cycloalkyl, C₅-C₈hetcycloalkyl, aryl, hetaryl, arylC₁-C₆alkyl, hetarylC₁-C₆alkyl, arylR⁸C₁-C₆alkyl or hetarylR⁸C₁-C₆alkyl; wherein the alkyl, cycloalkyl, hetcycloalkyl, aryl and hetaryl groups independently are optionally substituted with one or more of R⁹;
R⁴ and R⁵ independently are hydrogen, C₁-C₆alkyl, C₅-C₁₀cycloalkyl, C₅-C₈hetcycloalkyl, aryl, hetaryl, arylC₁-C₆alkyl or hetarylC₁-C₆alkyl; wherein the alkyl, cycloalkyl, hetcycloalkyl, aryl, hetaryl, arylalkyl or hetarylalkyl groups independently are optionally substituted with one or more of R¹¹; or
R⁴ and R⁵ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, arylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkylcarboxy;
R⁶, R⁷, R⁹ and R¹¹ independently are hydrogen, halo, NO₂, NH₂, cyano, trihalomethyl, trihalomethoxy, hydroxy, oxo, C₁-C₆alkyl, aryl, hetaryl, arylC₁-C₆alkyl, hetarylC₁-C₆alkyl, SO₂NR¹⁵R¹⁶, C₁-C₆alkyloxy, aryloxy, hetaryloxy, arylC₁-C₆alkyloxy, C₁-C₆alkylcarbonyl, arylcarbonyl, arylC₁-C₆alkylcarbonyl, arylcarbonylNR¹⁵, carboxyC₁-C₆alkyl or carboxyarylC₁-C₆alkyl;
R⁸ is oxygen, NR¹⁰, C(=O)NR¹⁰ or SOₙNR¹⁰; wherein n is 1 or 2;
R¹⁰ is hydrogen, C₁-C₆alkyl, aryl, hetaryl, arylC₁-C₆alkyl or hetarylC₁-C₆alkyl;
R¹⁵ and R¹⁶ independently are hydrogen, C₁-C₆alkyl, C₅-C₁₀cycloalkyl, C₅-C₈hetcycloalkyl, aryl or hetaryl; wherein the alkyl, cycloalkyl, hetcycloalkyl, aryl or hetaryl groups independently are optionally substituted with one or more of R¹¹; or
R¹⁵ and R¹⁶ together with the nitrogen to which they are attached, are forming a saturated or partially saturated cyclic, bicyclic or tricyclic ring system containing from 4 to 8 carbon atoms and from 0 to 2 additional heteroatoms selected from nitrogen, oxygen or sulphur, the ring system optionally being substituted with at least one of C₁-C₆alkyl, aryl, arylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkylcarboxy; or
a salt thereof with a pharmaceutically acceptable acid or base, or any optical isomer or mixture of optical isomers, including a racemic mixture, or any tautomeric forms.

In another embodiment of the present invention said substituted 1,2,4-triazole, or a prodrug thereof, as a component of the combination therapy is of the general formula (V) wherein X is O.

In another embodiment of the present invention said substituted 1,2,4-triazole, or a prodrug thereof, as a component of the combination therapy is of the general formula (V) wherein X is S.

In another embodiment of the present invention said substituted 1,2,4-triazole, or a prodrug thereof, as a component of the combination therapy is of the general formula (V) wherein R¹ is arylC₁-C₆alkyl, hetarylC₁-C₆alkyl, R⁴R⁵NcarbonylC₁-C₆alkyl, arylcarbonylC₁-C₆alkyl, hetarylcarbonylC₁-C₆alkyl; wherein aryl and hetaryl groups independently are optionally substituted with one or more of R⁶.

In another embodiment of the present invention said substituted 1,2,4-triazole, or a prodrug thereof, as a component of the combination therapy is of the general formula (V) wherein R² is C₁-C₆alkyl, arylC₁-C₆alkyl, C₃-C₁₀cycloalkyl or hetarylC₁-C₆alkyl; all of which is optionally substituted with one or more of R⁷.

In another embodiment of the present invention said substituted 1,2,4-triazole, or a prodrug thereof, as a component of the combination therapy is of the general formula (V) wherein R³ is C₃-C₁₀cycloalkyl, C₃-C₁₀hetcycloalkyl, aryl, hetaryl, arylC₁-C₆alkyl, hetarylC₁-C₆alkyl, arylR⁸C₁-C₆alkyl or hetarylR⁸C₁-C₆alkyl, wherein all groups indenpendently are optionally substituted with one or more of R⁹.

In another embodiment of the present invention said substituted 1,2,4-triazole, or a prodrug thereof, as a component of the combination therapy is of the general formula (V) wherein R³ is C₃-C₁₀hetcycloalkyl, aryl, hetaryl, arylC₁-C₆alkyl, hetarylC₁-C₆alkyl, arylR⁸C₁-C₆alkyl or hetarylR⁸C₁-C₆alkyl, wherein all groups indenpendently are optionally substituted with one or more of R⁹.

In another embodiment of the present invention said substituted 1,2,4-triazole, or a prodrug thereof, as a component of the combination therapy is of the general formula (V) wherein R⁴ and R⁵ independently are aryl or hetaryl wherein both groups indenpendently are optionally substituted with one or more of R¹¹.

In another embodiment of the present invention said substituted 1,2,4-triazole, or a prodrug thereof, as a component of the combination therapy is of the general formula (V) wherein R⁴ and R⁵ together with the nitrogen to which they are attached, are forming a saturated or partially saturated cyclic, bicyclic or tricyclic ring system containing from 4 to 10 carbon atoms and from 0 to 2 additional nitrogen atoms, the ring system optionally being substituted with at least one of C₁-C₆alkyl, aryl, arylC₁-C₆alkyl, hydroxy, oxo, C₁-C₆alkyloxy, arylC₁-C₆alkyloxy, C₁-C₆alkyloxyC₁-C₆alkyl, C₁-C₆alkylcarbonyl, arylcarbonyl, arylC₁-C₆alkylcarbonyl, C₁-C₆alkylcarboxy, arylcarboxy or arylC₁-C₆alkylcarboxy.

In another embodiment of the present invention said substituted 1,2,4-triazole, or a prodrug thereof, as a component of the combination therapy is of the general formula (V) wherein R⁶, R⁷, R⁹ and R¹¹ independently are hydrogen, halo, NO₂, NH₂, cyano, NR⁴R⁵, CONR⁴R⁵, trihalomethyl, trihalomethoxy, hydroxy, oxo, C₁-C₆alkyl, C₃-C₁₀cycloalkyl, C₃-C₁₀hetcycloalkyl, aryl, hetaryl, arylC₁-C₆alkyl, hetarylC₁-C₆alkyl, C₁-C₆alkylSO₂, R¹⁵R¹⁶NSO₂, C₁-C₆alkyloxy, aryloxy, hetaryloxy, arylC₁-C₆alkyloxy, C₁-C₆alkylcarbonyl, arylcarbonyl, arylC₁-C₆alkylcarbonyl, arylcarbonylNR¹⁵, carboxyC₁-C₆alkyl or carboxyarylC₁-C₆alkyl.

In another embodiment of the present invention said substituted 1,2,4-triazole, or a prodrug thereof, as a component of the combination therapy is of the general formula (V) wherein R⁸ is oxygen, NR¹⁰, C(=O)NR¹⁰ or SOₙNR¹⁰; wherein n is 1 or 2.

In another embodiment of the present invention said substituted 1,2,4-triazole, or a prodrug thereof, as a component of the combination therapy is of the general formula (V) wherein R¹⁰ is hydrogen, C₁-C₆alkyl, aryl, hetaryl, arylC₁-C₆alkyl or hetarylC₁-C₆alkyl.

In another embodiment of the present invention said substituted 1,2,4-triazole, or a prodrug thereof, as a component of the combination therapy is selected from the group consisting of:
3-(4-Allyl-5-mercapto-4H-[1,2,4]triazol-3-yl)-1-(2,4-dichloro-benzyl)-1H-pyridin-2-one;
4-Methyl-3-(4-methyl-2-phenyl-thiazol-5-yl)-5-prop-2-ynylsulfanyl-4H-[1,2,4]triazole;
N-{1-[5-(4-*tert*-Butyl-benzylsulfanyl)-4-ethyl-4H-[1,2,4]triazol-3-yl]-ethyl}-4-chlorobenzenesulfonamide;
4-Methyl-3-methylsulfanyl-5-[3-(3-trifluoromethyl-benzyloxy)-thiophen-2-yl]-4H-[1,2,4]triazole;
N-(4-Chloro-phenyl)-2-(4-methyl-5-thiophen-3-ylmethyl-4H-[1,2,4]triazol-3-ylsulfanyl)-acetamide;
2-[4-Methyl-5-(2-oxo-2-phenyl-ethylsulfanyl)-4H-[1,2,4]triazol-3-ylmethyl]-4H-benzo[1,4]thiazin-3-one;
3-(4-Fluoro-benzylsulfanyl)-5-(2-fluoro-phenyl)-4-methyl-4H-[1,2,4]triazole;
3-(2-Fluoro-phenyl)-4-methyl-5-(4-methyl-benzylsulfanyl)-4H-[1,2,4]triazole;
4-Methyl-3-(4-methyl-benzylsulfanyl)-5-(3-trifluoromethyl-phenyl)-4H-[1,2,4]triazole;
3-(2,4-Dichloro-phenyl)-4-furan-2-ylmethyl-5-methylsulfanyl-4H-[1,2,4]triazole;
3-(4-tert-Butyl-phenyl)-5-(2,6-dichloro-benzylsulfanyl)-4-furan-2-ylmethyl-4H-[1,2,4]triazole;
4-Methyl-3-(4-methyl-benzylsulfanyl)-5-(5-methyl-isoxazol-3-yl)-4H-[1,2,4]triazole;
3-(3-Methoxy-benzylsulfanyl)-4-methyl-5-thiophen-2-yl-4H-[1,2,4]triazole;
3-(4-Fluoro-benzylsulfanyl)-4-methyl-5-thiophen-2-yl-4H-[1,2,4]triazole;
3-(4-tert-Butyl-benzylsulfanyl)-4-methyl-5-thiophen-2-yl-4H-[1,2,4]triazole;
4-Methyl-3-(2-methyl-benzylsulfanyl)-5-thiophen-2-yl-4H-[1,2,4]triazole;
2-(5-Benzylsulfanyl-4-methyl-4H-[1,2,4]triazol-3-ylmethyl)-4H-benzo[1,4]thiazin-3-one;
2-[5-(4-Chloro-benzylsulfanyl)-4-methyl-4H-[1,2,4]triazol-3-ylmethyl]-4H-benzo[1,4]thiazin-3-one;
5-[5-(2,6-Dichloro-benzylsulfanyl)-4-methyl-4H-[1,2,4]triazol-3-yl]-benzo[1,2,5]oxadiazole;
1-(4-Chloro-phenyl)-2-[4-methyl-5-(4-trifluoromethyl-pyridin-3-yl)-4H-[1,2,4]triazol-3-ylsulfanyl]-ethanone;
3-[5-(4-Chloro-benzylsulfanyl)-4-methyl-4H-[1,2,4]triazol-3-yl]-2-methyl-imidazo[1,2-a]pyridine;
3-[5-(2,4-Dichloro-benzylsulfanyl)-4-methyl-4H-[1,2,4]triazol-3-yl]-2,8-dimethyl-imidazo[1,2-a]pyridine;
4-Allyl-5-(2,4-dichloro-phenyl)-4H-[1,2,4]triazole-3-thiol ;
3-(5-Allylsulfanyl-4-methyl-4H-[1,2,4]triazol-3-yl)-1-benzyl-1 H-pyridin-2-one; 3-(4-Allyl-5-methylsulfanyl-4H-[1,2,4]triazol-3-yl)-1-(2,4-dichloro-benzyl)-1 H-pyridin-2-one; 3-(4-Allyl-5-mercapto-4H-[1,2,4]triazol-3-yl)-1-(4-chloro-benzyl)-1 H-pyridin-2-one; 3-(4-Allyl-5-prop-2-ynylsulfanyl-4H-[1,2,4]triazol-3-yl)-1-(4-chloro-benzyl)-1 H-pyridin-2-one; 3-(5-Allylsulfanyl-4-ethyl-4H-[1,2,4]triazol-3-yl)-1-(2,4-dichloro-benzyl)-1 H-pyridin-2-one;
1-(2,4-Dichloro-benzyl)-3-[4-ethyl-5-(2-oxo-2-phenyl-ethylsulfanyl)-4H-[1,2,4]triazol-3-yl]-1H-pyridin-2-one;
3-(4-Allyl-5-prop-2-ynylsulfanyl-4H-[1,2,4]triazol-3-yl)-1-(3,4-dichloro-benzyl)-1H-pyridin-2-one;
4-Allyl-5-(4-methyl-2-phenyl-thiazol-5-yl)-4H-[1,2,4]triazole-3-thiol;
3-Allylsulfanyl-4-methyl-5-(4-methyl-2-phenyl-thiazol-5-yl)-4H-[1,2,4]triazole;
4-Allyl-5-[2-(3,4-dimethoxy-phenyl)-thiazol-4-yl]-4H-[1,2,4]triazole-3-thiol;
N-[5-(4-tert-Butyl-benzylsulfanyl)-4-methyl-4H-[1,2,4]triazol-3-ylmethyl]-4-methyl-benzenesulfonamide;
4-Methyl-3-[3-(4-methyl-benzyloxy)-thiophen-2-yl]-5-methylsulfanyl-4H-[1,2,4]triazole;
5-[3-(2,4-Dichloro-benzyloxy)-thiophen-2-yl]-4-methyl-4H-[1,2,4]triazole-3-thiol;
3-[3-(2,4-Dichloro-benzyloxy)-thiophen-2-yl]-4-methyl-5-methylsulfanyl-4H-[1,2,4]triazole;
4-Methyl-5-[3-(3-trifluoromethyl-benzyloxy)-thiophen-2-yl]-4H-[1,2,4]triazole-3-thiol;
2-(5-Benzyl-4-methyl-4H-[1,2,4]triazol-3-ylsulfanyl)-N-(4-iodo-phenyl)-acetamide;
3-(5-Allylsulfanyl-4-methyl-4H-[1,2,4]triazol-3-yl)-2,7-dimethyl-imidazo[1,2-a]pyridine;
3-(5-Benzylsulfanyl-4-methyl-4H-[1,2,4]triazol-3-yl)-2,7-dimethyl-imidazo[1,2-a]pyridine;
3-(5-Benzylsulfanyl-4-methyl-4H-[1,2,4]triazol-3-yl)-2-methyl-imidazo[1,2-a]pyridine;
4-Butyl-5-(3-chloro-phenyl)-4H-[1,2,4]triazole-3-thiol;
4-Allyl-5-(3-chloro-phenyl)-4H-[1,2,4]triazole-3-thiol;
N-(5-{2-[5-(4-Methoxy-phenyl)-3-thiophen-2-yl-4,5-dihydro-pyrazol-1-yl]-2-oxo-ethylsulfanyl}-4-methyl-4H-[1,2,4]triazol-3-ylmethyl)-benzamide;
N-(5-{2-[3,5-Bis-(4-methoxy-phenyl)-4,5-dihydro-pyrazol-1-yl]-2-oxo-ethylsulfanyl}-4-methyl-4H-[1,2,4]triazol-3-ylmethyl)-3-methoxy-benzamide;
N-(5-{2-[5-(4-Methoxy-phenyl)-3-thiophen-2-yl-4,5-dihydro-pyrazol-1-yl]-2-oxo-ethylsulfanyl}-4-methyl-4H-[1,2,4]triazol-3-ylmethyl)-4-(pyrrolidine-1-sulfonyl)-benzamide;
N-(5-{2-[5-(4-Methoxy-phenyl)-3-thiophen-2-yl-4,5-dihydro-pyrazol-1-yl]-2-oxo-ethylsulfanyl}-4-methyl-4H-[1,2,4]triazol-3-ylmethyl)-4-(piperidine-1-sulfonyl)-benzamide;
N-(5-{2-[5-(4-Methoxy-phenyl)-3-thiophen-2-yl-4,5-dihydro-pyrazol-1-yl]-2-oxo-ethylsulfanyl}-4-methyl-4H-[1,2,4]triazol-3-ylmethyl)-4-(morpholine-4-sulfonyl)-benzamide;
4-Benzyl-3-(4-fluoro-naphthalen-1-ylmethylsulfanyl)-5-phenyl-4H-[1,2,4]triazole;
2-[4-Allyl-5-(4-methoxy-phenyl)-4H-[1,2,4]triazol-3-ylsulfanyl]-1-naphthalen-1-yl-ethanone;
1-(4-Fluoro-phenyl)-4-(4-methyl-5-phenyl-4H-[1,2,4]triazol-3-ylsulfanyl)-butan-1-one
2-(4-Methyl-5-phenyl-4H-[1,2,4]triazol-3-ylsulfanyl)-1-(5,6,7,8-tetrahydro-naphthalen-2-yl)-ethanone;
5-(2-Carbazol-9-yl-ethyl)-4-(2-methyl-allyl)-4H-[1,2,4]triazole-3-thiol;
1-(4-Methoxy-phenyl)-2-[4-(2-methyl-allyl)-5-phenyl-4H-[1,2,4]triazol-3-ylsulfanyl]-ethanone;
2-{2-[4-Allyl-5-(3-methoxy-phenyl)-4H-[1,2,4]triazol-3-ylsulfanyl]-acetylamino}-benzoic acid methyl ester;
5-(2,4-Dichloro-phenyl)-4-(2-methyl-allyl)-4H-[1,2,4]triazole-3-thiol;
4-Allyl-3-(4-methoxy-benzylsulfanyl)-5-(3-methoxy-phenyl)-4H-[1,2,4]triazole;
2-[4-Allyl-5-(3-methoxy-phenyl)-4H-[1,2,4]triazol-3-ylsulfanyl]-1-(9H-fluoren-2-yl)-ethanone;
3-(4-Methoxy-benzylsulfanyl)-4-methyl-5-phenyl-4H-[1,2,4]triazole;
2-[4-Allyl-5-(3-methoxy-phenyl)-4H-[1,2,4]triazol-3-ylsulfanyl]-1-(4-ethyl-phenyl)-ethanone;
2-(4-Allyl-5-phenyl-4H-[1,2,4]triazol-3-ylsulfanyl)-N-(4-chloro-phenyl)-acetamide;
2-[4-Allyl-5-(4-tert-butyl-phenyl)-4H-[1,2,4]triazol-3-ylsulfanyl]-1-(4-chloro-phenyl)-ethanone;
2-(4-Allyl-5-phenylaminomethyl-4H-[1,2,4]triazol-3-ylsulfanyl)-N-(2,4-difluoro-phenyl)-acetamide;
2-(4-Allyl-5-phenoxymethyl-4H-[1,2,4]triazol-3-ylsulfanyl)-1-(4-methoxy-phenyl)-ethanone;
2-(4-Allyl-5-phenoxymethyl-4H-[1,2,4]triazol-3-ylsulfanyl)-1-(4-bromo-phenyl)-ethanone;
(4-Allyl-5-benzylsulfanyl-4H-[1,2,4]triazol-3-ylmethyl)-phenyl-amine;
2-(4-Allyl-5-benzotriazol-1-ylmethyl-4H-[1,2,4]triazol-3-ylsulfanyl)-1-(4-methoxy-phenyl)-ethanone;
2-(4-Allyl-5-phenylaminomethyl-4H-[1,2,4]triazol-3-ylsulfanyl)-N-(2-chloro-phenyl)-acetamide;
1-(4-Methoxy-phenyl)-2-[4-(2-methyl-allyl)-5-phenyl-4H-[1,2,4]triazol-3-ylsulfanyl]-ethanone;
2-[4-Ethyl-5-(2-methoxy-phenyl)-4H-[1,2,4]triazol-3-ylsulfanyl]-N-(4-methyl-3-nitro-phenyl)-acetamide;
[5-(2-Methoxy-phenyl)-4-methyl-4H-[1,2,4]triazol-3-ylsulfanyl]-acetic acid isopropyl ester;
N-(4-Chloro-phenyl)-2-[4-ethyl-5-(2-methoxy-phenyl)-4H-[1,2,4]triazol-3-ylsulfanyl]-acetamide;
2-[4-Ethyl-5-(2-methoxy-phenyl)-4H-[1,2,4]triazol-3-ylsulfanyl]-N-(4-methoxy-phenyl)-acetamide;
N-(3-Cyano-4,5,6,7-tetrahydro-benzo[b]thiophen-2-yl)-2-[4-ethyl-5-(2-methoxy-phenyl)-4H-[1,2,4]triazol-3-ylsulfanyl]-acetamide;
N-(4-Acetyl-phenyl)-2-[4-ethyl-5-(2-methoxy-phenyl)-4H-[1,2,4]triazol-3-ylsulfanyl]-acetamide;
4-[5-(2,6-Dichloro-benzylsulfanyl)-4-furan-2-ylmethyl-4H-[1,2,4]triazol-3-yl]-pyridine;
N-(4-Methyl-3-nitro-phenyl)-2-[4-methyl-5-(3,4,5-trimethoxy-phenyl)-4H-[1,2,4]triazol-3-ylsulfanyl]-acetamide;
N-(3-Cyano-4,5,6,7-tetrahydro-benzo[b]thiophen-2-yl)-2-(4-furan-2-ylmethyl-5-pyridin-4-yl-4H-[1,2,4]triazol-3-ylsulfanyl)-acetamide;
2-[2-(4-Ethyl-5-m-tolyl-4H-[1,2,4]triazol-3-ylsulfanyl)-acetylamino]-benzoic acid methyl ester;
2-(4-Methyl-5-phenyl-4H-[1,2,4]triazol-3-ylsulfanyl)-N-thiazol-2-yl-acetamide;
N-(4-Chloro-phenyl)-2-(4-ethyl-5-m-tolyl-4H-[1,2,4]triazol-3-ylsulfanyl)-acetamide;
N-(4-Ethoxy-phenyl)-2-(4-ethyl-5-m-tolyl-4H-[1,2,4]triazol-3-ylsulfanyl)-acetamide;
2-(4-Ethyl-5-m-tolyl-4H-[1,2,4]triazol-3-ylsulfanyl)-N-(2-nitro-phenyl)-acetamide;
2-(5-Benzyl-4-methyl-4H-[1,2,4]triazol-3-ylsulfanyl)-N-(4-bromo-phenyl)-acetamide;
2-{5-[(2,6-Dimethyl-phenylamino)-methyl]-4-methyl-4H-[1,2,4]triazol-3-ylsulfanyl}-1-phenyl-ethanone;
2-[5-(2-Bromo-phenyl)-4-ethyl-4H-[1,2,4]triazol-3-ylsulfanyl]-N-(4-fluoro-phenyl)-acetamide;
2-[4-Ethyl-5-(4-methoxy-phenyl)-4H-[1,2,4]triazol-3-ylsulfanyl]-1-(4-methoxy-phenyl)-ethanone;
N-(4-Iodo-2-methyl-phenyl)-2-[5-(2-methoxy-phenyl)-4-methyl-4H-[1,2,4]triazol-3-ylsulfanyl]-acetamide;
2-(5-Benzyl-4-methyl-4H-[1,2,4]triazol-3-ylsulfanyl)-1-(3,4-dihydro-2H-quinolin-1-yl)-ethanone;
N-(2-Bromo-4-methyl-phenyl)-2-(4-furan-2-ylmethyl-5-pyridin-4-yl-4H-[1,2,4]triazol-3-ylsulfanyl)-acetamide;
N-(4-Chloro-phenyl)-2-(4-phenethyl-5-pyridin-4-yl-4H-[1,2,4]triazol-3-ylsulfanyl)-acetamide;
2-[4-Ethyl-5-(2-methoxy-phenyl)-4H-[1,2,4]triazol-3-ylsulfanyl]-N-(3-trifluoromethyl-phenyl)-acetamide;
2-[5-[(4-Fluoro-phenylamino)-methyl]-4-(tetrahydro-furan-2-ylmethyl)-4H-[1,2,4]triazol-3-ylsulfanyl]-1-p-tolyl-ethanone;
2-(5-Methyl-4-phenethyl-4H-[1,2,4]triazol-3-ylsulfanyl)-N-naphthalen-1-yl-acetamide;
N-(5-Ethyl-[1,3,4]thiadiazol-2-yl)-2-(5-methyl-4-phenethyl-4H-[1,2,4]triazol-3-ylsulfanyl)-acetamide;
2-(5-Methyl-4-phenethyl-4H-[1,2,4]triazol-3-ylsulfanyl)-N-phenyl-acetamide;
[5-(4-Bromo-phenyl)-4-methyl-4H-[1,2,4]triazol-3-ylsulfanyl]-acetic acid cyclohexyl ester;
2-[5-(2-Bromo-phenyl)-4-ethyl-4H-[1,2,4]triazol-3-ylsulfanyl]-N-(3-chloro-phenyl)-acetamide;
2-[5-(2-Bromo-phenyl)-4-ethyl-4H-[1,2,4]triazol-3-ylsulfanyl]-N-(4-chloro-phenyl)-acetamide;
2-[5-(2-Bromo-phenyl)-4-ethyl-4H-[1,2,4]triazol-3-ylsulfanyl]-N-m-tolyl-acetamide;
N-(3-Cyano-4,5,6,7-tetrahydro-benzo[b]thiophen-2-yl)-2-[5-(4-hydroxy-phenyl)-4-methyl-4H-[1,2,4]triazol-3-ylsulfanyl]-acetamide;
2-(5-Benzyl-4-ethyl-4H-[1,2,4]triazol-3-ylsulfanyl)-N-(2-methyl-4-nitro-phenyl)-acetamide;
3-Benzylsulfanyl-4-methyl-5-phenyl-4H-[1,2,4]triazole;
3-Butylsulfanyl-5-(4-methoxy-phenyl)-4-methyl-4H-[1,2,4]triazole;
2-[5-(2-Bromo-phenyl)-4-ethyl-4H-[1,2,4]triazol-3-ylsulfanyl]-N-(4-ethoxy-phenyl)-acetamide;
N-(4-Acetyl-phenyl)-2-(4-benzyl-5-pyrid i n-3-yl-4 H-[1,2,4]triazol-3-ylsulfanyl)-acetamide;
2-(4-Benzyl-5-pyridin-3-yl-4H-[1,2,4]triazol-3-ylsulfanyl)-N-(2,4-dimethyl-phenyl)-acetamide;
N-(3-Nitro-phenyl)-2-(4-phenethyl-5-pyridin-3-yl-4H-[1,2,4]triazol-3-ylsulfanyl)-acetamide;
2-(4-Phenethyl-5-pyridin-3-yl-4H-[1,2,4]triazol-3-ylsulfanyl)-N-phenyl-acetamide;
N-Naphthalen-1-yl-2-(4-phenethyl-5-pyridin-3-yl-4H-[1,2,4]triazol-3-ylsulfanyl)-acetamide;
(4-Benzyl-5-pyridin-3-yl-4H-[1,2,4]triazol-3-ylsulfanyl)-acetic acid cyclohexyl ester;
1-(3,4-Dihydro-2H-quinolin-1-yl)-2-(4-ethyl-5-phenyl-4H-[1,2,4]triazol-3-ylsulfanyl)-ethanone;
N-(3-Cyano-4,5,6,7-tetrahydro-benzo[b]thiophen-2-yl)-2-(4-ethyl-5-phenyl-4H-[1,2,4]triazol-3-ylsulfanyl)-acetamide;
2-[5-(4-Methoxy-phenyl)-4-methyl-4H-[1,2,4]triazol-3-ylsulfanyl]-1-phenyl-ethanone;
3-Benzylsulfanyl-5-(4-methoxy-phenyl)-4-methyl-4H-[1,2,4]triazole;
2-(4,5-Dibenzyl-4H-[1,2,4]triazol-3-ylsulfanyl)-N-thiazol-2-yl-acetamide;
2-(4-Phenethyl-5-pyridin-3-yl-4H-[1,2,4]triazol-3-ylsulfanyl)-N-m-tolyl-acetamide;
2-(4-Phenethyl-5-pyridin-3-yl-4H-[1,2,4]triazol-3-ylsulfanyl)-N-phenyl-propionamide;
3-(5-Benzylsulfanyl-4-phenethyl-4H-[1,2,4]triazol-3-yl)-pyridine;
(4-Phenethyl-5-pyridin-3-yl-4H-[1,2,4]triazol-3-ylsulfanyl)-acetic acid ethyl ester;
2-(4-Phenethyl-5-pyridin-3-yl-4H-[1,2,4]triazol-3-ylsulfanyl)-1-phenyl-ethanone;
2-(4-Phenethyl-5-pyridin-3-yl-4H-[1,2,4]triazol-3-ylsulfanyl)-N-p-tolyl-propionamide;
1-(3,4-Dihydro-2H-quinolin-1-yl)-2-{4-ethyl-5-[(4-fluoro-phenylamino)-methyl]-4H-[1,2,4]triazol-3-ylsulfanyl}-ethanone;
2-[5-(3,4-Dimethoxy-phenyl)-4-methyl-4H-[1,2,4]triazol-3-ylsulfanyl]-1-p-tolyl-ethanone;
N-(3-Chloro-2-methyl-phenyl)-2-(4-methyl-5-o-tolyl-4H-[1,2,4]triazol-3-ylsulfanyl)-acetamide;
2-(4-Ethyl-5-pyridin-3-yl-4H-[1,2,4]triazol-3-ylsulfanyl)-N, N-diphenyl-propionamide;
2-(5-Cyclohexyl-4-ethyl-4H-[1,2,4]triazol-3-ylsulfanyl)-N-(2-nitro-phenyl)-acetamide;
2-(5-Cyclohexyl-4-ethyl-4H-[1,2,4]triazol-3-ylsulfanyl)-N-(4-methyl-3-nitro-phenyl)-acetamide;
N-(2-Bromo-4-methyl-phenyl)-2-(4-ethyl-5-pyridin-4-yl-4H-[1,2,4]triazol-3-ylsulfanyl)-acetamide;
1-(3,4-Dihydro-2H-quinolin-1-yl)-2-(4-phenethyl-5-pyridin-4-yl-4H-[1,2,4]triazol-3-ylsulfanyl)-ethanone;
N-(4-Fluoro-phenyl)-2-(4-phenethyl-5-pyridin-4-yl-4H-[1,2,4]triazol-3-ylsulfanyl)-acetamide;
N-(4-Acetyl-phenyl)-2-(5-benzyl-4-ethyl-4H-[1,2,4]triazol-3-ylsulfanyl)-acetamide;
2-(5-Benzyl-4-ethyl-4H-[1,2,4]triazol-3-ylsulfanyl)-N-(2-chloro-phenyl)-acetamide;
N-(3-Chloro-2-methyl-phenyl)-2-[4-ethyl-5-(2-methoxy-phenyl)-4H-[1,2,4]triazol-3-ylsulfanyl]-acetamide;
2-[5-(2-Bromo-phenyl)-4-ethyl-4H-[1,2,4]triazol-3-ylsulfanyl]-N-(2-methyl-4-nitro-phenyl)-acetamide;
2-[5-(2-Methoxy-phenyl)-4-methyl-4H-[1,2,4]triazol-3-ylsulfanyl]-N-p-tolyl-propionamide;
[4-Ethyl-5-(2-methoxy-phenyl)-4H-[1,2,4]triazol-3-ylsulfanyl]-acetic acid ethyl ester;
2-(4-Methyl-5-o-tolyl-4H-[1,2,4]triazol-3-ylsulfanyl)-N-(3-nitro-phenyl)-acetamide;
2-[4-Ethyl-5-(2-methoxy-phenyl)-4H-[1,2,4]triazol-3-ylsulfanyl]-N-naphthalen-1-yl-propionamide;
2-[4-Ethyl-5-(2-methoxy-phenyl)-4H-[1,2,4]triazol-3-ylsulfanyl]-N-p-tolyl-propionamide;
2-[4-Ethyl-5-(2-methoxy-phenyl)-4H-[1,2,4]triazol-3-ylsulfanyl]-N-phenyl-propionamide;
2-[4-Ethyl-5-(2-methoxy-phenyl)-4H-[1,2,4]triazol-3-ylsulfanyl]-N-(4-phenyl-thiazol-2-yl)-acetamide;
2-[4-Ethyl-5-(2-methoxy-phenyl)-4H-[1,2,4]triazol-3-ylsulfanyl]-N-p-tolyl-acetamide;
N-(3-Chloro-4-methyl-phenyl)-2-[4-ethyl-5-(2-methoxy-phenyl)-4H-[1,2,4]triazol-3-ylsulfanyl]-acetamide;
2-[5-(4-Hydroxy-phenyl)-4-methyl-4H-[1,2,4]triazol-3-ylsulfanyl]-N-(4-nitro-phenyl)-acetamide;
4-{2-[5-(2-Bromo-phenyl)-4-methyl-4H-[1,2,4]triazol-3-ylsulfanyl]-acetylamino}-benzoic acid ethyl ester;
2-[5-(2-Bromo-phenyl)-4-methyl-4H-[1,2,4]triazol-3-ylsulfanyl]-N-o-tolyl-acetamide;
2-[5-(2-Bromo-phenyl)-4-methyl-4H-[1,2,4]triazol-3-ylsulfanyl]-N-(4-chloro-phenyl)-acetamide;
2-[5-(2-Bromo-phenyl)-4-methyl-4H-[1,2,4]triazol-3-ylsulfanyl]-N-(4-methoxy-2-nitro-phenyl)-acetamide;
2-[5-(2-Bromo-phenyl)-4-methyl-4H-[1,2,4]triazol-3-ylsulfanyl]-N-(2-methyl-4-nitro-phenyl)-acetamide;
1-(3,4-Dihydro-2H-quinolin-1-yl)-2-{5-[(2,4-dimethyl-phenylamino)-methyl]-4-ethyl-4H-[1,2,4]triazol-3-ylsulfanyl}-ethanone;
N-(4-Chloro-3-nitro-phenyl)-2-[5-(4-chloro-phenyl)-4-ethyl-4H-[1,2,4]triazol-3-ylsulfanyl]-acetamide;
2-[5-(2-Bromo-phenyl)-4-ethyl-4H-[1,2,4]triazol-3-ylsulfanyl]-1-(3,4-dihydro-2H-quinolin-1-yl)-ethanone;
2-(5-Benzyl-4-methyl-4H-[1,2,4]triazol-3-ylsulfanyl)-1-p-tolyl-ethanone;
2-(4-Benzyl-5-pyridin-3-yl-4H-[1,2,4]triazol-3-ylsulfanyl)-N-(4-methoxy-phenyl)-acetamide;
2-(4-Benzyl-5-pyridin-3-yl-4H-[1,2,4]triazol-3-ylsulfanyl)-N-(2-chloro-phenyl)-acetamide;
2-(4-Benzyl-5-pyridin-3-yl-4H-[1,2,4]triazol-3-ylsulfanyl)-N-(5-ethyl-[1,3,4]thiadiazol-2-yl)-acetamide;
4-{2-[5-(2-Bromo-phenyl)-4-ethyl-4H-[1,2,4]triazol-3-ylsulfanyl]-acetylamino}-benzoic acid methyl ester;
2-(5-Benzyl-4-methyl-4H-[1,2,4]triazol-3-ylsulfanyl)-N-(2-nitro-phenyl)-acetamide;
2-(5-Benzyl-4-methyl-4H-[1,2,4]triazol-3-ylsulfanyl)-N-(3-cyano-4,5,6,7-tetrahydro-benzo[b]thiophen-2-yl)-acetamide;
2-[5-(4-Chloro-phenyl)-4-ethyl-4H-[1,2,4]triazol-3-ylsulfanyl]-N-(2-nitro-phenyl)-acetamide;
(5-Benzylsulfanyl-4-ethyl-4H-[1,2,4]triazol-3-ylmethyl)-(4-chloro-phenyl)-amine;
N-(2-Methoxy-phenyl)-2-[5-(2-methoxy-phenyl)-4-methyl-4H-[1,2,4]triazol-3-ylsulfanyl]-acetamide;
2-(4-Allyl-5-pyridin-4-yl-4H-[1,2,4]triazol-3-ylsulfanyl)-N-(3,4-dichloro-phenyl)-acetamide;
2-(5-Benzyl-4-ethyl-4H-[1,2,4]triazol-3-ylsulfanyl)-N-p-tolyl-acetamide;
2-(5-Benzyl-4-methyl-4H-[1,2,4]triazol-3-ylsulfanyl)-N-(4-chloro-phenyl)-acetamide;
2-(5-Benzyl-4-ethyl-4H-[1,2,4]triazol-3-ylsulfanyl)-N-(4-methoxy-phenyl)-acetamide;
N-(5-Chloro-2-methyl-phenyl)-2-(4-methyl-5-o-tolyl-4H-[1,2,4]triazol-3-ylsulfanyl)-acetamide;
N-(4-Methoxy-phenyl)-2-(4-methyl-5-o-tolyl-4H-[1,2,4]triazol-3-ylsulfanyl)-acetamide;
N-(2,4-Dimethyl-phenyl)-2-(4-methyl-5-o-tolyl-4H-[1,2,4]triazol-3-ylsulfanyl)-acetamide;
N-(4-Ethoxy-phenyl)-2-(4-methyl-5-o-tolyl-4H-[1,2,4]triazol-3-ylsulfanyl)-acetamide;
4-[2-(4-Methyl-5-o-tolyl-4H-[1,2,4]triazol-3-ylsulfanyl)-acetylamino]-benzoic acid methyl ester;
N-(4-Chloro-phenyl)-2-(4-methyl-5-o-tolyl-4H-[1,2,4]triazol-3-ylsulfanyl)-acetamide;
2-[5-(4-Hydroxy-phenyl)-4-methyl-4H-[1,2,4]triazol-3-ylsulfanyl]-N-(4-phenyl-thiazol-2-yl)-acetamide;
2-(5-Benzyl-4-methyl-4H-[1,2,4]triazol-3-ylsulfanyl)-N-(2-bromo-4-methyl-phenyl)-acetamide;
2-(5-Benzyl-4-methyl-4H-[1,2,4]triazol-3-ylsulfanyl)-1-phenyl-ethanone;
2-(5-Benzyl-4-ethyl-4H-[1,2,4]triazol-3-ylsulfanyl)-N-(4-nitro-phenyl)-acetamide;
2-(5-Cyclohexyl-4-ethyl-4H-[1,2,4]triazol-3-ylsulfanyl)-N-p-tolyl-acetamide;
2-[5-(2-Bromo-phenyl)-4-methyl-4H-[1,2,4]triazol-3-ylsulfanyl]-N-(4-ethoxy-phenyl)-acetamide;
2-(5-Cyclohexyl-4-ethyl-4H-[1,2,4]triazol-3-ylsulfanyl)-N-naphthalen-1-yl-acetamide;
4-[5-(4-tert-Butyl-benzylsulfanyl)-4-ethyl-4H-[1,2,4]triazol-3-yl]-pyridine;
4-[5-(2,4-Dichloro-benzylsulfanyl)-4-ethyl-4H-[1,2,4]triazol-3-yl]-pyridine;
2-(5-Cyclohexyl-4-ethyl-4H-[1,2,4]triazol-3-ylsulfanyl)-N-(2-methyl-4-nitro-phenyl)-acetamide;
2-(5-Cyclohexyl-4-ethyl-4H-[1,2,4]triazol-3-ylsulfanyl)-N-(3-trifluoromethyl-phenyl)-acetamide;
2-[5-(2-Bromo-phenyl)-4-methyl-4H-[1,2,4]triazol-3-ylsulfanyl]-N-(4-fluoro-phenyl)-acetamide;
4-{2-[5-(2-Bromo-phenyl)-4-methyl-4H-[1,2,4]triazol-3-ylsulfanyl]-acetylamino}-benzoic acid methyl ester;
N-(3-Chloro-phenyl)-2-(5-cyclohexyl-4-ethyl-4H-[1,2,4]triazol-3-ylsulfanyl)-acetamide;
2-(5-Cyclohexyl-4-ethyl-4H-[1,2,4]triazol-3-ylsulfanyl)-N-(4-ethoxy-phenyl)-acetamide;
2-{5-[(4-Chloro-phenylamino)-methyl]-4-ethyl-4H-[1,2,4]triazol-3-ylsulfanyl}-N-(2-nitro-phenyl)-acetamide;
2-(5-Benzyl-4-ethyl-4H-[1,2,4]triazol-3-ylsulfanyl)-N-(3-chloro-2-methyl-phenyl)-acetamide;
2-[5-(2-Bromo-phenyl)-4-ethyl-4H-[1,2,4]triazol-3-ylsulfanyl]-N-phenyl-acetamide;
3-Benzyl-5-(2-chloro-6-fluoro-benzylsulfanyl)-4-methyl-4H-[1,2,4]triazole;
2-(5-Cyclohexyl-4-ethyl-4H-[1,2,4]triazol-3-ylsulfanyl)-N-(4-nitro-phenyl)-acetamide;
2-[5-(4-Hydroxy-phenyl)-4-methyl-4H-[1,2,4]triazol-3-ylsulfanyl]-1-phenyl-ethanone;
4-[5-(4-Chloro-benzylsulfanyl)-4-methyl-4H-[1,2,4]triazol-3-yl]-phenol;
2-(5-Benzyl-4-ethyl-4H-[1,2,4]triazol-3-ylsulfanyl)-N-(4-fluoro-phenyl)-acetamide;
2-(5-Cyclohexyl-4-ethyl-4H-[1,2,4]triazol-3-ylsulfanyl)-1-(4-methoxy-phenyl)-ethanone;
2-(5-Benzyl-4-ethyl-4H-[1,2,4]triazol-3-ylsulfanyl)-N-(4-bromo-phenyl)-acetamide;
2-(5-Benzyl-4-ethyl-4H-[1,2,4]triazol-3-ylsulfanyl)-N-phenyl-acetamide;
2-(5-Cyclohexyl-4-ethyl-4H-[1,2,4]triazol-3-ylsulfanyl)-N-(4-nitro-phenyl)-propionamide;
2-(5-Benzyl-4-ethyl-4H-[1,2,4]triazol-3-ylsulfanyl)-N-(2-trifluoromethyl-phenyl)-acetamide;
{5-[(3-Chloro-4-methyl-phenylamino)-methyl]-4-ethyl-4H-[1,2,4]triazol-3-ylsulfanyl}-acetic acid cyclohexyl ester;
3-[5-(2,4-Dichloro-benzylsulfanyl)-4-methyl-4H-[1,2,4]triazol-3-yl]-pyridine;
2-[5-(2-Bromo-phenyl)-4-methyl-4H-[1,2,4]triazol-3-ylsulfanyl]-N-phenyl-acetamide;
N-(3-Chloro-4-methyl-phenyl)-2-[5-(4-hydroxy-phenyl)-4-methyl-4H-[1,2,4]triazol-3-ylsulfanyl]-acetamide;
N-(4-Chloro-phenyl)-2-[5-(4-hydroxy-phenyl)-4-methyl-4H-[1,2,4]triazol-3-ylsulfanyl]-acetamide;
3-Benzyl-5-(4-chloro-benzylsulfanyl)-4-methyl-4H-[1,2,4]triazole;
2-[5-(2-Bromo-phenyl)-4-methyl-4H-[1,2,4]triazol-3-ylsulfanyl]-N-(4-nitro-phenyl)-acetamide;
N-(4-Ethoxy-phenyl)-2-[5-(4-hydroxy-phenyl)-4-methyl-4H-[1,2,4]triazol-3-ylsulfanyl]-acetamide;
2-[5-(2-Bromo-phenyl)-4-methyl-4H-[1,2,4]triazol-3-ylsulfanyl]-N-(4-methoxy-phenyl)-acetamide;
{5-[(4-Chloro-phenylamino)-methyl]-4-ethyl-4H-[1,2,4]triazol-3-ylsulfanyl}-acetic acid cyclohexyl ester;
3-Benzylsulfanyl-5-(2-methoxy-phenyl)-4-methyl-4H-[1,2,4]triazole;
N-(2-Chloro-phenyl)-2-(4-methyl-5-m-tolyl-4H-[1,2,4]triazol-3-ylsulfanyl)-acetamide;
N-(2-Methoxy-phenyl)-2-(4-methyl-5-o-tolyl-4H-[1,2,4]triazol-3-ylsulfanyl)-acetamide;
2-(5-Cyclohexyl-4-ethyl-4H-[1,2,4]triazol-3-ylsulfanyl)-N-phenyl-acetamide;
2-[5-(2-Bromo-phenyl)-4-ethyl-4H-[1,2,4]triazol-3-ylsulfanyl]-N-(3-nitro-phenyl)-acetamide;
2-[5-(2-Bromo-phenyl)-4-methyl-4H-[1,2,4]triazol-3-ylsulfanyl]-1-(3,4-dihydro-2H-quinolin-1-yl)-ethanone;
2-(5-Benzyl-4-ethyl-4H-[1,2,4]triazol-3-ylsulfanyl)-N-(2-bromo-4-methyl-phenyl)-acetamide;
N-(2-Chloro-phenyl)-2-[5-(2-methoxy-phenyl)-4-methyl-4H-[1,2,4]triazol-3-ylsulfanyl]-acetamide;
(5-Benzylsulfanyl-4-ethyl-4H-[1,2,4]triazol-3-ylmethyl)-(3-trifluoromethyl-phenyl)-amine;
2-[5-(2-Bromo-phenyl)-4-methyl-4H-[1,2,4]triazol-3-ylsulfanyl]-1-(4-methoxy-phenyl)-ethanone;
[4-Allyl-5-(3-bromo-benzylsulfanyl)-4H-[1,2,4]triazol-3-ylmethyl]-phenyl-amine;
2-(4-Furan-2-ylmethyl-5-pyridin-4-yl-4H-[1,2,4]triazol-3-ylsulfanyl)-1-(3-nitro-phenyl)-ethanone;
1-(4-Bromo-phenyl)-2-(4-furan-2-ylmethyl-5-pyridin-4-yl-4H-[1,2,4]triazol-3-ylsulfanyl)-ethanone;
4-{5-[2-(2-Methoxy-phenoxy)-ethylsulfanyl]-4-methyl-4H-[1,2,4]triazol-3-yl}-phenylamine;
4-[5-(4-Chloro-benzylsulfanyl)-4-methyl-4H-[1,2,4]triazol-3-yl]-phenylamine;
[4-Allyl-5-(1,3-dioxo-1H,3H-benzo[de]isoquinolin-2-ylmethyl)-4H-[1,2,4]triazol-3-ylsulfanyl]-acetic acid ethyl ester;
2-{4-Allyl-5-[(4-chloro-phenylamino)-methyl]-4H-[1,2,4]triazol-3-ylsulfanyl}-1-phenyl-ethanone;
5-(3-Bromo-phenyl)-4-(2-methyl-allyl)-4H-[1,2,4]triazole-3-thiol;
2-(4-Allyl-5-benzotriazol-1-ylmethyl-4H-[1,2,4]triazol-3-ylsulfanyl)-N-(4-chloro-phenyl)-acetamide;
1-(4-Fluoro-phenyl)-2-(4-methyl-5-phenyl-4H-[1,2,4]triazol-3-ylsulfanyl)-ethanone;
8-(4-Methyl-5-phenyl-4H-[1,2,4]triazol-3-ylsulfanyl)-5-nitro-quinoline;
3-(2-Bromo-benzylsulfanyl)-4-methyl-5-(4-nitro-phenyl)-4H-[1,2,4]triazole;
2-[4-Allyl-5-(3-methoxy-phenyl)-4H-[1,2,4]triazol-3-ylsulfanyl]-1-(2,4-dimethyl-phenyl)-ethanone;
1-(4-Bromo-phenyl)-2-(5-furan-2-yl-4-methyl-4H-[1,2,4]triazol-3-ylsulfanyl)-ethanone;
2-[5-(2-Bromo-phenyl)-4-ethyl-4H-[1,2,4]triazol-3-ylsulfanyl]-N-(5-methyl-thiazol-2-yl)-acetamide;
2-[4-Ethyl-5-(p-tolylamino-methyl)-4H-[1,2,4]triazol-3-ylsulfanyl]-1-p-tolyl-ethanone;
2-(5-Benzyl-4-ethyl-4H-[1,2,4]triazol-3-ylsulfanyl)-N-(4-ethyl-phenyl)-acetamide;
2-(5-Benzyl-4-ethyl-4H-[1,2,4]triazol-3-ylsulfanyl)-1-(4-methoxy-phenyl)-ethanone;
2-(5-Benzyl-4-ethyl-4H-[1,2,4]triazol-3-ylsulfanyl)-N-(5-ethyl-[1,3,4]thiadiazol-2-yl)-acetamide;
2-[5-(2-Bromo-phenyl)-4-methyl-4H-[1,2,4]triazol-3-ylsulfanyl]-1-p-tolyl-ethanone;
3-(2-Bromo-phenyl)-4-methyl-5-(4-nitro-benzylsulfanyl)-4H-[1,2,4]triazole;
2-[5-(2-Bromo-phenyl)-4-methyl-4H-[1,2,4]triazol-3-ylsulfanyl]-N-(4-phenyl-thiazol-2-yl)-acetamide;
2-[5-(2-Bromo-phenyl)-4-methyl-4H-[1,2,4]triazol-3-ylsulfanyl]-N-(2,4-dimethyl-phenyl)-acetamide;
N-(4-Bromo-phenyl)-2-[5-(2-bromo-phenyl)-4-ethyl-4H-[1,2,4]triazol-3-ylsulfanyl]-acetamide;
2-{5-[(2,4-Dimethyl-phenylamino)-methyl]-4-ethyl-4H-[1,2,4]triazol-3-ylsulfanyl}-1-(4-methoxyphenyl)-ethanone;
2-{5-[(2,4-Dimethyl-phenylamino)-methyl]-4-ethyl-4H-[1,2,4]triazol-3-ylsulfanyl}-1-p-tolyl-ethanone;
{5-[(2,4-Dimethyl-phenylamino)-methyl]-4-ethyl-4H-[1,2,4]triazol-3-ylsulfanyl}-acetic acid cyclohexyl ester;
2-{5-[(4-Fluoro-phenylamino)-methyl]-4-methyl-4H-[1,2,4]triazol-3-ylsulfanyl}-1-phenyl-ethanone;
2-{4-Ethyl-5-[(4-fluoro-phenylamino)-methyl]-4H-[1,2,4]triazol-3-ylsulfanyl}-1-(4-methoxyphenyl)-ethanone;
[4-Ethyl-5-(4-methyl-benzylsulfanyl)-4H-[1,2,4]triazol-3-ylmethyl]-(4-fluoro-phenyl)-amine;
(5-Benzylsulfanyl-4-ethyl-4H-[1,2,4]triazol-3-ylmethyl)-(4-fluoro-phenyl)-amine;
{4-Ethyl-5-[(4-fluoro-phenylamino)-methyl]-4H-[1,2,4]triazol-3-ylsulfanyl}-acetic acid cyclohexyl ester;
N-(4-Chloro-phenyl)-2-[4-ethyl-5-(4-hydroxy-phenyl)-4H-[1,2,4]triazol-3-ylsulfanyl]-acetamide;
N-(4-Acetyl-phenyl)-2-(5-cyclohexyl-4-ethyl-4H-[1,2,4]triazol-3-ylsulfanyl)-acetamide;
2-[4-Ethyl-5-(4-hydroxy-phenyl)-4H-[1,2,4]triazol-3-ylsulfanyl]-N-(4-methyl-3-nitro-phenyl)-acetamide;
N-Cyclohexyl-2-(4-phenethyl-5-pyridin-3-yl-4H-[1,2,4]triazol-3-ylsulfanyl)-acetamide;
N-(2,4-Dimethyl-phenyl)-2-[4-furan-2-ylmethyl-5-(4-hydroxy-phenyl)-4H-[1,2,4]triazol-3-ylsulfanyl]-acetamide;
4-[2-(5-Cyclohexyl-4-methyl-4H-[1,2,4]triazol-3-ylsulfanyl)-acetylamino]-benzoic acid ethyl ester;
2-[4-Furan-2-ylmethyl-5-(4-hydroxy-phenyl)-4H-[1,2,4]triazol-3-ylsulfanyl]-N-(4-methyl-3-nitrophenyl)-acetamide;
3-Isobutylsulfanyl-4-methyl-5-p-tolyl-4H-[1,2,4]triazole;
2-[2-(4-Methyl-5-m-tolyl-4H-[1,2,4]triazol-3-ylsulfanyl)-acetylamino]-benzoic acid methyl ester;
2-{4-Ethyl-5-[4-(morpholine-4-sulfonyl)-phenyl]-4H-[1,2,4]triazol-3-ylsulfanyl}-1-p-tolyl-ethanone;
4-Methyl-3-phenethylsulfanyl-5-p-tolyl-4H-[1,2,4]triazole;
2-(4-Benzyl-5-pyridin-3-yl-4 H-[1,2,4]triazol-3-ylsulfanyl)-N-p-tolyl-acetamide;
2-(4-Benzyl-5-pyridin-3-yl-4 H-[1,2,4]triazol-3-ylsulfanyl)-N-(4-nitro-phenyl)-acetamide;
2-(4-Benzyl-5-pyrid in-3-yl-4 H-[1,2,4]triazol-3-ylsulfanyl)-N-phenyl-acetamide;
2-(4-Benzyl-5-pyridin-3-yl-4H-[1,2,4]triazol-3-ylsulfanyl)-N-(3-chloro-phenyl)-acetamide;
2-(4-Benzyl-5-pyridin-3-yl-4H-[1,2,4]triazol-3-ylsulfanyl)-N-(4-nitro-phenyl)-propionamide;
2-(4-Benzyl-5-pyridin-3-yl-4 H-[1,2,4]triazol-3-ylsulfanyl)-N-(2,5-dichloro-phenyl)-acetamide;
N-(3-Chloro-phenyl)-2-(4-phenethyl-5-pyridin-3-yl-4H-[1,2,4]triazol-3-ylsulfanyl)-acetamide;
N-(4-Nitro-phenyl)-2-(4-phenethyl-5-pyridin-3-yl-4H-[1,2,4]triazol-3-ylsulfanyl)-propionamide;
N-(2-Bromo-4-methyl-phenyl)-2-[5-(3,4-dimethoxy-phenyl)-4-methyl-4H-[1,2,4]triazol-3-ylsulfanyl]-acetamide;
2-{4-Ethyl-5-[(4-fluoro-phenylamino)-methyl]-4H-[1,2,4]triazol-3-ylsulfanyl}-N-(4-nitro-phenyl)-acetamide;
3-Benzylsulfanyl-5-cyclohexyl-4-ethyl-4H-[1,2,4]triazole;
[5-(4-Hydroxy-phenyl)-4-methyl-4H-[1,2,4]triazol-3-ylsulfanyl]-acetic acid cyclohexyl ester;
2-(5-Cyclohexyl-4-ethyl-4H-[1,2,4]triazol-3-ylsulfanyl)-N-(5-ethyl-[1,3,4]thiadiazol-2-yl)-acetamide;
N-(3-Chloro-4-methyl-phenyl)-2-[4-ethyl-5-(4-hydroxy-phenyl)-4H-[1,2,4]triazol-3-ylsulfanyl]-acetamide;
[4-Benzyl-5-(4-methoxy-phenyl)-4H-[1,2,4]triazol-3-ylsulfanyl]-acetic acid ethyl ester;
3-Benzylsulfanyl-4-methyl-5-(3,4,5-trimethoxy-phenyl)-4H-[1,2,4]triazole;
N-(2-Chloro-phenyl)-2-[4-ethyl-5-(4-hydroxy-phenyl)-4H-[1,2,4]triazol-3-ylsulfanyl]-acetamide;
N-(2,4-Dimethoxy-phenyl)-2-(4-methyl-5-o-tolyl-4H-[1,2,4]triazol-3-ylsulfanyl)-acetamide;
2-(5-Cyclohexyl-4-methyl-4H-[1,2,4]triazol-3-ylsulfanyl)-N-(2-methyl-4-nitro-phenyl)-acetamide;
2-(5-Cyclohexyl-4-methyl-4H-[1,2,4]triazol-3-ylsulfanyl)-1-phenyl-ethanone;
3-Cyclohexyl-4-methyl-5-(4-methyl-benzylsulfanyl)-4H-[1,2,4]triazole;
N-(2-Bromo-phenyl)-2-(5-cyclohexyl-4-methyl-4H-[1,2,4]triazol-3-ylsulfanyl)-acetamide;
2-(5-Cyclohexyl-4-methyl-4H-[1,2,4]triazol-3-ylsulfanyl)-N-(4-iodo-2-methyl-phenyl)-acetamide;
N-(3-Cyano-4,5,6,7-tetrahydro-benzo[b]thiophen-2-yl)-2-(5-cyclohexyl-4-methyl-4H-[1,2,4]triazol-3-ylsulfanyl)-acetamide;
N-(4-Acetyl-phenyl)-2-(5-cyclohexyl-4-methyl-4H-[1,2,4]triazol-3-ylsulfanyl)-acetamide;
2-(5-Cyclohexyl-4-methyl-4H-[1,2,4]triazol-3-ylsulfanyl)-N-(4-methoxy-phenyl)-acetamide;
N-(4-Chloro-phenyl)-2-(5-cyclohexyl-4-methyl-4H-[1,2,4]triazol-3-ylsulfanyl)-acetamide;
2-(5-Cyclohexyl-4-methyl-4H-[1,2,4]triazol-3-ylsulfanyl)-N-phenyl-acetamide;
2-[5-(2-Bromo-phenyl)-4-ethyl-4H-[1,2,4]triazol-3-ylsulfanyl]-N-(2-methoxy-4-nitro-phenyl)-acetamide;
N-(3-Cyano-4,5,6,7-tetrahydro-benzo[b]thiophen-2-yl)-2-[5-(2-methoxy-phenyl)-4-methyl-4H-[1,2,4]triazol-3-ylsulfanyl]-acetamide;
N-(4-Bromo-phenyl)-2-(5-cyclohexyl-4-ethyl-4H-[1,2,4]triazol-3-ylsulfanyl)-acetamide;
2-[4-Ethyl-5-(4-hydroxy-phenyl)-4H-[1,2,4]triazol-3-ylsulfanyl]-N-phenyl-acetamide;
N-(3-Chloro-phenyl)-2-[4-ethyl-5-(4-hydroxy-phenyl)-4H-[1,2,4]triazol-3-ylsulfanyl]-acetamide;
N-(4-Acetyl-phenyl)-2-[4-ethyl-5-(4-hydroxy-phenyl)-4H-[1,2,4]triazol-3-ylsulfanyl]-acetamide;
N-(4-Bromo-phenyl)-2-[4-ethyl-5-(4-hydroxy-phenyl)-4H-[1,2,4]triazol-3-ylsulfanyl]-acetamide;
2-[4-Ethyl-5-(4-hydroxy-phenyl)-4H-[1,2,4]triazol-3-ylsulfanyl]-N-(4-iodo-2-methyl-phenyl)-acetamide;
N-(2,4-Dimethyl-phenyl)-2-[4-ethyl-5-(4-hydroxy-phenyl)-4H-[1,2,4]triazol-3-ylsulfanyl]-acetamide;
4-[5-(4-Chloro-benzylsulfanyl)-4-ethyl-4H-[1,2,4]triazol-3-yl]-phenol;
[4-Ethyl-5-(4-hydroxy-phenyl)-4H-[1,2,4]triazol-3-ylsulfanyl]-acetic acid cyclohexyl ester;
2-[5-(2-Methoxy-phenyl)-4-methyl-4H-[1,2,4]triazol-3-ylsulfanyl]-N-(4-phenyl-thiazol-2-yl)-acetamide;
N-(3-Chloro-phenyl)-2-[4-ethyl-5-(2-methoxy-phenyl)-4H-[1,2,4]triazol-3-ylsulfanyl]-acetamide;
2-[5-(2-Bromo-phenyl)-4-methyl-4H-[1,2,4]triazol-3-ylsulfanyl]-N-(2-nitro-phenyl)-acetamide;
2-[4-Methyl-5-(4-nitro-phenyl)-4H-[1,2,4]triazol-3-ylsulfanyl]-1-phenyl-ethanone;
N-(4-Bromo-phenyl)-2-{4-ethyl-5-[(4-fluoro-phenylamino)-methyl]-4H-[1,2,4]triazol-3-ylsulfanyl}-acetamide;
N-(4-Chloro-phenyl)-2-{4-ethyl-5-[(4-fluoro-phenylamino)-methyl]-4H-[1,2,4]triazol-3-ylsulfanyl}-acetamide;
2-{4-Ethyl-5-[(4-fluoro-phenylamino)-methyl]-4H-[1,2,4]triazol-3-ylsulfanyl}-N-(2-nitro-phenyl)-acetamide;
1-(4-Methoxy-phenyl)-2-[4-methyl-5-(4-nitro-phenyl)-4H-[1,2,4]triazol-3-ylsulfanyl]-ethanone;
[5-(4-Chloro-benzylsulfanyl)-4-ethyl-4H-[1,2,4]triazol-3-ylmethyl]-(4-fluoro-phenyl)-amine;
2-{4-Ethyl-5-[(4-fluoro-phenylamino)-methyl]-4H-[1,2,4]triazol-3-ylsulfanyl}-1-p-tolyl-ethanone;
2-{4-Ethyl-5-[(4-fluoro-phenylamino)-methyl]-4H-[1,2,4]triazol-3-ylsulfanyl}-N-(4-methyl-3-nitro-phenyl)-acetamide;
N-(2-Bromo-4-methyl-phenyl)-2-{4-ethyl-5-[(4-fluoro-phenylamino)-methyl]-4H-[1,2,4]triazol-3-ylsulfanyl}-acetamide;
4-Allyl-5-(3-bromo-phenyl)-4H-[1,2,4]triazole-3-thiol;
N-(2-Nitro-phenyl)-2-(4-phenethyl-5-pyridin-3-yl-4H-[1,2,4]triazol-3-ylsulfanyl)-acetamide;
2-{4-Ethyl-5-[4-(piperidine-1-sulfonyl)-phenyl]-4H-[1,2,4]triazol-3-ylsulfanyl}-1-p-tolyl-ethanone;
2-{4-Ethyl-5-[4-(piperidine-1-sulfonyl)-phenyl]-4H-[1,2,4]triazol-3-ylsulfanyl}-1-phenyl-ethanone;
N-Biphenyl-2-yl-2-(4-ethyl-5-phenyl-4H-[1,2,4]triazol-3-ylsulfanyl)-acetamide;
2-(4-Benzyl-5-pyridin-3-yl-4H-[1,2,4]triazol-3-ylsulfanyl)-N-(3-chloro-4-methyl-phenyl)-acetamide;
N-(2-Bromo-phenyl)-2-(4-ethyl-5-phenyl-4H-[1,2,4]triazol-3-ylsulfanyl)-acetamide;
4-Benzyl-5-cyclohexyl-4H-[1,2,4]triazole-3-thiol;
2-(5-Cyclohexyl-4-ethyl-4H-[1,2,4]triazol-3-ylsulfanyl)-1-phenyl-ethanone;
2-[5-(2-Bromo-phenyl)-4-methyl-4H-[1,2,4]triazol-3-ylsulfanyl]-1-phenyl-ethanone;
(5-Benzyl-4-methyl-4H-[1,2,4]triazol-3-ylsulfanyl)-acetic acid cyclohexyl ester;
4-Methyl-3-(4-nitro-benzylsulfanyl)-5-phenyl-4H-[1,2,4]triazole;
2-(4-Methyl-5-phenyl-4H-[1,2,4]triazol-3-ylsulfanyl)-1-p-tolyl-ethanone;
2-(4-Methyl-5-phenyl-4H-[1,2,4]triazol-3-ylsulfanyl)-1-phenyl-ethanone;
3-Benzylsulfanyl-4-methyl-5-(naphthalen-2-yloxymethyl)-4H-[1,2,4]triazole;
2-(5-Cyclohexyl-4-methyl-4H-[1,2,4]triazol-3-ylsulfanyl)-N-p-tolyl-acetamide;
3-Benzylsulfanyl-5-cyclohexyl-4-methyl-4H-[1,2,4]triazole;
2-(5-Benzyl-4-ethyl-4H-[1,2,4]triazol-3-ylsulfanyl)-N-(4-chloro-3-nitro-phenyl)-acetamide;
2-(5-Benzyl-4-ethyl-4H-[1,2,4]triazol-3-ylsulfanyl)-N-(4-methyl-3-nitro-phenyl)-acetamide;
2-(5-Benzyl-4-ethyl-4H-[1,2,4]triazol-3-ylsulfanyl)-N-(2-methoxy-4-nitro-phenyl)-acetamide;
N-(2-Bromo-4-methyl-phenyl)-2-(4-methyl-5-phenyl-4H-[1,2,4]triazol-3-ylsulfanyl)-acetamide;
3-Benzylsulfanyl-4-ethyl-5-(4-nitro-phenyl)-4H-[1,2,4]triazole;
4-Ethyl-3-(4-methyl-benzylsulfanyl)-5-(4-nitro-phenyl)-4H-[1,2,4]triazole;
2-[4-Ethyl-5-(4-nitro-phenyl)-4H-[1,2,4]triazol-3-ylsulfanyl]-1-p-tolyl-ethanone;
3-(4-Chloro-benzylsulfanyl)-4-ethyl-5-(4-nitro-phenyl)-4H-[1,2,4]triazole;
2-[4-Ethyl-5-(4-nitro-phenyl)-4H-[1,2,4]triazol-3-ylsulfanyl]-1-phenyl-ethanone;
2-[5-(2-Bromo-phenyl)-4-methyl-4H-[1,2,4]triazol-3-ylsulfanyl]-N-(3,4-dimethyl-phenyl)-acetamide;
(2,4-Dimethyl-phenyl)-[4-ethyl-5-(4-nitro-benzylsulfanyl)-4H-[1,2,4]triazol-3-ylmethyl]-amine
2-[5-(2-Bromo-phenyl)-4-methyl-4H-[1,2,4]triazol-3-ylsulfanyl]-N-(4-methyl-3-nitro-phenyl)-acetamide;
2-(4-Methyl-5-o-tolyl-4H-[1,2,4]triazol-3-ylsulfanyl)-N-(2-trifluoromethyl-phenyl)-acetamide;
2-[5-[(2,4-Dimethyl-phenylamino)-methyl]-4-(tetrahydro-furan-2-ylmethyl)-4H-[1,2,4]triazol-3-ylsulfanyl]-1-p-tolyl-ethanone;
4-[5-(2,6-Dichloro-benzylsulfanyl)-4-(tetrahydro-furan-2-ylmethyl)-4H-[1,2,4]triazol-3-yl]-pyridine;
3-Benzylsulfanyl-5-(4-chloro-phenoxymethyl)-4-ethyl-4H-[1,2,4]triazole;
3-Butylsulfanyl-4-methyl-5-p-tolyl-4H-[1,2,4]triazole;
1-(5-Chloro-2-methoxy-phenyl)-2-(4-methyl-5-p-tolyl-4H-[1,2,4]triazol-3-ylsulfanyl)-ethanone;
1-(4-Chloro-phenyl)-2-(4-methyl-5-p-tolyl-4H-[1,2,4]triazol-3-ylsulfanyl)-ethanone;
N-(2,4-Dimethoxy-phenyl)-2-(4-ethyl-5-phenyl-4H-[1,2,4]triazol-3-ylsulfanyl)-acetamide;
2-(4-Ethyl-5-phenyl-4H-[1,2,4]triazol-3-ylsulfanyl)-N-(2-methyl-4-nitro-phenyl)-acetamide;
N-(4-Ethoxy-phenyl)-2-(4-ethyl-5-phenyl-4H-[1,2,4]triazol-3-ylsulfanyl)-acetamide;
N-(4-Ethyl-phenyl)-2-(4-ethyl-5-phenyl-4H-[1,2,4]triazol-3-ylsulfanyl)-acetamide;
2-(4-Ethyl-5-phenyl-4H-[1,2,4]triazol-3-ylsulfanyl)-N-thiazol-2-yl-acetamide;
2-(4-Ethyl-5-phenyl-4H-[1,2,4]triazol-3-ylsulfanyl)-1-phenyl-ethanone;
2-(4-Ethyl-5-phenyl-4H-[1,2,4]triazol-3-ylsulfanyl)-1-p-tolyl-ethanone;
2-(4-Ethyl-5-phenyl-4H-[1,2,4]triazol-3-ylsulfanyl)-N-(2-nitro-phenyl)-acetamide;
N-(3-Chloro-phenyl)-2-(4-ethyl-5-phenyl-4H-[1,2,4]triazol-3-ylsulfanyl)-acetamide;
N-(2-Chloro-phenyl)-2-(4-ethyl-5-phenyl-4H-[1,2,4]triazol-3-ylsulfanyl)-acetamide;
2-[5-(2-Hydroxy-phenyl)-4-phenethyl-4H-[1,2,4]triazol-3-ylsulfanyl]-1-piperidin-1-yl-ethanone;
2-[5-(2-Morpholin-4-yl-ethylsulfanyl)-4-phenethyl-4H-[1,2,4]triazol-3-yl]-phenol;
N-(2-Methyl-4-nitro-phenyl)-2-(4-phenethyl-5-pyridin-4-yl-4H-[1,2,4]triazol-3-ylsulfanyl)-acetamide;
N-(2-Methyl-5-nitro-phenyl)-2-(4-phenethyl-5-pyridin-4-yl-4H-[1,2,4]triazol-3-ylsulfanyl)-acetamide;
N-(4-Bromo-phenyl)-2-(4-phenethyl-5-pyridin-4-yl-4H-[1,2,4]triazol-3-ylsulfanyl)-acetamide;
N-(3-Nitro-phenyl)-2-(4-phenethyl-5-pyridin-4-yl-4H-[1,2,4]triazol-3-ylsulfanyl)-acetamide;
2-(4-Ethyl-5-phenyl-4H-[1,2,4]triazol-3-ylsulfanyl)-N-(4-fluoro-phenyl)-acetamide;
2-(4-Ethyl-5-phenyl-4H-[1,2,4]triazol-3-ylsulfanyl)-N-(2-methoxy-4-nitro-phenyl)-acetamide;
N-(2,5-Dichloro-phenyl)-2-[4-ethyl-5-(2-methoxy-phenyl)-4H-[1,2,4]triazol-3-ylsulfanyl]-acetamide;
2-(5-Cyclohexyl-4-methyl-4H-[1,2,4]triazol-3-ylsulfanyl)-N-(3-nitro-phenyl)-acetamide;
2-[4-Benzyl-5-(4-hydroxy-phenyl)-4H-[1,2,4]triazol-3-ylsulfanyl]-N-(2-methyl-4-nitro-phenyl)-acetamide;
2-(4-Phenethyl-5-pyridin-4-yl-4H-[1,2,4]triazol-3-ylsulfanyl)-N-(3-trifluoromethyl-phenyl)-acetamide;
[4-Furan-2-ylmethyl-5-(4-hydroxy-phenyl)-4H-[1,2,4]triazol-3-ylsulfanyl]-acetic acid cyclohexyl ester;
2-[4-Furan-2-ylmethyl-5-(4-hydroxy-phenyl)-4H-[1,2,4]triazol-3-ylsulfanyl]-1-phenyl-ethanone;
2-[4-Ethyl-5-(2-methoxy-phenyl)-4H-[1,2,4]triazol-3-ylsulfanyl]-N-(2-ethyl-phenyl)-acetamide;
2-(5-Cyclohexyl-4-methyl-4H-[1,2,4]triazol-3-ylsulfanyl)-N-(3,4-dichloro-phenyl)-acetamide;
2-(5-Cyclohexyl-4-methyl-4H-[1,2,4]triazol-3-ylsulfanyl)-1-(4-methoxy-phenyl)-ethanone;
2-(4-Benzyl-5-phenyl-4H-[1,2,4]triazol-3-ylsulfanyl)-N-(4-methoxy-2-nitro-phenyl)-acetamide;
2-(4-Benzyl-5-phenyl-4H-[1,2,4]triazol-3-ylsulfanyl)-N-(5-methyl-thiazol-2-yl)-acetamide;
2-(4-Benzyl-5-phenyl-4H-[1,2,4]triazol-3-ylsulfanyl)-N-thiazol-2-yl-acetamide;
2-(4,5-Dibenzyl-4H-[1,2,4]triazol-3-ylsulfanyl)-N-(4-nitro-phenyl)-acetamide;
2-(4-Benzyl-5-phenyl-4H-[1,2,4]triazol-3-ylsulfanyl)-N-(2-nitro-phenyl)-acetamide;
3-Benzylsulfanyl-5-(4-bromo-phenyl)-4-methyl-4H-[1,2,4]triazole;
2-[4-Ethyl-5-(4-methoxy-phenyl)-4H-[1,2,4]triazol-3-ylsulfanyl]-N-(4-methyl-3-nitro-phenyl)-acetamide;
2-[4-Ethyl-5-(4-methoxy-phenyl)-4H-[1,2,4]triazol-3-ylsulfanyl]-1-phenyl-ethanone;
2-(4-Benzyl-5-methyl-4H-[1,2,4]triazol-3-ylsulfanyl)-1-phenyl-ethanone;
2-(5-Methyl-4-phenethyl-4H-[1,2,4]triazol-3-ylsulfanyl)-N-p-tolyl-acetamide;
2-(5-Methyl-4-phenethyl-4H-[1,2,4]triazol-3-ylsulfanyl)-N-(3-trifluoromethyl-phenyl)-acetamide;
N-(3-Chloro-phenyl)-2-(5-methyl-4-phenethyl-4H-[1,2,4]triazol-3-ylsulfanyl)-acetamide;
N-(4-Chloro-phenyl)-2-(5-methyl-4-phenethyl-4H-[1,2,4]triazol-3-ylsulfanyl)-acetamide;
2-[5-(2-Bromo-phenyl)-4-methyl-4H-[1,2,4]triazol-3-ylsulfanyl]-N-(2-methoxy-4-nitro-phenyl)-acetamide;
2-(5-Cyclohexyl-4-ethyl-4H-[1,2,4]triazol-3-ylsulfanyl)-N-(3-nitro-phenyl)-acetamide;
N-(2,3-Dichloro-phenyl)-2-[4-ethyl-5-(2-methoxy-phenyl)-4H-[1,2,4]triazol-3-ylsulfanyl]-acetamide;
N-(2,3-Dichloro-phenyl)-2-[4-ethyl-5-(4-hydroxy-phenyl)-4H-[1,2,4]triazol-3-ylsulfanyl]-acetamide;
2-[4-Ethyl-5-(2-methoxy-phenyl)-4H-[1,2,4]triazol-3-ylsulfanyl]-1-(4-methoxy-phenyl)-ethanone;
2-[4-Benzyl-5-(2-hydroxy-phenyl)-4H-[1,2,4]triazol-3-ylsulfanyl]-1-piperidin-1-yl-ethanone;
2-[4-Benzyl-5-(2-hydroxy-phenyl)-4H-[1,2,4]triazol-3-ylsulfanyl]-N-p-tolyl-acetamide;
2-{5-[(2,4-Dimethyl-phenylamino)-methyl]-4-furan-2-ylmethyl-4H-[1,2,4]triazol-3-ylsulfanyl}-1-phenyl-ethanone;
2-(4-Methyl-5-m-tolyl-4H-[1,2,4]triazol-3-ylsulfanyl)-N-p-tolyl-acetamide;
4-{4-Ethyl-5-[4-(piperidine-1-sulfonyl)-phenyl]-4H-[1,2,4]triazol-3-ylsulfanylmethyl}-benzonitrile;
N-(2-Methoxy-phenyl)-2-(4-methyl-5-m-tolyl-4H-[1,2,4]triazol-3-ylsulfanyl)-acetamide;
N-(4-Acetyl-phenyl)-2-(4-ethyl-5-m-tolyl-4H-[1,2,4]triazol-3-ylsulfanyl)-acetamide;
2-(4-Ethyl-5-m-tolyl-4H-[1,2,4]triazol-3-ylsulfanyl)-N-(4-methyl-3-nitro-phenyl)-acetamide;
2-[5-(4-Amino-phenyl)-4-methyl-4H-[1,2,4]triazol-3-ylsulfanyl]-1-(4-nitro-phenyl)-ethanone;
N-{4-[5-(2-Chloro-benzylsulfanyl)-4-methyl-4H-[1,2,4]triazol-3-yl]-phenyl}-2-methoxy-benzamide;
2-(4-Allyl-5-pyridin-4-yl-4H-[1,2,4]triazol-3-ylsulfanyl)-1-(4-fluoro-phenyl)-ethanone;
2-(4-Benzyl-5-pyridin-3-yl-4H-[1,2,4]triazol-3-ylsulfanyl)-N-(4-fluoro-phenyl)-acetamide;
2-[5-(4-Amino-phenyl)-4-methyl-4H-[1,2,4]triazol-3-ylsulfanyl]-1-(4-nitro-phenyl)-ethanone;
2-(4-Allyl-5-thiophen-2-yl-4H-[1,2,4]triazol-3-ylsulfanyl)-N-(4-chloro-phenyl)-acetamide;
(4-Benzyl-5-phenyl-4H-[1,2,4]triazol-3-ylsulfanyl)-acetic acid ethyl ester;
4-[5-(4-tert-Butyl-benzylsulfanyl)-4-methyl-4H-[1,2,4]triazol-3-yl]-pyridine;
2-{5-[(3-Chloro-phenylamino)-methyl]-4-ethyl-4H-[1,2,4]triazol-3-ylsulfanyl}-1-phenyl-ethanone;
{5-[(2-Chloro-phenylamino)-methyl]-4-ethyl-4H-[1,2,4]triazol-3-ylsulfanyl}-acetic acid isopropyl ester;
2-[4-Ethyl-5-(4-methoxy-phenoxymethyl)-4H-[1,2,4]triazol-3-ylsulfanyl]-1-phenyl-ethanone;
1-(4-Chloro-phenyl)-2-{5-[(2-chloro-phenylamino)-methyl]-4-ethyl-4H-[1,2,4]triazol-3-ylsulfanyl}-ethanone;
4-[5-(2-Chloro-6-fluoro-benzylsulfanyl)-4-methyl-4H-[1,2,4]triazol-3-yl]-pyridine;
3-Benzyl-5-benzylsulfanyl-4-ethyl-4H-[1,2,4]triazole;
3-Benzylsulfanyl-4-methyl-5-o-tolyl-4H-[1,2,4]triazole;
N-(4-Chloro-3-nitro-phenyl)-2-(4-methyl-5-o-tolyl-4H-[1,2,4]triazol-3-ylsulfanyl)-acetamide;
2-(4-Methyl-5-o-tolyl-4H-[1,2,4]triazol-3-ylsulfanyl)-1-p-tolyl-ethanone;
1-(4-Methoxy-phenyl)-2-(4-methyl-5-o-tolyl-4H-[1,2,4]triazol-3-ylsulfanyl)-ethanone;
[5-(2-Bromo-phenyl)-4-methyl-4H-[1,2,4]triazol-3-ylsulfanyl]-acetonitrile;
[5-(2-Hydroxy-phenyl)-4-phenethyl-4H-[1,2,4]triazol-3-ylsulfanyl]-acetonitrile;
1-[5-(2-Hydroxy-phenyl)-4-phenethyl-4H-[1,2,4]triazol-3-ylsulfanyl]-propan-2-one;
N-(2,3-Dichloro-phenyl)-2-[4-furan-2-ylmethyl-5-(2-methoxy-phenyl)-4H-[1,2,4]triazol-3-ylsulfanyl]-acetamide;
N-(3-Chloro-phenyl)-2-[4-furan-2-ylmethyl-5-(2-methoxy-phenyl)-4H-[1,2,4]triazol-3-ylsulfanyl]-acetamide;
N-(4-Chloro-phenyl)-2-[4-furan-2-ylmethyl-5-(2-methoxy-phenyl)-4H-[1,2,4]triazol-3-ylsulfanyl]-acetamide;
2-[5-(4-Methoxy-phenoxymethyl)-4-methyl-4H-[1,2,4]triazol-3-ylsulfanyl]-1-phenyl-ethanone;
2-{5-[(2-Chloro-phenylamino)-methyl]-4-ethyl-4H-[1,2,4]triazol-3-ylsulfanyl}-1-piperidin-1-yl-ethanone;
2-[5-(4-Methoxy-phenoxymethyl)-4-methyl-4H-[1,2,4]triazol-3-ylsulfanyl]-1-(4-methoxyphenyl)-ethanone;
N-(2,3-Dichloro-phenyl)-2-[4-furan-2-ylmethyl-5-(4-hydroxy-phenyl)-4H-[1,2,4]triazol-3-ylsulfanyl]-acetamide;
2-[4-Furan-2-ylmethyl-5-(4-hydroxy-phenyl)-4H-[1,2,4]triazol-3-ylsulfanyl]-1-(4-methoxyphenyl)-ethanone;
{5-[(2-Chloro-phenylamino)-methyl]-4-ethyl-4H-[1,2,4]triazol-3-ylsulfanyl}-acetic acid cyclohexyl ester;
2-(4-Phenethyl-5-pyridin-4-yl-4H-[1,2,4]triazol-3-ylsulfanyl)-N-thiazol-2-yl-acetamide;
2-(4-Ethyl-5-m-tolyl-4H-[1,2,4]triazol-3-ylsulfanyl)-N-(4-nitro-phenyl)-acetamide;
2-(4-Ethyl-5-phenyl-4H-[1,2,4]triazol-3-ylsulfanyl)-N-(2-methoxy-phenyl)-acetamide;
2-(4-Ethyl-5-phenyl-4H-[1,2,4]triazol-3-ylsulfanyl)-N-(2-trifluoromethyl-phenyl)-acetamide;
2-[4-Furan-2-ylmethyl-5-(2-methoxy-phenyl)-4H-[1,2,4]triazol-3-ylsulfanyl]-N-(2-methyl-4-nitro-phenyl)-acetamide;
N-(3-Chloro-2-methyl-phenyl)-2-[4-furan-2-ylmethyl-5-(2-methoxy-phenyl)-4H-[1,2,4]triazol-3-ylsulfanyl]-acetamide;
4-Allyl-5-m-tolyl-4H-[1,2,4]triazole-3-thiol;
4-(2-Methyl-allyl)-5-m-tolyl-4H-[1,2,4]triazole-3-thiol;
2-[4-Allyl-5-(3-bromo-phenyl)-4H-[1,2,4]triazol-3-ylsulfanyl]-N-(4-ethyl-phenyl)-acetamide;
4-Allyl-3-(3-bromo-phenyl)-5-(2-nitro-benzylsulfanyl)-4H-[1,2,4]triazole;
2-[4-Allyl-5-(3-bromo-phenyl)-4H-[1,2,4]triazol-3-ylsulfanyl]-1-biphenyl-4-yl-ethanone;
4-(2-Methyl-allyl)-5-(3-nitro-phenyl)-4H-[1,2,4]triazole-3-thiol;
4-Allyl-5-(2-carbazol-9-yl-ethyl)-4H-[1,2,4]triazole-3-thiol;
5-(4-Bromo-phenyl)-4-(2-methyl-allyl)-4H-[1,2,4]triazole-3-thiol;
4-(2-Methyl-allyl)-5-(3-methyl-2-nitro-phenyl)-4H-[1,2,4]triazole-3-thiol;
5-(4-Methoxy-phenyl)-4-(2-methyl-allyl)-4H-[1,2,4]triazole-3-thiol;
2-(4-Allyl-5-benzotriazol-1-ylmethyl-4H-[1,2,4]triazol-3-ylsulfanyl)-N-(4-bromo-phenyl)-acetamide;
2-(4-Allyl-5-benzotriazol-1-ylmethyl-4H-[1,2,4]triazol-3-ylsulfanyl)-N-pyridin-2-yl-acetamide;
{4-Allyl-5-[3-(1,3-dioxo-1H,3H-benzo[de]isoquinolin-2-yl)-propyl]-4H-[1,2,4]triazol-3-ylsulfanyl}-acetic acid ethyl ester; and
4-Allyl-5-(4-phenyl-piperazin-1-ylmethyl)-4H-[1,2,4]triazole-3-thiol; or
a salt thereof with a pharmaceutically acceptable acid or base, or any optical isomer or mixture of optical isomers, including a racemic mixture, or any tautomeric forms.

In another embodiment of the present invention said substituted 1,2,4-triazole, or a prodrug thereof, as a component of the combination therapy is selected from the group consisting of:
4-[2-(4-Ethyl-5-pyridin-4-yl-4*H*-[1,2,4]triazol-3-ylsulfanyl)ethyl]morpholine;
1-Benzothiazol-2-yl-2-(4-ethyl-5-phenyl-4*H*-[1,2,4]triazol-3-ylsulfanyl)-ethanone;
*N*-Cyclohexyl-2-(4-ethyl-5-phenyl-4*H*-[1,2,4]triazol-3-ylsulfanyl)-*N*-methyl-acetamide;
2-(4-Ethyl-5-phenyl-4*H*-[1,2,4]triazol-3-ylsulfanyl)-1-phenyl-ethanone;
2-(4-Ethyl-5-phenyl-4*H*-[1,2,4]triazol-3-ylsulfanyl)-1-(4-methoxy-phenyl)-ethanone;
2-(4-Ethyl-5-phenyl-4*H*-[1,2,4]triazol-3-ylsulfanyl)-1-pyridin-3-yl-ethanone;
2-(4-Ethyl-5-phenyl-4*H*-[1,2,4]triazol-3-ylsulfanyl)-1-(4-methanesulfonyl-phenyl)-ethanone;
2-(4-Phenethyl-5-phenyl-4*H*-[1,2,4]triazol-3-ylsulfanyl)-1-phenyl-ethanone;
1-(4-Methoxy-phenyl)-2-(4-phenethyl-5-phenyl-4*H*-[1,2,4]triazol-3-ylsulfanyl)-ethanone;
2-(4-Phenethyl-5-phenyl-4*H*-[1,2,4]triazol-3-ylsulfanyl)-1-pyridin-3-yl-ethanone;
1-(4-Methanesulfonyl-phenyl)-2-(4-phenethyl-5-phenyl-4H-[1,2,4]triazol-3-ylsulfanyl)-ethanone;
2-(4-Ethyl-5-pyridin-4-yl-4*H*-[1,2,4]triazol-3-ylsulfanyl)-1-phenyl-ethanone;
2-(4-Ethyl-5-pyridin-4-yl-4*H*-[1,2,4]triazol-3-ylsulfanyl)-1-(4-methoxy-phenyl)-ethanone;
2-(4-Ethyl-5-pyridin-4-yl-4*H*-[1,2,4]triazol-3-ylsulfanyl)-1-pyridin-3-yl-ethanone;
2-(4-Ethyl-5-pyridin-4-yl-4*H*-[1,2,4]triazol-3-ylsulfanyl)-1-(4-methanesulfonyl-phenyl)-ethanone;
2-(4-Phenethyl-5-pyridin-4-yl-4*H*-[1,2,4]triazol-3-ylsulfanyl)-1-phenyl-ethanone;
1-(4-Methoxy-phenyl)-2-(4-phenethyl-5-pyridin-4-yl-4*H*-[1,2,4]triazol-3-ylsulfanyl)-ethanone;
2-(4-Phenethyl-5-pyridin-4-yl-4*H*-[1,2,4]triazol-3-ylsulfanyl)-1-pyridin-3-yl-ethanone;
1-(4-Methanesulfonyl-phenyl)-2-(4-phenethyl-5-pyridin-4-yl-4*H*-[1,2,4]triazol-3-ylsulfanyl)-ethanone;
2-(4-Ethyl-5-furan-2-yl-4*H*-[1,2,4]thriazol-3-ylsulfanyl)-1-phenyl-ethanone;
2-(4-Ethyl-5-furan-2-yl-4*H*-[1,2,4]triazol-3-ylsulfanyl)-1-(4-methoxy-phenyl)-ethanone;
2-(4-Ethyl-5-furan-2-yl-4*H*-[1,2,4]triazol-3-ylsulfanyl)-1-pyridin-3-yl-ethanone;
2-(4-Ethyl-5-furan-2-yl-4*H*-[1,2,4]triazol-3-ylsulfanyl)-1-(4-methanesulfonyl-phenyl)-ethanone;
2-(5-Furan-2-yl-4-phenethyl-4*H*-[1,2,4]triazol-3-ylsulfanyl)-1-phenyl-ethanone;
2-(5-Furan-2-yl-4-phenethyl-4*H*-[1,2,4]triazol-3-ylsulfanyl)-1-(4-methoxy-phenyl)-ethanone;
2-(5-Furan-2-yl-4-phenethyl-4*H*-[1,2,4]triazol-3-ylsulfanyl)-1-pyridin-3-yl-ethanone;
2-(5-Furan-2-yl-4-phenethyl-4*H*-[1,2,4]triazol-3-ylsulfanyl)-1-(4-methanesulfonyl-phenyl)-ethanone;
1-Adamantan-1-yl-2-(4-methyl-4*H*-[1,2,4]triazol-3-ylsulfanyl)-ethanone;
3-[5-(2-Chloro-benzylsulfanyl)-4-ethyl-4*H*-[1,2,4]triazol-3-yl]-propan-1-ol;
4-[4-Ethyl-5-(3-hydroxy-propyl)-4*H*-[1,2,4]triazol-3-ylsulfanylmethyl]-benzonitrile;
3-[5-(6-Chloro-benzo[1,3]dioxol-5-ylmethylsulfanyl)-4-ethyl-4*H*-[1,2,4]triazol-3-yl]-propan-1-ol;
3-[5-(3,4-Dichloro-benzylsulfanyl)-4-ethyl-4*H*-[1,2,4]triazol-3-yl]-propan-1-ol;
3-(5-Cyclohexylmethylsulfanyl-4-ethyl-4*H*-[1,2,4]triazol-3-yl)-propan-1-ol;
3-[4-Ethyl-5-(4-trifluoromethyl-benzylsulfanyl)-4*H*-[1,2,4]triazol-3-yl]-propan-1-ol;
3-(5-Benzylsulfanyl-4-ethyl-4*H*-[1,2,4]triazol-3-yl)-propan-1-ol;
3-[4-Ethyl-5-(4-methanesulfonyl-benzylsulfanyl)-4*H*-[1,2,4]triazol-3-yl]-propan-1-ol;
3-[4-Ethyl-5-(3-nitro-benzylsulfanyl)-4*H*-[1,2,4]triazol-3-yl]-propan-1-ol;
3-[5-(2,6-Dichloro-benzylsulfanyl)-4-ethyl-4*H*-[1,2,4]triazol-3-yl]-propan-1-ol;
3-[4-Ethyl-5-(3-trifluoromethoxy-benzylsulfanyl)-4*H*-[1,2,4]triazol-3-yl]-propan-1-ol;
N-{4-[4-Ethyl-5-(3-hydroxy-propyl)-4*H*-[1,2,4]triazol-3-ylsulfanylmethyl]-phenyl}-acetamide;
3-[4-Ethyl-5-(5-methyl-2-nitro-benzylsulfanyl)-4*H*-[1,2,4]triazol-3-yl]-propan-1-ol;
3-[4-Ethyl-5-(2-nitro-benzylsulfanyl)-4*H*-[1,2,4]triazol-3-yl]-propan-1-ol;
4-[4-Ethyl-5-(4-hydroxy-phenyl)-4*H*-[1,2,4]triazol-3-ylsulfanylmethyl]-benzonitrile;
4-[5-(6-Chloro-benzo[1,3]dioxol-5-ylmethylsulfanyl)-4-ethyl-4*H*-[1,2,4]triazol-3-yl]-phenol;
4-[5-(4-Bromo-benzylsulfanyl)-4-ethyl-4*H*-[1,2,4]triazol-3-yl]-phenol;
4-[4-Ethyl-5-(4-trifluoromethyl-benzylsulfanyl)-4*H*-[1,2,4]triazol-3-yl]-phenol;
4-(5-Benzylsulfanyl-4-ethyl-4*H*-[1,2,4]triazol-3-yl)-phenol;
4-[4-Ethyl-5-(4-methanesulfonyl-benzylsulfanyl)-4*H*-[1,2,4]triazol-3-yl]-phenol;
4-[4-Ethyl-5-(3-nitro-benzylsulfanyl)-4*H*-[1,2,4]triazol-3-yl]-phenol;
4-[4-Ethyl-5-(4-hydroxy-phenyl)-4*H*-[1,2,4]triazol-3-ylsulfanylmethyl]-benzoic acid methyl etser;
4-[5-(4-Benzyloxy-benzylsulfanyl)-4-ethyl-4H-[1,2,4]triazol-3-yl]-phenol;
N-{4-[4-Ethyl-5-(4-hydroxy-phenyl)-4*H*-[1,2,4]triazol-3-ylsulfanylmethyl]-phenyl}-acetamide;
4-[4-Ethyl-5-(4-methoxy-benzylsulfanyl)-4*H*-[1,2,4]triazol-3-yl]-phenol;
4-[4-Ethyl-5-(5-methyl-2-nitro-benzylsulfanyl)-4*H*-[1,2,4]triazol-3-yl]-phenol;
4-[4-Ethyl-5-(2-nitro-benzylsulfanyl)-4*H*-[1,2,4]triazol-3-yl]-phenol;
4-[5-(2,4-Dichloro-benzylsulfanyl)-4-ethyl-4*H*-[1,2,4]triazol-3-yl]-phenol;
3-Benzo[1,3]dioxol-5-yl-5-(2-chloro-benzylsulfanyl)-4-ethyl-4H-[1,2,4]triazole;
4-(5-Benzo[1,3]dioxol-5-yl-4-ethyl-4*H*-[1,2,4]triazol-3-ylsulfanylmethyl)-benzonitrile;
3-Benzo[1,3]dioxol-5-yl-5-(6-chloro-benzo[1,3]dioxol-5-ylmethylsulfanyl)-4-ethyl-4H-[1,2,4]triazole;
3-Benzo[1,3]dioxol-5-yl-5-(4-bromo-benzylsulfanyl)-4-ethyl-4*H*-[1,2,4]triazole;
4-[4-(5-Benzo[1,3]dioxol-5-yl-4-ethyl-4*H*-[1,2,4]triazol-3-ylsulfanylmethyl)-phenyl]-[1,2,3]thiadiazole;
3-Benzo[1,3]dioxol-5-yl-5-cyclohexylmethylsulfanyl-4-ethyl-4*H*-[1,2,4]triazole;
3-Benzo[1,3]dioxol-5-yl-4-ethyl-5-(4-trifluoromethoxy-benzylsulfanyl)-4*H*-[1,2,4]triazole;
3-Benzo[1,3]dioxol-5-yl-5-(3,5-dimethoxy-benzylsulfanyl)-4-ethyl-4*H*-[1,2,4]triazole;
3-Benzo[1,3]dioxol-5-yl-4-ethyl-5-(4-methanesulfonyl-benzylsulfanyl)-4*H*-[1,2,4]triazole;
4-(5-Benzo[1,3]dioxol-5-yl-4-ethyl-4*H*-[1,2,4]triazol-3-ylsulfanylmethyl)-benzoic acid methyl etser;
3-Benzo[1,3]dioxol-5-yl-5-(2,6-dichloro-benzylsulfanyl)-4-ethyl-4*H*-[1,2,4]triazole;
3-Benzo[1,3]dioxol-5-yl-5-(4-benzyloxy-benzylsulfanyl)-4-ethyl-4*H*-[1,2,4]triazole;
N-[4-(5-Benzo[1,3]dioxol-5-yl-4-ethyl-4*H*-[1,2,4]triazol-3-ylsulfanylmethyl)-phenyl]-acetamide;
3-Benzo[1,3]dioxol-5-yl-4-ethyl-5-(4-methoxy-benzylsulfanyl)-4*H*-[1,2,4]triazole;
3-Benzo[1,3]dioxol-5-yl-4-ethyl-5-(5-methyl-2-nitro-benzylsulfanyl)-4*H*-[1,2,4]triazole;
3-Benzo[1,3]dioxol-5-yl-4-ethyl-5-(2-nitro-benzylsulfanyl)-4*H*-[1,2,4]triazole;
3-Benzo[1,3]dioxol-5-yl-5-(2,4-dichloro-benzylsulfanyl)-4-ethyl-4*H*-[1,2,4]triazole;
4-[4-Ethyl-5-(2-phenoxy-phenyl)-4*H*-[1,2,4]triazol-3-ylsulfanylmethyl]-benzonitrile;
3-(6-Chloro-benzo[1,3]dioxol-5-ylmethylsulfanyl)-4-ethyl-5-(2-phenoxy-phenyl)-4*H-*[1,2,4]triazole;
3-(3,4-Dichloro-benzylsulfanyl)-4-ethyl-5-(2-phenoxy-phenyl)-4*H*-[1,2,4]triazole;
3-(4-Bromo-benzylsulfanyl)-4-ethyl-5-(2-phenoxy-phenyl)-4*H*-[1,2,4]triazole;
4-{4-[4-Ethyl-5-(2-phenoxy-phenyl)-4*H*-[1,2,4]triazol-3-ylsulfanylmethyl]-phenyl}-[1,2,3]thiadiazole;
3-(3,5-Dimethoxy-benzylsulfanyl)-4-ethyl-5-(2-phenoxy-phenyl)-4*H*-[1,2,4]triazole;
4-Ethyl-3-(2-phenoxy-phenyl)-5-(4-trifluoromethyl-benzylsulfanyl)-4*H*-[1,2,4]triazole;
4-Ethyl-3-(4-methanesulfonyl-benzylsulfanyl)-5-(2-phenoxy-phenyl)-4*H*-[1,2,4]triazole;
4-[4-Ethyl-5-(2-phenoxy-phenyl)-4*H*-[1,2,4]triazol-3-ylsulfanylmethyl]-benzoic acid methyl etser;
3-(2,6-Dichloro-benzylsulfanyl)-4-ethyl-5-(2-phenoxy-phenyl)-4*H*-[1,2,4]triazole;
3-(4-Benzyloxy-benzylsulfanyl)-4-ethyl-5-(2-phenoxy-phenyl)-4*H*-[1,2,4]triazole;
4-Ethyl-3-(4-methyl-benzylsulfanyl)-5-(2-phenoxy-phenyl)-4*H*-[1,2,4]triazole;
4-Ethyl-3-(2-phenoxy-phenyl)-5-(3-trifluoromethoxy-benzylsulfanyl)-4*H*-[1,2,4]triazole;
4-Ethyl-3-(4-methoxy-benzylsulfanyl)-5-(2-phenoxy-phenyl)-4*H*-[1,2,4]triazole;
4-Ethyl-3-(2-nitro-benzylsulfanyl)-5-(2-phenoxy-phenyl)-4*H*-[1,2,4]triazole;
3-(2,4-Dichloro-benzylsulfanyl)-4-ethyl-5-(2-phenoxy-phenyl)-4*H*-[1,2,4]triazole;
3-Cyclohexylmethylsulfanyl-4-ethyl-5-naphthalen-1-yl-4*H*-[1,2,4]triazole;
4-Ethyl-3-naphthalen-1-yl-5-(4-trifluoromethyl-benzylsulfanyl)-4*H*-[1,2,4]triazole;
4-Ethyl-3-(4-methanesulfonyl-benzylsulfanyl)-5-naphthalen-1-yl-4*H*-[1,2,4]triazole;
4-Ethyl-3-naphthalen-1-yl-5-(3-nitro-benzylsulfanyl)-4*H*-[1,2,4]triazole;
4-(4-Ethyl-5-naphthalen-1-yl-4*H*-[1,2,4]triazol-3-ylsulfanylmethyl)-benzoic acid methyl etser;
4-Ethyl-3-naphthalen-1-yl-5-(4-nitro-benzylsulfanyl)-4*H*-[1,2,4]triazole;
3-(2,6-Dichloro-benzylsulfanyl)-4-ethyl-5-naphthalen-1-yl-4*H*-[1,2,4]triazole;
4-Ethyl-3-(4-methyl-benzylsulfanyl)-5-naphthalen-1-yl-4*H*-[1,2,4]triazole;
4-Ethyl-3-(4-methoxy-benzylsulfanyl)-5-naphthalen-1-yl-4*H*-[1,2,4]triazole;
4-Ethyl-3-(5-methyl-2-nitro-benzylsulfanyl)-5-naphthalen-1-yl-4*H*-[1,2,4]triazole;
3-(2,4-Dichloro-benzylsulfanyl)-4-ethyl-5-naphthalen-1-yl-4*H*-[1,2,4]triazole;
[4-(5-Cyclohexylmethylsulfanyl-4-ethyl-4*H*-[1,2,4]triazol-3-yl)-phenyl]-dimethyl-amine
{4-[5-(3,5-Dimethoxy-benzylsulfanyl)-4-ethyl-4*H*-[1,2,4]triazol-3-yl]-phenyl}-dimethyl-amine
{4-[4-Ethyl-5-(4-trifluoromethyl-benzylsulfanyl)-4*H*-[1,2,4]triazol-3-yl]-phenyl}-dimethyl-amine
4-[5-(4-Dimethylamino-phenyl)-4-ethyl-4H-[1,2,4]triazol-3-ylsulfanylmethyl]-benzoic acid methyl etser;
{4-[4-Ethyl-5-(4-nitro-benzylsulfanyl)-4*H*-[1,2,4]triazol-3-yl]-phenyl}-dimethyl-amine;
{4-[4-Ethyl-5-(4-methoxy-benzylsulfanyl)-4*H*-[1,2,4]triazol-3-yl]-phenyl}-dimethyl-amine;
{4-[4-Ethyl-5-(5-methyl-2-nitro-benzylsulfanyl)-4*H*-[1,2,4]triazol-3-yl]-phenyl}-dimethyl-amine;
{4-[5-(2,4-Dichloro-benzylsulfanyl)-4-ethyl-4*H*-[1,2,4]triazol-3-yl]-phenyl}-dimethyl-amine;
4-(4-Ethyl-5-thiophen-2-yl-4*H*-[1,2,4]triazol-3-ylsulfanylmethyl)-benzonitrile;
3-(6-Chloro-benzo[1,3]dioxol-5-ylmethylsulfanyl)-4-ethyl-5-thiophen-2-yl-4*H*-[1,2,4]triazole;
3-(3,4-Dichloro-benzylsulfanyl)-4-ethyl-5-thiophen-2-yl-4*H*-[1,2,4]triazole;
3-(4-Bromo-benzylsulfanyl)-4-ethyl-5-thiophen-2-yl-4*H*-[1,2,4]triazole;
4-[4-(4-Ethyl-5-thiophen-2-yl-4*H*-[1,2,4]triazol-3-ylsulfanylmethyl)-phenyl]-[1,2,3]thiadiazole;
4-Ethyl-3-thiophen-2-yl-5-(4-trifluoromethoxy-benzylsulfanyl)-4*H*-[1,2,4]triazole;
3-(3,5-Dimethoxy-benzylsulfanyl)-4-ethyl-5-thiophen-2-yl-4*H*-[1,2,4]triazole;
4-Ethyl-3-thiophen-2-yl-5-(4-trifluoromethyl-benzylsulfanyl)-4*H*-[1,2,4]triazole;
3-Benzylsulfanyl-4-ethyl-5-thiophen-2-yl-4*H*-[1,2,4]triazole;
4-Ethyl-3-(4-methanesulfonyl-benzylsulfanyl)-5-thiophen-2-yl-4*H*-[1,2,4]triazole;
4-(4-Ethyl-5-thiophen-2-yl-4*H*-[1,2,4]triazol-3-ylsulfanylmethyl)-benzoic acid methyl etser;
4-Ethyl-3-(4-nitro-benzylsulfanyl)-5-thiophen-2-yl-4*H*-[1,2,4]triazole;
3-(2,6-Dichloro-benzylsulfanyl)-4-ethyl-5-thiophen-2-yl-4*H*-[1,2,4]triazole;
4-Ethyl-3-(4-methyl-benzylsulfanyl)-5-thiophen-2-yl-4*H*-[1,2,4]triazole;
4-Ethyl-3-(4-methoxy-benzylsulfanyl)-5-thiophen-2-yl-4*H*-[1,2,4]triazole;
4-Ethyl-3-(5-methyl-2-nitro-benzylsulfanyl)-5-thiophen-2-yl-4*H*-[1,2,4]triazole;
3-(2-Chloro-benzylsulfanyl)-4-ethyl-5-(2-methoxy-phenyl)-4*H*-[1,2,4]triazole;
3-(6-Chloro-benzo[1,3]dioxol-5-ylmethylsulfanyl)-4-ethyl-5-(2-methoxy-phenyl)-4*H-*[1,2,4]triazole;
4-Ethyl-3-(2-methoxy-phenyl)-5-(4-trifluoromethoxy-benzylsulfanyl)-4*H*-[1,2,4]triazole;
3-(3,5-Dimethoxy-benzylsulfanyl)-4-ethyl-5-(2-methoxy-phenyl)-4*H*-[1,2,4]triazole;
4-Ethyl-3-(2-methoxy-phenyl)-5-(4-trifluoromethyl-benzylsulfanyl)-4*H*-[1,2,4]triazole;
3-Benzylsulfanyl-4-ethyl-5-(2-methoxy-phenyl)-4*H*-[1,2,4]triazole;
4-Ethyl-3-(4-methanesulfonyl-benzylsulfanyl)-5-(2-methoxy-phenyl)-4*H*-[1,2,4]triazole;
4-[4-Ethyl-5-(2-methoxy-phenyl)-4*H*-[1,2,4]triazol-3-ylsulfanylmethyl]-benzoic acid methyl etser;
4-Ethyl-3-(2-methoxy-phenyl)-5-(4-nitro-benzylsulfanyl)-4*H*-[1,2,4]triazole;
3-(2,6-Dichloro-benzylsulfanyl)-4-ethyl-5-(2-methoxy-phenyl)-4*H*-[1,2,4]triazole;
3-(4-Benzyloxy-benzylsulfanyl)-4-ethyl-5-(2-methoxy-phenyl)-4*H*-[1,2,4]triazole;
4-Ethyl-3-(2-methoxy-phenyl)-5-(4-methyl-benzylsulfanyl)-4*H*-[1,2,4]triazole;
4-Ethyl-3-(2-methoxy-phenyl)-5-(3-trifluoromethoxy-benzylsulfanyl)-4*H*-[1,2,4]triazole;
4-Ethyl-3-(2-methoxy-phenyl)-5-(5-methyl-2-nitro-benzylsulfanyl)-4*H*-[1,2,4]triazole;
4-Ethyl-3-(2-methoxy-phenyl)-5-(2-nitro-benzylsulfanyl)-4*H*-[1,2,4]triazole;
3-(2-Chloro-benzylsulfanyl)-4-ethyl-5-(4-methoxy-phenyl)-4*H*-[1,2,4]triazole;
4-[4-Ethyl-5-(4-methoxy-phenyl)-4H-[1,2,4]triazol-3-ylsulfanylmethyl]-benzonitrile;
3-(6-Chloro-benzo[1,3]dioxol-5-ylmethylsulfanyl)-4-ethyl-5-(4-methoxy-phenyl)-4*H-*[1,2,4]triazole;
3-(3,4-Dichloro-benzylsulfanyl)-4-ethyl-5-(4-methoxy-phenyl)-4*H*-[1,2,4]triazole;
3-(4-Bromo-benzylsulfanyl)-4-ethyl-5-(4-methoxy-phenyl)-4*H*-[1,2,4]triazole;
4-{4-[4-Ethyl-5-(4-methoxy-phenyl)-4*H*-[1,2,4]triazol-3-ylsulfanylmethyl]-phenyl}-[1,2,3]thiadiazole;
3-Cyclohexylmethylsulfanyl-4-ethyl-5-(4-methoxy-phenyl)-4*H*-[1,2,4]triazole;
4-Ethyl-3-(4-methoxy-phenyl)-5-(4-trifluoromethyl-benzylsulfanyl)-4*H*-[1,2,4]triazole;
4-Ethyl-3-(4-methanesulfonyl-benzylsulfanyl)-5-(4-methoxy-phenyl)-4*H*-[1,2,4]triazole;
4-[4-Ethyl-5-(4-methoxy-phenyl)-4*H*-[1,2,4]triazol-3-ylsulfanylmethyl]-benzoic acid methyl etser;
3-(2,6-Dichloro-benzylsulfanyl)-4-ethyl-5-(4-methoxy-phenyl)-4*H*-[1,2,4]triazole;
3-(4-Benzyloxy-benzylsulfanyl)-4-ethyl-5-(4-methoxy-phenyl)-4*H*-[1,2,4]triazole;
4-Ethyl-3-(4-methoxy-phenyl)-5-(3-trifluoromethoxy-benzylsulfanyl)-4*H*-[1,2,4]triazole;
N-{4-[4-Ethyl-5-(4-methoxy-phenyl)-4*H*-[1,2,4]triazol-3-ylsulfanylmethyl]-phenyl}-acetamide;
4-Ethyl-3-(4-methoxy-benzylsulfanyl)-5-(4-methoxy-phenyl)-4*H*-[1,2,4]triazole;
4-Ethyl-3-(4-methoxy-phenyl)-5-(2-nitro-benzylsulfanyl)-4*H*-[1,2,4]triazole;
4-(4-Ethyl-5-pyridin-4-yl-4*H*-[1,2,4]triazol-3-ylsulfanylmethyl)-benzonitrile;
4-[5-(4-Bromo-benzylsulfanyl)-4-ethyl-4*H*-[1,2,4]triazol-3-yl]-pyridine;
4-[5-(3,5-Dimethoxy-benzylsulfanyl)-4-ethyl-4*H*-[1,2,4]triazol-3-yl]-pyridine;
4-[4-Ethyl-5-(4-methanesulfonyl-benzylsulfanyl)-4*H*-[1,2,4]triazol-3-yl]-pyridine;
4-(4-Ethyl-5-pyridin-4-yl-4H-[1,2,4]triazol-3-ylsulfanylmethyl)-benzoic acid methyl etser;
4-[4-Ethyl-5-(4-methyl-benzylsulfanyl)-4*H*-[1,2,4]triazol-3-yl]-pyridine;
4-[4-Ethyl-5-(3-trifluoromethoxy-benzylsulfanyl)-4*H*-[1,2,4]triazol-3-yl]-pyridine;
4-[5-(2,4-Dichloro-benzylsulfanyl)-4-ethyl-4*H*-[1,2,4]triazol-3-yl]-pyridine;
3-[5-(2-Chloro-benzylsulfanyl)-4-ethyl-4*H*-[1,2,4]triazol-3-yl]-pyridine;
4-(4-Ethyl-5-pyridin-3-yl-4*H*-[1,2,4]triazol-3-ylsulfanylmethyl)-benzonitrile;
3-[5-(6-Chloro-benzo[1,3]dioxol-5-ylmethylsulfanyl)-4-ethyl-4*H*-[1,2,4]triazol-3-yl]-pyridine;
3-[5-(3,4-Dichloro-benzylsulfanyl)-4-ethyl-4*H*-[1,2,4]triazol-3-yl]-pyridine;
3-[5-(4-Bromo-benzylsulfanyl)-4-ethyl-4*H*-[1,2,4]triazol-3-yl]-pyridine;
3-[4-Ethyl-5-(4-[1,2,3]thiadiazol-4-yl-benzylsulfanyl)-4*H*-[1,2,4]triazol-3-yl]-pyridine;
3-[4-Ethyl-5-(4-trifluoromethoxy-benzylsulfanyl)-4*H*-[1,2,4]triazol-3-yl]-pyridine;
3-[5-(3,5-Dimethoxy-benzylsulfanyl)-4-ethyl-4*H*-[1,2,4]triazol-3-yl]-pyridine;
3-[4-Ethyl-5-(4-trifluoromethyl-benzylsulfanyl)-4*H*-[1,2,4]triazol-3-yl]-pyridine;
3-(5-Benzylsulfanyl-4-ethyl-4*H*-[1,2,4]triazol-3-yl)-pyridine;
3-[4-Ethyl-5-(4-methanesulfonyl-benzylsulfanyl)-4*H*-[1,2,4]triazol-3-yl]-pyridine;
3-[4-Ethyl-5-(3-nitro-benzylsulfanyl)-4*H*-[1,2,4]triazol-3-yl]-pyridine;
4-(4-Ethyl-5-pyridin-3-yl-4*H*-[1,2,4]triazol-3-ylsulfanylmethyl)-benzoic acid methyl etser;
3-[5-(2,6-Dichloro-benzylsulfanyl)-4-ethyl-4*H*-[1,2,4]triazol-3-yl]-pyridine;
3-[5-(4-Benzyloxy-benzylsulfanyl)-4-ethyl-4*H*-[1,2,4]triazol-3-yl]-pyridine;
3-[4-Ethyl-5-(4-methyl-benzylsulfanyl)-4*H*-[1,2,4]triazol-3-yl]-pyridine;
3-[4-Ethyl-5-(3-trifluoromethoxy-benzylsulfanyl)-4*H*-[1,2,4]triazol-3-yl]-pyridine;
N-[4-(4-Ethyl-5-pyridin-3-yl-4*H*-[1,2,4]triazol-3-ylsulfanylmethyl)-phenyl]-acetamide;
3-[4-Ethyl-5-(4-methoxy-benzylsulfanyl)-4*H*-[1,2,4]triazol-3-yl]-pyridine;
3-[4-Ethyl-5-(5-methyl-2-nitro-benzylsulfanyl)-4*H*-[1,2,4]triazol-3-yl]-pyridine;
3-[4-Ethyl-5-(2-nitro-benzylsulfanyl)-4*H*-[1,2,4]triazol-3-yl]-pyridine;
3-[5-(2,4-Dichloro-benzylsulfanyl)-4-ethyl-4*H*-[1,2,4]triazol-3-yl]-pyridine;
3-(2-Chloro-benzylsulfanyl)-5-(4-chloro-phenyl)-4-ethyl-4*H*-[1,2,4]triazole;
4-[5-(4-Chloro-phenyl)-4-ethyl-4*H*-[1,2,4]triazol-3-ylsulfanylmethyl]-benzonitrile;
3-(4-Chloro-phenyl)-5-(3,4-dichloro-benzylsulfanyl)-4-ethyl-4*H*-[1,2,4]triazole;
4-{4-[5-(4-Chloro-phenyl)-4-ethyl-4*H*-[1,2,4]triazol-3-ylsulfanylmethyl]-phenyl}-[1,2,3]thiadiazole;
3-(4-Chloro-phenyl)-4-ethyl-5-(4-methanesulfonyl-benzylsulfanyl)-4*H*-[1,2,4]triazole;
3-(4-Chloro-phenyl)-4-ethyl-5-(3-nitro-benzylsulfanyl)-4*H*-[1,2,4]triazole;
4-[5-(4-Chloro-phenyl)-4-ethyl-4*H*-[1,2,4]triazol-3-ylsulfanylmethyl]-benzoic acid methyl etser;
3-(4-Chloro-phenyl)-5-(2,6-dichloro-benzylsulfanyl)-4-ethyl-4*H*-[1,2,4]triazole;
3-(4-Benzyloxy-benzylsulfanyl)-5-(4-chloro-phenyl)-4-ethyl-4*H*-[1,2,4]triazole;
3-(4-Chloro-phenyl)-4-ethyl-5-(4-methyl-benzylsulfanyl)-4*H*-[1,2,4]triazole;
3-(4-Chloro-phenyl)-4-ethyl-5-(3-trifluoromethoxy-benzylsulfanyl)-4*H*-[1,2,4]triazole;
N-{4-[5-(4-Chloro-phenyl)-4-ethyl-4*H*-[1,2,4]triazol-3-ylsulfanylmethyl]-phenyl}-acetamide;
3-(4-Chloro-phenyl)-4-ethyl-5-(5-methyl-2-nitro-benzylsulfanyl)-4*H*-[1,2,4]triazole;
3-(4-Chloro-phenyl)-5-(2,4-dichloro-benzylsulfanyl)-4-ethyl-4*H*-[1,2,4]triazole;
5-[5-(2-Chloro-benzylsulfanyl)-4-ethyl-4*H*-[1,2,4]triazol-3-yl]-4-phenyl-[1,2,3]thiadiazole;
4-[4-Ethyl-5-(4-phenyl-[1,2,3]thiadiazol-5-yl)-4*H*-[1,2,4]triazol-3-ylsulfanylmethyl]-benzonitrile;
5-[5-(3,4-Dichloro-benzylsulfanyl)-4-ethyl-4*H*-[1,2,4]triazol-3-yl]-4-phenyl-[1,2,3]thiadiazole;
5-[4-Ethyl-5-(4-trifluoromethoxy-benzylsulfanyl)-4*H*-[1,2,4]triazol-3-yl]-4-phenyl-[1,2,3]thiadiazole;
5-[5-(3,5-Dimethoxy-benzylsulfanyl)-4-ethyl-4*H*-[1,2,4]triazol-3-yl]-4-phenyl-[1,2,3]thiadiazole;
5-[4-Ethyl-5-(4-trifluoromethyl-benzylsulfanyl)-4*H*-[1,2,4]triazol-3-yl]-4-phenyl-[1,2,3]thiadiazole;
5-(5-Benzylsulfanyl-4-ethyl-4*H*-[1,2,4]triazol-3-yl)-4-phenyl-[1,2,3]thiadiazole;
5-[4-Ethyl-5-(4-methanesulfonyl-benzylsulfanyl)-4*H*-[1,2,4]triazol-3-yl]-4-phenyl-[1,2,3]thiadiazole;
5-[4-Ethyl-5-(4-nitro-benzylsulfanyl)-4*H*-[1,2,4]triazol-3-yl]-4-phenyl-[1,2,3]thiadiazole;
5-[5-(2,6-Dichloro-benzylsulfanyl)-4-ethyl-4*H*-[1,2,4]triazol-3-yl]-4-phenyl-[1,2,3]thiadiazole;
5-[4-Ethyl-5-(3-trifluoromethoxy-benzylsulfanyl)-4H-[1,2,4]triazol-3-yl]-4-phenyl-[1,2,3]thiadiazole;
5-[4-Ethyl-5-(4-methoxy-benzylsulfanyl)-4*H*-[1,2,4]triazol-3-yl]-4-phenyl-[1,2,3]thiadiazole;
5-[4-Ethyl-5-(2-nitro-benzylsulfanyl)-4*H*-[1,2,4]triazol-3-yl]-4-phenyl-[1,2,3]thiadiazole;
4-{5-[4-(2,5-Dimethyl-pyrrol-1-yl)-phenyl]-4-ethyl-4*H*-[1,2,4]triazol-3-ylsulfanylmethyl}-benzonitrile;
3-(6-Chloro-benzo[1,3]dioxol-5-ylmethylsulfanyl)-5-[4-(2,5-dimethyl-pyrrol-1-yl)-phenyl]-4-ethyl-4H-[1,2,4]triazole;
3-(3,4-Dichloro-benzylsulfanyl)-5-[4-(2,5-dimethyl-pyrrol-1-yl)-phenyl]-4-ethyl-4*H-*[1,2,4]triazole;
3-[4-(2,5-Dimethyl-pyrrol-1-yl)-phenyl]-4-ethyl-5-(4-trifluoromethoxy-benzylsulfanyl)-4*H-*[1,2,4]triazole;
3-(3,5-Dimethoxy-benzylsulfanyl)-5-[4-(2,5-dimethyl-pyrrol-1-yl)-phenyl]-4-ethyl-4*H-*[1,2,4]triazole;
3-[4-(2,5-Dimethyl-pyrrol-1-yl)-phenyl]-4-ethyl-5-(4-trifluoromethyl-benzylsulfanyl)-4*H-*[1,2,4]triazole;
3-Benzylsulfanyl-5-[4-(2,5-dimethyl-pyrrol-1-yl)-phenyl]-4-ethyl-4*H*-[1,2,4]triazole;
3-[4-(2,5-Dimethyl-pyrrol-1-yl)-phenyl]-4-ethyl-5-(4-methanesulfonyl-benzylsulfanyl)-4*H-*[1,2,4]triazole;
3-[4-(2,5-Dimethyl-pyrrol-1-yl)-phenyl]-4-ethyl-5-(3-nitro-benzylsulfanyl)-4*H*-[1,2,4]triazole;
4-{5-[4-(2,5-Dimethyl-pyrrol-1-yl)-phenyl]-4-ethyl-4*H*-[1,2,4]triazol-3-ylsulfanylmethyl}-benzoic acid methyl etser;
3-[4-(2,5-Dimethyl-pyrrol-1-yl)-phenyl]-4-ethyl-5-(4-nitro-benzylsulfanyl)-4*H*-[1,2,4]triazole;
3-(4-Benzyloxy-benzylsulfanyl)-5-[4-(2,5-dimethyl-pyrrol-1-yl)-phenyl]-4-ethyl-4*H-*[1,2,4]triazole;
N-(4-{5-[4-(2,5-Dimethyl-pyrrol-1-yl)-phenyl]-4-ethyl-4*H*-[1,2,4]triazol-3-ylsulfanylmethyl}-phenyl)-acetamide;
3-[4-(2,5-Dimethyl-pyrrol-1-yl)-phenyl]-4-ethyl-5-(4-methoxy-benzylsulfanyl)-4*H-*[1,2,4]triazole;
3-[4-(2,5-Dimethyl-pyrrol-1-yl)-phenyl]-4-ethyl-5-(5-methyl-2-nitro-benzylsulfanyl)-4*H-*[1,2,4]triazole;
3-[4-(2,5-Dimethyl-pyrrol-1-yl)-phenyl]-4-ethyl-5-(2-nitro-benzylsulfanyl)-4*H*-[1,2,4]triazole;
3-(2,4-Dichloro-benzylsulfanyl)-5-[4-(2,5-dimethyl-pyrrol-1-yl)-phenyl]-4-ethyl-4*H-*[1,2,4]triazole;
3-Benzo[b]thiophen-2-yl-5-(2-chloro-benzylsulfanyl)-4-ethyl-4*H*-[1,2,4]triazole;
4-(5-Benzo[b]thiophen-2-yl-4-ethyl-4*H*-[1,2,4]triazol-3-ylsulfanylmethyl)-benzonitrile;
3-Benzo[b]thiophen-2-yl-5-(6-chloro-benzo[1,3]dioxol-5-ylmethylsulfanyl)-4-ethyl-4*H-*[1,2,4]triazole;
3-Benzo[b]thiophen-2-yl-5-(4-bromo-benzylsulfanyl)-4-ethyl-4*H*-[1,2,4]triazole;
4-[4-(5-Benzo[b]thiophen-2-yl-4-ethyl-4*H*-[1,2,4]triazol-3-ylsulfanylmethyl)-phenyl]-[1,2,3]thiadiazole;
3-Benzo[b]thiophen-2-yl-4-ethyl-5-(3-nitro-benzylsulfanyl)-4H-[1,2,4]triazole;
4-(5-Benzo[b]thiophen-2-yl-4-ethyl-4*H*-[1,2,4]triazol-3-ylsulfanylmethyl)-benzoic acid methyl etser;
3-Benzo[b]thiophen-2-yl-4-ethyl-5-(4-nitro-benzylsulfanyl)-4*H*-[1,2,4]triazole;
3-Benzo[b]thiophen-2-yl-5-(4-benzyloxy-benzylsulfanyl)-4-ethyl-4*H*-[1,2,4]triazole;
3-Benzo[b]thiophen-2-yl-4-ethyl-5-(3-trifluoromethoxy-benzylsulfanyl)-4*H*-[1,2,4]triazole;
N-[4-(5-Benzo[b]thiophen-2-yl-4-ethyl-4*H*-[1,2,4]triazol-3-ylsulfanylmethyl)-phenyl]-acetamide;
3-Benzo[b]thiophen-2-yl-4-ethyl-5-(4-methoxy-benzylsulfanyl)-4H-[1,2,4]triazole;
3-Benzo[b]thiophen-2-yl-4-ethyl-5-(2-nitro-benzylsulfanyl)-4*H*-[1,2,4]triazole;
3-(2-Chloro-benzylsulfanyl)-4-ethyl-5-(1-phenyl-5-trifluoromethyl-1*H*-pyrazol-4-yl)-4*H-*[1,2,4]triazole;
3-(6-Chloro-benzo[1,3]dioxol-5-ylmethylsulfanyl)-4-ethyl-5-(1-phenyl-5-trifluoromethyl-1*H-*pyrazol-4-yl)-4*H*-[1,2,4]triazole;
4-{4-[4-Ethyl-5-(1-phenyl-5-trifluoromethyl-1*H*-pyrazol-4-yl)-4*H*-[1,2,4]triazol-3-ylsulfanylmethyl]-phenyl}-[1,2,3]thiadiazole;
4-Ethyl-3-(1-phenyl-5-trifluoromethyl-1*H*-pyrazol-4-yl)-5-(4-trifluoromethoxy-benzylsulfanyl)-4*H*-[1,2,4]triazole;
3-(3,5-Dimethoxy-benzylsulfanyl)-4-ethyl-5-(1-phenyl-5-trifluoromethyl-1 *H*-pyrazol-4-yl)-4*H-*[1,2,4]triazole;
3-Benzylsulfanyl-4-ethyl-5-(1-phenyl-5-trifluoromethyl-1*H*-pyrazol-4-yl)-4*H*-[1,2,4]triazole;
4-Ethyl-3-(4-methanesulfonyl-benzylsulfanyl)-5-(1-phenyl-5-trifluoromethyl-1*H*-pyrazol-4-yl)-4*H*-[1,2,4]triazole;
4-Ethyl-3-(3-nitro-benzylsulfanyl)-5-(1-phenyl-5-trifluoromethyl-1 *H*-pyrazol-4-yl)-4*H-*[1,2,4]triazole;
4-[4-Ethyl-5-(1-phenyl-5-trifluoromethyl-1*H*-pyrazol-4-yl)-4*H*-[1,2,4]triazol-3-ylsulfanylmethyl]-benzoic acid methyl etser;
3-(2,6-Dichloro-benzylsulfanyl)-4-ethyl-5-(1-phenyl-5-trifluoromethyl-1*H*-pyrazol-4-yl)-4*H-*[1,2,4]triazole;
3-(4-Benzyloxy-benzylsulfanyl)-4-ethyl-5-(1-phenyl-5-trifluoromethyl-1*H*-pyrazol-4-yl)-4*H-*[1,2,4]triazole;
4-Ethyl-3-(4-methyl-benzylsulfanyl)-5-(1-phenyl-5-trifluoromethyl-1*H*-pyrazol-4-yl)-4*H-*[1,2,4]triazole;
N-{4-[4-Ethyl-5-(1-phenyl-5-trifluoromethyl-1*H*-pyrazol-4-yl)-4*H*-[1,2,4]triazol-3-ylsulfanylmethyl]-phenyl}-acetamide;
4-Ethyl-3-(5-methyl-2-nitro-benzylsulfanyl)-5-(1-phenyl-5-trifluoromethyl-1*H*-pyrazol-4-yl)-4*H-*[1,2,4]triazole;
4-Ethyl-3-(2-nitro-benzylsulfanyl)-5-(1-phenyl-5-trifluoromethyl-1*H*-pyrazol-4-yl)-4H-[1,2,4]triazole;
3-(2,4-Dichloro-benzylsulfanyl)-4-ethyl-5-(1-phenyl-5-trifluoromethyl-1*H*-pyrazol-4-yl)-4*H-*[1,2,4]triazole;
3-(2-Chloro-benzylsulfanyl)-5-(3-chloro-4-methyl-thiophen-2-yl)-4-ethyl-4*H*-[1,2,4]triazole;
4-[5-(3-Chloro-4-methyl-thiophen-2-yl)-4-ethyl-4*H*-[1,2,4]triazol-3-ylsulfanylmethyl]-benzonitrile;
3-(3-Chloro-4-methyl-thiophen-2-yl)-5-(3,4-dichloro-benzylsulfanyl)-4-ethyl-4*H*-[1,2,4]triazole;
4-{4-[5-(3-Chloro-4-methyl-thiophen-2-yl)-4-ethyl-4*H*-[1,2,4]triazol-3-ylsulfanylmethyl]-phenyl}-[1,2,3]thiadiazole;
3-(3-Chloro-4-methyl-thiophen-2-yl)-5-cyclohexylmethylsulfanyl-4-ethyl-4*H*-[1,2,4]triazole;
3-(3-Chloro-4-methyl-thiophen-2-yl)-4-ethyl-5-(4-trifluoromethoxy-benzylsulfanyl)-4H-[1,2,4]triazole;
3-(3-Chloro-4-methyl-thiophen-2-yl)-4-ethyl-5-(4-trifluoromethyl-benzylsulfanyl)-4*H-*[1,2,4]triazole;
3-(3-Chloro-4-methyl-thiophen-2-yl)-4-ethyl-5-(3-nitro-benzylsulfanyl)-4*H*-[1,2,4]triazole;
4-[5-(3-Chloro-4-methyl-thiophen-2-yl)-4-ethyl-4*H*-[1,2,4]triazol-3-ylsulfanylmethyl]-benzoic acid methyl etser;
3-(3-Chloro-4-methyl-thiophen-2-yl)-5-(2,6-dichloro-benzylsulfanyl)-4-ethyl-4*H*-[1,2,4]triazole;
3-(3-Chloro-4-methyl-thiophen-2-yl)-4-ethyl-5-(4-methyl-benzylsulfanyl)-4*H*-[1,2,4]triazole;
3-(3-Chloro-4-methyl-thiophen-2-yl)-4-ethyl-5-(3-trifluoromethoxy-benzylsulfanyl)-4*H-*[1,2,4]triazole;
3-(3-Chloro-4-methyl-thiophen-2-yl)-4-ethyl-5-(5-methyl-2-nitro-benzylsulfanyl)-4*H-*[1,2,4]triazole;
3-(3-Chloro-4-methyl-thiophen-2-yl)-4-ethyl-5-(2-nitro-benzylsulfanyl)-4*H*-[1,2,4]triazole;
4-(4-Ethyl-5-pyridin-2-yl-4*H*-[1,2,4]triazol-3-ylsulfanylmethyl)-benzonitrile;
2-[5-(6-Chloro-benzo[1,3]dioxol-5-ylmethylsulfanyl)-4-ethyl-4*H*-[1,2,4]triazol-3-yl]-pyridine
2-[5-(3,4-Dichloro-benzylsulfanyl)-4-ethyl-4H-[1,2,4]triazol-3-yl]-pyridine;
2-[5-(4-Bromo-benzylsulfanyl)-4-ethyl-4*H*-[1,2,4]triazol-3-yl]-pyridine;
2-[4-Ethyl-5-(4-[1,2,3]thiadiazol-4-yl-benzylsulfanyl)-4*H*-[1,2,4]triazol-3-yl]-pyridine;
2-[5-(3,5-Dimethoxy-benzylsulfanyl)-4-ethyl-4*H*-[1,2,4]triazol-3-yl]-pyridine;
2-[4-Ethyl-5-(4-trifluoromethyl-benzylsulfanyl)-4*H*-[1,2,4]triazol-3-yl]-pyridine;
2-[4-Ethyl-5-(4-methanesulfonyl-benzylsulfanyl)-4*H*-[1,2,4]triazol-3-yl]-pyridine;
2-[4-Ethyl-5-(3-nitro-benzylsulfanyl)-4*H*-[1,2,4]triazol-3-yl]-pyridine;
4-(4-Ethyl-5-pyridin-2-yl-4H-[1,2,4]triazol-3-ylsulfanylmethyl)-benzoic acid methyl etser;
2-[4-Ethyl-5-(4-nitro-benzylsulfanyl)-4*H*-[1,2,4]triazol-3-yl]-pyridine;
2-[5-(4-Benzyloxy-benzylsulfanyl)-4-ethyl-4*H*-[1,2,4]triazol-3-yl]-pyridine;
2-[4-Ethyl-5-(4-methyl-benzylsulfanyl)-4*H*-[1,2,4]triazol-3-yl]-pyridine;
2-[4-Ethyl-5-(3-trifluoromethoxy-benzylsulfanyl)-4*H*-[1,2,4]triazol-3-yl]-pyridine;
2-[4-Ethyl-5-(5-methyl-2-nitro-benzylsulfanyl)-4*H*-[1,2,4]triazol-3-yl]-pyridine;
2-[4-Ethyl-5-(2-nitro-benzylsulfanyl)-4*H*-[1,2,4]triazol-3-yl]-pyridine;
3-[5-(3,4-Dichloro-benzylsulfanyl)-4-ethyl-4*H*-[1,2,4]triazol-3-yl]-2-methylsulfanyl-pyridine;
3-[4-Ethyl-5-(4-[1,2,3]thiadiazol-4-yl-benzylsulfanyl)-4*H*-[1,2,4]triazol-3-yl]-2-methylsulfanyl-pyridine;
3-(5-Cyclohexylmethylsulfanyl-4-ethyl-4*H*-[1,2,4]triazol-3-yl)-2-methylsulfanyl-pyridine;
3-[4-Ethyl-5-(4-trifluoromethoxy-benzylsulfanyl)-4*H*-[1,2,4]triazol-3-yl]-2-methylsulfanyl-pyridine;
3-[5-(3,5-Dimethoxy-benzylsulfanyl)-4-ethyl-4*H*-[1,2,4]triazol-3-yl]-2-methylsulfanyl-pyridine;
3-[4-Ethyl-5-(4-trifluoromethyl-benzylsulfanyl)-4*H*-[1,2,4]triazol-3-yl]-2-methylsulfanyl-pyridine;
3-(5-Benzylsulfanyl-4-ethyl-4*H*-[1,2,4]triazol-3-yl)-2-methylsulfanyl-pyridine;
3-[4-Ethyl-5-(4-methanesulfonyl-benzylsulfanyl)-4*H*-[1,2,4]triazol-3-yl]-2-methylsulfanyl-pyridine;
4-[4-Ethyl-5-(2-methylsulfanyl-pyridin-3-yl)-4*H*-[1,2,4]triazol-3-ylsulfanylmethyl]-benzoic acid methyl etser;
3-[5-(2,6-Dichloro-benzylsulfanyl)-4-ethyl-4*H*-[1,2,4]triazol-3-yl]-2-methylsulfanyl-pyridine;
3-[5-(4-Benzyloxy-benzylsulfanyl)-4-ethyl-4*H*-[1,2,4]triazol-3-yl]-2-methylsulfanyl-pyridine;
3-[4-Ethyl-5-(4-methyl-benzylsulfanyl)-4*H*-[1,2,4]triazol-3-yl]-2-methylsulfanyl-pyridine;
3-[4-Ethyl-5-(3-trifluoromethoxy-benzylsulfanyl)-4*H*-[1,2,4]triazol-3-yl]-2-methylsulfanyl-pyridine;
N-{4-[4-Ethyl-5-(2-methylsulfanyl-pyridin-3-yl)-4*H*-[1,2,4]triazol-3-ylsulfanylmethyl]-phenyl}-acetamide;
3-[4-Ethyl-5-(5-methyl-2-nitro-benzylsulfanyl)-4*H*-[1,2,4]triazol-3-yl]-2-methylsulfanyl-pyridine;
3-[4-Ethyl-5-(2-nitro-benzylsulfanyl)-4*H*-[1,2,4]triazol-3-yl]-2-methylsulfanyl-pyridine;
3-(2-Chloro-benzylsulfanyl)-4-ethyl-5-(5-methyl-thiophen-2-yl)-4*H*-[1,2,4]triazole;
4-[4-Ethyl-5-(5-methyl-thiophen-2-yl)-4*H*-[1,2,4]triazol-3-ylsulfanylmethyl]-benzonitrile;
3-(6-Chloro-benzo[1,3]dioxol-5-ylmethylsulfanyl)-4-ethyl-5-(5-methyl-thiophen-2-yl)-4*H-*[1,2,4]triazole;
3-(3,4-Dichloro-benzylsulfanyl)-4-ethyl-5-(5-methyl-thiophen-2-yl)-4*H*-[1,2,4]triazole;
4-{4-[4-Methyl-5-(5-methyl-thiophen-2-yl)-4*H*-[1,2,4]triazol-3-ylsulfanylmethyl]-phenyl}-[1,2,3]thiadiazole;
3-Cyclohexylmethylsulfanyl-4-ethyl-5-(5-methyl-thiophen-2-yl)-4*H*-[1,2,4]triazole;
4-Ethyl-3-(5-methyl-thiophen-2-yl)-5-(4-trifluoromethoxy-benzylsulfanyl)-4*H*-[1,2,4]triazole;
3-(3,5-Dimethoxy-benzylsulfanyl)-4-ethyl-5-(5-methyl-thiophen-2-yl)-4*H*-[1,2,4]triazole;
4-Ethyl-3-(5-methyl-thiophen-2-yl)-5-(4-trifluoromethyl-benzylsulfanyl)-4*H*-[1,2,4]triazole;
3-Benzylsulfanyl-4-ethyl-5-(5-methyl-thiophen-2-yl)-4*H*-[1,2,4]triazole;
4-Ethyl-3-(4-methanesulfonyl-benzylsulfanyl)-5-(5-methyl-thiophen-2-yl)-4*H*-[1,2,4]triazole;
4-Ethyl-3-(5-methyl-thiophen-2-yl)-5-(3-nitro-benzylsulfanyl)-4*H*-[1,2,4]triazole;
4-[4-Ethyl-5-(5-methyl-thiophen-2-yl)-4*H*-[1,2,4]triazol-3-ylsulfanylmethyl]-benzoic acid methyl etser;
4-Ethyl-3-(5-methyl-thiophen-2-yl)-5-(4-nitro-benzylsulfanyl)-4*H*-[1,2,4]triazole;
3-(2,6-Dichloro-benzylsulfanyl)-4-ethyl-5-(5-methyl-thiophen-2-yl)-4*H*-[1,2,4]triazole;
4-Ethyl-3-(4-methyl-benzylsulfanyl)-5-(5-methyl-thiophen-2-yl)-4*H*-[1,2,4]triazole;
N-{4-[4-Ethyl-5-(5-methyl-thiophen-2-yl)-4*H*-[1,2,4]triazol-3-ylsulfanylmethyl]-phenyl}-acetamide;
4-Ethyl-3-(4-methoxy-benzylsulfanyl)-5-(5-methyl-thiophen-2-yl)-4*H*-[1,2,4]triazole;
4-Ethyl-3-(5-methyl-thiophen-2-yl)-5-(2-nitro-benzylsulfanyl)-4*H*-[1,2,4]triazole;
3-(2,4-Dichloro-benzylsulfanyl)-4-ethyl-5-(5-methyl-thiophen-2-yl)-4*H*-[1,2,4]triazole;
4-[5-(5-Chloro-thiophen-2-yl)-4-ethyl-4*H*-[1,2,4]triazol-3-ylsulfanylmethyl]-benzonitrile;
3-(5-Chloro-thiophen-2-yl)-5-(3,4-dichloro-benzylsulfanyl)-4-ethyl-4*H*-[1,2,4]triazole;
3-(4-Bromo-benzylsulfanyl)-5-(5-chloro-thiophen-2-yl)-4-ethyl-4*H*-[1,2,4]triazole;
4-{4-[5-(5-Chloro-thiophen-2-yl)-4-ethyl-4*H*-[1,2,4]triazol-3-ylsulfanylmethyl]-phenyl}-[1,2,3]thiadiazole;
3-(5-Chloro-thiophen-2-yl)-5-cyclohexylmethylsulfanyl-4-ethyl-4*H*-[1,2,4]triazole;
3-(5-Chloro-thiophen-2-yl)-4-ethyl-5-(4-trifluoromethoxy-benzylsulfanyl)-4*H*-[1,2,4]triazole;
3-(5-Chloro-thiophen-2-yl)-5-(3,5-dimethoxy-benzylsulfanyl)-4-ethyl-4*H*-[1,2,4]triazole;
3-(5-Chloro-thiophen-2-yl)-4-ethyl-5-(4-trifluoromethyl-benzylsulfanyl)-4*H*-[1,2,4]triazole;
3-(5-Chloro-thiophen-2-yl)-4-ethyl-5-(3-nitro-benzylsulfanyl)-4*H*-[1,2,4]triazole;
4-[5-(5-Chloro-thiophen-2-yl)-4-ethyl-4H-[1,2,4]triazol-3-ylsulfanylmethyl]-benzoic acid methyl etser;
3-(5-Chloro-thiophen-2-yl)-5-(2,6-dichloro-benzylsulfanyl)-4-ethyl-4*H*-[1,2,4]triazole;
3-(5-Chloro-thiophen-2-yl)-4-ethyl-5-(4-methyl-benzylsulfanyl)-4*H*-[1,2,4]triazole;
3-(5-Chloro-thiophen-2-yl)-4-ethyl-5-(3-trifluoromethoxy-benzylsulfanyl)-4*H*-[1,2,4]triazole;
N-{4-[5-(5-Chloro-thiophen-2-yl)-4-ethyl-4*H*-[1,2,4]triazol-3-ylsulfanylmethyl]-phenyl}-acetamide;
3-(5-Chloro-thiophen-2-yl)-4-ethyl-5-(4-methoxy-benzylsulfanyl)-4*H*-[1,2,4]triazole;
3-(5-Chloro-thiophen-2-yl)-4-ethyl-5-(5-methyl-2-nitro-benzylsulfanyl)-4*H*-[1,2,4]triazole;
3-(5-Chloro-thiophen-2-yl)-4-ethyl-5-(2-nitro-benzylsulfanyl)-4*H*-[1,2,4]triazole;
3-(5-Chloro-thiophen-2-yl)-5-(2,4-dichloro-benzylsulfanyl)-4-ethyl-4*H*-[1,2,4]triazole;
4-[4-Ethyl-5-(1*H*-indol-3-ylmethyl)-4*H*-[1,2,4]triazol-3-ylsulfanylmethyl]-benzonitrile;
3-[5-(3,4-Dichloro-benzylsulfanyl)-4-ethyl-4*H*-[1,2,4]triazol-3-ylmethyl]-1 *H*-indole;
3-[5-(3,5-Dimethoxy-benzylsulfanyl)-4-ethyl-4*H*-[1,2,4]triazol-3-ylmethyl]-1 *H*-indole;
3-[4-Ethyl-5-(4-methanesulfonyl-benzylsulfanyl)-4*H*-[1,2,4]triazol-3-ylmethyl]-1 *H*-indole;
3-[4-Ethyl-5-(3-nitro-benzylsulfanyl)-4*H*-[1,2,4]triazol-3-ylmethyl]-1 *H*-indole;
3-[4-Ethyl-5-(4-nitro-benzylsulfanyl)-4*H*-[1,2,4]triazol-3-ylmethyl]-1 *H*-indole;
3-[5-(2,6-Dichloro-benzylsulfanyl)-4-ethyl-4*H*-[1,2,4]triazol-3-ylmethyl]-1 *H*-indole;
3-[5-(4-Benzyloxy-benzylsulfanyl)-4-ethyl-4*H*-[1,2,4]triazol-3-ylmethyl]-1*H*-indole;
3-[4-Ethyl-5-(3-trifluoromethoxy-benzylsulfanyl)-4*H*-[1,2,4]triazol-3-ylmethyl]-1*H*-indole;
N-{4-[4-Ethyl-5-(1*H*-indol-3-ylmethyl)-4*H*-[1,2,4]triazol-3-ylsulfanylmethyl]-phenyl}-acetamide;
3-[4-Ethyl-5-(4-methoxy-benzylsulfanyl)-4*H*-[1,2,4]triazol-3-ylmethyl]-1*H*-indole;
3-[4-Ethyl-5-(5-methyl-2-nitro-benzylsulfanyl)-4*H*-[1,2,4]triazol-3-ylmethyl]-1*H*-indole;
3-[4-Ethyl-5-(2-nitro-benzylsulfanyl)-4*H*-[1,2,4]triazol-3-ylmethyl]-1*H*-indole;
3-[5-(2,4-Dichloro-benzylsulfanyl)-4-ethyl-4*H*-[1,2,4]triazol-3-ylmethyl]-1*H*-indole;
2-[5-(6-Chloro-benzo[1,3]dioxol-5-ylmethylsulfanyl)-4-ethyl-4*H*-[1,2,4]triazol-3-yl]-phenol;
2-[4-Ethyl-5-(4-[1,2,3]thiadiazol-4-yl-benzylsulfanyl)-4*H*-[1,2,4]triazol-3-yl]-phenol;
2-(5-Cyclohexylmethylsulfanyl-4-ethyl-4*H*-[1,2,4]triazol-3-yl)-phenol;
2-[5-(3,5-Dimethoxy-benzylsulfanyl)-4-ethyl-4*H*-[1,2,4]triazol-3-yl]-phenol;
2-[4-Ethyl-5-(4-trifluoromethyl-benzylsulfanyl)-4*H*-[1,2,4]triazol-3-yl]-phenol;
2-(5-Benzylsulfanyl-4-ethyl-4*H*-[1,2,4]triazol-3-yl)-phenol;
2-[4-Ethyl-5-(4-methanesulfonyl-benzylsulfanyl)-4*H*-[1,2,4]triazol-3-yl]-phenol;
2-[4-Ethyl-5-(3-nitro-benzylsulfanyl)-4*H*-[1,2,4]triazol-3-yl]-phenol;
4-[4-Ethyl-5-(2-hydroxy-phenyl)-4*H*-[1,2,4]triazol-3-ylsulfanylmethyl]-benzoic acid methyl etser;
2-[4-Ethyl-5-(4-nitro-benzylsulfanyl)-4*H*-[1,2,4]triazol-3-yl]-phenol;
2-[5-(2,6-Dichloro-benzylsulfanyl)-4-ethyl-4*H*-[1,2,4]triazol-3-yl]-phenol;
2-[4-Ethyl-5-(4-methyl-benzylsulfanyl)-4*H*-[1,2,4]triazol-3-yl]-phenol;
2-[4-Ethyl-5-(3-trifluoromethoxy-benzylsulfanyl)-4*H*-[1,2,4]triazol-3-yl]-phenol;
N-{4-[4-Ethyl-5-(2-hydroxy-phenyl)-4*H*-[1,2,4]triazol-3-ylsulfanylmethyl]-phenyl}-acetamide;
2-[4-Ethyl-5-(5-methyl-2-nitro-benzylsulfanyl)-4*H*-[1,2,4]triazol-3-yl]-phenol;
2-[4-Ethyl-5-(2-nitro-benzylsulfanyl)-4*H*-[1,2,4]triazol-3-yl]-phenol;
2-[5-(2,4-Dichloro-benzylsulfanyl)-4-ethyl-4*H*-[1,2,4]triazol-3-yl]-phenol;
5-[5-(3,4-Dichloro-benzylsulfanyl)-4-ethyl-4*H*-[1,2,4]triazol-3-yl]-4-methyl-[1,2,3]thiadiazole;
5-[5-(4-Bromo-benzylsulfanyl)-4-ethyl-4*H*-[1,2,4]triazol-3-yl]-4-methyl-[1,2,3]thiadiazole;
5-(5-Cyclohexylmethylsulfanyl-4-ethyl-4*H*-[1,2,4]triazol-3-yl)-4-methyl-[1,2,3]thiadiazole;
5-[5-(3,5-Dimethoxy-benzylsulfanyl)-4-ethyl-4*H*-[1,2,4]triazol-3-yl]-4-methyl-[1,2,3]thiadiazole;
5-(5-Benzylsulfanyl-4-ethyl-4*H*-[1,2,4]triazol-3-yl)-4-methyl-[1,2,3]thiadiazole;
5-[4-Ethyl-5-(3-nitro-benzylsulfanyl)-4*H*-[1,2,4]triazol-3-yl]-4-methyl-[1,2,3]thiadiazole;
4-[4-Ethyl-5-(4-methyl-[1,2,3]thiadiazol-5-yl)-4*H*-[1,2,4]triazol-3-ylsulfanylmethyl]-benzoic acid methyl etser;
5-[4-Ethyl-5-(4-nitro-benzylsulfanyl)-4*H*-[1,2,4]triazol-3-yl]-4-methyl-[1,2,3]thiadiazole;
5-[5-(4-Benzyloxy-benzylsulfanyl)-4-ethyl-4*H*-[1,2,4]triazol-3-yl]-4-methyl-[1,2,3]thiadiazole;
5-[4-Ethyl-5-(4-methyl-benzylsulfanyl)-4*H*-[1,2,4]triazol-3-yl]-4-methyl-[1,2,3]thiadiazole;
5-[4-Ethyl-5-(3-trifluoromethoxy-benzylsulfanyl)-4*H*-[1,2,4]triazol-3-yl]-4-methyl-[1,2,3]thiadiazole;
5-[4-Ethyl-5-(5-methyl-2-nitro-benzylsulfanyl)-4*H*-[1,2,4]triazol-3-yl]-4-methyl-[1,2,3]thiadiazole;
5-[5-(2,4-Dichloro-benzylsulfanyl)-4-ethyl-4*H*-[1,2,4]triazol-3-yl]-4-methyl-[1,2,3]thiadiazole;
4-(5-Benzyl-4-ethyl-4*H*-[1,2,4]triazol-3-ylsulfanylmethyl)-benzonitrile;
3-Benzyl-5-(3,4-dichloro-benzylsulfanyl)-4-ethyl-4*H*-[1,2,4]triazole;
4-[4-(5-Benzyl-4-ethyl-4*H*-[1,2,4]triazol-3-ylsulfanylmethyl)-phenyl]-[1,2,3]thiadiazole;
3-Benzyl-5-(3,5-dimethoxy-benzylsulfanyl)-4-ethyl-4*H*-[1,2,4]triazole;
3-Benzyl-4-ethyl-5-(4-trifluoromethyl-benzylsulfanyl)-4*H*-[1,2,4]triazole;
3-Benzyl-5-benzylsulfanyl-4-ethyl-4*H*-[1,2,4]triazole;
3-Benzyl-5-(2,6-dichloro-benzylsulfanyl)-4-ethyl-4*H*-[1,2,4]triazole;
3-Benzyl-4-ethyl-5-(4-methyl-benzylsulfanyl)-4*H*-[1,2,4]triazole;
3-Benzyl-4-ethyl-5-(4-methoxy-benzylsulfanyl)-4*H*-[1,2,4]triazole;
3-Benzyl-4-ethyl-5-(5-methyl-2-nitro-benzylsulfanyl)-4*H*-[1,2,4]triazole;
3-Benzyl-4-ethyl-5-(2-nitro-benzylsulfanyl)-4*H*-[1,2,4]triazole;
3-Benzyl-5-(2,4-dichloro-benzylsulfanyl)-4-ethyl-4*H*-[1,2,4]triazole;
3-(2-Bromo-phenyl)-5-(2-chloro-benzylsulfanyl)-4-ethyl-4*H*-[1,2,4]triazole;
4-[5-(2-Bromo-phenyl)-4-ethyl-4*H*-[1,2,4]triazol-3-ylsulfanylmethyl]-benzonitrile;
3-(2-Bromo-phenyl)-5-(6-chloro-benzo[1,3]dioxol-5-ylmethylsulfanyl)-4-ethyl-4*H-*[1,2,4]triazole;
3-(2-Bromo-phenyl)-5-(3,4-dichloro-benzylsulfanyl)-4-ethyl-4*H*-[1,2,4]triazole;
3-(2-Bromo-phenyl)-5-cyclohexylmethylsulfanyl-4-ethyl-4*H*-[1,2,4]triazole;
3-(2-Bromo-phenyl)-4-ethyl-5-(4-trifluoromethoxy-benzylsulfanyl)-4*H*-[1,2,4]triazole;
3-(2-Bromo-phenyl)-5-(3,5-dimethoxy-benzylsulfanyl)-4-ethyl-4*H*-[1,2,4]triazole;
3-(2-Bromo-phenyl)-4-ethyl-5-(4-trifluoromethyl-benzylsulfanyl)-4*H*-[1,2,4]triazole;
3-(2-Bromo-phenyl)-4-ethyl-5-(3-nitro-benzylsulfanyl)-4*H*-[1,2,4]triazole;
3-(4-Benzyloxy-benzylsulfanyl)-5-(2-bromo-phenyl)-4-ethyl-4*H*-[1,2,4]triazole;
3-(2-Bromo-phenyl)-4-ethyl-5-(4-methoxy-benzylsulfanyl)-4*H*-[1,2,4]triazole;
3-(2-Bromo-phenyl)-4-ethyl-5-(5-methyl-2-nitro-benzylsulfanyl)-4*H*-[1,2,4]triazole;
3-(2-Bromo-phenyl)-5-(2,4-dichloro-benzylsulfanyl)-4-ethyl-4*H*-[1,2,4]triazole;
2-[4-(3-Methoxy-propyl)-5-(1-methyl-piperidin-2-yl)-4*H*-[1,2,4]triazol-3-ylsulfanylmethyl]-benzonitrile;
2-[4-(3-Methoxy-propyl)-5-(1-methyl-piperidin-2-yl)-4*H*-[1,2,4]triazol-3-ylsulfanylmethyl]-pyridine;
2-[5-(2-Methoxy-ethylsulfanyl)-4-(3-methoxy-propyl)-4*H*-[1,2,4]triazol-3-yl]-1-methylpiperidine;
2-[4-(2-Methoxy-ethyl)-5-(1-methyl-piperidin-2-yl)-4*H*-[1,2,4]triazol-3-ylsulfanylmethyl]-benzonitrile;
2-[4-(2-Methoxy-ethyl)-5-(1-methyl-piperidin-2-yl)-4*H*-[1,2,4]triazol-3-ylsulfanylmethyl]-pyridine;
2-[4-(2-Methoxy-ethyl)-5-(2-methoxy-ethylsulfanyl)-4*H*-[1,2,4]triazol-3-yl]-1-methyl-piperidine;
2-[4-Cyclopropyl-5-(1-methyl-piperidin-2-yl)-4*H*-[1,2,4]triazol-3-ylsulfanylmethyl]-benzonitrile;
2-[4-Cyclopropyl-5-(1-methyl-piperidin-2-yl)-4*H*-[1,2,4]triazol-3-ylsulfanylmethyl]-pyridine;
2-[4-Cyclopropyl-5-(2-methoxy-ethylsulfanyl)-4*H*-[1,2,4]triazol-3-yl]-1-methyl-piperidine;
2-[5-(1-Methyl-piperidin-2-yl)-4-(2-piperidin-1-yl-ethyl)-4*H*-[1,2,4]triazol-3-ylsulfanylmethyl]-benzonitrile;
2-[5-(1-Methyl-piperidin-2-yl)-4-(2-piperidin-1-yl-ethyl)-4*H*-[1,2,4]triazol-3-ylsulfanylmethyl]-pyridine;
2-[4-Cyclopropylmethyl-5-(1-methyl-piperidin-2-yl)-4*H*-[1,2,4]triazol-3-ylsulfanylmethyl]-benzonitrile;
2-[4-Cyclopropylmethyl-5-(1-methyl-piperidin-2-yl)-4*H*-[1,2,4]triazol-3-ylsulfanylmethyl]-pyridine;
2-[4-Cyclopropylmethyl-5-(2-methoxy-ethylsulfanyl)-4*H*-[1,2,4]triazol-3-yl]-1-methylpiperidine;
2-[5-(2-Hydroxy-phenyl)-4-(3-methoxy-propyl)-4*H*-[1,2,4]triazol-3-ylsulfanylmethyl]-benzonitrile;
2-[4-(3-Methoxy-propyl)-5-(pyridin-2-ylmethylsulfanyl)-4*H*-[1,2,4]triazol-3-yl]-phenol;
2-[5-(2-Methoxy-ethylsulfanyl)-4-(3-methoxy-propyl)-4*H*-[1,2,4]triazol-3-yl]-phenol;
2-[5-(2-Hydroxy-phenyl)-4-(2-methoxy-ethyl)-4*H*-[1,2,4]triazol-3-ylsulfanylmethyl]-benzonitrile;
2-[4-(2-Methoxy-ethyl)-5-(pyridin-2-ylmethylsulfanyl)-4*H*-[1,2,4]triazol-3-yl]-phenol;
2-[4-(2-Methoxy-ethyl)-5-(2-methoxy-ethylsulfanyl)-4*H*-[1,2,4]triazol-3-yl]-phenol;
2-[4-Cyclopropyl-5-(2-hydroxy-phenyl)-4*H*-[1,2,4]triazol-3-ylsulfanylmethyl]-benzonitrile;
2-[4-Cyclopropyl-5-(pyridin-2-ylmethylsulfanyl)-4*H*-[1,2,4]triazol-3-yl]-phenol;
2-[4-Cyclopropyl-5-(2-methoxy-ethylsulfanyl)-4*H*-[1,2,4]triazol-3-yl]-phenol;
2-[5-(2-Hydroxy-phenyl)-4-(2-piperidin-1-yl-ethyl)-4*H*-[1,2,4]triazol-3-ylsulfanylmethyl]-benzonitrile;
2-[4-(2-Piperidin-1-yl-ethyl)-5-(pyridin-2-ylmethylsulfanyl)-4*H*-[1,2,4]triazol-3-yl]-phenol;
2-[5-(2-Methoxy-ethylsulfanyl)-4-(2-piperidin-1-yl-ethyl)-4*H*-[1,2,4]triazol-3-yl]-phenol;
2-[4-Cyclopropylmethyl-5-(2-hydroxy-phenyl)-4*H*-[1,2,4]triazol-3-ylsulfanylmethyl]-benzonitrile;
2-[4-Cyclopropylmethyl-5-(pyridin-2-ylmethylsulfanyl)-4*H*-[1,2,4]triazol-3-yl]-phenol;
2-[4-Cyclopropylmethyl-5-(2-methoxy-ethylsulfanyl)-4*H*-[1,2,4]triazol-3-yl]-phenol;
2-[5-(2-Methoxy-phenyl)-4-(3-methoxy-propyl)-4*H*-[1,2,4]triazol-3-ylsulfanylmethyl]-benzonitrile;
2-[5-(2-Methoxy-phenyl)-4-(3-methoxy-propyl)-4*H*-[1,2,4]triazol-3-ylsulfanylmethyl]-pyridine;
3-(2-Methoxy-ethylsulfanyl)-5-(2-methoxy-phenyl)-4-(3-methoxy-propyl)-4*H*-[1,2,4]triazole;
2-[4-(2-Methoxy-ethyl)-5-(2-methoxy-phenyl)-4*H*-[1,2,4]triazol-3-ylsulfanylmethyl]-benzonitrile;
2-[4-(2-Methoxy-ethyl)-5-(2-methoxy-phenyl)-4*H*-[1,2,4]triazol-3-ylsulfanylmethyl]-pyridine;
4-(2-Methoxy-ethyl)-3-(2-methoxy-ethylsulfanyl)-5-(2-methoxy-phenyl)-4*H*-[1,2,4]triazole;
2-[4-Cyclopropyl-5-(2-methoxy-phenyl)-4*H*-[1,2,4]triazol-3-ylsulfanylmethyl]-benzonitrile;
2-[4-Cyclopropyl-5-(2-methoxy-phenyl)-4*H*-[1,2,4]triazol-3-ylsulfanylmethyl]-pyridine;
4-Cyclopropyl-3-(2-methoxy-ethylsulfanyl)-5-(2-methoxy-phenyl)-4*H*-[1,2,4]triazole;
2-[5-(2-Methoxy-phenyl)-4-(2-piperidin-1-yl-ethyl)-4*H*-[1,2,4]triazol-3-ylsulfanylmethyl]-benzonitrile;
2-[5-(2-Methoxy-phenyl)-4-(2-piperidin-1-yl-ethyl)-4*H*-[1,2,4]triazol-3-ylsulfanylmethyl]-pyridine;
1-{2-[3-(2-Methoxy-ethylsulfanyl)-5-(2-methoxy-phenyl)-[1,2,4]triazol-4-yl]-ethyl}-piperidine;
2-[4-Cyclopropylmethyl-5-(2-methoxy-phenyl)-4*H*-[1,2,4]triazol-3-ylsulfanylmethyl]-benzonitrile;
2-[4-Cyclopropylmethyl-5-(2-methoxy-phenyl)-4*H*-[1,2,4]triazol-3-ylsulfanylmethyl]-pyridine;
4-Cyclopropylmethyl-3-(2-methoxy-ethylsulfanyl)-5-(2-methoxy-phenyl)-4H-[1,2,4]triazole;
2-[4-(3-Methoxy-propyl)-5-thiophen-2-yl-4*H*-[1,2,4]triazol-3-ylsulfanylmethyl]-benzonitrile;
2-[4-(3-Methoxy-propyl)-5-thiophen-2-yl-4*H*-[1,2,4]triazol-3-ylsulfanylmethyl]-pyridine;
3-(2-Methoxy-ethylsulfanyl)-4-(3-methoxy-propyl)-5-thiophen-2-yl-4H-[1,2,4]triazole;
2-[4-(2-Methoxy-ethyl)-5-thiophen-2-yl-4*H*-[1,2,4]triazol-3-ylsulfanylmethyl]-benzonitrile;
2-[4-(2-Methoxy-ethyl)-5-thiophen-2-yl-4*H*-[1,2,4]triazol-3-ylsulfanylmethyl]-pyridine;
4-(2-Methoxy-ethyl)-3-(2-methoxy-ethylsulfanyl)-5-thiophen-2-yl-4*H*-[1,2,4]triazole;
2-(4-Cyclopropyl-5-thiophen-2-yl-4*H*-[1,2,4]triazol-3-ylsulfanylmethyl)-benzonitrile;
2-(4-Cyclopropyl-5-thiophen-2-yl-4*H*-[1,2,4]triazol-3-ylsulfanylmethyl)-pyridine;
4-Cyclopropyl-3-(2-methoxy-ethylsulfanyl)-5-thiophen-2-yl-4*H*-[1,2,4]triazole;
2-[4-(2-Piperidin-1-yl-ethyl)-5-thiophen-2-yl-4*H*-[1,2,4]triazol-3-ylsulfanylmethyl]-benzonitrile;
2-[4-(2-Piperidin-1-yl-ethyl)-5-thiophen-2-yl-4*H*-[1,2,4]triazol-3-ylsulfanylmethyl]-pyridine;
1-{2-[3-(2-Methoxy-ethylsulfanyl)-5-thiophen-2-yl-[1,2,4]triazol-4-yl]-ethyl}-piperidine;
2-(4-Cyclopropylmethyl-5-thiophen-2-yl-4*H*-[1,2,4]triazol-3-ylsulfanylmethyl)-benzonitrile;
2-(4-Cyclopropylmethyl-5-thiophen-2-yl-4*H*-[1,2,4]triazol-3-ylsulfanylmethyl)-pyridine; 4-Cyclopropylmethyl-3-(2-methoxy-ethylsulfanyl)-5-thiophen-2-yl-4*H*-[1,2,4]triazole; or
a salt thereof with a pharmaceutically acceptable acid or base, or any optical isomer or mixture of optical isomers, including a racemic mixture, or any tautomeric forms.

The compounds of the present invention have asymmetric centers and may occur as racemates, racemic mixtures, and as individual enantiomers or diastereoisomers, with all isomeric forms being included in the present invention as well as mixtures thereof.

The present invention also encompasses pharmaceutically acceptable salts of the present compounds. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci., 66, 2 (1977), which is incorporated herein by reference. Examples of metal salts include lithium, sodium, potassium, barium, calcium, magnesium, zinc, calcium salts and the like. Examples of amines and organic amines include ammonium, methylamine, dimethylamine, trimethylamine, ethylamine, diethylamine, propylamine, butylamine, tetramethylamine, ethanolamine, diethanolamine, triethanolamine, meglumine, ethylenediamine, choline, N,N'-dibenzylethylenediamine, N-benzylphenylethylamine, N-methyl-D-glucamine, guanidine and the like. Examples of cationic amino acids include lysine, arginine, histidine and the like.

Further, some of the compounds of the present invention may form solvates with water or common organic solvents. Such solvates are encompassed within the scope of the invention.

The pharmaceutically acceptable salts are prepared by reacting a compound of the present invention with 1 to 4 equivalents of a base such as sodium hydroxide, sodium methoxide, sodium hydride, potassium tert-butoxide, calcium hydroxide, magnesium hydroxide and the like, in solvents like ether, THF, methanol, tert-butanol, dioxane, isopropanol, ethanol etc. Mixtures of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, enzymatic resolution, resolving the diastereomeric salts formed with chiral acids such as mandelic acid, camphorsulfonic acid, tartaric acid, lactic acid, and the like wherever applicable or chiral bases such as brucine, (R)- or (S)-phenylethylamine, cinchona alkaloids and their derivatives and the like. Commonly used methods are compiled by Jaques et al. in "Enantiomers, Racemates and Resolution" (Wiley Interscience, 1981). More specifically the compound of the present invention may be converted to a 1:1 mixture of diastereomeric amides by treating with chiral amines, aminoacids, aminoalcohols derived from aminoacids; conventional reaction conditions may be employed to convert acid into an amide; the diastereomers may be separated either by fractional crystallization or chromatography and the stereoisomers of compound of formula I may be prepared by hydrolysing the pure diastereomeric amide.

Various polymorphs of the compounds forming part of this invention may be prepared by crystallization of said compounds under different conditions. For example, using different solvents commonly used or their mixtures for recrystallization; crystallizations at different temperatures; and various modes of cooling, ranging from very fast to very slow cooling during crystallizations. Polymorphs may also be obtained by heating or melting the compound followed by gradual or fast cooling. The presence of polymorphs may be determined by solid probe NMR spectroscopy, IR spectroscopy, differential scanning calorimetry, powder X-ray diffraction or such other techniques.

The invention also encompasses prodrugs of the present compounds, which on administration undergo chemical conversion by metabolic processes before becoming active pharmacological substances. In general, such prodrugs will be functional derivatives of the present compounds, which are readily convertible *in vivo* into the required compound of the present invention. Conventional procedures for the selection and preparation of suitable prodrug derivatives are described, for example, in "Design of Prodrugs", ed. H. Bundgaard, Elsevier, 1985.

It is a well known problem in drug discovery that compounds, such as enzyme inhibitors, may be very potent and selective in biochemical assays, yet be inactive *in vivo.* This lack of so-called bioavailability may be ascribed to a number of different factors such as lack of or poor absorption in the gut, first pass metabolism in the liver and/or poor uptake in cells. Although the factors determining bioavailability are not completely understood, there are many examples in the scientific literature, well known to those skilled in the art, of how to modify compounds, which are potent and selective in biochemical assays but show low or no activity *in vivo,* into drugs that are biologically active.

It is within the scope of the invention to modify the compounds of the present invention, termed the "original compound", by attaching chemical groups that will improve the bioavailability of said compounds in such a way that the uptake in cells or mammals is facilitated.

Examples of said modifications, which are not intended in any way to limit the scope of the invention, include changing of one or more carboxy groups to esters (for instance methyl esters, ethyl esters, *tert-*butyl, acetoxymethyl, pivaloyloxymethyl esters or other acyloxymethyl esters). Compounds of the invention, original compounds, such modified by attaching chemical groups are termed "modified compounds".

The invention also encompasses active metabolites of the present compounds.

The compounds according to the invention alters, and more specifically, reduces the level of active intracellular glucocorticoid and are accordingly useful for the treatment, prevention and/or prophylaxis of disorders and diseases in which such a modulation or reduction is beneficial.

Accordingly, the present compounds may be applicable for the treatment, prevention and/or prophylaxis of the metabolic syndrome, insulin resistance, dyslipidemia, hypertension, obesity, type 2 diabetes, impaired glucose tolerance (IGT), impaired fasting glucose (IFG), Latent Autoimmune Diabetes in the Adult (LADA), type 1 diabetes, diabetic late complications including cardiovascular diseases, cardiovascular disorders, disorders of lipid metabolism, neurodegenerative and psychiatric disorders, dysregulation of intraocular pressure including glaucoma, immune disorders, inappropriate immune responses, musculo-skeletal disorders, gastrointestinal disorders, polycystic ovary syndrome (PCOS), reduced hair growth or other diseases, disorders or conditions that are influenced by intracellular glucocorticoid levels, adverse effects of increased blood levels of active endogenous or exogenous glucocorticoid, and any combination thereof, adverse effects of increased plasma levels of endogenous active glucocorticoid, Cushing's disease, Cushing's syndrome, adverse effects of glucocorticoid receptor agonist treatment of autoimmune diseases, adverse effects of glucocorticoid receptor agonist treatment of inflammatory diseases, adverse effects of glucocorticoid receptor agonist treatment of diseases with an inflammatory component, adverse effects of glucocorticoid receptor agonist treatment as a part of cancer chemotherapy, adverse effects of glucocorticoid receptor agonist treatment for surgical/post-surgical or other trauma, adverse effects of glucocorticoid receptor agonist therapy in the context of organ or tissue transplantation or adverse effects of glucocorticoid receptor agonist treatment in other diseases, disorders or conditions where glucocorticoid receptor agonists provide clinically beneficial effects.

More specifically the present compounds may be applicable for the treatment, prevention and/or prophylaxis of the metabolic syndrome, type 2 diabetes, diabetes as a consequence of obesity, insulin resistance, hyperglycemia, prandial hyperglycemia, hyperinsuline-mia, inappropriately low insulin secretion, impaired glucose tolerance (IGT), impaired fasting glucose (IFG), increased hepatic glucose production, type 1 diabetes, LADA, pediatric diabetes, dyslipidemia, diabetic dyslipidemia, hyperlipidemia, hypertriglyceridemia, hyperlipoproteinemia, hypercholesterolemia, decreased HDL cholesterol, impaired LDL/HDL ratio, other disorders of lipid metabolism, obesity, visceral obesity, obesity as a consequence of diabetes, increased food intake, hypertension, diabetic late complications, micro-/macroalbuminuria, nephropathy, retinopathy, neuropathy, diabetic ulcers, cardiovascular diseases, arteriosclerosis, atherosclerosis, coronary artery disease, cardiac hypertrophy, myocardial ischemia, heart insufficiency, congestional heart failure, stroke, myocardial infarction, arrythmia, decreased blood flow, erectile dysfunction (male or female), myopathy, loss of muscle tissue, muscle wasting, muscle catabolism, osteoporosis, decreased linear growth, neurodegenerative and psychiatric disorders, Alzheimers disease, neuronal death, impaired cognitive function, depression, anxiety, eating disorders, appetite regulation, migraine, epilepsia, addiction to chemical substances, disorders of intraocular pressure, glaucoma, polycystic ovary syndrome (PCOS), inappropriate immune responses, inappropriate T helper-1/T helper-2 polarisation, bacterial infections, mycobacterial infections, fungal infections, viral infections, parasitic infestations, suboptimal responses to immunizations, immune dysfunction, partial or complete baldness, or other diseases, disorders or conditions that are influenced by intracellular glucocorticoid levels and any combination thereof, adverse effects of glucocorticoid receptor agonist treatment of allergic-inflammatory diseases such as asthma and atopic dermatitis, adverse effects of glucocorticoid receptor agonist treatment of disorders of the respiratory system e.g. asthma, cystic fibrosis, emphysema, bronchitis, hypersensitivity, pneumonitis, eosinophilic pneumonias, pulmonary fibrosis, adverse effects of glucocorticoid receptor agonist treatment of inflammatory bowel disease such as Crohn's disease and ulcerative colitis; adverse effects of glucocorticoid receptor agonist treatment of disorders of the immune system, connective tissue and joints e.g. reactive arthritis, rheumatoid arthritis, Sjögren's syndrome, systemic lupus erythematosus, lupus nephritis, Henoch-Schönlein purpura, Wegener's granulomatosis, temporal arteritis, systemic sclerosis, vasculitis, sarcoidosis, dermatomyositis-polymyositis, pemphigus vulgaris; adverse effects of glucocorticoid receptor agonist treatment of endocrinological diseases such as hyperthyroidism, hypoaldosteronism, hypopituitarism; adverse effects of glucocorticoid receptor agonist treatment of hematological diseases e.g. hemolytic anemia, thrombocytopenia, paroxysmal nocturnal hemoglobinuria; adverse effects of glucocorticoid receptor agonist treatment of cancer such as spinal cord diseases, neoplastic compression of the spinal cord, brain tumours, acute lymphoblastic leukemia, Hodgkin's disease, chemotherapy-induced nausea, adverse effects of glucocorticoid receptor agonist treatment of diseases of muscle and at the neuro-muscular joint e.g. myasthenia gravis and heriditary myopathies (e.g. Duchenne muscular dystrophy), adverse effects of glucocorticoid receptor agonist treatment in the context of surgery & transplantation e.g. trauma, post-surgical stress, surgical stress, renal transplantation, liver transplantation, lung transplantation, pancreatic islet transplantation, blood stem cell transplantation, bone marrow transplantation, heart transplantation, adrenal gland transplantation, tracheal transplantation, intestinal transplantation, corneal transplantation, skin grafting, keratoplasty, lens implantation and other procedures where immunosuppression with glucocorticoid receptor agonists is beneficial; adverse effects of glucocorticoid receptor agonist treatment of brain absess, nausea/vomiting, infections, hypercalcemia, adrenal hyperplasia, autoimmune hepatitis, spinal cord diseases, saccular aneurysms; or adverse effects to glucocorticoid receptor agonist treatment in other diseases, disorders and conditions where glucocorticoid receptor agonists provide clinically beneficial effects.

Accordingly, in a further aspect the invention relates to a compound according to the invention for use as a pharmaceutical composition.

The invention also relates to pharmaceutical compositions comprising, as an active ingredient, at least one compound according to the invention together with one or more pharmaceutically acceptable carriers or diluents.

The pharmaceutical composition is preferably in unit dosage form, comprising from about 0.05 mg/day to about 2000 mg/day, preferably from about 1 mg/day to about 500 mg/day of a compound according to the invention.

In another embodiment, the patient is treated with a compound according to the invention for at least about 1 week, for at least about 2 weeks, for at least about 4 weeks, for at least about 2 months or for at least about 4 months.

In yet another embodiment, the pharmaceutical composition is for oral, nasal, transdermal, pulmonal or parenteral administration.

Furthermore, the invention relates to the use of a compound according to the invention for the preparation of a pharmaceutical composition for the treatment, prevention and/or prophylaxis of disorders and diseases wherein a modulation or an inhibition of the activity of 11 β-HSD1 is beneficial.

The invention also relates to a method for the treatment, prevention and/or prophylaxis of disorders and diseases wherein a modulation or an inhibition of the activity of 11β-HSD1 is beneficial, the method comprising administering to a subject in need thereof an effective amount of a compound according to the invention.

In a preferred embodiment of the invention the present compounds are used for the preparation of a medicament for the treatment, prevention and/or prophylaxis of any diseases and conditions that are influenced by intracellular glucocorticoid levels as mentioned above.

Thus, in a preferred embodiment of the invention the present compounds are used for the preparation of a medicament for the treatment, prevention and/or prophylaxis of conditions and disorders where a decreased level of active intracellular glucocorticoid is desirable, such as the conditions and diseases mentioned above.

In yet a preferred embodiment of the invention the present compounds are used for the preparation of a medicament for the treatment, prevention and/or prophylaxis of the metabolic syndrome including insulin resistance, dyslipidemia, hypertension and obesity.

In yet another preferred embodiment of the invention the present compounds are used for the preparation of a medicament for the treatment, prevention and/or prophylaxis of type 2 diabetes, impaired glucose tolerance (IGT), impaired fasting glucose (IFG).

In yet another preferred embodiment of the invention the present compounds are used for the preparation of a pharmaceutical composition for the delaying or prevention of the progression from IGT to type 2 diabetes.

In yet another preferred embodiment of the invention the present compounds are used for the preparation of a pharmaceutical composition for the delaying or prevention of the progression of the metabolic syndrome into type 2 diabetes.

In still another preferred embodiment of the invention the present compounds are used for the preparation of a pharmaceutical composition for the treatment, prevention and/or prophylaxis of diabetic late complications including cardiovascular diseases; arteriosclerosis; atherosclerosis.

In a further preferred embodiment of the invention the present compounds are used for the preparation of a pharmaceutical composition for the treatment, prevention and/or prophylaxis of neurodegenerative and psychiatric disorders.

In yet a further preferred embodiment of the invention the present compounds are used for the preparation of a pharmaceutical composition for the treatment, prevention and/or prophylaxis of adverse effects of glucocorticoid receptor agonist therapy.

In another embodiment of the present invention, the route of administration may be any route which effectively transports a compound according to the invention to the appropriate or desired site of action, such as oral, nasal, buccal, transdermal, pulmonal, or parenteral.

In still a further aspect of the invention the present compounds are administered in combination with one or more further active substances in any suitable ratios. Such further active substances may e.g. be selected from antiobesity agents, antidiabetics, agents modifying the lipid metabolism, antihypertensive agents, glucocorticoid receptor agonists, agents for the treatment and/or prevention of complications resulting from or associated with diabetes and agents for the treatment and/or prevention of complications and disorders resulting from or associated with obesity.

Thus, in a further aspect of the invention the present compounds may be administered in combination with one or more antiobesity agents or appetite regulating agents.

Such agents may be selected from the group consisting of CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, PPAR (peroxisome proliferator-activated receptor) modulators, RXR (retinoid X receptor) modulators, TR β agonists, AGRP (Agouti related protein) inhibitors, H3 histamine antagonists, opioid antagonists (such as naltrexone), exendin-4, GLP-1 and ciliary neurotrophic factor.

In one embodiment of the invention the antiobesity agent is leptin; dexamphetamine or amphetamine; fenfluramine or dexfenfluramine; sibutramine; orlistat; mazindol or phen-termine.

Suitable antidiabetic agents include insulin, insulin analogues and derivatives such as those disclosed in EP 792 290 (Novo Nordisk A/S), eg N^{εB29}-tetradecanoyl des (B30) human insulin, EP 214 826 and EP 705 275 (Novo Nordisk A/S), eg Asp^{B28} human insulin, US 5,504,188 (Eli Lilly), eg Lys^{B28} Pro^{B29} human insulin, EP 368 187 (Aventis), eg Lantus, which are all incorporated herein by reference, GLP-1 (glucagon like peptide-1) and GLP-1 derivatives such as those disclosed in WO 98/08871 to Novo Nordisk A/S, which is incorporated herein by reference as well as orally active hypoglycaemic agents.

The orally active hypoglycaemic agents preferably comprise sulphonylureas, biguanides, meglitinides, glucosidase inhibitors, glucagon antagonists such as those disclosed in WO 99/01423 to Novo Nordisk A/S and Agouron Pharmaceuticals, Inc., GLP-1 agonists, potassium channel openers such as those disclosed in WO 97/26265 and WO 99/03861 to Novo Nordisk A/S which are incorporated herein by reference, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as PPARα modulators, PPARδ modulators, cholesterol absorption inhibitors, HSL (hormone-sensitive lipase) inhibitors and HMG CoA inhibitors (statins), nicotinic acid, fibrates, anion exchangers, compounds lowering food intake, bile acid resins, RXR agonists and agents acting on the ATP-dependent potassium channel of the β-cells.

In one embodiment, the present compounds are administered in combination with insulin or an insulin analogue or derivative, such as N^{εB29}-tetradecanoyl des (B30) human insulin, Asp^{B28} human insulin, Lys^{B28} Pro^{B29} human insulin, Lantus®, or a mix-preparation comprising one or more of these.

In a further embodiment the present compounds are administered in combination with a sulphonylurea e.g. tolbutamide, glibenclamide, glipizide or glicazide.

In another embodiment the present compounds are administered in combination with a biguanide e.g. metformin.

In yet another embodiment the present compounds are administered in combination with a meglitinide e.g. repaglinide or senaglinide.

In still another embodiment the present compounds are administered in combination with a thiazolidinedione e.g. troglitazone, ciglitazone, pioglitazone, rosiglitazone or compounds disclosed in WO 97/41097 such as 5-[[4-[3-Methyl-4-oxo-3,4-dihydro-2-quinazolinyl]methoxy]phenyl-methyl]thiazolidine-2,4-dione or a pharmaceutically acceptable salt thereof, preferably the potassium salt.

In yet another embodiment the present compounds may be administered in combination with the insulin sensitizers disclosed in WO 99/19313 such as (-) 3-[4-[2-Phenoxazin-10-yl)ethoxy]phenyl]-2-ethoxypropanoic acid or a pharmaceutically acceptable salts thereof, preferably the arginine salt.

In a further embodiment the present compounds are administered in combination with an α-glucosidase inhibitor e.g. miglitol or acarbose.

In another embodiment the present compounds are administered in combination with an agent acting on the ATP-dependent potassium channel of the β-cells e.g. tolbutamide, glibenclamide, glipizide, glicazide or repaglinide.

Furthermore, the present compounds may be administered in combination with nateglinide.

In still another embodiment the present compounds are administered in combination with an antihyperlipidemic agent or antilipidemic agent e.g. cholestyramine, colestipol, clofibrate, gemfibrozil, fenofibrate, bezafibrate, tesaglitazar, EML-4156, LY-818, MK-767, atorvastatin, fluvastatin, lovastatin, pravastatin, simvastatin, acipimox, probucol, ezetimibe or dextrothyroxine.

In a further embodiment the present compounds are administered in combination with more than one of the above-mentioned compounds e.g. in combination with a sulphonylurea and metformin, a sulphonylurea and acarbose, repaglinide and metformin, insulin and a sulphonylurea, insulin and metformin, insulin, insulin and lovastatin, etc.

Further, the present compounds may be administered in combination with one or more antihypertensive agents. Examples of antihypertensive agents are β-blockers such as alprenolol, atenolol, timolol, pindolol, propranolol, metoprolol, bisoprololfumerate, esmolol, acebutelol, metoprolol, acebutolol, betaxolol, celiprolol, nebivolol, tertatolol, oxprenolol, amusolalul, carvedilol, labetalol, β2-receptor blockers e.g. S-atenolol, OPC-1085, ACE (angiotensin converting enzyme) inhibitors such as quinapril, lisinopril, enalapril, captopril, benazepril, perindopril, trandolapril, fosinopril, ramipril, cilazapril, delapril, imidapril, moexipril, spirapril, temocapril, zofenopril, S-5590, fasidotril, Hoechst-Marion Roussel: 100240 (EP 00481522), omapatrilat, gemopatrilat and GW-660511, calcium channel blockers such as nifedipine, felodipine, nicardipine, isradipine, nimodipine, diltiazem, amlodipine, nitrendipine, verapamil, lacidipine, lercanidipine, aranidipine, cilnidipine, clevidipine, azelnidipine, barnidipine, efonodipine, iasidipine, iemildipine, iercanidipine, manidipine, nilvadipine, pranidipine, furnidipine, α-blockers such as doxazosin, urapidil, prazosin, terazosin, bunazosin and OPC-28326, diuretics such as thiazides/sulphonamides (e.g. bendroflumetazide, chlorothalidone, hydrochlorothiazide and clopamide), loop-diuretics (e.g. bumetanide, furosemide and torasemide) and potassium sparing diuretics (e.g. amiloride, spironolactone), endothelin ET-A antagonists such as ABT-546, ambrisetan, atrasentan, SB-234551, CI-1034, S-0139 and YM-598, endothelin antagonists e.g. bosentan and J-104133, renin inhibitors such as aliskiren, vasopressin V1 antagonists e.g. OPC-21268, vasopressin V2 antagonists such as tolvaptan, SR-121463 and OPC-31260, B-type natriuretic peptide agonists e.g. Nesiritide, angiotensin II antagonists such as irbesartan, candesartancilexetil, losartan, valsartan, telmisartan, eprosartan, candesartan, CL-329167, eprosartan, iosartan, olmesartan, pratosartan, TA-606, and YM-358, 5-HT2 agonists e.g. fenoldopam and ketanserin, adenosine A1 antagonists such as naftopidil, N-0861 and FK-352, thromboxane A2 antagonists such as KT2-962, endopeptidase inhibitors e.g. ecadotril, nitric oxide agonists such as LP-805, dopamine D1 antagonists e.g. MYD-37, dopamine D2 agonists such as nolomirole, n-3 fatty acids e.g. omacor, prostacyclin agonists such as treprostinil, beraprost, PGE1 agonists e.g. ecraprost, Na+/K+ ATPase modulators e.g. PST-2238, Potassium channel activators e.g. KR-30450, vaccines such as PMD-3117, Indapamides, CGRP-unigene, guanylate cyclase stimulators, hydralazines, methyldopa, docarpamine, moxonidine, CoAprovel, MondoBiotech-811.

Further reference can be made to Remington: The Science and Practice of Pharmacy, 19th Edition, Gennaro, Ed., Mack Publishing Co., Easton, PA, 1995.

Furthermore, the present compounds may be administered in combination with one or more glucocorticoid receptor agonists. Examples of such glucocorticoid receptor agonists are betametasone, dexamethasone, hydrocortisone, methylprednisolone, prednisolone, prednisone, beclomethasone, butixicort, clobetasol, flunisolide, flucatisone (and analogues), momethasone, triamcinolonacetonide, triamcinolonhexacetonide GW-685698, NXC-1015, NXC-1020, NXC-1021, NS-126, P-4112, P-4114, RU-24858 and T-25 series.

It should be understood that any suitable combination of the compounds according to the invention with one or more of the above-mentioned compounds and optionally one or more further pharmacologically active substances are considered to be within the scope of the present invention.

### PHARMACEUTICAL COMPOSITIONS

The compounds of the present invention may be administered alone or in combination with pharmaceutically acceptable carriers or excipients, in either single or multiple doses as part of combination therapy comprising antihypertensive agents. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well-known in the art.

Liquid dosage forms for oral administration include solutions, emulsions, suspensions, syrups and elixirs.
Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 2000 mg, e.g. from about 0.1 to about 1000 mg, from about 0.5 mg to about 500 mg., from about 1 mg to about 200 mg, e.g. about 100 mg.

For parenteral routes, such as intravenous, intrathecal, intramuscular and similar administration, typically doses are in the order of about half the dose employed for oral administration.

The compounds of this invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of the compounds for use according to the present invention which are generally prepared by reacting the free base with a suitable organic or inorganic acid or by reacting the acid with a suitable organic or inorganic base. When a compound for use according to the present invention, contains a free base such salts are prepared in a conventional manner by treating a solution or suspension of the compound with a chemical equivalent of a pharmaceutically acceptable acid. When a compounds for use according to the present invention, contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds for use according to the present invention and these form a further aspect of the present invention.

For parenteral administration, solutions of the present compounds in sterile aqueous solution, aqueous propylene glycol or sesame or peanut oil may be employed. Such aqueous solutions should be suitable buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of suitable carriers are water, salt solutions, alcohols, polyethylene glycols, polyhydroxyethoxylated castor oil, peanut oil, olive oil, syrup, phospholipids, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents.

The pharmaceutical compositions formed by combining the compounds of the invention and the pharmaceutically acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. These formulations may be in the form of powder or granules, as a solution or suspension in an aqueous or non-aqueous liquid, or as an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or a soft gelatine capsule wherein the active ingredient is mixed with water or an oil medium, for example peanut oil, liquid paraffin, or olive oil.

Aqueous suspensions may contain the active compounds in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions comprising a compound for use according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, preservative, flavouring and colouring agents. The pharmaceutical compositions may be in the form of a sterile injectable aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the present invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the present invention are contemplated. For the purpose of this application, topical applications shall include mouth washes and gargles.

The compounds for use according to the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds for use according to the present invention may form solvates with water or common organic solvents. Such solvates are also encompassed within the scope of the present invention.

Thus, in a further embodiment, there is provided a pharmaceutical composition comprising a compound and antihypertensive agent(s) for use according to the present invention, or a pharmaceutically acceptable salt, solvate, or prodrug thereof, and one or more pharmaceutically acceptable carriers, excipients, or diluents.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

A typical tablet which may be prepared by conventional tabletting techniques may contain:

### Core:

| | |
|---|---|
| Active compound (as free compound or salt thereof) | 5.0 mg |
| Lactosum Ph. Eur. | 67.8 mg |
| Cellulose, microcryst. (Avicel) | 31.4 mg |
| Amberlite^{®}IRP88* | 1.0 mg |
| Magnesii stearas Ph. Eur. | q.s. |

### Coating:

| | |
|---|---|
| Hydroxypropyl methylcellulose | approx. 9 mg |
| Mywacett 9-40 T** | approx. 0.9 mg |
| * Polacrillin potassium NF, tablet disintegrant, Rohm and Haas. ** Acylated monoglyceride used as plasticizer for film coating. | |

The compounds of the invention may be administered to a patient which is a mammal, especially a human. Such mammals include animals, both domestic animals, e.g. household pets, and non-domestic animals such as wildlife.

### PHARMACOLOGICAL METHODS

### Materials:

³H-cortisone, ³H-cortisol and scintillation proximity assay (SPA) beads were purchased from Amersham Pharmacia Biotech, β-NADPH and β-NAD were from Sigma Chemical Co, rabbit anti-cortisol antibodies were from Fitzgerald and sheep-anti-11-dehydrocorticosterone antibodies were from Chemicon International INC. Hepes and phosphate-buffered saline were purchased from Life Technologies. The test compounds were dissolved in DMSO (10 mM). All dilutions were performed in a buffer containing 50 mM TRIS-HCI (Sigma Chemical Co), 4 mM EDTA (Sigma Chemical Co), 0.1 % BSA (Sigma Chemical Co), 0.01 % Tween-20 (Sigma Chemical Co) and 0.005% bacitracin (Novo Nordisk A/S), pH=7.4. Optiplate 96 wells plates were supplied by Packard. The amount of radioligand bound to the SPA beads was measured on TopCount NXT, Packard.

### Experimental procedures in vitro:

### 11β-HSD1 enzymatic activity assay:

An extract of yeast transformed with h-11 β HSD1 (Hult et al., FEBS Lett., 441, 25 (1998)) was used as the source of enzyme. Enzyme preparation, 120 nM ³H-cortisone, 4 mM β-NADPH, rabbit-anti cortisol antibody (1:200), serial dilutions of test compound and anti-rabbit Ig coated SPA particles (2 mg/well) were added to the wells. The reaction was initiated by mixing the different components and was allowed to proceed under shaking for 60 min at 30°C. The reaction was stopped be the addition of 10 fold excess of a stopping buffer containing 500 µM carbenoxolone and 1.0 µM cortisone. Data was analysed using GraphPad Prism software.

### Selectivity vs. h-11βHSD2:

Baby hamster kidney (BHK) cells were transfected with full-length h-11βHSD2. The cells were sonicated in phosphate-buffered saline containing 10 mM HEPES (Life Technologies). The cell lysate was used as the source of enzyme. Enzyme preparation, 50 nM ³H-cortisol, 4 mM β-NAD, antibody (1:40), serial dilutions of test compound and anti-sheep lg coated SPA particles (1 mg/well) were added to the wells of 96-well plates. The reaction was initiated by mixing the different components and was allowed to proceed under shaking for 30 min at 30°C. The reaction was stopped be the addition of 10 fold excess of a stopping buffer containing 5 µM carbenoxolone. Data was analysed using GraphPad Prism software.

### Animal studies

### Pharmacological activity may be determined in an in vivo test procedure in rats as follows:

Sprague-Dawley rats are allowed to acclimatise in the test facility for at least two weeks. Throughout the experiment, the rats are kept in their home cages at a standard 12 hour light - 12 hour dark cycle at 20 ± 2°C with free access to drinking water and standard rat chow containing calcium, phosphorus and vitamin D3. The combination according to the present invention or the active ingredients alone are dissolved in a vehicle appropriate for the chosen route of administration and administered in fixed amounts alone or in combination to the test animals. After predetermined time, the change in desired end-point is measured. An example of such an end-point could be the prevention by an 11 β-HSD1 inhibitor of glucocorticoid receptor agonist induced bone loss measured by:
- Total-, cortical-, and trabecular bone mineral content (BMC) and density (BMD) determined by peripheral quantitative computed tomography (pQCT) in proximal and midshaft tibia.
- Cancellous bone volume in proximal tibia determined by histomorphometry.
- Biochemical markers: Bone turnover rates assessed using the marker serum osteo-calcin (an osteoblast specific marker) and the urinary marker RatLaps (a degradation product of type I collagen derived from bone - an osteoclast specific marker). Cartilage turnover assessed using the urine marker CartiLaps (a degradation product of type II collagen).
While the invention has been described and illustrated with reference to certain particular embodiments thereof, those skilled in the art will appreciate that various adaptations, changes, modifications, substitutions, deletions, or additions of procedures and protocols may be made without departing from the spirit and scope of the invention. All references cited herein are hereby incorporated by reference in their entirety.

## Claims

1. A combination therapy which comprises an 11 β-hydroxysteroid dehydrogenase type 1 (11β-HSD1) inhibitor and a glucocorticoid receptor agonist.

2. The combination according to claim 1 wherein the 11β-HSD1 inhibitor is of the Formula (II): 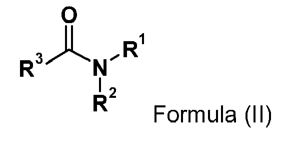 wherein
R¹ is C₃-C₁₀cycloalkyl, C₃-C₁₀hetcycloalkyl, C₁-C₈alkyl, aryl, hetaryl, arylC₁-C₆alkyl or hetarylC₁-C₆alkyl, wherein the cycloalkyl, hetcycloalkyl, alkyl, arylalkyl and hetarylalkyl groups independently are optionally substituted with one or more of R⁴.
R² is hydrogen, C₁-C₈alkyl, aryl, hetaryl, arylC₁-C₆alkyl, C₃-C₁₀cycloalkylC₁-C₆alkyl, C₁-C₆alkyl-carboxyC₁-C₆alkyl wherein the alkyl, aryl and cycloalkyl groups independently are optionally substituted with one or more of R⁵; or
R¹ and R² together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹⁴;
R³ is C₁-C₈alkyl, C₁-C₆alkenyl, C₁-C₆alkynyl, C₃-C₁₀cycloalkyl, C₃-C₁₀hetcycloalkyl, aryl, hetaryl, arylC₁-C₆alkyl, C₁-C₆alkyloxyC₁-C₆alkyl, hetarylC₁-C₆alkyl, aryl-R⁶-C₁-C₆alkyl, hetaryl-R⁶-C₁-C₆alkyl or arylC₁-C₆alkyl-R⁶-C₁-C₆alkyl wherein the alkyl, cycloalkyl, hetcycloalkyl, alkenyl, alkynyl, aryl and hetaryl groups independently are optionally substituted with one or more of R⁷;
R⁴ and R⁵ independently are hydrogen, hydroxy, oxo, cyano, halo, methylendioxo, NR⁸R⁹, C₁-C₈alkyl, C₁-C₆alkyloxy, trihalomethyl, trihalomethyloxy, C₃-C₁₀cycloalkyl, C₃-C₁₀hetcycloalkyl, C₃-C₁₀cycloalkenyl, aryl, hetaryl, hetarylSOₙ, arylC₁-C₆alkyloxy, hetarylC₁-C₆alkyloxy, C₁-C₆alkyl-R⁶-C₁-C₆alkyl, arylC₁-C₆alkyl-R⁶-C₁-C₆alkyl, C₁-C₆alkylcarbonyl, arylcarbonyl, arylC₁-C₆alkylcarbonyl, hetarylcarbonyl, hetarylC₁-C₆alkyl-carbonyl, C₁-C₆alkylSOₙ, C₁-C₆alkyl-carboxy, arylcarboxy, hetarylcarboxy, arylC₁-C₆alkylcarboxy or hetarylC₁-C₆alkylcarboxy wherein the alkyl, cycloalkyl, hetcycloalkyl, aryl and hetaryl groups independently are optionally substituted with one ore more of R¹⁵;
R⁶ is oxygen, sulphur, SOₙ or NR¹⁶;
R⁷ is hydrogen, halo, hydroxy, cyano, nitro, COOR¹⁷, C₁-C₈alkyl, C₃-C₁₀cycloalkyl, C₃-C₁₀hetcycloalkyl, methylendioxo, trihalomethyl, trihalomethyloxy, aryl, arylC₁-C₆alkyl, C₁-C₆alkyloxy, C₁-C₆alkyloxyC₁-C₆alkyl, aryloxy, arylC₁-C₆alkyloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyl-oxyC₁-C₆alkyl, NR⁸R⁹, SO₂NR⁸R⁹, NR⁴R⁵carbonylC₁-C₆alkyl, arylthio, hetarylthio, R¹⁸carbonylNR⁸, arylSOₙ, hetarylSOₙ, R¹⁹SOₘNR⁸, arylthioC₁-C₆alkyl, hetarylthioC₁-C₆alkyl or arylC₁-C₆alkylR⁶C₁-C₆alkyl; wherein the aryl and hetaryl groups independently are optionally substituted with one or more R¹⁰;
R⁸ and R⁹ independently are hydrogen, C₁-C₈alkyl, aryl, hetaryl, arylC₁-C₆alkyl or hetarylC₁-C₆alkyl wherein the alkyl, aryl and hetaryl groups independently are optionally substituted with one or more of R¹¹; or
R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one halo, cyano, C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R¹⁰ and R¹¹ independently are hydrogen, hydroxy, oxo, halo, cyano, nitro, C₁-C₈alkyl, C₁-C₆alkyloxy, NR¹²R¹³, methylendioxo, trihalomethyl or trihalomethyloxy;
R¹² and R¹³ independently are hydrogen, C₁-C₈alkyl or arylC₁-C₆alkyl;
R¹⁴ is hydrogen, halo, hydroxy, oxo, nitro, cyano, C₁-C₈alkyl, C₁-C₆alkyloxy or aryloxy;
R¹⁵ is hydrogen, halo, hydroxy, oxo, nitro, cyano, CONR⁸R⁹ or COOR¹⁷;
R¹⁶ is hydrogen, C₁-C₈alkyl, C₃-C₁₀cycloalkyl, C₃-C₁₀hetcycloalkyl, aryl, arylC₁-C₆alkyl, hetaryl, hetarylC₁-C₆alkyl, alkylcarbonyl, arylcarbonyl, arylC₁-C₆alkylcarbonyl, aryloxyC₁-C₆alkyl, hetaryloxyC₁-C₆alkyl, arylthioC₁-C₆alkyl or hetarylthioC₁-C₆alkyl; wherein the alkyl, cycloalkyl, hetcycloalkyl, aryl and hetaryl groups independently are optionally substituted with one or more of R¹⁰;
R¹⁷ is hydrogen, C₁-C₈alkyl, aryl or arylC₁-C₆alkyl;
R¹⁸ is C₁-C₆alkyl, C₂-C₆alkenyl, aryl, arylC₁-C₆alkyl, hetaryl, hetarylC₁-C₆alkyl, C₃-C₁₀cycloalkyl, C₃-C₁₀hetcycloalkyl, C₁-C₆alkyloxy, aryloxy, arylC₁-C₆alkyloxy, arylC₁-C₆alkyloxyC₁-C₆alkyl, hetaryloxy, hetarylC₁-C₆alkyloxy, hetarylC₁-C₆alkyloxyC₁-C₆alkyl or R⁸R⁹NC₁-C₆alkyl wherein the alkyl, alkenyl, cycloalkyl, hetcycloalkyl, aryl and hetaryl groups are optionally substituted with R¹⁵;
R¹⁹ is C₁-C₆alkyl, C₃-C₁₀cycloalkyl, C₃-C₁₀hetcycloalkyl, aryl, arylC₁-C₆alkyl, hetaryl, hetaryl-C₁-C₆alkyl;
m is 1 or 2;
n is 0, 1 or 2; or
a salt thereof with a pharmaceutically acceptable acid or base, or any optical isomer or mixture of optical isomers, including a racemic mixture, or any tautomeric forms.

3. The combination according to claim 2 wherein the 11 β-HSD1 inhibitor is of formula (II) wherein:
R¹ is C₃-C₁₀cycloalkyl, C₃-C₁₀hetcycloalkyl, C₁-C₈alkyl, aryl, hetaryl, arylC₁-C₆alkyl or hetarylC₁-C₆alkyl, wherein the cycloalkyl, hetcycloalkyl, alkyl, arylalkyl and hetarylalkyl groups independently are optionally substituted with one or more of R⁴;
R² is hydrogen, C₁-C₈alkyl, aryl, hetaryl, arylC₁-C₆alkyl, C₃-C₁₀cycloalkylC₁-C₆alkyl, C₁-C₆alkylcarboxyC₁-C₆alkyl wherein the alkyl, aryl and cycloalkyl groups independently are optionally substituted with one or more of R⁵; or
R¹ and R² are together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁₋₆alkylcarboxy wherein the alkyl and aryl groups independently are optionally substituted with one ore more of R¹⁴;
R³ is C₁-C₈alkyl, C₁-C₆alkenyl, C₁-C₆alkynyl, C₃-C₁₀cycloalkyl, C₃-C₁₀hetcycloalkyl, aryl, hetaryl, arylC₁-C₆alkyl, C₁-C₆alkyloxyC₁-C₆alkyl, hetarylC₁-C₆alkyl, aryl-R⁶-C₁-C₆alkyl, hetaryl-R⁶-C₁-C₆alkyl or arylC₁-C₆alkyl-R⁶-C₁-C₆alkyl wherein the alkyl, cycloalkyl, hetcycloalkyl, alkenyl, alkynyl, aryl and hetaryl groups independently are optionally substituted with one or more of R⁷;
R⁴ and R⁵ independently are hydrogen, hydroxy, oxo, cyano, halo, methylendioxo, NR⁸R⁹, C₁-C₈alkyl, C₁-C₆alkyloxy, trihalomethyl, trihalomethyloxy, C₃-C₁₀cycloalkyl, C₃-C₁₀hetcycloalkyl, C₃-C₁₀cycloalkenyl, aryl, hetaryl, hetarylSOₙ, arylC₁-C₆alkyloxy, hetarylC₁-C₆alkyloxy, C₁-C₆alkyl-R⁶-C₁-C₆alkyl, arylC₁-C₆alkyl-R⁶-C₁-C₆alkyl, C₁-C₆alkylcarbonyl, arylcarbonyl, arylC₁-C₆alkylcarbonyl, hetarylcarbonyl, hetarylC₁-C₆alkyl-carbonyl, C₁-C₆alkylSOₙ, C₁-C₆alkyl-carboxy, arylcarboxy, hetarylcarboxy, arylC₁-C₆alkylcarboxy or hetarylC₁-C₆alkylcarboxy wherein the alkyl, cycloalkyl, hetcycloalkyl, aryl and hetaryl groups independently are optionally substituted with one ore more of R¹⁵;
R⁶ is oxygen, sulphur, SOₙ, NR¹⁶;
R⁷ is hydrogen, halo, hydroxyl, cyano, nitro, COOR¹⁷, C₁-C₈alkyl, C₃-C₁₀cycloalkyl, C₃-C₁₀hetcycloalkyl, methylendioxo, trihalomethyl, trihalomethyloxy, aryl, arylC₁-C₆alkyl, C₁-C₆alkyloxy, C₁-C₆alkyloxyC₁-C₆alkyl, aryloxy, aryloxyC₁-C₆alkyl, arylC₁-C₆alkyloxyC₁-C₆alkyl, hetaryl, hetarylC₁-C₆alkyl, hetaryloxy, hetarylC₁-C₆alkyloxy, hetaryloxyC₁-C₆alkyl, hetarylC₁-C₆alkyloxyC₁-C₆alkyl, NR⁸R⁹, SO₂NR⁸R⁹, NR⁴R⁵carbonylalkyl, arylcarbonylNR⁸, arylthio, hetarylthio, arylSOₘ, hetarylSOₘ, arylSOₘNR⁸, arylthioC₁-C₆alkyl, hetarylthioC₁-C₆alkyl or arylC₁-C₆alkyl-R⁶C₁-C₆alkyl; wherein the aryl and hetaryl groups independently are optionally substituted with one or more R¹⁰;
R⁸ and R⁹ independently are hydrogen, C₁-C₈alkyl, aryl, hetaryl, arylC₁-C₆alkyl or hetarylC₁-C₆alkyl wherein the alkyl, aryl and hetaryl groups independently are optionally substituted with one or more of R¹¹; or
R⁸ and R⁹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulfur, the ring system optionally being substituted with at least one C₁-C₈alkyl, aryl, hetaryl, arylC₁-C₆alkyl, hetarylC₁-C₆alkyl, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkyl-carboxy or hetarylC₁-C₆alkylcarboxy;
R¹⁰ and R¹¹ independently are hydrogen, hydroxy, oxo, halo, cyano, nitro, C₁-C₆alkyl, C₁-C₆alkyloxy, NR¹²R¹³, methylendioxo, trihalomethyl or trihalomethyloxy;
R¹² and R¹³ independently are hydrogen, C₁-C₈alkyl or arylC₁-C₆alkyl;
R¹⁴ is hydrogen, halo, hydroxy, oxo, nitro, cyano, C₁-C₈alkyl, C₁-C₆alkyloxy or aryloxy;
R¹⁵ is hydrogen, halo, hydroxy, oxo, nitro, cyano or COOR¹⁷;
R¹⁶ is hydrogen, C₁-C₈alkyl, C₃-C₁₀cycloalkyl, C₃-C₁₀hetcycloalkyl, aryl, arylC₁-C₆alkyl, hetaryl, hetarylC₁-C₆alkyl, alkylcarbonyl, arylcarbonyl, arylC₁-C₆alkylcarbonyl, aryloxyC₁-C₆alkyl, hetaryloxyC₁-C₆alkyl, arylthioC₁-C₆alkyl or hetarylthioC₁-C₆alkyl; wherein the alkyl, cycloalkyl, hetcycloalkyl, aryl and hetaryl groups independently are optionally substituted with one or more of R¹⁰;
R¹⁷ is hydrogen, C₁-C₈alkyl, aryl or arylC₁-C₆alkyl;
m is 1 or 2;
n is 0, 1 or 2; or
a salt thereof with a pharmaceutically acceptable acid or base, or optical isomer, mixture of optical isomers, racemic mixture, or tautomeric form thereof.

4. The combination according to claim 2 or 3 wherein the 11β-HSD1 inhibitor is selected from the group:
3-(10,11-Dihydro-dibenzo[b,f]azepin-5-yl)-1-[4-(1H-imidazol-4-yl)-piperidin-1-yl]-propan-1-one;
4-(10,11-Dihydro-dibenzo[b,f]azepin-5-yl)-1-[4-(3H-imidazol-4-yl)-piperidin-1-yl]-butan-1-one;
2,4-Bis-benzyloxy-benzamide;
(1H-Indol-4-yl)-piperidin-1-yl-methanone;
N-(1,4-Dioxo-1,4-dihydro-naphthalen-2-yl)-benzamide;
N-(2,3-Dihydroxy-propyl)-2-(2-phenyl-adamantan-2-yl)-acetamide;
(6-Fluoro-2-methyl-3,4-dihydro-2H-quinolin-1-yl)-phenyl-methanone;
(2-Chloro-phenyl)-(6-fluoro-2-methyl-3,4-dihydro-2H-quinolin-1-yl)-methanone;
3-Cyclopentyl-1-(6-fluoro-2-methyl-3,4-dihydro-2H-quinolin-1-yl)-propan-1-one;
(3-Chloro-thieno[2,3-b]thiophen-2-yl)-thiomorpholin-4-yl-methanone;
2-[2-(4-Chloro-phenyl)-adamantan-2-yl]-1-[4-(4-methoxy-phenyl)-piperazin-1-yl]-ethanone;
1-(4-Benzyl-piperazin-1-yl)-2-[2-(4-chloro-phenyl)-adamantan-2-yl]-ethanone;
2-[2-(4-Chloro-phenyl)-adamantan-2-yl]-1-(4-methyl-piperazin-1-yl)-ethanone;
1-[4-(6-Chloro-pyridin-2-yl)-piperazin-1-yl]-2-(2-phenyl-adamantan-2-yl)-ethanone;
4-Chloro-N-(1,7,7-trimethyl-bicyclo[2.2.1]hept-2-yl)-benzamide;
3-Chloro-benzo[b]thiophene-2-carboxylic acid (2-cyano-ethyl)-cyclohexyl-amide;
2-[2-(Bicyclo[2.2.1]hept-5-en-2-ylamino)-4-oxo-4,5-dihydro-thiazol-5-yl]-N-(2-chloro-phenyl)-acetamide;
[3-(4-sec-Butyl-phenoxy)-phenyl]-piperidin-1-yl-methanone;
3-(6-Chloro-pyridin-2-yloxy)-N-ethyl-benzamide;
N-Benzyl-2,4-dichloro-N-pyridin-2-yl-benzamide;
2-[Benzoyl-(3-chloro-4-fluoro-phenyl)-amino]-propionic acid butyl ester; 2-[Benzoyl-(3-chloro-4-fluoro-phenyl)-amino]-propionic acid pentyl ester;
3-(4-Fluoro-phenyl)-1-(4-phenyl-piperidin-1-yl)-but-2-en-1-one;
N-(1,7,7-Trimethyl-bicyclo[2.2.1]hept-2-yl)-benzamide;
1-(3-Cyclopentyl-propionyl)-piperidine-3-carboxylic acid ethyl ester;
4-Phenyl-1-phenylacetyl-piperidine-4-carbonitrile;
1-Octanoyl-4-phenyl-piperidine-4-carbonitrile;
2,2-Dimethyl-1-(1,3,3-thrimethyl-6-aza-bicyclo[3.2.1]oct-6-yl)-propan-1-one;
(4-Chloro-phenyl)-(1,3,3-trimethyl-6-aza-bicyclo[3.2.1]oct-6-yl)-methanone;
N-[1-(4-Methanesulfonyl-phenyl)-ethyl]-N-(tetrahydro-furan-2-ylmethyl)-benzamide;
2-(2-Amino-phenylsulfanyl)-1-(5-fluoro-2,3-dihydro-indol-1-yl)-ethanone;
N-(1-Methyl-2,3-dihydro-1H-indol-5-ylmethyl)-N-(tetrahydro-furan-2-ylmethyl)-benzamide;
1-Benzoyl-piperidine-2-carboxylic acid ethyl ester;
N-(2-Chloro-phenyl)-2-(1,2,3,4-tetrahydro-isoquinolin-1-yl)-acetamide;
(Decahydro-naphthalen-1-yl)-(4-methyl-piperazin-1-yl)-methanone;
(4-Methyl-piperazin-1-yl)-(2-p-tolylsulfanyl-phenyl)-methanone;
Adamantane-1-carboxylic acid (3-benzyloxy-2-ethyl-6-methyl-pyridin-4-yl)-amide;
(6-Fluoro-2-methyl-3,4-dihydro-2H-quinolin-1-yl)-(3,4,5-trimethoxy-phenyl)-methanone;
N-Bicyclo[2.2.1]hept-2-yl-3-cyclopentyl-propionamide;
(2-Benzo[1,2,5]oxadiazol-5-yl-thiazol-4-yl)-piperidin-1-yl-methanone;
Thiophene-2-carboxylic acid [4-(4-fluoro-phenyl)-4-hydroxy-butyl]-isopropyl-amide;
N-Cyclohexyl-3-(1,3-dioxo-1,3-dihydro-isoindol-2-yl)-propionamide;
2-[(Adamantane-1-carbonyl)-amino]-3-(1H-indol-3-yl)-propionic acid methyl ester; Adamantane-1-carboxylic acid [3-(1 H-benzoimidazol-2-ylsulfanyl)-5-nitro-phenyl]-amide;
N-Benzyl-N-(1-cyclopropyl-ethyl)-4-fluoro-benzamide;
Thiophene-2-carboxylic acid 2-[2-(2-phenyl-adamantan-2-yl)-acetylamino]-ethyl ester;
N-(4-Acetyl-phenyl)-2-(2-phenyl-adamantan-2-yl)-acetamide;
2-[2-(4-Chloro-phenyl)-adamantan-2-yl]-N-(2-hydroxy-ethyl)-acetamide;
(4-Benzoyl-piperidin-1-yl)-thiophen-2-yl-methanone;
N-(2-Benzoyl-4-methyl-phenyl)-3-phenyl-acrylamide;
N-(2-Benzoyl-4-methyl-phenyl)-2-fluoro-benzamide;
Adamantane-1-carboxylic acid (4-ethoxy-benzothiazol-2-yl)-amide;
Adamantane-1-carboxylic acid (5-benzoyl-4-phenyl-thiazol-2-yl)-amide;
3-(2-Hydroxy-phenyl)-1,3-di-piperidin-1-yl-propan-1-one;
N-(1-Methyl-2-phenyl-ethyl)-3-phenyl-propionamide;
4-Oxo-4-piperidin-1-yl-butyric acid 4-tert-butyl-cyclohexyl ester;
N-tert-Butyl-N-(4-methoxy-benzyl)-4-nitro-benzamide;
{4-[(Adamantane-1-carbonyl)-amino]-phenoxy}-acetic acid;
2-(4-Isobutyl-phenyl)-N-(1-phenyl-ethyl)-propionamide;
Adamantane-1-carboxylic acid 4-[(adamantane-1-carbonyl)-amino]-2,6-dimethyl-pyridin-3-yl ester;
2-Phenyl-1-(3-phenyl-pyrrolidin-1-yl)-ethanone;
Adamantane-1-carboxylic acid 4-[(adamantane-1-carbonyl)-amino]-2-ethyl-6-methyl-pyridin-3-yl ester;
N-(1,3-Dioxo-1,3-dihydro-isoindol-2-ylmethyl)-N-(4-hydroxy-phenyl)-benzamide;
Biphenyl-4-yl-piperidin-1-yl-methanone;
Azepan-1-yl-(3,5-dichloro-phenyl)-methanone;
Azepan-1-yl-biphenyl-4-yl-methanone;
Azepan-1-yl-(4-chloro-phenyl)-methanone;
3-Heptylcarbamoyl-bicyclo[2.2.1]hept-5-ene-2-carboxylic acid;
Adamantan-1-yl-azepan-1-yl-methanone;
N,N-Dibenzyl-3,4-dimethoxy-benzamide;
N-Benzyl-N-isopropyl-4-nitro-benzamide;
N-[2-(1H-Indol-3-yl)-1-methyl-ethyl]-2-(4-isobutyl-phenyl)-propionamide;
N-tert-Butyl-2-(4-isobutyl-phenyl)-propionamide;
Adamantane-1-carboxylic acid (2-acetyl-phenyl)-amide;
N-(1,3-Dioxo-1,3-dihydro-isoindol-2-ylmethyl)-N-(4-fluoro-phenyl)-benzamide;
(Octahydro-quinolin-1-yl)-phenyl-methanone;
(7-Hydroxy-octahydro-quinolin-1-yl)-phenyl-methanone;
N-(1,3-Dioxo-1,3-dihydro-isoindol-2-ylmethyl)-N-p-tolyl-benzamide;
N,N-Dibenzyl-4-methyl-benzamide;
(2-Chloro-phenyl)-(2-methyl-piperidin-1-yl)-methanone;
[4-Bromo-3-(piperidine-1-sulfonyl)-phenyl]-piperidin-1-yl-methanone;
2-Chloro-N-(3,4-dimethyl-phenyl)-benzamide;
1-Azepan-1-yl-2-(3,3-dimethyl-3,4-dihydro-2H-isoquinolin-1-ylidene)-ethanone;
N-Cyclohexyl-4-(2,4-dichloro-phenoxy)-butyramide;
N-Benzo[1,3]dioxol-5-yl-2-chloro-benzamide;
(4-Benzyl-piperidin-1-yl)-(2-chloro-phenyl)-methanone;
2-(Benzothiazol-2-ylsulfanyl)-N-cyclohexyl-acetamide;
Cyclohexanecarboxylic acid (7,7-dimethyl-5-oxo-5,6,7,8-tetrahydro-quinazolin-2-yl)-amide;
2,4-Dichloro-N-ethyl-N-o-tolyl-benzamide;
(4-Benzyl-piperidin-1-yl)-(4-fluoro-phenyl)-methanone;
N-Cyclohexyl-4-(2,4-dichloro-phenoxy)-N-methyl-butyramide;
3-[2-(1,3-Dioxo-1,3-dihydro-isoindol-2-yl)-ethyl]-adamantane-1-carboxylic acid;
Morpholin-4-yl-(3-p-tolyl-adamantan-1-yl)-methanone;
N-Benzyl-2,4-dichloro-N-methyl-benzamide;
Thiophene-2-carboxylic acid dibenzylamide;
Azepan-1-yl-(2-bromo-phenyl)-methanone;
(3,4-Dichloro-phenyl)-(4-methyl-piperidin-1-yl)-methanone;
N,N-Dibenzyl-3,4-dichloro-benzamide;
4-(2,4-Dichloro-phenoxy)-1-piperidin-1-yl-butan-1-one;
N,N-Dibenzyl-2-fluoro-benzamide;
(2-Chloro-phenyl)-piperidin-1-yl-methanone;
2-Chloro-N-(3-trifluoromethyl-phenyl)-benzamide;
N-Benzyl-N-ethyl-2-phenyl-acetamide;
(3,4-Dihydro-2H-quinolin-1-yl)-p-tolyl-methanone;
Thiophene-2-carboxylic acid benzyl-ethyl-amide;
N-Adamantan-1-yl-2-dibenzylamino-acetamide;
N-Cyclohexyl-2-(1,3-dioxo-1,3-dihydro-isoindol-2-yl)-3-phenyl-propionamide;
Thiophene-2-carboxylic acid cycloheptylamide;
N-Cyclohexyl-3-diethylsulfamoyl-4-methyl-benzamide;
4-Benzoyl-N-methyl-N-phenyl-benzamide;
N-Benzyl-2-bromo-N-methyl-benzamide;
2-Chloro-N-methyl-N-phenyl-benzamide;
4-Chloro-N-ethyl-N-o-tolyl-benzamide;
N-Benzyl-4, N-dimethyl-benzamide;
2-(4-Chloro-3,5-dimethyl-phenoxy)-N-cyclohexyl-N-methyl-acetamide;
N-Benzyl-2-bromo-N-isopropyl-benzamide;
3-(2-Chloro-phenyl)-N-cyclohexyl-N-methyl-acrylamide;
N-Phenyl-N-(2,2,5-trimethyl-hex-4-enyl)-acetamide;
N-m-Tolyl-N-(2,2,5-trimethyl-hex-4-enyl)-acetamide;
(3-Chloro-benzo[b]thiophen-2-yl)-(4-methyl-piperazin-1-yl)-methanone;
Adamantane-1-carboxylic acid (5-methyl-pyridin-2-yl)-amide;
3-Bromo-N-[2-methyl-1-(1,3,3-trimethyl-6-aza-bicyclo[3.2.1]octane-6-carbonyl)-butyl]-benzamide;
4-Chloro-N-[2-methyl-1-(1,3,3-trimethyl-6-aza-bicyclo[3.2.1]octane-6-carbonyl)-butyl]-benzamide;
4-Methyl-N-[2-methyl-1-(1,3,3-trimethyl-6-aza-bicyclo[3.2.1]octane-6-carbonyl)-butyl]-benzamide;
Cyclohexanecarboxylic acid [2-methyl-1-(1,3,3-trimethyl-6-aza-bicyclo[3.2.1]octane-6-carbonyl)-butyl]-amide;
3-Cyclopentyl-N-[2-methyl-1-(1,3,3-trimethyl-6-aza-bicyclo[3.2.1]octane-6-carbonyl)-butyl]-propionamide;
2-Chloro-N-[2-(4-ethyl-benzoylamino)-ethyl]-N-(4-fluoro-phenyl)-benzamide;
N-{1-Benzyl-2-[4-(3-cyclopentyl-propionyl)-piperazin-1-yl]-2-oxo-ethyl}-3-cyclopentyl-propionamide;
N-Bicyclo[2.2.1]hept-5-en-2-ylmethyl-3-cyclopentyl-N-[2-(1H-indol-3-yl)-ethyl]-propionamide;
N-Bicyclo[2.2.1]hept-5-en-2-ylmethyl-2, 4-dichloro-N-[2-(1H-indol-3-yl)-ethyl]-benzamide;
Naphthalene-2-carboxylic acid (2-oxo-azepan-3-yl)-thiophen-3-ylmethyl-amide;
3,4,5-Trimethoxy-N-(4-methyl-benzyl)-N-[6-(pyridin-2-ylamino)-hexyl]-benzamide;
3-Cyclopentyl-N-(4-methyl-benzyl)-N-[6-(pyridin-2-ylamino)-hexyl]-propionamide;
N-(3,4-Dimethoxy-benzyl)-3-methoxy-N-[6-(pyridin-2-ylamino)-hexyl]-benzamide;
N,N-Dimethyl-2-[3-(4-nitro-phenyl)-adamantan-1-yl]-acetamide;
Adamantane-1-carboxylic acid [4-(1,3-dioxo-1,3-dihydro-isoindol-2-yl)-butyl]-p-tolyl-amide;
2-(1,3-Dioxo-1,3-dihydro-isoindol-2-yl)-3-methyl-N-(2-trifluoromethyl-phenyl)-butyramide;
2-(4-Chloro-2-methyl-phenoxy)-N-(2-trifluoromethyl-phenyl)-propionamide;
4-(2,4-Dichloro-phenoxy)-1-[4-(4-fluoro-phenyl)-piperazin-1-yl]-butan-1-one;
(3,4-Dihydro-2H-quinolin-1-yl)-[3-(piperidine-1-sulfonyl)-phenyl]-methanone;
Acetic acid 4-(azepane-1-carbonyl)-phenyl ester;
N-Adamantan-1-ylmethyl-benzamide;
[3-(4-Nitro-phenyl)-adamantan-1-yl]-piperidin-1-yl-methanone;
N-(1,1-Dimethyl-hexyl)-2-morpholin-4-yl-acetamide;
Adamantyl-1-carboxylic acid (2-methoxy-ethyl)-amide;
N-(4-Adamantan-1-yl-2-methyl-phenyl)-acetamide;
3-p-Tolyl-adamantane-1-carboxylic acid (2,5-dichloro-phenyl)-amide;
(3-Chloro-adamantan-1-yl)-pyrrolidin-1-yl-methanone;
2-Amino-5-cyclohexylcarbamoyl-4-methyl-thiophene-3-carboxylic acid ethyl ester;
N-(2-Chloro-phenyl)-2-{3-[(2-chloro-phenylcarbamoyl)-methyl]-adamantan-1-yl}-acetamide;
3-p-Tolyl-adamantane-1-carboxylic acid (2,4-difluoro-phenyl)-amide;
Adamantyl-1-carboxylic acid tert-butylamide;
2-Adamantan-1-yl-N-tert-butyl-acetamide;
N-Methyl-N-phenyl-4-(pyrrolidine-1-sulfonyl)-benzamide;
N-(1-Adamantan-1-yl-ethyl)-2-fluoro-benzamide;
Adamantane-1-carboxylic acid [2-(3,4-dimethoxy-phenyl)-ethyl]-amide;
Adamantane-1-carboxylic acid dimethylamide;
N-Benzyl-4-chloro-N-(1-cyclopropyl-vinyl)-benzamide;
3,5-Dimethyl-adamantane-1-carboxylic acid benzylamide;
2,4-Dichloro-N-cyclohexyl-N-(2-hydroxy-ethyl)-benzamide;
N-Adamantan-1-yl-2,4-dichloro-N-ethyl-benzamide;
2-[(3-p-Tolyl-adamantane-1-carbonyl)-amino]-propionic acid methyl ester; N-Adamantan-1-yl-3-morpholin-4-yl-propionamide;
3-p-Tolyl-adamantane-1-carboxylic acid isopropylamide;
N-Adamantan-1-yl-2-benzylamino-acetamide;
N-Benzyl-2,4-dichloro-N-(1-cyclopropyl-ethyl)-5-methyl-benzamide;
2-[(Adamantane-1-carbonyl)-amino]-benzoic acid ethyl ester;
N-Benzyl-N-isopropyl-4-methyl-3-nitro-benzamide;
(3,4-Dihydro-2H-quinolin-1-yl)-(2-fluoro-phenyl)-methanone;
N-Ethyl-2-fluoro-N-phenyl-benzamide;
2-Phenethyl-N-(2-trifluoromethyl-phenyl)-benzamide;
1-(3,4-Dihydro-2H-quinolin-1-yl)-2-o-tolyloxy-ethanone;
2-(1-Benzyl-1 H-imidazol-2-ylsulfanyl)-N-cyclohexyl-acetamide;
Cyclohexanecarboxylic acid (2-propoxy-phenyl)-amide;
2-{3-[4-(2-Chloro-phenyl)-piperazin-1-yl]-3-oxo-propyl}-isoindole-1,3-dione;
N-Cyclopentyl-2-(2,4-dichloro-phenoxy)-propionamide;
Adamantane-1-carboxylic acid (2-trifluoromethyl-phenyl)-amide;
(4-Chloro-3-nitro-phenyl)-(2,6-dimethyl-piperidin-1-yl)-methanone;
4-(2-Ethyl-phenyl)-4-aza-tricyclo[5.2.2.0^{2,6}]undec-8-ene-3,5-dione;
2-Phenyl-N-(2-trifluoromethyl-phenyl)-butyramide;
N-Adamantan-1-yl-4-chloro-2-nitro-benzamide;
3-p-Tolyl-adamantane-1-carboxylic acid (2,3-dimethyl-phenyl)-amide;
N-Benzyl-3-methyl-4-p-tolyl-butyramide;
N-(2-Cyclohex-1-enyl-ethyl)-2-(1,3-dioxo-1,3-dihydro-isoindol-2-yl)-propionamide;
(4-tert-Butyl-phenyl)-(3,4-dihydro-1 H-isoquinolin-2-yl)-methanone;
2-[1-(4-Chloro-benzenesulfonyl)-1 H-benzoimidazol-2-ylsulfanyl]-N-thiophen-2-ylmethylacetamide;
2-Phenoxy-1-[4-(2-trifluoromethyl-benzyl)-piperazin-1-yl]-ethanone;
Cyclohexanecarboxylic acid [5-(2-fluoro-benzylsulfanylmethyl)-[1,3,4]thiadiazol-2-yl]-amide;
4-Methyl-2-phenyl-thiazole-5-carboxylic acid naphthalen-1-ylamide;
4-Fluoro-N-[4-(1,3,3-trimethyl-6-aza-bicyclo[3.2.1]octane-6-carbonyl)-phenyl]-benzenesulfonamide;
(3-Methoxy-phenyl)-(4-o-tolyl-piperazin-1-yl)-methanone;
N-Adamantan-1-yl-3-(1,3-dioxo-1,3-dihydro-isoindol-2-yl)-propionamide;
N-Cyclooctyl-2-methoxy-3-methyl-benzamide;
2-[4-(2,3,3-Trimethyl-6-aza-bicyclo[3.2.1]octane-6-carbonyl)-phenyl]-isoindole-1,3-dione;
(2,3-Diphenyl-quinoxalin-6-yl)-(2,3,3-trimethyl-6-aza-bicyclo[3.2.1]oct-6-yl)-methanone;
Adamantan-1-yl-(1,3,4,5-tetrahydro-pyrido[4,3-b]indol-2-yl)-methanone;
N-{4-[1-(Naphthalene-2-sulfonyl)-piperidin-3-yl]-butyl}-N'-p-tolyl-oxalamide;
N-Benzyl-N-(2-oxo-2-pyrrolidin-1-yl-ethyl)-benzenesulfonamide;
(4-Amino-phenyl)-(1,3,3-trimethyl-6-aza-bicyclo[3.2.1]oct-6-yl)-methanone;
1-[4-(4-Fluoro-phenyl)-piperazin-1-yl]-2-(2-isopropyl-5-methyl-phenoxy)-ethanone;
Adamantane-1-carboxylic acid benzyl-pyridin-2-yl-amide;
Adamantan-1-yl-piperidin-1-yl-methanone;
Adamantan-1-yl-pyrrolidin-1-yl-methanone;
(3,4-Dihydro-2H-quinolin-1-yl)-o-tolyl-methanone;
Adamantyl-1-carboxylic acid benzylamide;
Pyridine-2-carboxylic acid adamantan-2-ylamide;
(3-Chloro-adamantan-1-yl)-piperidin-1-yl-methanone;
Adamantan-1-yl-(4-methyl-piperidin-1-yl)-methanone;
2-[3-(Azepane-1-carbonyl)-phenyl]-isoindole-1,3-dione;
2-[3-(Piperidine-1-carbonyl)-phenyl]-isoindole-1,3-dione;
4-(Benzyl-methanesulfonyl-amino)-N-furan-2-ylmethyl-benzamide;
(4-Nitro-phenyl)-(1,3,3-trimethyl-6-aza-bicyclo[3.2.1]oct-6-yl)-methanone;
1-Cyclohexyl-5-oxo-pyrrolidine-3-carboxylic acid (4-chloro-3-nitro-phenyl)-amide;
N-(2-Chloro-phenyl)-2-(2-oxo-2-phenyl-ethylsulfanyl)-acetamide;
3-(4-Hydroxy-phenyl)-N-isochroman-1-ylmethyl-3-phenyl-propionamide;
(4-Ethoxy-phenyl)-(2-methyl-piperidin-1-yl)-methanone;
1-Cyclohexyl-5-oxo-pyrrolidine-3-carboxylic acid (3-chloro-phenyl)-amide;
N-[4-(Benzyl-isopropyl-sulfamoyl)-phenyl]-acetamide;
N-(3,4-Dimethyl-phenyl)-N-[4-(piperidine-1-carbonyl)-benzyl]-methanesulfonamide;
2-(5-Phenyl-1 H-imidazol-2-ylsulfanyl)-N-(1,1,3,3-tetramethyl-butyl)-acetamide;
2-(Benzothiazol-2-ylsulfanyl)-N-(1,1,3,3-tetramethyl-butyl)-acetamide;
2-(Benzooxazol-2-ylsulfanyl)-N-(1,1,3,3-tetramethyl-butyl)-acetamide;
2-(Naphthalen-2-ylcarbamoylmethylsulfanyl)-N-(1,1,3,3-tetramethyl-butyl)-acetamide;
Acetic acid 4-(3,5-dimethyl-piperidine-1-carbonyl)-phenyl ester;
(4-Chloro-3-nitro-phenyl)-(2,6-dimethyl-piperidin-1-yl)-methanone;
[1-(4-Chloro-benzenesulfonyl)-piperidin-3-yl]-(octahydro-quinolin-1-yl)-methanone;
2,4-Dichloro-N-cyclohexyl-N-(2-hydroxy-ethyl)-benzamide;
(4-Fluoro-phenyl)-(3,4,4a,8a-tetrahydro-2H-quinolin-1-yl)-methanone;
N-Adamantan-1-yl-2-ethoxy-acetamide;
2-(2-Oxo-2-phenothiazin-10-yl-ethyl)-hexahydro-isoindole-1,3-dione;
Adamantane-1-carboxylic acid (tetrahydro-furan-2-ylmethyl)-amide;
2-Bromo-N-cycloheptyl-benzamide;
Bicyclo[2.2.1]hept-2-yl-[4-(2-ethoxy-phenyl)-piperazin-1-yl]-methanone;
N-Furan-2-ylmethyl-2-phenyl-2-phenylsulfanyl-acetamide;
Adamantane-1-carboxylic acid benzyl-methyl-amide;
1-(3,4-Dihydro-2H-quinolin-1-yl)-3-(4-fluoro-phenyl)-propenone;
Adamantan-1-yl-(2,6-dimethyl-piperidin-1-yl)-methanone;
4-Methyl-N-homoadamantyl-3-yl-benzamide;
(3,5-Dimethyl-piperidin-1-yl)-(3-methyl-4-nitro-phenyl)-methanone;
Quinoline-2-carboxylic acid cyclooctylamide;
Adamantane-1-carboxylic acid [2-(2,4-dimethoxy-phenyl)-ethyl]-amide;
(3,4-Dihydro-1 H-isoquinolin-2-yl)-o-tolyl-methanone;
(3,6-Dichloro-benzo[b]thiophen-2-yl)-(4-methyl-piperazin-1-yl)-methanone;
3-(1-Benzyl-1 H-imidazol-2-ylsulfanyl)-N-cyclohexyl-propionamide;
Propionic acid 2-amino-4-methyl-5-p-tolylcarbamoyl-thiophen-3-yl ester;
2-Cyclohexyl-N-(2,6-dimethyl-phenyl)-N-furan-2-ylmethyl-acetamide;
(3-Methoxy-phenyl)-(2,2,4-trimethyl-4-phenyl-3,4-dihydro-2H-quinolin-1-yl)-methanone;
1-[4-(2,3,3-Trimethyl-6-aza-bicyclo[3.2.1]octane-6-carbonyl)-phenyl]-pyrrolidine-2,5-dione;
1-(3,4-Dihydro-2H-quinolin-1-yl)-2-(1-naphthalen-1-yl-1H-tetrazol-5-ylsulfanyl)-ethanone;
[4-(2,3-Dimethyl-phenyl)-piperazin-1-yl]-o-tolyl-methanone;
(4-Benzyl-piperidin-1-yl)-(4-methyl-3-nitro-phenyl)-methanone;
N-(2-Cyano-phenyl)-2-(9-ethyl-9H-1,3,4,9-tetraaza-fluoren-2-ylsulfanyl)-acetamide;
N-(2-Cyano-phenyl)-2-(9-methyl-9H-1,3,4,9-tetraaza-fluoren-2-ylsulfanyl)-acetamide;
1-(Thiophene-2-carbonyl)-2,3-dihydro-1 H-quinolin-4-one;
(3-Chloro-6-nitro-benzo[b]thiophen-2-yl)-piperidin-1-yl-methanone;
(4-Bromo-phenyl)-(3,5-dimethyl-piperidin-1-yl)-methanone;
2-Morpholin-4-yl-N-(1-phenyl-cyclopentylmethyl)-acetamide;
9-Oxo-9H-fluorene-1-carboxylic acid (3-methyl-butyl)-amide;
[4-(2,5-Dimethyl-pyrrol-1-yl)-phenyl]-(4-methyl-piperidin-1-yl)-methanone;
N-Cycloheptyl-3-diethylsulfamoyl-benzamide;
(4-Methoxy-phenyl)-(3-phenyl-piperidin-1-yl)-methanone;
3-Amino-N-cyclohexyl-N-methyl-benzamide;
N-Ethyl-3,4-dimethyl-N-phenyl-benzamide;
N-Benzyl-3,4, N-trimethyl-benzamide;
(4-Fluoro-phenyl)-(3-phenyl-piperidin-1-yl)-methanone;
[4-(2,3-Dimethyl-phenyl)-piperazin-1-yl]-(3-methoxy-phenyl)-methanone;
Furan-2-carboxylic acid [4-(4-methyl-piperidine-1-sulfonyl)-phenyl]-amide;
N-(2-Cyclohex-1-enyl-ethyl)-2-o-tolyloxy-acetamide;
5-(2-Chloro-phenoxymethyl)-furan-2-carboxylic acid (1-bicyclo[2.2.1]hept-2-yl-ethyl)-amide;
3-(2-Chloro-phenyl)-1-[4-(2,3-dimethyl-phenyl)-piperazin-1-yl]-propenone;
N-[3-(Azepane-1-carbonyl)-phenyl]-benzamide;
[3-(Piperidine-1-carbonyl)-pyrazol-1-yl]-o-tolyl-methanone;
N-(1-Phenyl-cyclopentylmethyl)-2-piperidin-1-yl-propionamide;
2-Morpholin-4-yl-N-(1-phenyl-cyclopentylmethyl)-propionamide;
N-[4-(Azepane-1-sulfonyl)-phenyl]-2,2,2-trifluoro-acetamide;
2,3-Dihydro-benzo[1,4]dioxine-6-carboxylic acid (1-adamantan-1-yl-ethyl)-amide;
N-Adamantan-1-yl-2-(3-methoxy-phenoxy)-acetamide;
3-Chloro-benzo[b]thiophene-2-carboxylic acid (2-cyano-ethyl)-phenyl-amide;
[4-(4-Nitro-benzyl)-piperidin-1-yl]-phenyl-methanone;
[4-(2-Nitro-benzyl)-piperidin-1-yl]-phenyl-methanone;
3-[5-(4-Fluoro-phenyl)-furan-2-yl]-1-(1,3,3-trimethyl-6-aza-bicyclo[3.2.1]oct-6-yl)-propenone;
2-(3-Fluoro-benzoylamino)-4-methyl-5-(piperidine-1-carbonyl)-thiophene-3-carboxylic acid methyl ester;
N-(2-Ethyl-phenyl)-2-(3-methyl-piperidin-1-yl)-acetamide;
2-(2-Methoxy-benzoylamino)-4-methyl-5-(piperidine-1-carbonyl)-thiophene-3-carboxylic acid methyl ester;
1-Phenyl-cyclopentanecarboxylic acid (4-phenyl-tetrahydro-pyran-4-ylmethyl)-amide;
4-(2,4-Dichloro-phenoxy)-1-(4-phenyl-piperazin-1-yl)-butan-1-one;
Naphthalene-1-carboxylic acid cycloheptylamide;
N-Indan-5-yl-2-methyl-3-nitro-benzamide;
N-Cyclohexyl-3-(2,2,2-trifluoro-ethoxymethyl)-benzamide;
2-Methoxy-N-(1-phenyl-cyclopentylmethyl)-benzamide;
[5-(2,5-Dichloro-phenoxymethyl)-furan-2-yl]-(2,6-dimethyl-morpholin-4-yl)-methanone;
[5-(2-Bromo-phenoxymethyl)-furan-2-yl]-(2-methyl-piperidin-1-yl)-methanone;
5-(2-Methoxy-phenoxymethyl)-furan-2-carboxylic acid cycloheptylamide;
(3,4-Dihydro-1H-isoquinolin-2-yl)-[1-(2-nitro-benzenesulfonyl)-piperidin-3-yl]-methanone;
N-Cyclooctyl-2-(4-methoxy-phenoxy)-acetamide;
N-(2,3-Dimethyl-phenyl)-4-[methyl-(toluene-4-sulfonyl)-amino]-butyramide;
N-Phenyl-N-[4-(piperidine-1-carbonyl)-benzyl]-benzenesulfonamide;
N-[4-(3,4-Dihydro-1 H-isoquinoline-2-carbonyl)-benzyl]-N-(3,4-dimethyl-phenyl)-methanesulfonamide;
2,3-Dihydro-benzo[1,4]dioxine-2-carboxylic acid bicyclo[2.2.1]hept-2-ylamide; 4,5,6,7-Tetrahydro-benzo[b]thiophene-3-carboxylic acid cycloheptylamide;
N-(2-Azepan-1-yl-2-oxo-ethyl)-N-benzyl-4-fluoro-benzenesulfonamide;
1-(2,6-Dimethyl-morpholin-4-yl)-3,3-diphenyl-propan-1-one;
N-Bicyclo[2.2.1]hept-2-yl-4-morpholin-4-ylmethyl-benzamide;
[3-(2-Chloro-6-nitro-phenoxy)-phenyl]-piperidin-1-yl-methanone;
N-Adamantan-1-yl-2-(4-methyl-quinolin-2-ylsulfanyl)-acetamide;
Cyclohexanecarboxylic acid (2-phenylsulfanyl-phenyl)-amide;
(4-Hydroxy-4-phenyl-octahydro-quinolin-1-yl)-phenyl-methanone;
3-Cyclohexyl-N-(3-phenyl-propyl)-propionamide;
2-[1-(2,5-Dimethyl-phenyl)-1H-tetrazol-5-ylsulfanyl]-N-isopropyl-N-phenyl-acetamide;
N-{2-[4-(3,4-Dihydro-1H-isoquinoline-2-sulfonyl)-phenyl]-ethyl}-acetamide;
N-Benzyl-N-[2-oxo-2-(4-phenyl-piperazin-1-yl)-ethyl]-benzenesulfonamide;
[4-(2-Chloro-6-nitro-phenoxy)-phenyl]-piperidin-1-yl-methanone;
N-Cycloheptyl-3-phenyl-propionamide;
(3-Chloro-6-methyl-benzo[b]thiophen-2-yl)-piperidin-1-yl-methanone;
N-Cycloheptyl-2,4-dimethoxy-benzamide;
N-(3-Chloro-phenyl)-2-(8,11,11-trimethyl-3,4,6-triaza-tricyclo[6.2.1.0^{2,7}]undeca-2(7),3,5-trien-5-ylsulfanyl)-acetamide;
N-(2-Nitro-phenyl)-2-(8,11,11-trimethyl-3,4,6-triaza-tricyclo[6.2.1.0^{2,7}]undeca-2(7),3,5-trien-5-ylsulfanyl)-acetamide;
N-Phenyl-2-(8,11,11-trimethyl-3,4,6-triaza-tricyclo[6.2.1.0^{2,7}]undeca-2(7),3,5-trien-5-ylsulfanyl)-acetamide;
N-Ethyl-3-methyl-N-o-tolyl-benzamide;
N-[5-(2,4-Dichloro-benzylsulfanyl)-[1,3,4]thiadiazol-2-yl]-2,2-dimethyl-propionamide;
4-Fluoro-N-[4-(1,3,3-trimethyl-6-aza-bicyclo[3.2.1]octane-6-carbonyl)-phenyl]-benzenesulfonamide;
4-Bromo-1-ethyl-1H-pyrazole-3-carboxylic acid (2-methylsulfanyl-phenyl)-amide;
5-Benzoyl-furan-2-carboxylic acid diisopropylamide;
2-[3-(Piperidine-1-carbonyl)-phenyl]-isoindole-1,3-dione;
N-{2-[2-(4-Bromo-phenyl)-2-oxo-ethylsulfanyl]-benzothiazol-6-yl}-acetamide;
2-(6-Amino-benzothiazol-2-ylsulfanyl)-N-cyclohexyl-acetamide;
N-(2-Cyclohexylcarbamoylmethylsulfanyl-benzothiazol-6-yl)-2-methoxy-benzamide;
Benzofuran-2-yl-(4-phenyl-piperidin-1-yl)-methanone;
1-(2-Nitro-phenyl)-piperidine-3-carboxylic acid diethylamide;
1-(4-Nitro-phenyl)-piperidine-3-carboxylic acid diethylamide;
5-Bromo-furan-2-carboxylic acid adamantan-2-ylamide;
3,3-Dimethyl-pentanedioic acid bis-[(2,4-difluoro-phenyl)-amide];
2-(3-Bromo-benzylsulfanyl)-1-(4-phenyl-piperazin-1-yl)-ethanone;
N-(2-Azepan-1-yl-2-oxo-ethyl)-N-benzyl-4-bromo-benzenesulfonamide;
1-(2,3-Dihydro-indol-1-yl)-2-p-tolylsulfanyl-ethanone;
[4-(4-Bromo-benzenesulfonyl)-piperazin-1-yl]-furan-2-yl-methanone;
[5-(2-Bromo-phenoxymethyl)-furan-2-yl]-(2,6-dimethyl-morpholin-4-yl)-methanone;
5-(2-Chloro-phenoxymethyl)-furan-2-carboxylic acid diethylamide;
5-(2-Bromo-phenoxymethyl)-furan-2-carboxylic acid diethylamide;
5-(2-Chloro-phenoxymethyl)-furan-2-carboxylic acid methyl-phenyl-amide;
[5-(2-Chloro-phenoxymethyl)-furan-2-yl]-(4-methyl-piperidin-1-yl)-methanone;
[3-(2,5-Dichloro-phenoxymethyl)-phenyl]-pyrrolidin-1-yl-methanone;
[5-(4-Ethoxy-phenoxymethyl)-furan-2-yl]-(4-methyl-piperidin-1-yl)-methanone;
3-(1,3-Dioxo-1,3-dihydro-isoindol-2-yl)-N-(2-methyl-cyclohexyl)-propionamide;
3-(3,4-Dihydro-2H-quinoline-1-carbonyl)-N-phenyl-benzenesulfonamide;
[3-(2,3-Dihydro-indole-1-sulfonyl)-phenyl]-(3,4-dihydro-2H-quinolin-1-yl)-methanone;
[3-(2,5-Dimethyl-pyrrol-1-yl)-phenyl]-(4-methyl-piperidin-1-yl)-methanone;
N-Cyclohexyl-3-(2-hydroxy-4-methyl-phenyl)-3-phenyl-propionamide;
2-Diethylamino-N-(1-phenyl-cyclopentylmethyl)-propionamide;
(6-Fluoro-2-methyl-3,4-dihydro-2H-quinolin-1-yl)-(3-trifluoromethyl-phenyl)-methanone;
(2,6-Dimethyl-morpholin-4-yl)-[4-(naphthalen-1-yloxymethyl)-phenyl]-methanone;
N-Benzyl-4-bromo-N-ethyl-benzamide;
(3-Methyl-piperidin-1-yl)-[4-(naphthalen-1-yloxymethyl)-phenyl]-methanone;
Azepan-1-yl-[5-(2-chloro-phenoxymethyl)-furan-2-yl]-methanone;
(4-Methyl-piperidin-1-yl)-[4-(naphthalen-1-yloxymethyl)-phenyl]-methanone;
Azepan-1-yl-[5-(2,4-dichloro-phenoxymethyl)-furan-2-yl]-methanone;
N-Cycloheptyl-4-(4-methoxy-2-methyl-phenyl)-butyramide;
2-(4-Benzothiazol-2-yl-piperazin-1-yl)-N-cyclohexyl-acetamide;
N-Cycloheptyl-2-(2,6-dimethyl-phenoxy)-acetamide;
(3,4-Dihydro-2H-quinolin-1-yl)-(3-iodo-phenyl)-methanone;
N-Cycloheptyl-3-(2,2,2-trifluoro-ethoxymethyl)-benzamide;
Azepan-1-yl-[4-(2-chloro-phenoxymethyl)-phenyl]-methanone;
(2,6-Dimethyl-morpholin-4-yl)-[4-(naphthalen-2-yloxymethyl)-phenyl]-methanone;
Azepan-1-yl-[3-(4-ethoxy-phenoxymethyl)-phenyl]-methanone;
Benzo[b]thiophene-3-carboxylic acid (1,2,3,4-tetrahydro-naphthalen-1-yl)-amide;
2-(4-Chloro-2-methyl-phenoxy)-N-cycloheptyl-acetamide;
2,4-Dichloro-N-cyclohexyl-N-methyl-benzamide;
N-Cyclooctyl-2-p-tolyloxy-acetamide;
(3,5-Dimethyl-piperidin-1-yl)-(4-methyl-3-nitro-phenyl)-methanone;
Biphenyl-4-yl-(2,6-dimethyl-piperidin-1-yl)-methanone;
N-Cyclohexyl-4-fluoro-N-methyl-benzamide;
N-[4-(Azepane-1-carbonyl)-phenyl]-N-methyl-benzenesulfonamide;
N-Cycloheptyl-2-fluoro-benzamide;
N-Cycloheptyl-4-methyl-benzamide;
(3-Methyl-piperidin-1-yl)-p-tolyl-methanone;
[2-(3,4-Dimethoxy-phenylcarbamoyl)-piperidin-1-yl]-acetic acid benzyl ester;
N-[4-(2-Methyl-piperidine-1-sulfonyl)-phenyl]-acetamide;
2-(2,4-Dichloro-phenoxy)-N-(2-methyl-butyl)-propionamide;
[4-(2-Chloro-6-nitro-phenyl)-piperazin-1-yl]-(4-methoxy-phenyl)-methanone;
N-Cyclohexyl-4-(4-methoxy-3-methyl-phenyl)-butyramide;
(3-Chloro-6-methoxy-benzo[b]thiophen-2-yl)-(3,4-dihydro-1H-isoquinolin-2-yl)-methanone;
2-(4-Methyl-benzylsulfanyl)-1-piperidin-1-yl-ethanone;
N-Cyclohexyl-N-[(4-phenyl-thiazol-2-ylcarbamoyl)-methyl]-benzamide;
N-(2-Azepan-1-yl-2-oxo-ethyl)-N-(4-isopropyl-phenyl)-methanesulfonamide;
N-Adamantan-1-yl-3-p-tolylsulfanyl-propionamide;
6-(2,4-Dichloro-phenylcarbamoyl)-3,4-dimethyl-cyclohex-3-enecarboxylic acid;
(4-Butyl-cyclohexyl)-morpholin-4-yl-methanone;
(3,4-Dichloro-phenyl)-(3,4-dihydro-2H-quinolin-1-yl)-methanone;
N-(2-Cyclohex-1-enyl-ethyl)-3-methoxy-benzamide;
N-Adamantan-2-yl-3-(1,5-dimethyl-1H-pyrazol-4-yl)-acrylamide;
N-Adamantan-1-yl-N-methyl-4-(4-nitro-pyrazol-1-ylmethyl)-benzamide;
5-(4-Chloro-3,5-dimethyl-pyrazol-1-ylmethyl)-furan-2-carboxylic acid adamantan-2-ylamide;
2-(4-Chloro-phenoxy)-N-(2-fluoro-5-methyl-phenyl)-2-methyl-propionamide;
N-Adamantan-1-yl-2-(4-chloro-3,5-dimethyl-phenoxy)-acetamide;
2-[(3-Carboxy-bicyclo[2.2.1]heptane-2-carbonyl)-amino]-5,6-dihydro-4H-cyclopenta[b]thiophene-3-carboxylic acid propyl ester;
2-Adamantan-1-yl-N-[1-(2,5-dimethyl-phenyl)-ethyl]-acetamide;
3-Methyl-thiophene-2-carboxylic acid cyclooctylamide;
N-p-Tolyl-2-(8,11,11-trimethyl-3,4,6-triaza-tricyclo[6.2.1.0^{2,7}]undeca-2,4,6-trien-5-ylsulfanyl)-propionamide;
Azepan-1-yl-[5-(4-chloro-5-methyl-3-nitro-pyrazol-1-ylmethyl)-furan-2-yl]-methanone;
N-Adamantan-2-yl-3-(4-bromo-3-nitro-pyrazol-1-yl methyl)-benzamide;
N-Bicyclo[2.2.1]hept-2-yl-2-chloro-benzamide;
[5-(3-Chloro-phenoxymethyl)-furan-2-yl]-piperidin-1-yl-methanone;
1-(4-Ethyl-benzoyl)-6-methoxy-2-methyl-2,3-dihydro-1 H-quinolin-4-one;
6-Fluoro-2-methyl-1-{3-[4-(propane-1-sulfonyl)-phenoxymethyl]-benzoyl}-2,3-dihydro-1H-quinolin-4-one;
N-Cycloheptyl-2-(naphthalen-1-yloxy)-acetamide;
N-Cyclohexyl-4-o-tolyloxy-butyramide;
(2-Benzylsulfanyl-phenyl)-morpholin-4-yl-methanone;
(2-Chloro-5,6-difluoro-3-methyl-phenyl)-(4-methyl-piperidin-1-yl)-methanone;
(3-Bromo-phenyl)-[4-(4-chloro-2-nitro-phenyl)-piperazin-1-yl]-methanone;
2-Bromo-N-(1,1,3,3-tetramethyl-butyl)-benzamide;
N-Adamantan-1-yl-2-(2-benzoyl-5-methoxy-phenoxy)-acetamide;
N-Cyclohexyl-3-methyl-4-p-tolyl-butyramide;
[5-(4-Methyl-2-nitro-phenoxymethyl)-furan-2-yl]-thiomorpholin-4-yl-methanone;
[5-(2,5-Dichloro-phenoxymethyl)-furan-2-yl]-thiomorpholin-4-yl-methanone;
5-(4-Chloro-2-nitro-phenoxymethyl)-furan-2-carboxylic acid adamantan-1-ylamide; 4,5,6,7-Tetrahydro-benzo[b]thiophene-3-carboxylic acid cyclohexylamide;
4-Chloro-1,5-dimethyl-1H-pyrazole-3-carboxylic acid adamantan-1-yl-methyl-amide;
4-(4-Methoxy-3-methyl-phenyl)-N-(2-methyl-cyclohexyl)-butyramide;
3-Benzo[1,3]dioxol-5-yl-1-(3,4-dihydro-1H-isoquinolin-2-yl)-propenone;
N-Bicyclo[2.2.1]hept-2-yl-3-phenylsulfanyl-propionamide;
Azepan-1-yl-[5-(2-nitro-phenoxymethyl)-furan-2-yl]-methanone;
N-Benzyl-2-(4-chloro-phenylsulfanyl)-N-methyl-acetamide;
1-(4-Benzyl-piperidin-1-yl)-2-benzylsulfanyl-ethanone;
2-(4-tert-Butyl-phenoxy)-1-(4-ethyl-piperazin-1-yl)-ethanone;
[4-(4-Ethoxy-phenoxymethyl)-phenyl]-(4-methyl-piperidin-1-yl)-methanone;
5-(4-Bromo-3,5-dimethyl-pyrazol-1-ylmethyl)-furan-2-carboxylic acid adamantan-2-ylamide;
1-Azepan-1-yl-3-(4-chloro-phenylsulfanyl)-propan-1-one;
N-Bicyclo[2.2.1]hept-2-yl-2-(2-chloro-benzylsulfanyl)-acetamide;
2-(2-Methyl-benzylsulfanyl)-1-(4-phenyl-piperazin-1-yl)-ethanone;
N-[2-(1-Benzo[1,3]dioxol-5-yl-3-furan-2-yl-3-oxo-propylsulfanyl)-phenyl]-acetamide;
(3,5-Dimethyl-piperidin-1-yl)-(3-iodo-phenyl)-methanone;
[5-(2-Bromo-phenoxymethyl)-furan-2-yl]-(6-fluoro-2-methyl-3,4-dihydro-2H-quinolin-1-yl)-methanone;
N-Benzyl-N-cyclohex-1-enyl-isonicotinamide;
1-[4-(4-Fluoro-phenyl)-piperazin-1-yl]-2-(2-methyl-benzylsulfanyl)-ethanone;
2-(2-Bromo-4-methyl-phenoxy)-N-(2-cyclohex-1-enyl-ethyl)-acetamide;
2-[5-(2-Hydroxy-phenyl)-[1,3,4]oxadiazol-2-ylsulfanyl]-1-piperidin-1-yl-ethanone;
5-(4-Nitro-pyrazol-1-ylmethyl)-furan-2-carboxylic acid adamantan-2-ylamide;
3-Benzo[1,3]dioxol-5-yl-3-(2-methoxy-phenyl)-1-pyrrolidin-1-yl-propan-1-one;
N-Adamantan-2-yl-3,4-dichloro-benzamide;
Benzo[b]thiophen-3-yl-(6-fluoro-2-methyl-3,4-dihydro-2H-quinolin-1-yl)-methanone;
2-Adamantan-1-yl-1-(3,4-dihydro-1H-isoquinolin-2-yl)-ethanone;
4,5,6,7-Tetrahydro-benzo[b]thiophene-3-carboxylic acid (2-cyclohex-1-enyl-ethyl)-amide; Benzo[b]thiophene-3-carboxylic acid (3,3,5-trimethyl-cyclohexyl)-amide;
2-(2,6-Dimethyl-phenoxy)-N-(2-isopropyl-phenyl)-acetamide;
4-Bromo-N-[3-(piperidine-1-carbonyl)-phenyl]-benzamide;
N-Benzo[1,3]dioxol-5-ylmethyl-2-(2-cyano-phenylsulfanyl)-benzamide;
N-Adamantan-1-yl-2-(naphthalen-2-yloxy)-acetamide;
[4-(4-Chloro-phenylsulfanylmethyl)-phenyl]-morpholin-4-yl-methanone;
Thiophene-2-carboxylic acid (3,3,5-trimethyl-cyclohexyl)-amide;
Benzo[1,3]dioxol-5-yl-(3,4-dihydro-2H-quinolin-1-yl)-methanone;
3-Chloro-benzo[b]thiophene-2-carboxylic acid cyclooctylamide;
2-[2-Morpholin-4-yl-1-(4-nitro-benzyl)-2-oxo-ethyl]-isoindole-1,3-dione;
2-Hydroxy-4,4-dimethyl-6-oxo-cyclohex-1-enecarboxylic acid phenylamide;
(2,4-Dichloro-phenyl)-(2,6-dimethyl-piperidin-1-yl)-methanone;
Adamantane-1-carboxylic acid furan-2-ylmethyl-p-tolyl-amide;
Azocan-1-yl-(4-tert-butyl-phenyl)-methanone;
3-Chloro-benzo[b]thiophene-2-carboxylic acid benzyl-methyl-amide;
Adamantane-1-carboxylic acid (2-fluoro-phenyl)-amide;
2-(Piperidine-1-carbonyl)-5-piperidin-1-yl-oxazole-4-carbonitrile;
N-(4,6-Dimethyl-5-nitro-pyridin-3-yl)-benzamide;
Adamantan-1-yl-[4-(2-methoxy-phenyl)-piperazin-1-yl]-methanone;
(2-Methyl-piperidin-1-yl)-o-tolyl-methanone;
N-Benzyl-4-chloro-N-isopropyl-3-nitro-benzamide;
N-(3-Hexylsulfanyl-[1,2,4]thiadiazol-5-yl)-3-methyl-butyramide;
4,N-Dimethyl-N-[4-(piperidine-1-carbonyl)-phenyl]-benzenesulfonamide;
Azepan-1-yl-(5-tert-butyl-2H-pyrazol-3-yl)-methanone;
2-Amino-4-methyl-5-(piperidine-1-carbonyl)-thiophene-3-carboxylic acid ethyl ester; 5-Methyl-furan-2-carboxylic acid (1-adamantan-1-yl-ethyl)-amide;
(3-Chloro-6-methyl-benzo[b]thiophen-2-yl)-(3,4-dihydro-1H-isoquinolin-2-yl)-methanone;
N-Adamantan-1-yl-2-trifluoromethyl-benzamide;
(3-Bromo-phenyl)-(2,2,4-trimethyl-4-phenyl-3,4-dihydro-2H-quinolin-1-yl)-methanone;
Benzo[1,3]dioxole-5-carboxylic acid dipropylamide;
N-(3,3-Diphenyl-propyl)-4-methoxy-benzamide;
[4-(2-Chloro-6-nitro-phenyl)-piperazin-1-yl]-p-tolyl-methanone;
Furan-2-yl-[4-(toluene-4-sulfonyl)-piperazin-1-yl]-methanone;
3-(2-Chloro-6-fluoro-phenyl)-1-(3,4-dihydro-2H-quinolin-1-yl)-propenone;
2-Chloro-N-cycloheptyl-benzamide;
1-[4-(4-Nitro-phenyl)-piperazin-1-yl]-3-phenyl-propan-1-one;
(3,4-Dihydro-1H-isoquinolin-2-yl)-(3,4-dimethyl-phenyl)-methanone;
(1-Adamantan-1-yl-4-bromo-1 H-pyrazol-3-yl)-morpholin-4-yl-methanone;
2-Phenyl-cyclopropanecarboxylic acid cyclooctylamide;
3-[4-(2-Ethoxy-phenyl)-piperazine-1-carbonyl]-isochromen-1-one;
[3-(4-Bromo-pyrazol-1-ylmethyl)-phenyl]-(4-methyl-piperidin-1-yl)-methanone;
2-Azepan-1-yl-N-biphenyl-2-yl-acetamide;
N-[5-(3,4-Dimethoxy-benzyl)-[1,3,4]thiadiazol-2-yl]-3-methyl-butyramide;
Adamantan-1-yl-(4-phenyl-piperidin-1-yl)-methanone;
N-(2-Azepan-1-yl-2-oxo-ethyl)-N-(4-ethoxy-phenyl)-4-methylsulfanyl-benzenesulfonamide;
1-Adamantan-1-yl-4-bromo-1H-pyrazole-3-carboxylic acid diethylamide;
(6-Fluoro-2-methyl-3,4-dihydro-2H-quinolin-1-yl)-(2-fluoro-phenyl)-methanone;
3-[4-(2,3-Dimethyl-phenyl)-piperazine-1-carbonyl]-isochromen-1-one;
N-Cyclooctyl-2-(2-methoxy-phenoxy)-acetamide;
N-Cyclohexyl-N-methyl-2-nitro-benzamide;
Adamantane-1-carboxylic acid (1,1 -dioxo-tetrahydro-thiophen-3-yl)-amide;
N-Adamantan-2-yl-2-(4-chloro-phenyl)-acetamide;
(2,4-Dichloro-phenyl)-(3-methyl-piperidin-1-yl)-methanone;
2-(4-tert-Butyl-phenoxy)-N-cyclooctyl-acetamide;
(4-Hydroxy-4-phenyl-octahydro-quinolin-1-yl)-phenyl-methanone;
(2-Chloro-phenyl)-(2-methyl-piperidin-1-yl)-methanone;
(10,11-Dihydro-dibenzo[b,f]azepin-5-yl)-(2-methoxy-phenyl)-methanone;
(3-Chloro-phenyl)-(2-methyl-piperidin-1-yl)-methanone;
(3-Chloro-6-nitro-benzo[b]thiophen-2-yl)-(3-methyl-piperidin-1-yl)-methanone;
(2,5-Dichloro-phenyl)-(4-methyl-piperidin-1-yl)-methanone;
N-[5-(3,4-Dichloro-benzyl)-[1,3,4]thiadiazol-2-yl]-2,2-dimethyl-propionamide;
4-(4-Chloro-2-methyl-phenoxy)-1-(3,4-dihydro-2H-quinolin-1-yl)-butan-1-one;
(3,4-Dichloro-phenyl)-[4-(2,3-dimethyl-phenyl)-piperazin-1-yl]-methanone;
Cyclooctanecarboxylic acid [1-(naphthalene-2-sulfonyl)-pyrrolidin-2-yl]-amide;
1-Butyl-pyrrolidine-2-carboxylic acid benzo[1,3]dioxol-4-ylamide;
5-Methyl-furan-2-carboxylic acid dibenzylamide;
(3,4-Dihydro-2H-quinolin-1-yl)-[3-(4-phenyl-piperazine-1-sulfonyl)-phenyl]-methanone;
Bicyclo[2.2.1]hept-2-yl-[4-(2,3-dimethyl-phenyl)-piperazin-1-yl]-methanone;
N-Adamantan-1-yl-2-benzoyl-benzamide;
[5-(2-Chloro-phenoxymethyl)-furan-2-yl]-(3-methyl-piperidin-1-yl)-methanone;
(3,5-Dimethyl-piperidin-1-yl)-(2-iodo-phenyl)-methanone;
1-Benzenesulfonyl-pyrrolidine-2-carboxylic acid cyclooctylamide;
(3,4-Dimethoxy-phenyl)-(6-fluoro-2-methyl-3,4-dihydro-2H-quinolin-1-yl)-methanone;
3-(2,6-Dichloro-phenyl)-1-(2-ethyl-piperidin-1-yl)-propenone;
N-(3,4-Difluoro-phenyl)-2,6-difluoro-benzamide;
2,6-Difluoro-N-naphthalen-1-yl-benzamide;
(4-Chloro-phenyl)-(3,5-dimethyl-piperidin-1-yl)-methanone;
N-[4-(2,6-Dimethyl-piperidine-1-carbonyl)-phenyl]-2-(naphthalen-2-yloxy)-acetamide;
(2-Chloro-phenyl)-(3-methyl-piperidin-1-yl)-methanone;
N-{2-[4-(Piperidine-1-sulfonyl)-phenyl]-ethyl}-acetamide;
N-Biphenyl-2-yl-2-(pyridin-2-ylsulfanyl)-acetamide;
Azepan-1-yl-[5-(4-chloro-3,5-dimethyl-pyrazol-1-ylmethyl)-furan-2-yl]-methanone;
Acetic acid 4-(4-methyl-piperidine-1-carbonyl)-phenyl ester;
Acetic acid 4-(4-benzyl-piperidine-1-carbonyl)-phenyl ester;
Benzo[1,3]dioxole-5-carboxylic acid cycloheptylamide;
2-(2,4-Dimethyl-phenoxy)-1-(6-fluoro-2-methyl-3,4-dihydro-2H-quinolin-1-yl)-ethanone;
Acetic acid 4-(3,4-dihydro-2H-quinoline-1-carbonyl)-phenyl ester;
Azepan-1-yl-(3,5-dibromo-phenyl)-methanone;
(3,5-Dibromo-phenyl)-[4-(2-methoxy-phenyl)-piperazin-1-yl]-methanone;
N-Cyclooctyl-4-isopropyl-benzamide;
N-Cyclooctyl-2-(4-methoxy-phenyl)-acetamide;
(4-tert-Butyl-piperidin-1-yl)-phenyl-methanone;
N-(4-tert-Butyl-3-nitro-phenyl)-acetamide;
(2,6-Dimethyl-piperidin-1-yl)-[5-(2,3,5,6-tetrafluoro-phenoxymethyl)-furan-2-yl]-methanone;
2-(4-Chloro-3,5-dimethyl-phenoxy)-N-cyclohexyl-N-methyl-acetamide;
N-Cyclohexyl-3-(1,3-dioxo-1,3-dihydro-isoindol-2-yl)-N-methyl-propionamide;
2-(4-Chloro-3-methyl-phenoxy)-N-cyclohexyl-N-methyl-acetamide;
N-Cyclopentyl-3-(3,4-dihydro-2H-quinoline-1-carbonyl)-benzenesulfonamide;
(3,4-Dihydro-1 H-isoquinolin-2-yl)-(3-dimethylamino-phenyl)-methanone;
3-Cyclohexylcarbamoyl-bicyclo[2.2.1]hept-5-ene-2-carboxylic acid isopropyl ester;
1-(3,4-Dihydro-1 H-isoquinolin-2-yl)-2-(2-methoxy-phenyl)-ethanone;
N-Benzyl-N-cyclohex-1-enyl-benzamide;
[1-(Thiophene-2-sulfonyl)-piperidin-4-yl]-(1,3,3-trimethyl-6-aza-bicyclo[3.2.1]oct-6-yl)-methanone;
N-Adamantan-1-yl-2-(1-phenyl-1H-tetrazol-5-ylsulfanyl)-acetamide;
(3,4-Dihydro-2H-quinolin-1-yl)-[4-(morpholine-4-sulfonyl)-phenyl]-methanone;
(3,4-Dihydro-2H-quinolin-1-yl)-[4-(pyrrolidine-1-sulfonyl)-phenyl]-methanone;
(3,4-Dihydro-2H-quinolin-1-yl)-[1-(thiophene-2-sulfonyl)-piperidin-4-yl]-methanone;
(1-Benzenesulfonyl-piperidin-3-yl)-(3,4-dihydro-1H-isoquinolin-2-yl)-methanone;
6,7-Dimethyl-4-oxa-tricyclo[4.3.0.0^{3,7}]nonane-3-carboxylic acid cyclohexylamide; 6,7-Dimethyl-4-oxa-tricyclo[4.3.0.0^{3,7}]nonane-3-carboxylic acid (2-chloro-phenyl)-amide;
(6,7-Dimethyl-4-oxa-tricyclo[4.3.0.0^{3,7}]non-3-yl)-piperidin-1-yl-methanone;
2-[(Adamantane-1-carbonyl)-amino]-3-(1H-indol-3-yl)-propionic acid methyl ester;
2-(5,6-Dimethyl-4-oxo-3,4-dihydro-thieno[2,3-d]pyrimidin-2-ylsulfanyl)-N-furan-2-ylmethylacetamide;
N-Allyl-2-(5,6-dimethyl-4-oxo-3,4-dihydro-thieno[2,3-d]pyrimidin-2-ylsulfanyl)-acetamide;
N-Adamantan-1-yl-2-(5,6,7,8-tetrahydro-benzo[4,5]thieno[2,3-d]pyrimidin-4-ylsulfanyl)-acetamide;
1-(3,4-Dihydro-2H-quinoline-1-carbonyl)-4,7,7-trimethyl-2-oxa-bicyclo[2.2.1]heptan-3-one;
1-(3,4-Dihydro-1 H-isoquinoline-2-carbonyl)-4,7,7-trimethyl-2-oxa-bicyclo[2.2.1]heptan-3-one;
Azepan-1-yl-(6,7-dimethyl-4-oxa-tricyclo[4.3.0.0^{3,7}]non-3-yl)-methanone;
2,5-Dimethyl-furan-3-carboxylic acid (1-adamantan-1-yl-ethyl)-amide;
1-Cyclohexyl-5-oxo-pyrrolidine-3-carboxylic acid (3-cyano-4,5,6,7-tetrahydro-benzo[b]thiophen-2-yl)-amide;
6-(2,4-Dichloro-phenylcarbamoyl)-3,4-dimethyl-cyclohex-3-enecarboxylic acid;
2-(2-Cyano-phenylsulfanyl)-N-cyclopentyl-benzamide;
[5-(2-Methoxy-4-propyl-phenoxymethyl)-furan-2-yl]-(3-methyl-piperidin-1-yl)-methanone;
(4-tert-Butyl-phenyl)-(3,5-dimethyl-piperidin-1-yl)-methanone;
[4-(2-Methoxy-naphthalen-1-ylmethyl)-piperazin-1-yl]-(4-methoxy-phenyl)-methanone;
(3,4-Dichloro-phenyl)-(3,5-dimethyl-piperidin-1-yl)-methanone;
(4-Ethoxy-phenyl)-(4-methyl-piperidin-1-yl)-methanone;
2-Phenethyl-N-(tetrahydro-furan-2-ylmethyl)-benzamide;
N-Cycloheptyl-2-phenoxy-benzamide;
Adamantane-1-carboxylic acid (2-ethoxy-phenyl)-amide;
N-Adamantan-2-yl-2-o-tolyloxy-acetamide;
(2-Chloro-phenyl)-(3,5-dimethyl-piperidin-1-yl)-methanone;
1-Morpholin-4-yl-2-[3-(4-nitro-phenyl)-adamantan-1-yl]-ethanone;
2-Dimethylamino-N-(2-nitro-phenyl)-benzamide;
N-Benzyl-2-(4,4,6-trimethyl-1-p-tolyl-1,4-dihydro-pyrimidin-2-ylsulfanyl)-acetamide;
[4-(3,5-Dinitro-phenoxy)-phenyl]-(2-ethyl-piperidin-1-yl)-methanone;
1-(4-Chloro-benzoyl)-2,3-dihydro-1H-benzo[g]quinolin-4-one;
2-[(Adamantane-1-carbonyl)-amino]-3-phenyl-propionic acid methyl ester;
[Benzyl-(4-nitro-benzoyl)-amino]-acetic acid ethyl ester;
9-Oxo-9H-fluorene-3-carboxylic acid methyl-(4-nitro-phenyl)-amide;
Adamantane-1-carboxylic acid [2-(4-methoxy-phenyl)-ethyl]-amide;
(10,11-Dihydro-dibenzo[b,f]azepin-5-yl)-(4-fluoro-phenyl)-methanone;
2-Benzylsulfanyl-N-[2-(2-methoxy-phenoxy)-ethyl]-acetamide;
N-Adamantan-1-yl-2-(2-oxo-4-phenyl-pyrrolidin-1-yl)-acetamide;
2-Bromo-N-tricyclo[3.2.1.0^{2,4}]oct-6-ylmethyl-benzamide;
Adamantane-1-carboxylic acid (2,6-dimethoxy-pyrimidin-4-yl)-amide;
Hexanedioic acid (2,7,7-trimethyl-bicyclo[2.2.1]hept-1-yl)-amide (1,7,7-trimethyl-bicyclo[2.2.1]hept-2-yl)-amide;
2-Chloro-N-(2-cyclohexyl-ethyl)-benzamide;
2-[3-(2-Ethyl-piperidin-1-yl)-3-oxo-propyl]-isoindole-1,3-dione;
N-Adamantan-1-yl-2-(2-oxo-4-phenyl-pyrrolidin-1-yl)-acetamide;
N-Adamantan-1-yl-2-hydroxy-2,2-diphenyl-acetamide;
Adamantane-1-carboxylic acid (naphthalen-1-ylmethyl)-amide;
Adamantane-1-carboxylic acid (benzo[1,3]dioxol-5-ylmethyl)-amide;
1-(Azepane-1-carbonyl)-fluoren-9-one;
2-(Quinolin-2-ylsulfanyl)-N-p-tolyl-acetamide;
2,4-Dichloro-N-[3-(piperidine-1-carbonyl)-phenyl]-benzamide;
2-Chloro-4,5-difluoro-N-(3,3,5-trimethyl-cyclohexyl)-benzamide;
2-(2-Chloro-benzylsulfanyl)-N-p-tolyl-acetamide;
[4-(4-Chloro-phenylsulfanylmethyl)-phenyl]-pyrrolidin-1-yl-methanone;
N-Adamantan-1-yl-N-methyl-isonicotinamide;
Azepan-1-yl-[4-(4-chloro-phenylsulfanylmethyl)-phenyl]-methanone;
(2-Chloro-phenyl)-(1,5,7-trimethyl-3,7-diaza-bicyclo[3.3.1]non-3-yl)-methanone;
(3-Chloro-benzo[b]thiophen-2-yl)-(4-methyl-piperidin-1-yl)-methanone;
Benzoic acid 1-benzoyl-decahydro-quinolin-4-yl ester;
2-(3-Bromo-benzylsulfanyl)-1-[4-(2-methoxy-phenyl)-piperazin-1-yl]-ethanone;
4-Methyl-N-[2-(phenoxazine-10-carbonyl)-phenyl]-benzenesulfonamide;
2-[1-(Azepane-1-carbonyl)-2-methyl-propyl]-isoindole-1,3-dione;
2-(3-Bromo-benzylsulfanyl)-1-piperidin-1-yl-ethanone;
1-[3-(4-Bromo-phenyl)-1-furan-2-yl-3-oxo-propyl]-pyrrolidin-2-one;
2-Chloro-N-cyclooctyl-4,5-difluoro-benzamide;
2,4-Dichloro-N-(2-furan-2-ylmethyl-cyclohexyl)-benzamide;
N-(4-Benzoyl-furazan-3-yl)-2-fluoro-benzamide;
N-Adamantan-1-yl-2-(3-cyano-4-methoxymethyl-6-methyl-pyridin-2-ylsulfanyl)-acetamide;
4-tert-Butyl-N-cyclooctyl-benzamide;
N-Adamantan-1-yl-2-phenyl-butyramide;
(3-Chloro-6-methoxy-benzo[b]thiophen-2-yl)-piperidin-1-yl-methanone;
(3,7-Dichloro-6-methoxy-benzo[b]thiophen-2-yl)-piperidin-1-yl-methanone;
Acetic acid 1-benzoyl-decahydro-quinolin-4-yl ester;
2-Bromo-N-methyl-N-phenyl-benzamide;
N-Benzo[1,3]dioxol-5-yl-2,4-dichloro-benzamide;
(3-Chloro-6-fluoro-benzo[b]thiophen-2-yl)-piperidin-1-yl-methanone;
N-(1,2,3,5,6,7-Hexahydro-s-indacen-1-yl)-2-piperidin-1-yl-acetamide;
2-[(Adamantane-1-carbonyl)-amino]-3-(1H-indol-3-yl)-propionic acid methyl ester; 2-[2-(1,3-Dioxo-1,3-dihydro-isoindol-2-yl)-3-phenyl-propionylamino]-3-methyl-butyric acid methyl ester;
2-(6-Oxo-6-piperidin-1-yl-hexyl)-isoindole-1,3-dione;
2-[2-(1,3-Dioxo-1,3-dihydro-isoindol-2-yl)-3-phenyl-propionylamino]-3-methyl-butyric acid methyl ester;
Adamantane-1-carboxylic acid (2,6-dihydroxy-pyrimidin-4-yl)-amide;
Adamantane-1-carboxylic acid [3-(1 H-benzoimidazol-2-ylsulfanyl)-5-nitro-phenyl]-amide; Adamantane-1-carboxylic acid methyl-phenyl-amide;
3-Chloro-benzo[b]thiophene-2-carboxylic acid dibenzylamide;
N-Adamantan-1-yl-2-(3-cyano-6-methyl-4-trifluoromethyl-pyridin-2-ylsulfanyl)-acetamide;
2-(3-Oxo-3-phenyl-propenyl)-isoindole-1,3-dione;
Adamantane-1-carboxylic acid (4-ethoxy-benzothiazol-2-yl)-amide;
N-[5-(5-Chloro-benzooxazol-2-yl)-2-methyl-phenyl]-2-methoxy-benzamide;
N-[2-(2-Bromo-phenyl)-benzooxazol-5-yl]-2-methoxy-benzamide;
2-(4-Chloro-phenoxy)-N-(4-chloro-3-trifluoromethyl-phenyl)-acetamide;
2,2-Dimethyl-N-(5-propyl-[1,3,4]thiadiazol-2-yl)-propionamide;
2-[2-(2,6-Dimethyl-morpholin-4-yl)-1-methyl-2-oxo-ethyl]-isoindole-1,3-dione;
2-(2-Cyano-phenylsulfanyl)-N-(2-trifluoromethyl-phenyl)-benzamide;
Azepan-1-yl-(3,6-dichloro-benzo[b]thiophen-2-yl)-methanone;
Benzo[1,3]dioxol-5-yl-(4-benzyl-piperidin-1-yl)-methanone;
Azepan-1-yl-(3-chloro-6-methyl-benzo[b]thiophen-2-yl)-methanone;
N-(5-Hexyl-[1,3,4]thiadiazol-2-yl)-isobutyramide;
(3-Chloro-phenyl)-(10,11-dihydro-dibenzo[b,f]azepin-5-yl)-methanone;
(2-Chloro-phenyl)-(10,11-dihydro-dibenzo[b,f]azepin-5-yl)-methanone;
2-Amino-5-(azepane-1-carbonyl)-4-methyl-thiophene-3-carboxylic acid ethyl ester;
Adamantan-1-yl-(4-cyclopropyl-1,4,6,7-tetrahydro-imidazo[4,5-c]pyridin-5-yl)-methanone;
Adamantan-1-yl-(4-trifluoromethyl-1,4,6,7-tetrahydro-imidazo[4,5-c]pyridin-5-yl)-methanone;
Adamantan-1-yl-[4-(1 H-benzoimidazol-2-ylsulfanyl)-piperidin-1-yl]-methanone;
Adamantan-1-yl-(1,4,6,7-tetrahydro-imidazo[4,5-c]pyridin-5-yl)-methanone;
[4-(1H-Imidazol-4-yl)-piperidin-1-yl]-(4-pentyl-phenyl)-methanone;
3-Cyclohexyl-1-[4-(1 H-imidazol-4-yl)-piperidin-1-yl]-propan-1-one;
1-(4-Propyl-piperazin-1-yl)-3-(4-trifluoromethyl-phenyl)-propan-1-one;
N-(2-Hydroxy-benzyl)-3-thiophen-3-yl-N-(2-thiophen-2-yl-ethyl)-acrylamide;
N-(1,3-Dimethyl-pentyl)-2-(3-fluoro-phenyl)-N-(4-hydroxy-benzyl)-acetamide;
N-Cyclobutyl-2-(3-fluoro-phenyl)-N-(4-hydroxy-benzyl)-acetamide;
N-Cyclobutyl-N-(4-hydroxy-benzyl)-4-trifluoromethyl-benzamide;
N-(3-Hydroxy-benzyl)-2-methyl-3-nitro-N-(4-sulfamoyl-benzyl)-benzamide;
N--(4-Bromo-benzyl)-N-(4-hydroxy-benzyl)-2-naphthalen-1-yl-acetamide;
6-(2-Bromo-phenylsulfanyl)-hexanoic acid (3-amino-2,2-dimethyl-propyl)-amide;
N-(3-Amino-2,2-dimethyl-propyl)-4-[2-(2-isopropyl-phenylsulfanyl)-ethyl]-benzamide;
N-(3-Amino-2,2-dimethyl-propyl)-4-[4-(4-chloro-phenyl)-pyrimidin-2-ylsulfanylmethyl]-3-nitrobenzamide;
4-(4-Bromo-phenyl)-N-(2-hydroxy-benzyl)-4-oxo-N-thiophen-2-ylmethyl-butyramide;
N-[2-(2,4-Dichloro-phenyl)-ethyl]-N-(4-hydroxy-benzyl)-2-thiophen-3-yl-acetamide;
N-(2-Chloro-benzyl)-N-(4-hydroxy-benzyl)-2-thiophen-2-yl-acetamide;
Heptanoic acid benzyl-(4-hydroxy-benzyl)-amide;
N-(4-Fluoro-benzyl)-N-(4-hydroxy-benzyl)-2-thiophen-3-yl-acetamide;
4-Methyl-pentanoic acid (4-fluoro-benzyl)-(4-hydroxy-benzyl)-amide;
N-Allyl-2-(4-chloro-phenyl)-N-(4-hydroxy-benzyl)-acetamide;
N-Allyl-2-benzo[b]thiophen-3-yl-N-(4-hydroxy-benzyl)-acetamide;
Heptanoic acid (3-ethoxy-propyl)-(4-hydroxy-benzyl)-amide;
Dec-3-enoic acid (4-hydroxy-benzyl)-(4-trifluoromethyl-benzyl)-amide;
6-Oxo-6-phenyl-hexanoic acid (4-hydroxy-benzyl)-(4-trifluoromethyl-benzyl)-amide;
2-(3,4-Difluoro-phenyl)-N-(4-hydroxy-benzyl)-N-thiophen-2-ylmethyl-acetamide;
2-Methyl-pent-4-enoic acid (3-hydroxy-benzyl)-[2-(2-methoxy-phenyl)-ethyl]-amide; Heptanoic acid (3-hydroxy-benzyl)-(4-isopropyl-benzyl)-amide;
5-(2,6-Dichloro-phenylsulfanyl)-pentanoic acid (naphthalen-1-ylmethyl)-amide;
N-(6,6-Dimethyl-bicyclo[3.1.1]hept-2-ylmethyl)-4-[2-(5-methyl-1H-benzoimidazol-2-ylsulfanyl)-ethyl]-benzamide;
N-(6,6-Dimethyl-bicyclo[3.1.1]hept-2-ylmethyl)-4-[2-(4-phenoxy-pyrimidin-2-ylsulfanyl)-ethyl]-benzamide;
N-(6,6-Dimethyl-bicyclo[3.1.1]hept-2-ylmethyl)-4-[2-(4-fluoro-phenylsulfanyl)-ethyl]-benzamide;
4-(2,6-Dichloro-phenylsulfanyl)-N-(6,6-dimethyl-bicyclo[3.1.1]hept-2-ylmethyl)-butyramide;
N-(6,6-Dimethyl-bicyclo[3.1.1]hept-2-ylmethyl)-4-[2-(5-methyl-1H-benzoimidazol-2-ylsulfanyl)-ethyl]-benzamide;
N-(6,6-Dimethyl-bicyclo[3.1.1]hept-2-ylmethyl)-4-[2-(4-fluoro-phenylsulfanyl)-ethyl]-benzamide;
5-(3-Methylsulfanyl-[1,2,4]thiadiazol-5-ylsulfanyl)-pentanoic acid (6,6-dimethyl-bicyclo[3.1.1]hept-2-ylmethyl)-amide;
5-(2,6-Dichloro-phenylsulfanyl)-pentanoic acid [2-(3-trifluoromethyl-phenyl)-ethyl]-amide;
4-[2-(2,6-Dichloro-phenylsulfanyl)-ethyl]-N-[2-(2-fluoro-phenyl)-ethyl]-benzamide;
2-Cyclohexylamino-thiazole-4-carboxylic acid (1,2,3,4-tetrahydro-naphthalen-1-yl)-amide;
2-Cyclohexylamino-thiazole-4-carboxylic acid (3-chloro-4-hydroxy-phenyl)-amide;
2-Cyclohexylamino-thiazole-4-carboxylic acid (1,2-dimethyl-propyl)-amide;
2-Cyclohexylamino-thiazole-4-carboxylic acid (1-ethyl-propyl)-amide;
2-Cyclohexylamino-thiazole-4-carboxylic acid [3-(1-hydroxy-ethyl)-phenyl]-amide;
2-Cyclohexylamino-thiazole-4-carboxylic acid (1-ethynyl-cyclohexyl)-amide;
2-Cyclohexylamino-thiazole-4-carboxylic acid (2-methoxy-dibenzofuran-3-yl)-amide;
2-Cyclohexylamino-thiazole-4-carboxylic acid [2-(4-hydroxy-phenyl)-ethyl]-amide;
2-Cyclohexylamino-thiazole-4-carboxylic acid (4-hydroxy-cyclohexyl)-amide;
2-(2,6-Difluoro-benzylamino)-N-[2-(3-trifluoromethyl-phenyl)-ethyl]-acetamide;
4-{4-[2-(4-Dimethylamino-phenyl)-acetyl]-piperazin-1-yl}-3-(2-phenyl-propylamino)-benzamide;
2-(2-Ethyl-phenylsulfanyl)-3-[methyl-(2-pyridin-4-yl-ethyl)-amino]-N-prop-2-ynyl-propionamide;
4-Methyl-cyclohexanecarboxylic acid {[2-(2-chloro-6-fluoro-benzylsulfanyl)-ethylcarbamoyl]-methyl}-prop-2-ynyl-amide;
2-Benzylsulfanyl-N-{[2-(2-chloro-6-fluoro-benzylsulfanyl)-ethylcarbamoyl]-methyl}-N-(2-methoxy-ethyl)-acetamide;
4-[2-(5-Cyclopropylmethylsulfanyl-[1,3,4]thiadiazol-2-ylsulfanyl)-ethyl]-N-(6,6-dimethyl-bicyclo[3.1.1]hept-2-ylmethyl)-benzamide;
N-(6,6-Dimethyl-bicyclo[3.1.1]hept-2-ylmethyl)-4-[2-(4-phenoxy-pyrimidin-2-ylsulfanyl)-ethyl]-benzamide;
N-(6,6-Dimethyl-bicyclo[3.1.1]hept-2-ylmethyl)-2-p-tolyloxy-acetamide;
Bicyclo[2.2.1]hept-5-ene-2-carboxylic acid [4-(2,5-difluoro-phenoxy)-butyl]-amide;
4-Trifluoromethyl-cyclohexanecarboxylic acid [6-(2,6-difluoro-phenoxy)-hexyl]-amide;
N-Cyclopropyl-3-methoxy-N-(2-piperidin-4-yl-ethyl)-5-(pyridine-2-carbonyl)-benzamide;
3-Methoxy-N-(2-methoxy-ethyl)-N-(2-piperidin-4-yl-ethyl)-5-(pyridine-2-carbonyl)-benzamide;
3-Methoxy-N-(2-piperidin-4-yl-ethyl)-5-(pyridine-2-carbonyl)-N-(tetrahydro-furan-2-ylmethyl)-benzamide;
3-Methoxy-N-(2-oxo-azepan-3-yl)-N-(2-piperidin-4-yl-ethyl)-5-(pyridine-2-carbonyl)-benzamide;
3-Methoxy-N-[3-(2-oxo-pyrrolidin-1-yl)-propyl]-N-(2-piperidin-4-yl-ethyl)-5-(pyridine-2-carbonyl)-benzamide;
3-Methoxy-N-methyl-N-(2-piperidin-4-yl-ethyl)-5-(pyridine-2-carbonyl)-benzamide;
[2-({Cyclopropyl-[3-methoxy-5-(pyridine-2-carbonyl)-benzoyl]-amino}-methyl)-phenoxy]-acetic acid;
(2-{[[3-Methoxy-5-(pyridine-2-carbonyl)-benzoyl]-(3-methyl-butyl)-amino]-methyl}-phenoxy)-acetic acid;
[2-({Cyclopentyl-[3-methoxy-5-(pyridine-2-carbonyl)-benzoyl]-amino}-methyl)-phenoxy]-acetic acid;
[2-({(2-Methoxy-ethyl)-[3-methoxy-5-(pyridine-2-carbonyl)-benzoyl]-amino}-methyl)-phenoxy]-acetic acid;
[2-({Carbamoylmethyl-[3-methoxy-5-(pyridine-2-carbonyl)-benzoyl]-amino}-methyl)-phenoxy]-acetic acid;
[2-({[3-Methoxy-5-(pyridine-2-carbonyl)-benzoyl]-pyridin-4-yl-amino}-methyl)-phenoxy]-acetic acid;
[2-({Cyclopropylmethyl-[3-methoxy-5-(pyridine-2-carbonyl)-benzoyl]-amino}-methyl)-phenoxy]-acetic acid;
[2-({[3-Methoxy-5-(pyridine-2-carbonyl)-benzoyl]-methyl-amino}-methyl)-phenoxy]-acetic acid;
[4-(4-Hydroxy-benzyl)-piperazin-1-yl]-[3-methoxy-5-(pyridine-2-carbonyl)-phenyl]-methanone;
{Carbamoylmethyl-[3-methoxy-5-(pyridine-2-carbonyl)-benzoyl]-amino}-acetic acid;
{(3-Imidazol-1-yl-propyl)-[3-methoxy-5-(pyridine-2-carbonyl)-benzoyl]-amino}-acetic acid;
{[3-Methoxy-5-(pyridine-2-carbonyl)-benzoyl]-pyridin-4-yl-amino}-acetic acid;
[[3-Methoxy-5-(pyridine-2-carbonyl)-benzoyl]-(2-oxo-azepan-3-yl)-amino]-acetic acid;
3-Methoxy-N-(2-methoxy-ethyl)-N-piperidin-3-ylmethyl-5-(pyridine-2-carbonyl)-benzamide;
4-[3-Methoxy-5-(pyridine-2-carbonyl)-benzoylamino]-piperidine-1-carboxylic acid ethyl ester;
3-Methoxy-N-[3-(2-oxo-pyrrolidin-1-yl)-propyl]-5-(pyridine-2-carbonyl)-benzamide;
3-({Carbamoylmethyl-[3-methoxy-5-(pyridine-2-carbonyl)-benzoyl]-amino}-methyl)-benzoic acid;
3-({(3-Imidazol-1-yl-propyl)-[3-methoxy-5-(pyridine-2-carbonyl)-benzoyl]-amino}-methyl)-benzoic acid;
4-Amino-N-(3-hydroxy-benzyl)-N-indan-2-yl-2-propionylamino-butyramide;
5-Amino-2-propionylamino-pentanoic acid (3-hydroxy-benzyl)-indan-2-yl-amide;
N-Ethyl-2-hexylamino-N-(4-hydroxy-benzyl)-acetamide;
2-Hexylamino-N-(4-hydroxy-benzyl)-N-methyl-acetamide;
1-[1-(6-Phenyl-hexanoyl)-piperidin-4-yl]-1,3-dihydro-benzoimidazol-2-one;
1-[1-(3-Cyclohexyl-propionyl)-piperidin-4-yl]-1,3-dihydro-benzoimidazol-2-one;
N-(2-Hydroxy-benzyl)-N-isobutyl-benzamide;
N-(2-Hydroxy-benzyl)-2-(4-hydroxy-phenyl)-N-isobutyl-acetamide;
N-(2-Hydroxy-benzyl)-N-(3-methyl-butyl)-benzamide;
N-(4-Hydroxy-benzyl)-N-isobutyl-benzamide;
4-Hydroxy-N-(4-hydroxy-benzyl)-N-isobutyl-benzamide;
N-(4-Hydroxy-benzyl)-2-(4-hydroxy-phenyl)-N-isobutyl-acetamide;
N-(4-Hydroxy-benzyl)-N-(3-methyl-butyl)-benzamide;
N-(2-Ethoxy-ethyl)-4-hydroxy-N-(4-hydroxy-benzyl)-benzamide;
N-(4-Hydroxy-benzyl)-N-(3-isopropoxy-propyl)-benzamide;
N-(3-Hydroxy-benzyl)-N-(4-methyl-pentyl)-benzamide;
N-(3-Hydroxy-benzyl)-2-(4-hydroxy-phenyl)-N-(4-methyl-pentyl)-acetamide;
N-(3-Hydroxy-benzyl)-N-(3-isopropoxy-propyl)-benzamide;
N-(2-Hydroxy-benzyl)-N-(3-methyl-butyl)-4-propyl-benzamide;
N-(4-Hydroxy-benzyl)-N-(6-hydroxy-hexyl)-4-propyl-benzamide ;
N-(4-Hydroxy-benzyl)-N-(3-methyl-butyl)-4-propyl-benzamide; and
N-[2-(4-Fluoro-benzylamino)-thiazol-4-ylmethyl]-N-phenethyl-butyramide; or
a salt thereof with a pharmaceutically acceptable acid or base, or an optical isomer, mixture of optical isomers, racemic mixture, or tautomeric form thereof.

5. The combination according to claim 2 or 3 wherein the 11 β-HSD1 inhibitor is selected from the group ::
(4-Tetrazol-1-yl-phenyl)-(1,3,3-trimethyl-6-aza-bicyclo[3.2.1]oct-6-yl)-methanone;
N-Cyclohexyl-N-methyl-2-phenoxymethyl-benzamide;
4-Amino-N-cyclohexyl-N-methyl-benzamide;
N-Cycloheptyl-N-methyl-2-phenoxymethyl-benzamide;
N-Cyclohexyl-N-methyl-benzamide;
2-Chloro-N-cyclohexyl-6-fluoro-N-methyl-benzamide;
N-Cyclohexyl-N-methyl-4-trifluoromethoxy-benzamide;
N-Cyclohexyl-2,3, N-trimethyl-benzamide;
3,5-Dichloro-N-cyclohexyl-N-methyl-benzamide;
N-Cyclohexyl-N-methyl-2-phenoxy-benzamide;
2,4-Bis-benzyloxy-N-cyclohexyl-N-methyl-benzamide;
2-Benzyloxy-N-cyclohexyl-N-methyl-benzamide;
N-Cyclohexyl-N-methyl-4-phenoxy-benzamide;
4-Benzyloxy-N-cyclohexyl-N-methyl-benzamide;
N-Cyclohexyl-N-methyl-4-phenoxymethyl-benzamide;
2-Chloro-N-cyclohexyl-N-ethyl-4-nitro-benzamide;
4-Chloro-N-cyclohexyl-N-ethyl-3-nitro-benzamide;
6-Fluoro-4H-benzo[1,3]dioxine-8-carboxylic acid cyclohexyl-methyl-amide;
Azepan-1-yl-(2-chloro-phenyl)-methanone;
Azepan-1-yl-(3-chloro-phenyl)-methanone;
Azepan-1-yl-phenyl-methanone;
2-(Biphenyl-4-yloxy)-N-cyclohexyl-N-methyl-benzamide;
N-Cyclohexyl-2-(3,5-dimethoxy-phenoxy)-N-methyl-benzamide;
N-Cyclohexyl-2-(2,3-dimethoxy-phenoxy)-N-methyl-benzamide;
2,4-Dichloro-N-(3,3-dimethyl-1,5-dioxa-spiro[5.5]undec-9-yl)-N-methyl-benzamide;
2,4-Dichloro-N-methyl-N-(4-oxo-cyclohexyl)-benzamide;
N-Cyclohexyl-2-hydroxy-N-methyl-benzamide;
N-Cyclohexyl-3-methoxy-N-methyl-benzamide;
Benzo[1,3]dioxole-5-carboxylic acid cyclohexyl-methyl-amide;
3-Benzyloxy-N-cyclohexyl-N-methyl-benzamide;
N-Cyclohexyl-3-hydroxy-N-methyl-benzamide;
[4-(Morpholine-4-sulfonyl)-phenyl]-(1,3,3-trimethyl-6-aza-bicyclo[3.2.1]oct-6-yl)-methanone;
N-Benzyl-3-(1,3,3-trimethyl-6-aza-bicyclo[3.2.1]octane-6-carbonyl)-benzenesulfonamide;
[4-Fluoro-3-(morpholine-4-sulfonyl)-phenyl]-(1,3,3-trimethyl-6-aza-bicyclo[3.2.1]oct-6-yl)-methanone;
Thiophene-2-sulfonic acid [4-(1,3,3-trimethyl-6-aza-bicyclo[3.2.1]octane-6-carbonyl)-phenyl]-amide;
N-Phenyl-4-(1,3,3-trimethyl-6-aza-bicyclo[3.2.1]octane-6-carbonyl)-benzenesulfonamide;
(4-Phenoxy-phenyl)-(1,3,3-trimethyl-6-aza-bicyclo[3.2.1]oct-6-yl)-methanone;
N-(2,4-Dimethyl-phenyl)-3-(1,3,3-trimethyl-6-aza-bicyclo[3.2.1]octane-6-carbonyl)-benzenesulfonamide;
(2-Phenoxymethyl-phenyl)-(1,3,3-trimethyl-6-aza-bicyclo[3.2.1]oct-6-yl)-methanone;
4-(3-Aza-bicyclo[3.2.2]nonane-3-carbonyl)-N, N-dipropyl-benzenesulfonamide;
2-Bromo-N-cyclohexyl-N-methyl-benzamide;
N-[4-(1,3,3-Trimethyl-6-aza-bicyclo[3.2.1]octane-6-carbonyl)-phenyl]-acetamide;
(4-Dimethylamino-phenyl)-(1,3,3-trimethyl-6-aza-bicyclo[3.2.1]oct-6-yl)-methanone;
(4-Pyrrol-1-yl-phenyl)-(1,3,3-trimethyl-6-aza-bicyclo[3.2.1]oct-6-yl)-methanone;
(4-Imidazol-1-yl-phenyl)-(1,3,3-trimethyl-6-aza-bicyclo[3.2.1]oct-6-yl)-methanone;
(4-Amino-2-methoxy-phenyl)-(trimethyl-6-aza-bicyclo[3.2.1]oct-6-yl)-methanone;
(4-Methanesulfonyl-phenyl)-(1,3,3-trimethyl-6-aza-bicyclo[3.2.1]oct-6-yl)-methanone;
(3-Methanesulfonyl-phenyl)-(1,3,3-trimethyl-6-aza-bicyclo[3.2.1]oct-6-yl)-methanone;
(4-Benzenesulfonyl-phenyl)-(1,3,3-trimethyl-6-aza-bicyclo[3.2.1]oct-6-yl)-methanone;
Azepan-1-yl-[4-(3,4-dihydro-1 H-isoquinolin-2-ylmethyl)-phenyl]-methanone;
Azepan-1-yl-(4-morpholin-4-ylmethyl-phenyl)-methanone;
[4-(3-Trifluoromethyl-pyrazol-1-yl)-phenyl]-(1,3,3-trimethyl-6-aza-bicyclo[3.2.1]oct-6-yl)-methanone;
(4-[1,2,4]Triazol-1-yl-phenyl)-(1,3,3-trimethyl-6-aza-bicyclo[3.2.1]oct-6-yl)-methanone;
(4-Pyrazol-1-yl-phenyl)-(1,3,3-trimethyl-6-aza-bicyclo[3.2.1]oct-6-yl)-methanone;
2-Benzyloxymethyl-N-cyclohexyl-N-methyl-benzamide;
N-Cyclohexyl-N-methyl-4-(3-methyl-5-oxo-4,5-dihydro-pyrazol-1-yl)-benzamide;
5-Methyl-2-[4-(1,3,3-trimethyl-6-aza-bicyclo[3.2.1]octane-6-carbonyl)-phenyl]-2,4-dihydro-pyrazol-3-one;
(9H-Carbazol-3-yl)-(1,3,3-trimethyl-6-aza-bicyclo[3.2.1]oct-6-yl)-methanone;
[4-(3,5-Dimethyl-pyrazol-1-yl)-phenyl]-(1,3,3-trimethyl-6-aza-bicyclo[3.2.1]oct-6-yl)-methanone;
Phenyl-(1,3,3-trimethyl-6-aza-bicyclo[3.2.1]oct-6-yl)-methanone;
Azepan-1-yl-(2-bromo-phenyl)-methanone;
(3-Aza-bicyclo[3.2.2]non-3-yl)-(2-bromo-phenyl)-methanone;
(4-Benzyl-piperidin-1-yl)-quinolin-2-yl-methanone;
(2-Methyl-piperidin-1-yl)-quinolin-2-yl-methanone;
(3-Aza-bicyclo[3.2.2]non-3-yl)-quinolin-2-yl-methanone;
Quinoline-2-carboxylic acid cyclohexyl-methyl-amide;
Quinolin-2-yl-(1,3,3-trimethyl-6-aza-bicyclo[3.2.1]oct-6-yl)-methanone;
1-[4-(1,3,3-Trimethyl-6-aza-bicydo[3.2.1]octane-6-carbonyl)-phenyl]-pyrrolidine-2,5-dione;
Pyridin-3-yl-(1,3,3-trimethyl-6-aza-bicyclo[3.2.1]oct-6-yl)-methanone;
Pyridin-4-yl-(1,3,3-trimethyl-6-aza-bicyclo[3.2.1]oct-6-yl)-methanone;
Pyridin-2-yl-(1,3,3-trimethyl-6-aza-bicyclo[3.2.1]oct-6-yl)-methanone;
(6-Pyrazol-1-yl-pyridin-3-yl)-(1,3,3-trimethyl-6-aza-bicyclo[3.2.1]oct-6-yl)-methanone;
4-(1,3,3-Trimethyl-6-aza-bicyclo[3.2.1]octane-6-carbonyl)-benzoic acid;
Imidazo[2,1-b]thiazol-6-yl-(1,3,3-trimethyl-6-aza-bicyclo[3.2.1]oct-6-yl)-methanone;
8-(4-Dimethylamino-benzoyl)-8-aza-bicyclo[3.2.1]octan-3-one;
(4-Dimethylamino-phenyl)-(3-hydroxy-8-aza-bicyclo[3.2.1]oct-8-yl)-methanone;
(4-Dimethylamino-phenyl)-(3-hydroxy-3-methyl-8-aza-bicyclo[3.2.1]oct-8-yl)-methanone;
Trifluoro-acetic acid 8-(4-dimethylamino-benzoyl)-8-aza-bicyclo[3.2.1]oct-3-yl ester; or
a salt thereof with a pharmaceutically acceptable acid or base, or any optical isomer or mixture of optical isomers, including a racemic mixture, or any tautomeric forms.

6. The combination according to claim 2 or 3 wherein the 11 β-HSD1 inhibitor is of the Formula (IIa): 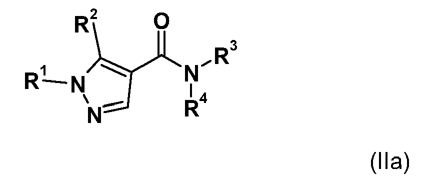 wherein
R¹ is aryl, arylC₁-C₆alkyl, hetaryl or hetarylC₁-C₆alkyl optionally substituted with one or more of R⁶ independently;
R² is halo, C₁-C₆alkyl, C₂-C₆alkenyl, C₂-C₆alkynyl, C₃-C₁₀cycloalkyl, C₃-C₁₀cycloalkylC₁-C₆alkyl, trihalomethyl, aryl, arylC₁-C₆alkyl, C₁-C₆alkyloxyC₁-C₆alkyl, arylC₁-C₆alkyloxyC₁-C₆alkyl, C₁-C₆alkylNR⁵C₁-C₆alkyl, arylC₁-C₆alkylNR⁵C₁-C₆alkyl, hetaryl or hetarylC₁-C₆alkyl wherein the alkyl, alkenyl, alkynyl, cycloalkyl and aryl groups independently are optionally substituted with one or more R⁷;
R³ is C₁-C₆alkyl optionally substituted with one or more of R⁸;
R⁴ is C₆-C₁₀cydoalkyl, C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸; or
R³ and R⁴ together with the nitrogen to which they are attached, are forming a saturated or partially saturated bicyclic/bridge ring system containing from 7 to 12 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetaryl-C₁-C₆alkyl, hydroxy, oxo, C₁-C₆alkyloxy, arylC₁-C₆alkyloxy or hetarylC₁-C₆alkyloxy, wherein the alkyl and aryl groups independently are optionally substituted with one ore more of R⁹;
R⁵ is C₁-C₆alkyl, C₂-C₆alkenyl, C₂-C₆alkynyl, C₃-C₁₀cycloalkyl, C₃-C₁₀hetcycloalkyl, C₃-C₁₀cycloalkylC₁-C₆alkyl, C₃-C₁₀hetcycloalkylC₁-C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;
R⁶ and R⁷ independently are hydrogen, hydroxy, oxo, halo, nitro, cyano, C₁-C₆alkyl, C₁-C₆alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl, arylC₁-C₆alkyloxycarbonyl, C₁-C₆alkylcarboxy, arylcarboxy or arylC₁-C₆alkylcarboxy;
R⁸ and R⁹ independently are hydrogen, C₁-C₆alkyl, aryl, hetaryl, arylC₁-C₆alkyl, hetarylC₁-C₆alkyl, hydroxy, oxo, cyano, NR¹⁰R¹¹, C₁-C₆alkyloxy, aryloxy, arylC₁-C₆alkyloxy, hetaryloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R¹⁰ and R¹¹ independently are hydrogen, C₁-C₈alkyl, aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarbonyl, C₁-C₆alkylcarboxyC₁-C₆alkyl; or
R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁₋₆-alkylcarboxy; or
a salt thereof with a pharmaceutically acceptable acid or base, or any optical isomer or mixture of optical isomers, including a racemic mixture, or any tautomeric forms.

7. The combination according to claim 6 wherein the 11 β-HSD1 inhibitor is of the Formula (IIa) wherein:
R¹ is aryl or hetaryl optionally substituted with one or more R⁶ independently;
R² is halo, C₁-C₆alkyl, C₂-C₆alkenyl, C₂-C₆alkynyl, C₃-C₁₀cycloalkyl, C₃-C₁₀cycloalkylC₁-C₆alkyl, aryl, arylC₁-C₆alkyl, C₁-C₆alkyloxyC₁-C₆alkyl, arylC₁-C₆alkyloxyC₁-C₆alkyl, C₁-C₆alkylNR⁵C₁-C₆alkyl, arylC₁-C₆alkylNR⁵C₁-C₆alkyl, hetaryl or hetarylC₁-C₆alkyl wherein the alkyl, alkenyl, alkynyl, cycloalkyl and aryl groups independently are optionally substituted with one or more R⁷;
R³ is C₁-C₆alkyl optionally substituted with one or more of R⁸;
R⁴ is C₆-C₁₀cycloalkyl, C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸;
R⁵ is C₁-C₆alkyl, C₂-C₆alkenyl, C₂-C₆alkynyl, C₃-C₁₀cycloalkyl, C₃-C₁₀hetcycloalkyl, C₃-C₁₀cycloalkylC₁-C₆alkyl, C₃-C₁₀hetcycloalkylC₁-C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;
R⁶ and R⁷ independently are hydrogen, hydroxy, oxo, halo, nitro, cyano, C₁-C₆alkyl, C₁-C₆alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl, arylC₁-C₆alkyloxycarbonyl, C₁-C₆alkylcarboxy, arylcarboxy or arylC₁-C₆alkylcarboxy;
R⁸ and R⁹ independently are hydrogen, C₁-C₆alkyl, aryl, hetaryl, arylC₁-C₆alkyl, hetarylC₁-C₆alkyl, hydroxy, oxo, cyano, NR¹⁰R¹¹, C₁-C₆alkyloxy, aryloxy, arylC₁-C₆alkyloxy, hetaryloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylcarbonyl, hetarylC₁-C₆alkyl-carbonyl, C₁-C₆alkylcarboxy, arylcarboxy or arylC₁-C₆alkylcarboxy;
R¹⁰ and R¹¹ independently are hydrogen, C₁-C₈alkyl, aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl; or R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetaryl C₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁₋₆alkylcarboxy; or
a salt thereof with a pharmaceutically acceptable acid or base, or any optical isomer or mixture of optical isomers, including a racemic mixture, a prodrug thereof, or any tautomeric forms.

8. The combination according to claim 6 or 7 wherein the 11β-HSD1 inhibitor is selected from:
1-(4-Chloro-phenyl)-5-propyl-1 *H*-pyrazole-4-carboxylic acid cyclohexyl-methyl-amide; 1-(4-Chloro-phenyl)-5-trifluoromethyl-1H-pyrazole-4-carboxylic acid cyclohexyl-methyl-amide;
[1-(4-Methoxy-phenyl)-5-methyl-1*H*-pyrazol-4-yl]-(1,3,3-trimethyl-6-aza-bicyclo[3.2.1]oct-6-yl)-methanone; 1 [1-(4-Chloro-phenyl)-5-propyl-1*H*-pyrazol-4-yl]-(1,3,3-trimethyl-6-aza-bicyclo[3.2.1]oct-6-yl)-methanone;
[1-(3,5-Dichloro-phenyl)-5-propyl-1*H*-pyrazol-4-yl]-(1,3,3-trimethyl-6-aza-bicyclo[3.2.1]oct-6-yl)-methanone;
1-(Phenyl)-5-methyl-1 *H*-pyrazole-4-carboxylic acid cyclohexyl-methyl-amide;
1-(4-Fluoro-phenyl)-5-methyl-1*H*-pyrazole-4-carboxylic acid cyclohexyl-methyl-amide;
1-(4-Methoxy-phenyl)-5-methyl-1 *H*-pyrazole-4-carboxylic acid cyclohexyl-methyl-amide;
1-(4-Chloro-phenyl)-5-methyl-1*H*-pyrazole-4-carboxylic acid cyclohexyl-methyl-amide;
1-(2-Methyl-phenyl)-5-methyl-1 *H*-pyrazole-4-carboxylic acid cyclohexyl-methyl-amide;
1-(4-Amino-phenyl)-5-methyl-1 *H*-pyrazole-4-carboxylic acid cyclohexyl-methyl-amide;
1-(2-Pyridyl)-5-methyl-1 *H*-pyrazole-4-carboxylic acid cyclohexyl-methyl-amide;
1-(2-Pyridyl)-5-propyl-1 *H*-pyrazole-4-carboxylic acid cyclohexyl-methyl-amide; or
a salt thereof with a pharmaceutically acceptable acid or base, or any optical isomer or mixture of optical isomers, including a racemic mixture, or any tautomeric forms.

9. The combination according to claim 2 or 3 wherein the 11 β-HSD1 inhibitor is of the Formula (llb) 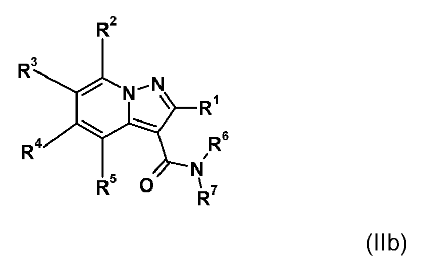 wherein
R¹ is hydrogen, trihalomethyl, C₁-C₆alkyl, C₁-C₆alkyloxy, C₁-C₆alkylthio, aryl, arylC₁-C₆alkyl, hetaryl or hetaralkyl, wherein the alkyl, aryl and hetaryl groups independently are optionally substituted with one or more of R⁸;
R² , R³, R⁴ and R⁵ independently are hydrogen, halo, nitro, cyano, hydroxy, NR⁹R¹⁰, trihalomethyl, C₁-C₆alkyl, C₁-C₆alkyloxy, C₁-C₆alkylthio, aryl, arylC₁-C₆alkyl, hetaryl or hetaralkyl, wherein the alkyl, aryl and hetaryl groups independently are optionally substituted with one or more of R⁸; or
R² together with R³ are forming a saturated or partially saturated cyclic ring system containing from 3 to 6 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy; or
R³ together with R⁴ are forming a saturated or partially saturated cyclic ring system containing from 3 to 6 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy; or
R⁴ together with R⁵ are forming a saturated or partially saturated cyclic ring system containing from 3 to 6 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, aryloxy, arylC₁-C₆alkyloxy or hetarylC₁-C₆alkyloxy;
R⁶ is aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl, wherein the alkyl, aryl and cycloalkyl groups independently are optionally substituted with one or more of R¹¹;
R⁷ is C₁-C₈alkyl, aryl, hetaryl, arylC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl, wherein the alkyl, aryl and cycloalkyl groups independently are optionally substituted with one or more of R¹¹; or
R⁶ and R⁷, together with the nitrogen to which they are attached, are forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁-C₆-alkylcarboxy wherein the alkyl and aryl groups independently are optionally substituted with one ore more of R⁸;
R⁹ and R¹⁰ independently are hydrogen, C₁-C₈alkyl, aryl, hetaryl, arylC₁-C₆alkyl, C₃-C₁₀-cycloalkyl, C₃-C₁₀hetcyloalkyl, C₃-C₁₀cycloalkylC₁-C₆alkyl, C₁-C₆alkylcarboxyC₁-C₆alkyl, wherein the alkyl, aryl and cycloalkyl groups independently are optionally substituted with one or more of R¹¹; or
R⁹ and R¹⁰, together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁸;
R⁸ and R¹¹ independently are hydrogen, halo, hydroxy, oxo, nitro, cyano, C₁-C₈alkyl, C₁-C₆alkyloxy or aryloxy; or
a salt thereof with a pharmaceutically acceptable acid or base, or any optical isomer or mixture of optical isomers, including a racemic mixture, or any tautomeric forms.

10. The combination according to claim 9 wherein the 11β-HSD1 inhibitor is selected frm the group:
pyrazolo[1,5-*a*]pyridin-3-yl-(1,3,3-trimethyl-6-aza-bicyclo[3.2.1]oct-6-yl)-methanone;
(2-Methyl-pyrazolo[1,5-a]pyridin-3-yl)-(1,3,3-trimethyl-6-aza-bicyclo[3.2.1]oct-6-yl)-methanone;
Pyrazolo[1,5-a]pyridine-3-carboxylic acid cyclohexyl-methyl-amide; or
a salt thereof with a pharmaceutically acceptable acid or base, or any optical isomer or mixture of optical isomers, including a racemic mixture, or any tautomeric forms.

11. The combination according to claim 2 or 3 wherein the 11 β-HSD1 inhibitor is of the Formula (IIg) 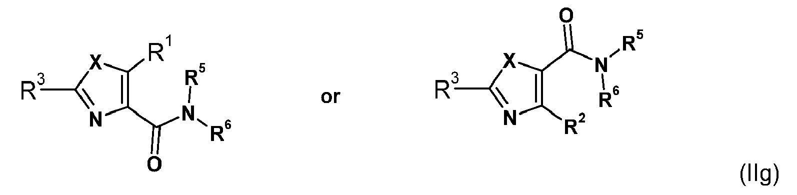 wherein
X is NR⁴, S or O;
R¹ and R² independently are hydrogen, halo, cyano, trihalomethyl, C₁-C₆alkyl or C₁-C₆alkyloxy, wherein the alkyl groups independently are optionally substituted with one or more of R⁷;
R³ is hydrogen, C₁-C₆alkyl, C₃-C₁₀cycloalkyl, C₁-C₆alkyloxy, C₁-C₆alkylthio, aryl, arylC₁-C₆alkyl, hetaryl or hetarylalkyl, wherein the alkyl, cycloalkyl, aryl, hetaryl and hetarylalkyl groups independently are optionally substituted with one or more of R⁷;
R⁴ is hydrogen, C₁-C₈alkyl, C₁-C₆alkyloxyC₁-C₆alkyl, aryl, hetaryl, hetarylC₁-C₆alkyl, arylC₁-C₆alkyl, arylC₁-C₆alkyloxyC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl wherein the alkyl, aryl, hetaryl, cycloalkyl and hetcycloalkyl groups independently are optionally substituted with one or more of R⁷;
R⁵ is hydrogen, and R⁶ is adamantyl optionally substituted with hydroxy, C₁-C₆alkyloxy, aryl, arylC₁-C₆alkyl, aryloxy, arylC₁-C₆alkyloxy, hetaryl, hetaryloxy or hetarylC₁-C₆alkyloxy wherein the alkyl, aryl and hetaryl groups independently are optionally substituted with one or more of R⁷; or
R⁵ and R⁶ are together with the nitrogen to which they are attached, forming a saturated or partially saturated cyclic, bicyclic or tricyclic ring system containing from 6 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylalkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁷;
R⁷ are independently hydrogen, halo, hydroxy, oxo, nitro, NR⁹R¹⁰, cyano, COOR⁸, CONR⁹R¹⁰, C₁-C₈alkyl, C₁-C₆alkyloxy, aryloxy, arylC₁-C₆alkyloxy, hetaryloxy or hetarylC₁-C₆alkyloxy;
R⁸ is hydrogen, C₁-C₆alkyl, aryl, arylC₁-C₆alkyl, hetarylalkyl, wherein the alkyl, aryl and hetarylalkyl groups independently are optionally substituted with one or more of R⁷;
R⁹ and R¹⁰ independently are hydrogen, C₁-C₈alkyl, C₃-C₁₀cycloalkyl, C₃-C₁₀hetcycloalkyl, C₃-C₁₀cycloalkylC₁-C₆alkyl, wherein the alkyl, cycloalkyl and hetcycloalkyl groups independently are optionally substituted with one or more of R⁷; or
R⁹ and R¹⁰ together with the nitrogen to which they are attached, are forming a saturated or partially saturated bicyclic or tricyclic ring system containing from 4 to 10 carbon atoms and from 0 to 2 additional heteroatoms selected from nitrogen or oxygen, the ring system optionally being substituted with at least one of C₁-C₈alkyl, arylC₁-C₆alkyl, hetarylC₁-C₆alkyl, hydroxy, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R¹¹;
R¹¹ is hydrogen, halo, oxo, hydroxy, C₁-C₆alkyl, aryl, arylC₁-C₆alkyl, hetaryl or hetarylalkyl; provided that hetcycloalkyl is not 7-aza[2,2,1]bicycleheptane; or
a salt thereof with a pharmaceutically acceptable acid or base, or any optical isomer or mixture of optical isomers, including a racemic mixture, or any tautomeric forms.

12. The combination according to claim 11 wherein the 11 P-HSD1 inhibitor is of the Formula (llg) wherein
X is NR⁴, S or O;
R¹ and R² independently are hydrogen, halo, cyano, trihalomethyl, C₁-C₆alkyl or C₁-C₆alkyloxy, wherein the alkyl groups independently are optionally substituted with one or more of R⁷;
R³ is hydrogen, C₁-C₆alkyl, C₃-C₁₀cycloalkyl, C₁-C₆alkyloxy, C₁-C₆alkylthio, aryl, arylC₁-C₆alkyl, hetaryl or hetarylalkyl, wherein the alkyl, cycloalkyl, aryl, hetaryl and hetarylalkyl groups independently are optionally substituted with one or more of R⁷;
R⁴ is hydrogen, C₁-C₈alkyl, C₁-C₆alkyloxyC₁-C₆alkyl, aryl, hetaryl, arylC₁-C₆alkyl, arylC₁-C₆alkyloxyC₁-C₆alkyl, C₃-C₁₀cycloalkyl, C₃-C₁₀hetcycloalkyl, C₃-C₁₀cycloalkylC₁-C₆alkyl, C₁-C₆alkylcarboxyC₁-C₆alkyl wherein the alkyl, aryl, hetaryl, cycloalkyl and hetcycloalkyl groups independently are optionally substituted with one or more of R⁷;
R⁵is hydrogen, and R⁶ is adamantyl optionally substituted with hydroxy, C₁-C₆alkyloxy, aryl, arylC₁-C₆alkyl, aryloxy, arylC₁-C₆alkyloxy, hetaryl, hetaryloxy or hetarylC₁-C₆alkyloxy wherein the alkyl, aryl and hetaryl groups independently are optionally substituted with one or more of R⁷; or
R⁵ and R⁶ are together with the nitrogen to which they are attached, forming a saturated or partially saturated cyclic, bicyclic or tricyclic ring system containing from 5 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylalkyl, hydroxy, oxo, cyano, C₁-C₆alkyloxy, arylC₁-C₆alkyloxy, hetarylC₁-C_{6_}alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy wherein the alkyl and aryl groups independently are optionally substituted with one ore more of R⁷;
R⁷ are independently hydrogen, halo, hydroxy, oxo, nitro, NR⁵R⁶ , cyano, COOR⁸, CONR⁵R⁶, C₁-C₈alkyl, C₁-C₆alkyloxy, aryloxy or hetaryloxy;
R⁸ is hydrogen, C₁-C₆alkyl, aryl, arylC₁-C₆alkyl, hetarylalkyl, wherein the alkyl, aryl and hetarylalkyl groups independently are optionally substituted with one or more of R⁷;
provided that hetcycloalkyl is not 7-aza[2,2,1]bicycleheptane; or
a salt thereof with a pharmaceutically acceptable acid or base, or any optical isomer or mixture of optical isomers, including a racemic mixture, or any tautomeric forms.

13. The combination according to claim 11 or 12 wherein the 11 β-HSD1 inhibitor is selected from the group
(4-Methyl-2-phenyl-thiazol-5-yl)-(1,3,3-trimethyl-6-aza-bicyclo[3.2.1]oct-6-yl)-methanone ;
(2,4-Dimethyl-thiazol-5-yl)-(1,3,3-trimethyl-6-aza-bicyclo[3.2.1]oct-6-yl)-methanone;
(4-Methyl-2-pyrazin-2-yl-thiazol-5-yl)-(1,3,3-trimethyl-6-aza-bicyclo[3.2.1]oct-6-yl)-methanone;
[4-Methyl-2-(4-trifluoromethyl-phenyl)-thiazol-5-yl]-(1,3,3-trimethyl-6-aza-bicyclo[3.2.1]oct-6-yl)-methanone;
(3-Aza-bicyclo[3.2.2]non-3-yl)-(2,4-dimethyl-thiazol-5-yl)-methanone;
(1 H-Imidazol-4-yl)-(1,3,3-trimethyl-6-aza-bicyclo[3.2.1]oct-6-yl)-methanone;
(3-Aza-bicyclo[3.2.2]non-3-yl)-(4-methyl-2-phenyl-thiazol-5-yl)-methanone;
2,4-Dimethyl-thiazole-5-carboxylic acid cycloheptylamide;
Azepan-1-yl-(2,4-dimethyl-thiazol-5-yl)-methanone;
2,4-Dimethyl-thiazole-5-carboxylic acid adamantan-1-ylamide;
(3-Aza-bicyclo[3.2.2]non-3-yl)-(1H-imidazol-4-yl)-methanone;
2,4-Dimethyl-thiazole-5-carboxylic acid (3-hydroxy-adamantan-1-yl)-amide;
(1-Methyl-1 H-imidazol-4-yl)-(1,3,3-trimethyl-6-aza-bicyclo[3.2.1]oct-6-yl)-methanone;
[1-(6-Methyl-pyridin-2-yl)-1 H-imidazol-4-yl]-(1,3,3-trimethyl-6-aza-bicyclo[3.2.1]oct-6-yl)-methanone;
[1-(4-Chloro-benzyl)-5-methyl-1 H-imidazol-4-yl]-(1,3,3-trimethyl-6-aza-bicyclo[3.2.1 ]oct-6-yl)-methanone; or
a salt thereof with a pharmaceutically acceptable acid or base, or any optical isomer or mixture of optical isomers, including a racemic mixture, or any tautomeric forms.

14. A pharmaceutical composition which comprises the combination according to any of the claims 1-13 and a pharmaceutically acceptable carrier.

15. A pharmaceutical composition made by combining an 11β-HSD1 inhibitor, a glucocorticoid receptor agonist and a pharmaceutically acceptable carrier.

16. A process for making a pharmaceutical composition comprising: combining an 11β-HSD1 inhibitor, a glucocorticoid receptor agonist and a pharmaceutically acceptable carrier.

17. The use of the combination according to any of the claims 1-13, for the preparation of a pharmaceutical composition for treating or preventing diseases or disorders where modulation or reduction of the level of active intracellular glucorticoid is beneficial

18. The use of the combination according to any of the claims 1-13, for the preparation of a pharmaceutical composition for reducing the adverse effects of glucocorticoid receptor agonist therapy in indications of Cushing's disease, Cushing's syndrome, allergic-inflammatory diseases, adverse effects of glucocorticoid receptor agonist treatment of disorders of the respiratory system, adverse effects of glucocorticoid receptor agonist treatment of inflammatory bowel disease; adverse effects of glucocorticoid receptor agonist treatment of disorders of the immune system, connective tissue and joints; adverse effects of glucocorticoid receptor agonist treatment of endocrinological diseases; adverse effects of glucocorticoid receptor agonist treatment of hematological diseases; adverse effects of glucocorticoid receptor agonist treatment of cancer, chemotherapy-induced nausea, adverse effects of glucocorticoid receptor agonist treatment of diseases of muscle and at the neuro-muscular joint; adverse effects of glucocorticoid receptor agonist treatment in the context of surgery & transplantation; adverse effects of glucocorticoid receptor agonist treatment of brain absess, nausea/vomiting, infections, hypercalcemia, adrenal hyperplasia, autoimmune hepatitis, spinal cord diseases, saccular aneurysms.

19. The use according to claim claim 18, wherein the indication is selected from the following group: Cushing's disease, Cushing's syndrome, asthma, atopic dermatitis, cystic fibrosis, emphysema, bronchitis, hypersensitivity, pneumonitis, eosinophilic pneumonias, pulmonary fibrosis, Crohn's disease, Ulcerative colitis, reactive arthritis, rheumatoid arthritis, Sjögren's syndrome, systemic lupus erythematosus, lupus nephritis, Henoch-Schönlein purpura, Wegener's granulomatosis, temporal arteritis, systemic sclerosis, vasculitis, sarcoidosis, dermatomyositis-polymyositis, pemphigus vulgaris, hyperthyroidism, hypoaldosteronism, hypopituitarism, hemolytic anemia, thrombocytopenia, paroxysmal nocturnal hemoglobinuria, neoplastic compression of the spinal cord, brain tumours, acute lymphoblastic leukemia, Hodgkin's disease, chemotherapy-induced nausea, myasthenia gravis, heriditary myopathies, Duchenne muscular dystrophy, trauma, post-surgical stress, surgical stress, renal transplantation, liver transplantation, lung transplantation, pancreatic islet transplantation, blood stem cell transplantation, bone marrow transplantation, heart transplantation, adrenal gland transplantation, tracheal transplantation, intestinal transplantation, corneal transplantation, skin grafting, keratoplasty, lens implantation, brain absess, nausea/vomiting, infections, hypercalcemia, adrenal hyperplasia, autoimmune hepatitis, spinal cord diseases, and saccular aneurysms.

20. The combination according to claim1 wherein the glucocorticoid receptor agonist is selected from the group consisting of: betametasone, dexamethasone, hydrocortisone, methylprednisolone, prednisolone, prednisone, beclomethasone, butixicort, clobetasol, flunisolide, flucatisone (and analogues), momethasone, triamcinolonacetonide, triamcinolonhexacetonide GW-685698, NXC-1015, NXC-1020, NXC-1021, NS-126, P-4112, P-4114, RU-24858 and T-25 series.

21. The combination according to any of the claims 2-13 wherein the glucocorticoid receptor agonist is selected from the group consisting of: betametasone, dexamethasone, hydrocortisone, methylprednisolone, prednisolone, prednisone, beclomethasone, butixicort, clobetasol, flunisolide, flucatisone (and analogues), momethasone, triamcinolonacetonide, triamcinolonhexacetonide GW-685698, NXC-1015, NXC-1020, NXC-1021, NS-126, P-4112, P-4114, RU-24858 and T-25 series.

22. A kit comprising an amount of an 11β-HSD1 inhibitor and a pharmaceutically acceptable carrier, vehicle, or diluent in a first unit dosage form; an amount of a glucocorticoid receptor agonist and a pharmaceutically acceptable carrier, vehicle, or diluent in a second unit dosage form; and a container.

23. The use of the combination according to any of the claims1-13, for the preparation of a pharmaceutical composition for preventing diseases or disorders where modulation or reduction of the level of active intracellular glucorticoid is beneficial, said use comprising administering to a patient in need of said treatment (i) a first amount of an 11 β-HSD1 inhibitor and (ii) a second amount of a glucocorticoid receptor agonist, wherein said first and second amounts together are effective for said treatment.
